# EUROPEAN PATENT SPECIFICATION

(11) **EP 3 788 379 B1**
(45) Date of publication and mention of the grant of the patent: **22.06.2022**
(21) Application number: 19722071.8
(22) Date of filing: 01.05.2019
(51) Int. Cl.: G01N 33/68

(54) **A METHOD OF DIAGNOSING OR PROGNOSING A NEUROLOGICAL DISORDER**
VERFAHREN ZUR DIAGNOSE ODER PROGNOSE EINER NEUROLOGISCHEN KRANKHEIT
MÉTHODE DE DIAGNOSTIC OU DE PRONOSTIC DE TROUBLE NEUROLOGIQUE

(30) Priority: 01.05.2018 GB 201807178
(43) Date of publication of application: 10.03.2021
(73) Proprietor: University Of Ulster, County Londonderry BT52 1SA (GB)
(72) Inventor: DUGUEZ, Stephanie, Derry BT47 2BS (GB); DUDDY, William, Derry BT47 2BS (GB)
(74) Representative: FRKelly
(86) International application number: PCT/EP2019/061169
(87) International publication number: WO 2019/211343

(56) References cited:
- WO-A1-2013/098786
- WO-A1-2015/006489
- WO-A1-2016/109449
- WO-A2-2005/067391
- WO-A2-2016/028699
- WOEHLBIER UTE ET AL: "ALS-linked protein disulfide isomerase variants cause motor dysfunction", THE EMBO JOURNAL, EUROPEAN MOLECULAR BIOLOGY ORGANIZATION, vol. 35, no. 8, 15 April 2016 (2016-04-15) , pages 845-865, XP009513699, ISSN: 0261-4189, DOI: 10.15252/EMBJ.201592224 [retrieved on 2016-02-11]
- LE GALL L ET AL: "HCB-27 Role of vesicle secretion in ALS transmission", AMYOTROPHIC LATERAL SCLEROSIS AND FRONTOTEMPORAL DEGENERATION -28TH INTERNATIONAL SYMPOSIUM ON ALS/MND, INFORMA HEALTHCARE, STOCKHOLM , vol. 18, no. Supplement 2 1 November 2017 (2017-11-01), page 169, XP009513704, ISSN: 2167-9223, DOI: 10.1080/21678421.2017.1371523 Retrieved from the Internet: URL:https://www.tandfonline.com/toc/iafd20 /18/sup2?nav=tocList [retrieved on 2017-11-07]

## Description

### Field of the Invention

The present invention relates to a method of diagnosing or prognosing amyotrophic lateral sclerosis in a subject. The method comprises determining the quantitative or qualitative level of DAG1 in a biological sample from the subject; and diagnosing or prognosing amyotrophic lateral sclerosis in the subject based on the quantitative or qualitative level of DAG1 in the biological sample.

### Background to the Invention

Amyotrophic lateral sclerosis (ALS), also known as motor neuron disease (MND), is a fatal motor neuron disorder resulting in progressive degeneration and death of upper and lower motor neurons, protein aggregation, severe muscle atrophy and respiratory insufficiency. Median survival is between 2 and 5 years from the onset of symptoms. ALS manifests as either familial ALS (FALS; -10% of cases) or sporadic ALS (SALS; -90% of cases). Incidence rate of ALS has been estimated at between 0.3 and 2.6 cases per 100,000 (2.16 per 100,000 person-years in Europe, 2.0 per 100,000 person-years in Ireland).

Studies in animal models and ALS patients show that motor neuron degeneration starts at the neuromuscular junction and that post-synaptic muscle changes may play an active role. Studies have shown that skeletal muscle has a functional secretory activity. The muscle secretome contains exosomes - vesicles that carry out intercellular transport of functional proteins, mRNA, and miRNA. The exosomes may act in an autocrine/paracrine manner on muscle cells or other types of cells and contribute to muscle growth and regeneration, body-wide metabolism, and other functions. Pathological aggregations of misfolded proteins, such as SOD1, TDP-43, or FUS proteins in affected neurons and also neighbouring glia can be considered as hallmarks of ALS. Several recent studies have shown that these intracellular proteins can be released through vesicle-mediated exocytosis, and then phagocytosed by neighbouring cells allowing a self-perpetuating transmission to adjacent motor neurons. In addition, in familial ALS, mutations of genes involved in autophagy pathways and in multi-vesicular biogenesis (such as ALSIN, VAPB, CHMP2B, and VCP) have been identified. Together, these data strongly suggest a disruption of the endosome and lysosome pathways in sporadic and familial ALS patients - both of these pathways are involved in exosome genesis. Diagnosis is slow, often requiring a patient to see several specialists over a period of months: the mean time from onset of symptoms to confirmation of diagnosis is 13-18 months. Thus the problem/need is to diagnose ALS more quickly.

### Summary of the Invention

The invention is defined in the appended claims.

Here is provided a method of diagnosing or prognosing a neurological disorder in a subject, the method comprising the steps of:
(a) determining the quantitative or qualitative level of one or more biomarkers in a biological sample from the subject; and
(b) diagnosing or prognosing the neurological disorder in the subject based on the quantitative or qualitative level of the or each biomarker in the biological sample;
wherein the or each biomarker is selected from: PDIA3; CCDC80; HSPA8; SERPINE2; PLEC; HSPA5; NCL; TPM1; TNC; IGFBP3; COL5A1; CLEC11A; MAP1B; NACA; PSMA2; RPL5; MYL6; CTSB; HSPD1; PTRF; NUCB1; PDIA4; P4HB; ACTN4; EEF1D; TPI1; SSBP1; PSMB8; PSAP; PSMB7; CAPZA1; PRDX1; PODN; MMP1; NUCB2; HMGB1; AHNAK; FNDC1; DENR; PENK; S100A11; RPL29; RPL27; RPL8; SRSF2; SF3B4; SBSN; LYAR; NUDC; CRLF1; TGFB1; CAT; CTSD; DLD; EPB41L2; PSMB9; RPS14; RPL7A; COLEC12; SMARCC2; TAGLN2; CCT4; ASPH; GPS1; CDK13; SH3GL2; HBA1; APEX1; PSMB5; TXN; AXL; RPS8; SET; RPS18; CFL1; DMKN; RPL22; RPL6; FKBP10; PAFAH1B1; S100A13; PSMA6; SERPINE1; CAPRIN1; SEPT7; VTN; ENPP2; NEO1; DKC1; CHI3L1; SKOR1; SSRP1; COL4A2; NME1; CFB; EIF2S3; DPYSL2; NUMA1; KTN1; CHID1; EFEMP1; YWHAZ; LDHB; SDCBP; TLN1; DES; APP; and DAG1.

Preferably, the or each biomarker is selected from: PDIA3; CCDC80; HSPA8; SERPINE2; PLEC; HSPA5; NCL; TPM1; TNC; IGFBP3; COL5A1; CLEC11A; MAP1B; NACA; PSMA2; RPL5; MYL6; CTSB; HSPD1; PTRF; NUCB1; PDIA4; P4HB; ACTN4; and CAPRIN1.

Further preferably, the or each biomarker is selected from: PDIA3; CCDC80; HSPA8; SERPINE2; PLEC; HSPA5; NCL; TPM1; TNC; IGFBP3; COL5A1; CLEC11A; MAP1B; NACA; PSMA2; RPL5; MYL6; CTSB; HSPD1; PTRF; NUCB1; PDIA4; P4HB; ACTN4; CAPRIN1; DES; APP; and DAG1. Further preferably, the or each biomarker is selected from: PDIA3; CCDC80; HSPA8; SERPINE2; PLEC; DES; APP; and DAG1.

Optionally, the determining step (a) comprises determining the quantitative or qualitative level of two or more biomarkers in the biological sample from the subject.

Further optionally, the determining step (a) comprises determining the quantitative or qualitative level of three, four, five, eight, ten, twenty, twenty five, twenty eight, thirty, forty, fifty, sixty, seventy, eighty, ninety, one hundred, or more biomarkers in the biological sample from the subject.

Optionally, the determining step (a) comprises determining the quantitative or qualitative level of all of the biomarkers in the biological sample from the subject.

Optionally or additionally, the determining step (a) comprises determining the quantitative or qualitative level of each of the biomarkers in the biological sample from the subject.

Optionally, the or each biomarker is a gene. Further optionally, the or each biomarker is a nucleic acid. Still further optionally, the or each biomarker is a deoxyribonucleic acid.

Optionally, the or each biomarker is a translation product of a gene.

Optionally, the or each biomarker is a translation product of a gene selected from: PDIA3; CCDC80; HSPA8; SERPINE2; PLEC; HSPA5; NCL; TPM1; TNC; IGFBP3; COL5A1; CLEC11A; MAP1B; NACA; PSMA2; RPL5; MYL6; CTSB; HSPD1; PTRF; NUCB1; PDIA4; P4HB; ACTN4; EEF1D; TPI1; SSBP1; PSMB8; PSAP; PSMB7; CAPZA1; PRDX1; PODN; MMP1; NUCB2; HMGB1; AHNAK; FNDC1; DENR; PENK; S100A11; RPL29; RPL27; RPL8; SRSF2; SF3B4; SBSN; LYAR; NUDC; CRLF1; TGFB1; CAT; CTSD; DLD; EPB41L2; PSMB9; RPS14; RPL7A; COLEC12; SMARCC2; TAGLN2; CCT4; ASPH; GPS1; CDK13; SH3GL2; HBA1; APEX1; PSMB5; TXN; AXL; RPS8; SET; RPS18; CFL1; DMKN; RPL22; RPL6; FKBP10; PAFAH1B1; S100A13; PSMA6; SERPINE1; CAPRIN1; SEPT7; VTN; ENPP2; NEO1; DKC1; CHI3L1; SKOR1; SSRP1; COL4A2; NME1; CFB; EIF2S3; DPYSL2; NUMA1; KTN1; CHID1; EFEMP1; YWHAZ; LDHB; SDCBP; TLN1; DES; APP; and DAG1.

Preferably, the or each biomarker is a translation product of a gene selected from: PDIA3; CCDC80; HSPA8; SERPINE2; PLEC; HSPA5; NCL; TPM1; TNC; IGFBP3; COL5A1; CLEC11A; MAP1B; NACA; PSMA2; RPL5; MYL6; CTSB; HSPD1; PTRF; NUCB1; PDIA4; P4HB; ACTN4; and CAPRIN1.

Further preferably, the or each biomarker is a translation product of a gene selected from: PDIA3; CCDC80; HSPA8; SERPINE2; PLEC; HSPA5; NCL; TPM1; TNC; IGFBP3; COL5A1; CLEC11A; MAP1B; NACA; PSMA2; RPL5; MYL6; CTSB; HSPD1; PTRF; NUCB1; PDIA4; P4HB; ACTN4; CAPRIN1; DES; APP; and DAG1.

Further preferably, the or each biomarker is a translation product of a gene selected from: PDIA3; CCDC80; HSPA8; SERPINE2; PLEC; DES; APP; and DAG1.

Optionally, the or each biomarker is a ribonucleic acid.

Optionally, the or each biomarker is a ribonucleic acid having a miRBase Accession Number selected from MIMAT0000244; ENSG00000239126; MIMAT0016865; MIMAT0002813; MIMAT0004697; MIMAT0000245; MIMAT0000737; MIMAT0000088; MIMAT0000414; MIMAT0004955; MIMAT0000076; MIMAT0000067; MIMAT0018084; and MIMAT0004813.

Optionally, the or each biomarker is a ribonucleic acid having a nucleic acid sequence selected from any one or more of SEQ ID Nos 109 - 122.

Optionally, the or each biomarker is a protein. Further optionally, the or each biomarker is a peptide. Still further optionally, the or each biomarker is a polypeptide.

Optionally, the or each biomarker is a protein encoded by a gene selected from: PDIA3; CCDC80; HSPA8; SERPINE2; PLEC; HSPA5; NCL; TPM1; TNC; IGFBP3; COL5A1; CLEC11A; MAP1B; NACA; PSMA2; RPL5; MYL6; CTSB; HSPD1; PTRF; NUCB1; PDIA4; P4HB; ACTN4; EEF1D; TPI1; SSBP1; PSMB8; PSAP; PSMB7; CAPZA1; PRDX1; PODN; MMP1; NUCB2; HMGB1; AHNAK; FNDC1; DENR; PENK; S100A11; RPL29; RPL27; RPL8; SRSF2; SF3B4; SBSN; LYAR; NUDC; CRLF1; TGFB1; CAT; CTSD; DLD; EPB41L2; PSMB9; RPS14; RPL7A; COLEC12; SMARCC2; TAGLN2; CCT4; ASPH; GPS1; CDK13; SH3GL2; HBA1; APEX1; PSMB5; TXN; AXL; RPS8; SET; RPS18; CFL1; DMKN; RPL22; RPL6; FKBP10; PAFAH1B1; S100A13; PSMA6; SERPINE1; CAPRIN1; SEPT7; VTN; ENPP2; NEO1; DKC1; CHI3L1; SKOR1; SSRP1; COL4A2; NME1; CFB; EIF2S3; DPYSL2; NUMA1; KTN1; CHID1; EFEMP1; YWHAZ; LDHB; SDCBP; TLN1; DES; APP; and DAG1.

Preferably, the or each biomarker is a protein encoded by a gene selected from: PDIA3; CCDC80; HSPA8; SERPINE2; PLEC; HSPA5; NCL; TPM1; TNC; IGFBP3; COL5A1; CLEC11A; MAP1B; NACA; PSMA2; RPL5; MYL6; CTSB; HSPD1; PTRF; NUCB1; PDIA4; P4HB; ACTN4; and CAPRIN1.

Further preferably, the or each biomarker is a protein encoded by a gene selected from: PDIA3; CCDC80; HSPA8; SERPINE2; PLEC; HSPA5; NCL; TPM1; TNC; IGFBP3; COL5A1; CLEC11A; MAP1B; NACA; PSMA2; RPL5; MYL6; CTSB; HSPD1; PTRF; NUCB1; PDIA4; P4HB; ACTN4; CAPRIN1; DES; APP; and DAG1.

Further preferably, the or each biomarker is a protein encoded by a gene selected from: PDIA3; CCDC80; HSPA8; SERPINE2; PLEC; DES; APP; and DAG1.

Optionally, the or each biomarker is a protein having a UniProtKB/Swiss-Prot Accession Number selected from: P30101; Q76M96; P11142; P07093; Q15149; P11021; P19338; P09493; P24821; P17936; P20908; Q9Y240; P46821; Q13765; P25787; P46777; P60660; P07858; P10809; Q6NZI2; Q02818; P13667; P07237; O43707; P29692; P60174; Q04837; P28062; P07602; Q99436; P52907; Q06830; Q7Z5L7; P03956; P80303; P09429; Q09666; Q4ZHG4; O43583; P01210; P31949; P47914; P61353; P62917; Q01130; Q15427; Q6UWP8; Q9NX58; Q9Y266; O75462; P01137; P04040; P07339; P09622; O43491; P28065; P62263; P62424; Q5KU26; Q8TAQ2; P37802; P50991; Q12797; Q13098; Q14004; Q99962; P69905; P27695; P28074; P10599; P30530; P62241; Q01105; P62269; P23528; Q6E0U4; P35268; Q02878; Q96AY3; P43034; Q99584; P60900; P05121; Q14444; Q16181; P04004; Q13822; Q92859; O60832; P36222; P84550; Q08945; P08572; P15531; P00751; P41091; Q16555; Q14980; Q86UP2; Q9BWS9; Q12805; P63104; P07195; 000560; Q9Y490; P17661; P05067; and Q14118.

Preferably, the or each biomarker is a protein having a UniProtKB/Swiss-Prot Accession Number selected from: P30101; Q76M96; P11142; P07093; Q15149; P11021; P19338; P09493; P24821; P17936; P20908; Q9Y240; P46821; Q13765; P25787; P46777; P60660; P07858; P10809; Q6NZI2; Q02818; P13667; P07237; O43707; and Q14444.

Further preferably, the or each biomarker is a protein having a UniProtKB/Swiss-Prot Accession Number selected from: P30101; Q76M96; P11142; P07093; Q15149; P11021; P19338; P09493; P24821; P17936; P20908; Q9Y240; P46821; Q13765; P25787; P46777; P60660; P07858; P10809; Q6NZI2; Q02818; P13667; P07237; O43707; Q14444; P17661; P05067; and Q14118.

Further preferably, the or each biomarker is a protein having a UniProtKB/Swiss-Prot Accession Number selected from: P30101; Q76M96; P11142; P07093; Q15149; P17661; P05067; and Q14118.

Optionally, the or each biomarker is a protein having an amino acid sequence selected from any one or more of SEQ ID Nos 1 - 108.

Optionally, the neurological disorder is a neurodegenerative disorder.

Optionally, the neurodegenerative disorder is selected from amyotrophic lateral sclerosis (ALS; Lou Gehrig's disease; motor neurone disease; MND), hereditary spastic paraplegia (HSP), primary lateral sclerosis (PLS), progressive muscular atrophy (PMA), progressive bulbar palsy (PBP), pseudobulbar palsy, spinal-bulbar muscular atrophy (SBMA), and spinal muscular atrophy.

Optionally, the diagnosing or prognosing step (b) comprises comparing the quantitative or qualitative level of the or each biomarker in the biological sample from the subject with the quantitative or qualitative level of the or each respective biomarker in a normal sample.

Optionally, the normal sample is a biological sample from a subject not suffering from a neurological disorder, optionally a neurodegenerative disorder, further optionally a neurodegenerative disorder selected from amyotrophic lateral sclerosis (ALS; Lou Gehrig's disease; motor neurone disease; MND), hereditary spastic paraplegia (HSP), primary lateral sclerosis (PLS), progressive muscular atrophy (PMA), progressive bulbar palsy (PBP), pseudobulbar palsy, spinal-bulbar muscular atrophy (SBMA), and spinal muscular atrophy.

Optionally, a quantitative or qualitative level of the or each biomarker in the biological sample from the subject greater than the quantitative or qualitative level of the or each respective biomarker in a normal sample is indicative of the quantitative or qualitative level of the neurological disorder, optionally the neurodegenerative disorder, further optionally the neurodegenerative disorder selected from amyotrophic lateral sclerosis (ALS; Lou Gehrig's disease; motor neurone disease; MND), hereditary spastic paraplegia (HSP), primary lateral sclerosis (PLS), progressive muscular atrophy (PMA), progressive bulbar palsy (PBP), pseudobulbar palsy, spinal-bulbar muscular atrophy (SBMA), and spinal muscular atrophy.

Optionally, a quantitative or qualitative level of the or each biomarker in the biological sample from the subject greater than the quantitative or qualitative level of the or each respective biomarker in a normal sample is indicative of the quantitative or qualitative presence of the neurological disorder, optionally the neurodegenerative disorder, further optionally the neurodegenerative disorder selected from amyotrophic lateral sclerosis (ALS; Lou Gehrig's disease; motor neurone disease; MND), hereditary spastic paraplegia (HSP), primary lateral sclerosis (PLS), progressive muscular atrophy (PMA), progressive bulbar palsy (PBP), pseudobulbar palsy, spinal-bulbar muscular atrophy (SBMA), and spinal muscular atrophy.

Optionally, the determining step (a) comprises determining the quantitative or qualitative level of one or more subsets of one or more biomarkers in the biological sample from the subject.

Optionally, the determining step (a) comprises determining the quantitative or qualitative level of two or more subsets of one or more biomarkers in the biological sample from the subject.

Optionally, the determining step (a) comprises determining the quantitative or qualitative level of three, four, five, six, seven, eight, nine, ten, eleven, twelve, thirteen, or fourteen subsets of one or more biomarkers in the biological sample from the subject.

Optionally, the determining step (a) comprises determining the quantitative or qualitative level of one or more of a first, second, third, fourth, fifth, sixth, seventh, eighth, ninth, tenth, eleventh, twelfth, thirteenth, or fourteenth subset of one or more biomarkers in the biological sample from the subject.

Optionally, the first subset comprises one or more biomarkers selected from: PDIA3; and CCDC80.

Optionally, the second subset comprises HSPA8.

Optionally, the third subset comprises one or more biomarkers selected from: SERPINE2; and PLEC.

Optionally, the fourth subset comprises one or more biomarkers selected from: HSPA5; and NCL.

Optionally, the fifth subset comprises one or more biomarkers selected from: TPM1; and TNC.

Optionally, the sixth subset comprises IGFBP3.

Optionally, the seventh subset comprises one or more biomarkers selected from: COL5A1; CLEC11A; MAP1B; NACA; and PSMA2.

Optionally, the eighth subset comprises one or more biomarkers selected from: RPL5; MYL6; CTSB; HSPD1; PTRF; NUCB1; PDIA4; P4HB; and ACTN4.

Optionally, the ninth subset comprises one or more biomarkers selected from: EEF1D; TPI1; SSBP1; PSMB8; PSAP; PSMB7; CAPZA1; PRDX1; PODN; MMP1; NUCB2; HMGB1; AHNAK; and FNDC1.

Optionally, the tenth subset comprises one or more biomarkers selected from: DENR; PENK; S100A11; RPL29; RPL27; RPL8; SRSF2; SF3B4; SBSN; LYAR; NUDC; CRLF1; TGFB1; CAT; CTSD; DLD; EPB41L2; PSMB9; RPS14; RPL7A; COLEC12; SMARCC2; TAGLN2; CCT4; ASPH; GPS1; CDK13; SH3GL2; HBA1; APEX1; PSMB5; TXN; AXL; RPS8; SET; RPS18; CFL1; DMKN; RPL22; RPL6; FKBP10; PAFAH1B1; S100A13; PSMA6; SERPINE1; CAPRIN1; SEPT7; VTN; ENPP2; NEO1; DKC1; CHI3L1; SKOR1; SSRP1; COL4A2; NME1; CFB; EIF2S3; DPYSL2; NUMA1; KTN1; CHID1; EFEMP1; YWHAZ; LDHB; SDCBP; and TLN1.

Optionally, the eleventh subset comprises one or more biomarkers selected from: DES; APP; and DAG1.

Optionally, the twelfth subset comprises one or more biomarkers selected from: PDIA3; CCDC80; HSPA8; SERPINE2; PLEC; HSPA5; NCL; TPM1; TNC; IGFBP3; COL5A1; CLEC11A; MAP1B; NACA; PSMA2; RPL5; MYL6; CTSB; HSPD1; PTRF; NUCB1; PDIA4; P4HB; ACTN4; and CAPRIN1.

Optionally, the thirteenth subset comprises one or more biomarkers selected from: PDIA3; CCDC80; HSPA8; SERPINE2; PLEC; HSPA5; NCL; TPM1; TNC; IGFBP3; COL5A1; CLEC11A; MAP1B; NACA; PSMA2; RPL5; MYL6; CTSB; HSPD1; PTRF; NUCB1; PDIA4; P4HB; ACTN4; CAPRIN1; DES; APP; and DAG1.

Optionally, the fourteenth subset comprises one or more biomarkers selected from: PDIA3; CCDC80; HSPA8; SERPINE2; PLEC; DES; APP; and DAG1.

Optionally, the determining step (a) comprises determining the quantitative or qualitative level of all of the biomarkers in one or more of the first, second, third, fourth, fifth, sixth, seventh, eighth, ninth, tenth, eleventh, twelfth, thirteenth, or fourteenth subsets.

Optionally, the determining step (a) comprises determining the quantitative or qualitative level of each of the biomarkers in one or more of the first, second, third, fourth, fifth, sixth, seventh, eighth, ninth, tenth, eleventh, twelfth, thirteenth, or fourteenth subsets.

Optionally, the diagnosing or prognosing step (b) comprises comparing the quantitative or qualitative level of the or each biomarker in the or each subset in the biological sample from the subject with the quantitative or qualitative level of the or each respective biomarker in a normal sample.

Optionally, the normal sample is a biological sample from a subject not suffering from a neurological disorder, optionally a neurodegenerative disorder, further optionally a neurodegenerative disorder selected from amyotrophic lateral sclerosis (ALS; Lou Gehrig's disease; motor neurone disease; MND), hereditary spastic paraplegia (HSP), primary lateral sclerosis (PLS), progressive muscular atrophy (PMA), progressive bulbar palsy (PBP), pseudobulbar palsy, spinal-bulbar muscular atrophy (SBMA), and spinal muscular atrophy.

Optionally, a quantitative or qualitative level of the or each biomarker in the or each subset in the biological sample from the subject greater than the quantitative or qualitative level of the or each respective biomarker in a normal sample is indicative of the quantitative or qualitative level of the neurological disorder, optionally the neurodegenerative disorder, further optionally the neurodegenerative disorder selected from amyotrophic lateral sclerosis (ALS; Lou Gehrig's disease; motor neurone disease; MND), hereditary spastic paraplegia (HSP), primary lateral sclerosis (PLS), progressive muscular atrophy (PMA), progressive bulbar palsy (PBP), pseudobulbar palsy, spinal-bulbar muscular atrophy (SBMA), and spinal muscular atrophy.

Optionally, a quantitative or qualitative level of the or each biomarker in the or each subset in the biological sample from the subject greater than the quantitative or qualitative level of the or each respective biomarker in a normal sample is indicative of the quantitative or qualitative presence of the neurological disorder, optionally the neurodegenerative disorder, further optionally the neurodegenerative disorder selected from amyotrophic lateral sclerosis (ALS; Lou Gehrig's disease; motor neurone disease; MND), hereditary spastic paraplegia (HSP), primary lateral sclerosis (PLS), progressive muscular atrophy (PMA), progressive bulbar palsy (PBP), pseudobulbar palsy, spinal-bulbar muscular atrophy (SBMA), and spinal muscular atrophy.

Optionally or additionally, the determining step (a) further comprises the additional step of determining the quantitative or qualitative level of one or more biomarkers in a biological sample from the subject; wherein the or each biomarker is a ribonucleic acid having a miRBase Accession Number selected from MIMAT0000244; ENSG00000239126; MIMAT0016865; MIMAT0002813; MIMAT0004697; MIMAT0000245; MIMAT0000737; MIMAT0000088; MIMAT0000414; MIMAT0004955; MIMAT0000076; MIMAT0000067; MIMAT0018084; and MIMAT0004813.

Optionally, the biological sample is selected from whole blood, serum, plasma, urine, interstitial fluid, peritoneal fluid, cervical swab, tears, saliva, buccal swab, skin, brain tissue, and cerebrospinal fluid.

Further optionally, the biological sample is selected from whole blood, serum, and plasma.

Still further optionally, the biological sample is whole blood.

PDIA3 is synonymous with Erp57.

### Brief Description of the Drawings

The present methods will now be described with reference to the following nonlimiting examples and the accompanying drawings, in which:
**Figure 1** is a schematic illustration of ALS signature in muscle stem cells;
**Figure 2** (A) illustrates immunostaining for exosome markers of differentiated muscle stem cells, (B) the secretion of exosomes by muscle cells, and (C) the multi-vesicular bodies of ALS muscle cells contain more exosomes than the controls;
**Figure 3** illustrates representative images of healthy myotubes treated with ALS or Control exosomes, wherein ALS exosomes induce cell stress;
**Figure 4** illustrates boxplots showing expression values of non-coding RNA microarrays (raw: left panel; background corrected: middle panel; background corrected and normalized: right panel);
**Figure 5** illustrates boxplots showing RLE values for non-coding RNA microarrays (average values close to 0 pass quality control);
**Figure 6** illustrates boxplots showing NUSE values for non-coding RNA microarrays (average values <1.05 pass quality control);
**Figure 7** illustrates DAG1 expression level in circulating serum exosomes, using anti-DAG antibody. Values are mean ± SD. Serum from 8 ALS patients (1 month after diagnose), 11 healthy subjects and 9 Parkinson patients. ANOVA 1 factor followed by a Tukey post-hoc test was performed. **, significantly different from healthy values, P<0.01;
**Figure 8** illustrates HSPA8 expression level in circulating serum exosomes. Values are mean ± SD. Serum from 5 ALS patients (1 month after diagnose), 9 healthy subjects and 7 Parkinson patients. ANOVA 1 factor followed by a Tukey post-hoc test was performed. *, **, significantly different from ALS values, P<0.05 and P<0.01 respectively; and
**Figure 9** illustrates levels of each biomarker in exosomes isolated from the blood serum of ALS patients, normalized against the quantity of total blood serum exosome (CD63), and plotted against the rate of progression of the pathology (delta ALSFRS-R).

### Examples

### Materials & Methods

### Participants and ethical approval

An open biopsy was performed in deltoid muscle on 18 ALS patients who attended the Motor Neuron Diseases center (Pitié Salpétrière, Paris). Twenty-one deltoid muscle biopsies from healthy age and gender-matched subjects were obtained from the BTR (Bank of Tissues for Research, a partner in the EU network EuroBioBank) in accordance with European recommendations and French legislation. The protocol (NCT01984957) was approved by the local ethical committee and all subjects completed informed consent in accordance with institutional guidelines.

### Muscle stem cell extraction and culture

Muscle biopsies were dissociated mechanically and plated in proliferation medium as previously described (Mamchaoui et al, Skeletal muscle, 2011, PMID: 22040608). CD56 magnetic bead sorting system (MACS, Miltenyl Biotech) was used to sort the myogenic cell population in accordance with the manufacturer's instructions. The myogenicity of the cell cultures was checked before and after sorting the cells by immuno-labelling using anti-desmin antibodies, and the cells were differentiated for 3 days in Dulbecco's Modified Eagle's medium (DMEM). A minimum of 80% of the cell population were positive for desmin.

### Exosomes extraction from the culture medium

Briefly, 10 million muscle stem cells (ALS and healthy subject, n=3 per group) were differentiated in DMEM for 3 days. After 3 days, the conditioned media were harvested, centrifuged at 300 × g for 5 min at room temperature (RT) and then at 4000 g for 20 min at 4°C in order to remove any dead cells and cell debris. The supernatants were filtered through 0.22 µm filter to remove the microparticles. The samples used for protein and miRNA extraction, and stored at -80°C.

### Protein extraction from exosomes

A volume of cell culture medium was mixed to 0.5 volumes of Total Exosome Isolation Reagent from cell culture media (lifeTechnologies^{®}) and incubated overnight at 4°C. The samples were then centrifugated at 10,000 × g for 1 hr at 4°C. The exosome pellets were re-suspended in 25 µl 8M Urea, 50 mM ammonium bicarbonate, pH 8.5, and reduced with dithiothreitol (DTT) for 1 h at 4°C. Protein concentrations were then quantified using Pierce BCA Protein Assay kit (ThermoFisher^{®}). Exosomal proteins were kept at -80°C.

### Proteome profile determined by Mass spectrometry

Approximately 20 µg of exosome protein were trypsin digested using a SmartDigest column (Thermo) for 2 hours at 70°C and 1400 rpm in accordance with the manufacturer's instructions. Peptides were then fractionated into 8 fractions using a high pH reverse phase spin column (Thermo) in accordance with the manufacturer's instructions. Fractioned peptides were vacuum dried, re-suspended and analyzed by data-dependent mass spectrometry on a Q Exactive HF (Thermo) with the following parameters: Positive Polarity, m/z 400-2000 MS Resolution 70,000, AGC 3e6, 100ms IT, MS/MS Resolution 17,500, AGC 5e5, 50ms IT, Isolation width 3 m/z, and NCE 30, cycle count 15.

### Database Search and Quantification

Resulting mass spectral files were searched for protein identification using ProteomeDiscoverer (Thermo) against the Uniprot human database for semi tryptic peptides and filtered based on a false discovery rate of <1%.

### Proteome profile analysis

Proteins were selected as candidates if the range of observed abundance of the 3 ALS replicates did not overlap with the range of the 3 Healthy control replicates. Candidates were ranked based on the difference in the median value of the 3 ALS replicates from that of the Healthy replicates.

### miRNA extraction from exosomes

A volume of cell culture medium was mixed to 0.5 volumes of Total Exosome Isolation Reagent from cell culture media (lifeTechnologies^{®}) and incubated overnight at 4°C. The samples were then centrifugated at 10,000 × g for 1 hr at 4°C. The exosome pellets were re-suspended with 1ml of trizol. After adding 0.2 mL of chloroform, the samples were incubated for 3 minutes at room temperature, then centrifugated for 15 minutes at 12,000 × g at 4°C. The aqueous upper phase was then mixed with 1/3 volume of 100% ethanol and mixed thoroughly. The samples were then placed on place a filter cartridge into a collection tube of total RNA and protein isolation kit from Invitrogen^{™} (LifeTechnologies^{™}). The miRNA extraction was performed following the manufacturer's instruction. RNA quality was determined using Eukaryote Total RNA Nano 2.6 (Agilent) and 2100 bioanalyzer. RNA quantity was determined using nanodrop, each in accordance with the manufacturer's instructions.

### miRNA profile and analysis

Total RNA (150 ng) was poly(A) tailed and biotin labelled using a FlashTag Biotin HSR RNA labelling kit and hybridized to Affymetrix GeneChip miRNA 4.0 arrays for 16 hours (48°C), following the manufacturer's instructions (ThermoFisher Scientific, Waltham, MA). Hybridization cocktails were then removed and the arrays washed and stained on a Fluidics Station 450 with the fluidics script for miRNA 4.0 arrays. Finally, arrays were scanned on a Affymetrix GCS3000 7G scanner and initial quality control data evaluated using Affymetrix Expression Console software (both from ThermoFisher Scientific).

### miRNome profile analysis

Further quality control of raw data was carried out using the R/Bioconductor packages oligo and pd.mirna.4.0. These packages were used to evaluate each sample's distribution of expression values, relative log expression values (RLE) values, and normalized unscaled standard errors (NUSE), all of which were consistent with good quality. Expression matrices were then normalized by quantile normalization and detection above background using Affymetrix Expression Console v 1.4.1.46. Differentially expressed miRNA (ALS v Healthy) were then identified as those having absolute fold-change > 2 and one-way, unpaired ANOVA p-value < 0.05.

### Serum samples from patients

Blood samples were collected using silica vacutainers, then gently inverted 8-10 times. After 2h of incubation on ice to allow clotting, samples were centrifugated at 1,500g for 15mn. Aliquots of 100µl of serum were transferred to cryovials and stored at -80°C. Eight ALS patients, 11 healthy subjects and 9 Parkinson patients aged and gender matched, were recruited (NCT01302600 and NCT02305147).

### Exosome extraction from serum

The exosomes were precipitated by adding 6µl Total Exosome Isolation Reagent, vortexing and incubating for 30mn on ice. Samples were then centrifugated for 10mn at 10,000g at RT. The pellets were resuspended in 13.5µl of Urea 4M/RIPA and kept on ice. In parallel, 6.3µl of Total Exosome Isolation Reagent was added to the remaining supernatants, vortexed and incubated for 30mn on ice. These second mix were then centrifugated for 10mn at 10,000g at RT. The second exosome pellets were resuspended in 13.5µl of Urea 4M/RIPA. The two exosome protein extracts were then mixed together and used for dot-blot analysis. The exosome protein extracts were stored at -80°C.

### Sample preparation for the dot blot

To 5µl of exosome protein extract was added 1xNuPAGE (Invitrogen^{™}). These non-reduced samples were used for CD63 analysis. To the remaining 22µl of the exosome protein extract was added 1xNuPAGE (Invitrogen^{™}) supplemented with 1x reducing reagent (Invitrogen^{™}). All samples were then heated at 70°C for 10mn.

### Dot blot analysis

1µl of reduced or non-reduced sample was loaded on the methanol pre-activated 0.2pm PVDF (Immobilon^{®}, Sigma-Aldrich^{®}). After drying for 5mn at RT, the membranes were re-activated quickly in methanol and staining with Ponceau S (Sigma-Aldrich^{®}) to control the loading. The membranes were then blocked overnight at 4°C in TBS-0.01 % tween (TBS-T) supplemented with 5%milk. The primary antibodies for CD63 (TS63, Invitrogen^{™}), for beta-dystroglycan (MANDAG2, 7D11, DSHB) and for HSPA8 (Millipore^{®}) were extemporaneously coupled with biotin using Zenon^{™} BiotinXX mouse IgG1 labelling kit following the manufacturer instructions. Briefly, to 1µg of antibody was added first 5µl of complex, then 5µl of blocking buffer. Anti-CD63, anti-betadystroglycan, and anti-HSPA8 were diluted in 1/1000, 1/133 and 1/500 respectively in TBS-T-5%milk.

The primary antibodies for Serpin E2 (TS63, Invitrogen^{™}), for ERP57 (PDIA3, Merck-Millipore^{®}), Desmin (Y66, Invitrogen^{™}), for Amyloid Precursor Protein (abcam), CCDC80 (Invitrogen^{™}), and Plectin (invitrogen^{™}) were extemporaneously coupled with biotin using Zenon^{™} BiotinXX rabbit IgG labelling kit following the manufacturer instructions. Briefly, to 1µg of antibody was added first 5µl of complex, then 5µl of blocking buffer. Anti-Serpin E2 anti- ERP57, anti-Desmin, anti-Amyloid Precursor Protein, anti-CCDC80, and anti-Plectin were diluted in 1/1000 in TBS-T-5%milk.

Membranes were incubated with diluted primary biotin-coupled antibodies for 45mn at RT. Membranes were then rinsed 3 times in TBS-T for 5mn. Membranes were then incubated for 45 min at RT with streptavidin-HRP (Invitrogen^{™}) diluted at 1/250 in TBS-T-5%milk. Membranes were then rinsed 3 times in TBS-T for 5mn. Membranes were revealed using SuperSignal^{™} West Pico PLUS Chemiluminescent Substrate (Thermo Scientific^{™}). Images were acquired every 15s for 15mn using UVP/ChemiDoc-It^{®}2 Imager. Images were then analysed using FIJI software.

### Statistical analysis

Values are mean±SD. An ANOVA 1 Factor followed by a Tukey posthoc-test was performed to assess the differences in biomarker levels between ALS, healthy and Parkinson subjects. The correlation between the biomarker levels and the progression of ALSFRS was tested using a Pearson correlation analysis. Cut-off for significant differences was set at P<0.05.

### Example 1 - Identification of ALS signature in muscle stem cells

Muscle stem cells were extracted from muscle biopsies of patients and healthy subjects (all male, 30-67 years old, n=5/6 per group), purified (>80% myogenic cells) and differentiated for 3 days into myotubes, wherein the samples were analyzed using GeneChip Human Exon 1.0ST array (Affymetrix, Inc., Cleveland, OH); and (A) illustrates a PCA plot showing clear separation of ALS patients on the 2nd component (y-axis), ALS: blue, SBMA: pink, SMA-III/IV: red, Control: green; (B) illustrates, of the 30 genes having expression signatures most strongly specific to ALS, many have been observed at exosomally-relevant subcellular localizations, according to analysis by the CellWhere tool.

### Example 2 - ALS muscle stem cells accumulate and secrete more exosomes than controls

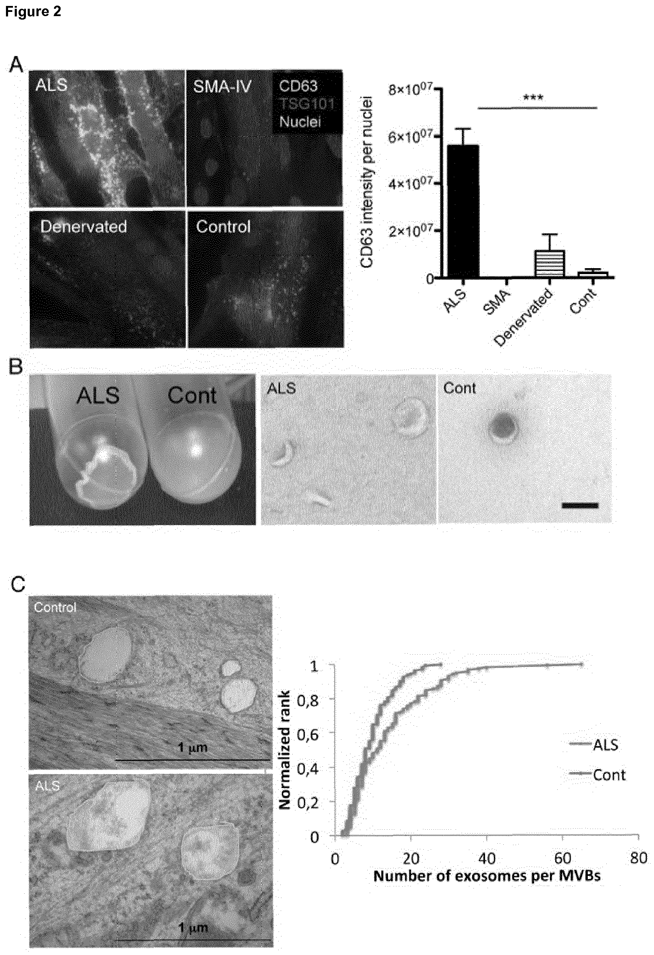

Referring to Figure 2(A), there is shown immunostaining for exosome markers of differentiated muscle stem cells. Left panel: Representative images of cultured muscle stem cells from ALS and SMA-III/IV patients, and from healthy control or denervated subjects labelled for exosomal markers (TSG101, CD63). Right Panel: quantification of CD63 fluorescence signal normalized per myonucleus. (n=8, 4, 2, 5 cell culture of different ALS, SMA-III/IV, polyneuritis and control subjects). ***, P<0.001.

Figure 2(B) illustrates exosomes are oversecreted by ALS muscle cells. Left panel: Representative picture of the exosome pellets from culture medium. The exosomes were extracted from the culture medium of 800,000 cells from ALS and control subjects. The experiment has been repeated 8 times on different cultures of muscle cells from ALS patients and control subjects. Right panel: electron microscopy confirming the presence of exosomes (cup-shape vesicles) in the pellets. The exosomes were more numerous in ALS pellets. Of note, the immunostaining performed on these vesicles confirmed that they are exosomes as they are positive for CD63 and CD82.

Figure 2(C) shows the multi-vesicular bodies of ALS muscle cells contain more exosomes than the controls. Left panel: Representative electron microscopy images showing an accumulation of exosomes in multi-vesicular bodies (MVBs, highlighted in green and orange) of ALS muscle cells compared to controls. Scale bar = 1 µm. Right panel: quantification of the number of exosome-like vesicles in the MVBs. Two sample Kolmogorov-Smirnov test confirmed the visual impression that the MVBs of ALS muscle cells contain a greater number of exosome-like vesicles compare to the control (P<0.05).

### Example 3 - Exosomes from ALS muscle cells are toxic to healthy myotubes and motor neurons

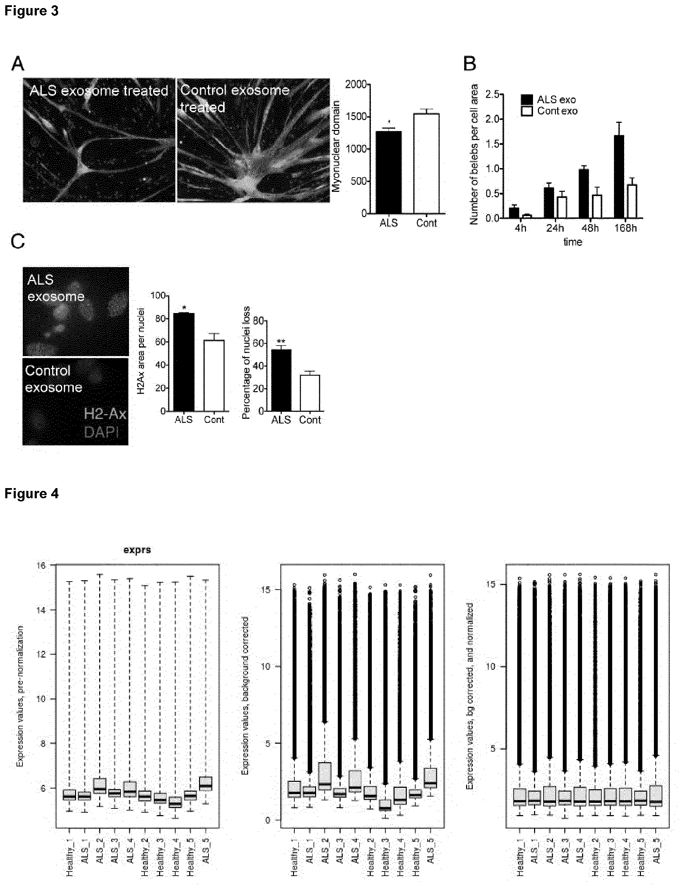

Referring to Figure 3(A), it is shown that ALS exosomes induce muscle cell atrophy. Left panel: Representative images of healthy myotubes treated with ALS or Control exosomes. Myotubes are stained with myosin heavy chain (green). Right panel: quantification of myonuclear domain size (area of myosin heavy chain staining divided by the number of nuclei). The ALS-exosome treated myotubes have a smaller myonuclear domain (* P<0.05; n=6/group).

Figure 3(B) illustrates ALS exosomes induce cell stress. Quantification of blebs numbers per healthy myotubes over time treated with ALS or control exosomes (n=6/group). ANOVA 2 factor interaction, time and treatment P<0.001.

Figure 3(C) illustrates ALS exosomes induce cell death. Left panel: representative immunostaining of H2-Ax immunostaining of healthy myotubes treated with ALS or control exosomes. Middle panel: quantification of H2-Ax signal per nucleus. Right panel: quantification of loss of motor neuron nuclei in culture.

### Example 4 - Identification of specific exosomal contents as biomarkers of ALS

Exosomal contents include various types of biomolecule, of which those potentially involved in signaling roles include proteins and their peptides, and non-coding RNAs. Muscle stem cells (primary myoblasts) were obtained from the Deltoid muscles of ALS patients and healthy controls and cultured to form differentiated myotubes. Exosomes were purified from the culture medium of the 10 million muscle cells following 3 days of culture, using total exosome isolation reagent (Invitrogen^{™} Life technologies^{™}). To identify the contents of exosomes, we carried out proteomic and non-coding RNA profiling of purified exosomes.

### Protein biomarkers

Proteomic mass spectrometry data identified peptides of 105 proteins (Table 1) having levels that were consistently increased in ALS muscle exosomes (n=3) compared to healthy control muscle exosomes (n=3).

Since the proteomic samples were normalized to protein concentration, it was also noted that proteins with consistently high levels in both ALS and Healthy muscle exosomes may also be good biomarkers, as these may still be elevated in ALS blood per volume due to our observation that muscle exosomes are secreted in greater abundance from ALS myotubes than from healthy controls. In this way, a further 3 candidates that had consistently high levels in both ALS and Healthy muscle exosomes, and had other features of mechanistic interest, were identified. Namely, these were DES, APP, and DAG1. APP is the primary component of amyloid plaques in Alzheimer's disease. DES is observed at high levels in all muscle tissue types but is entirely absent from other tissues. DAG1 is a major component of the dystroglycan complex, which is implicated in a number of muscular dystrophies.

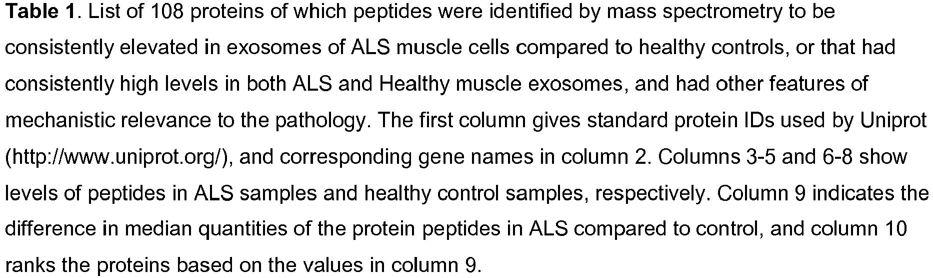

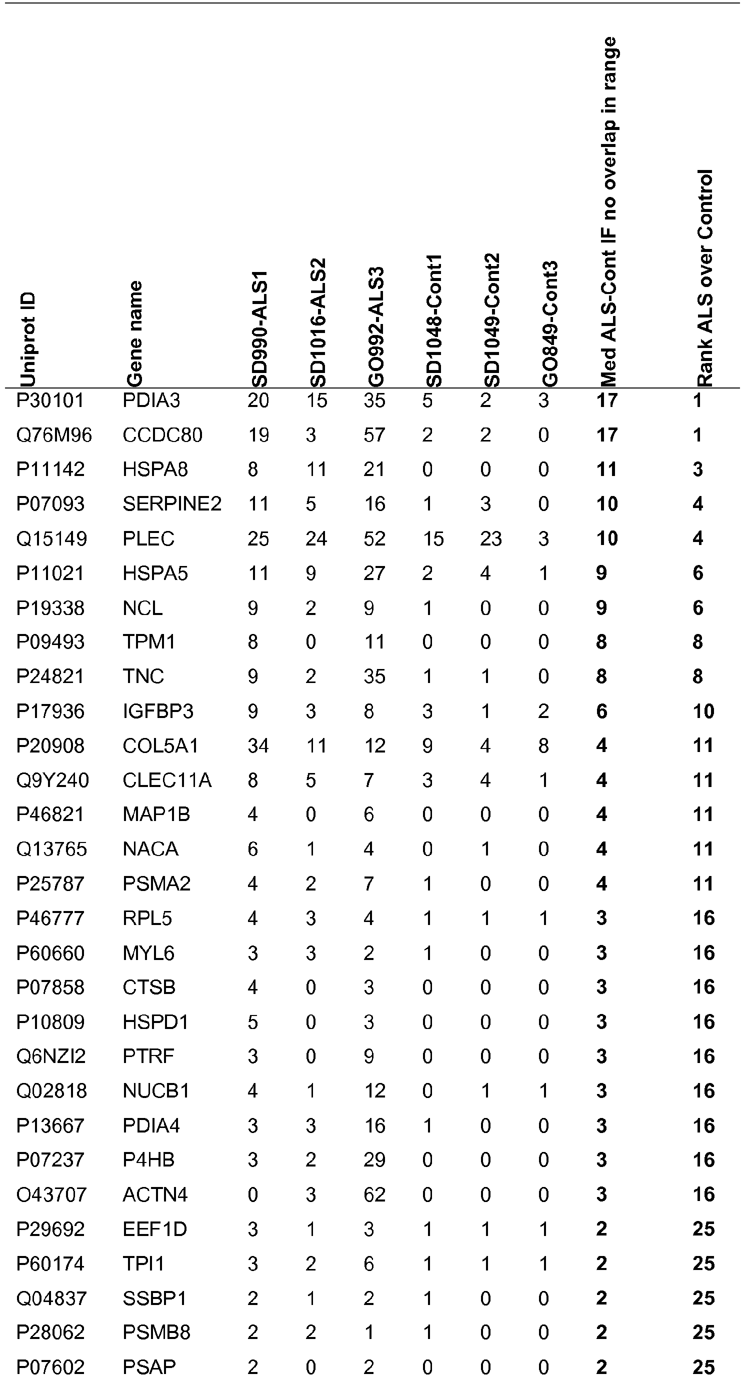

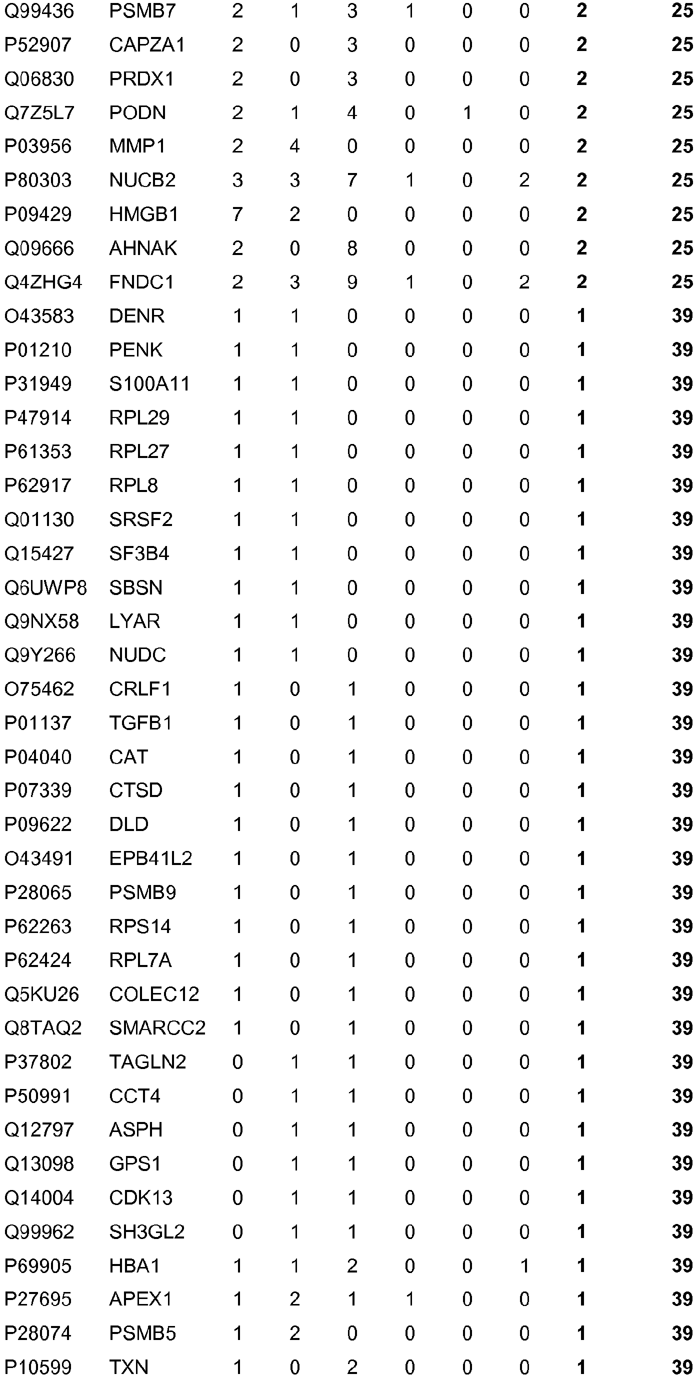

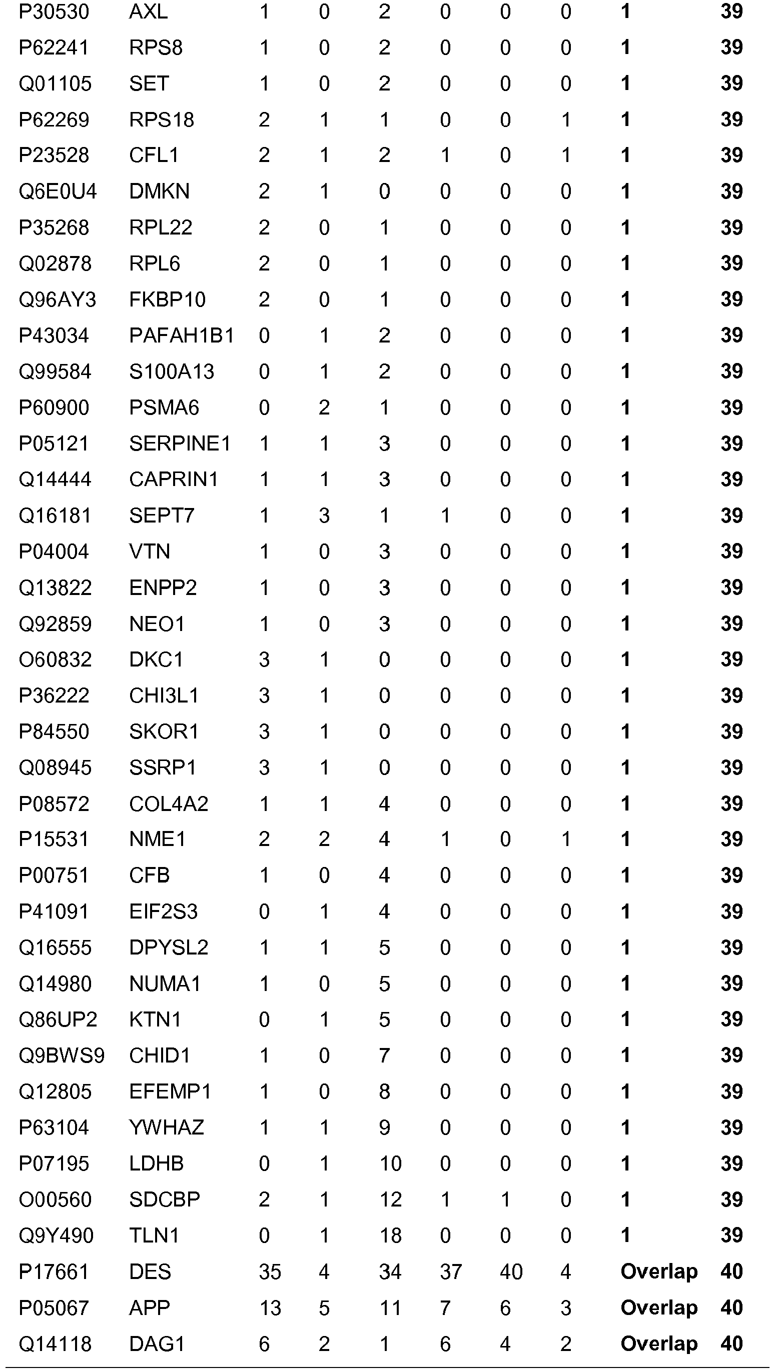

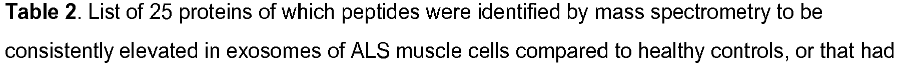

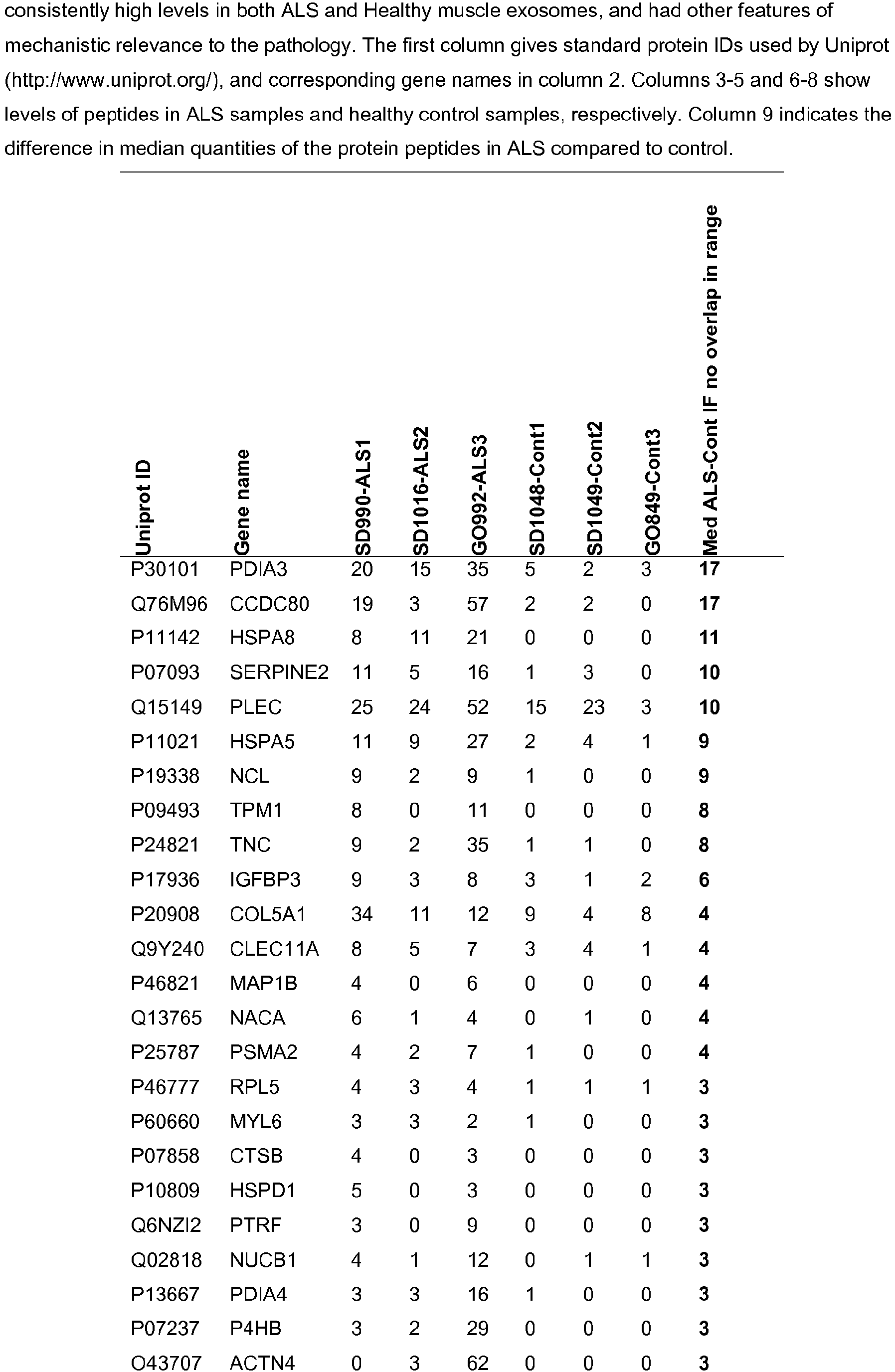

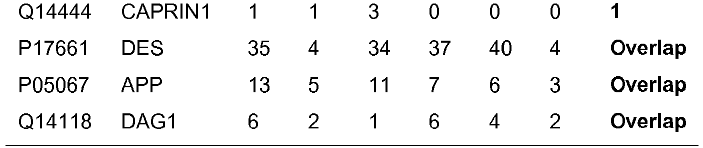

### Non-coding RNA biomarkers

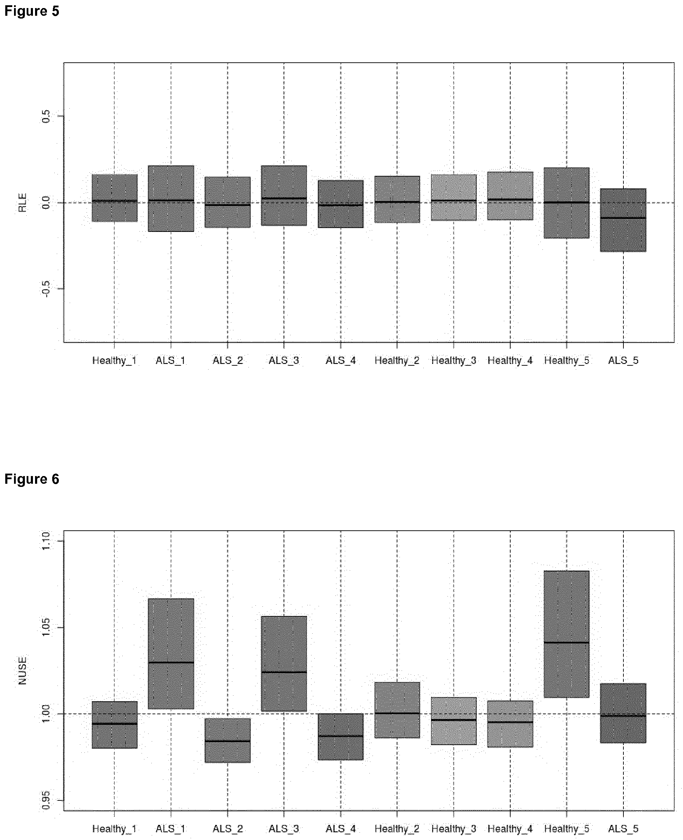

Profiling of non-coding RNA species was carried out by microarray (using Affymetrix GeneChip^{®} miRNA 4.0 Array) on ALS exosomes (n=5) compared to healthy controls (n=5). After quality control and normalization (see Fig. 4-6), one-way unpaired ANOVA identified 14 non-coding RNAs to be significantly dysregulated in ALS v healthy controls (Table 3: 12 up-regulated in ALS, 2 downregulated in ALS). Of these, 13 are microRNAs (indicated by their miRBase IDs hsa-*** and accession numbers MIMAT***), and 1 is the small nucleolar RNA snoU13 (ENSG00000239126).

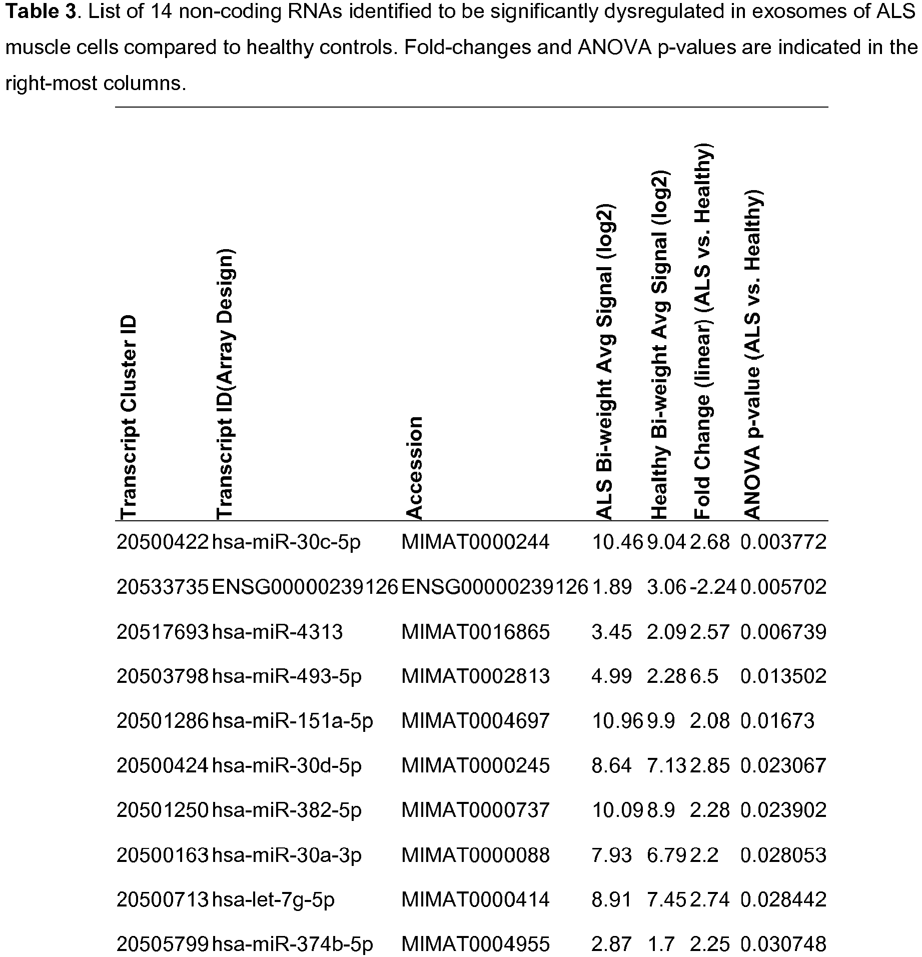

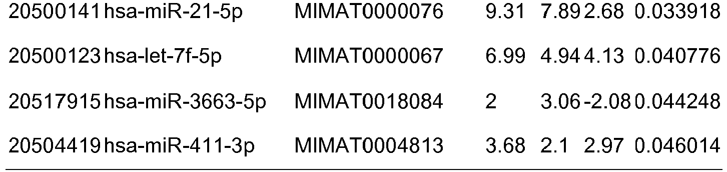

### Example 6 - Comparison of different biomarkers against different disease groups

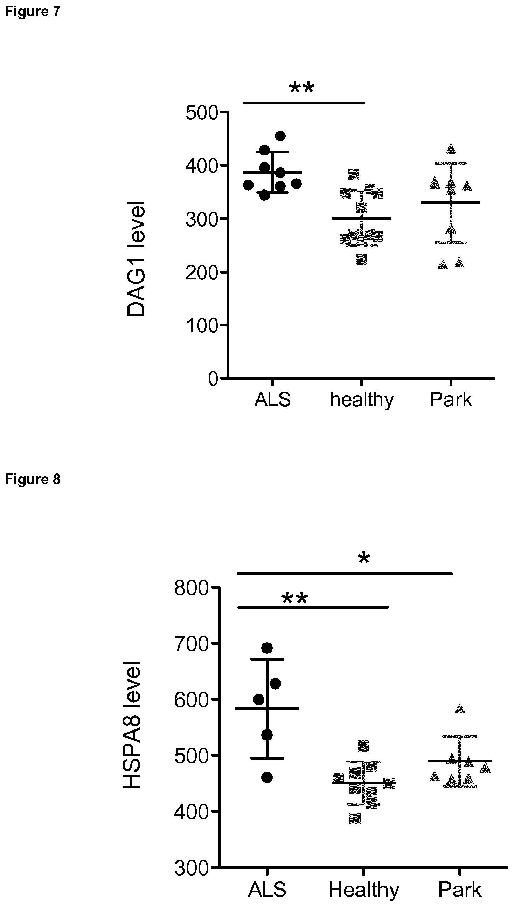

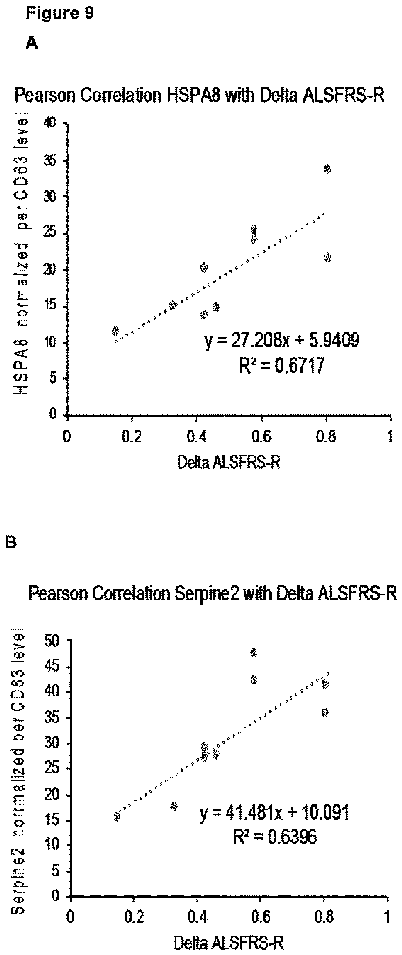

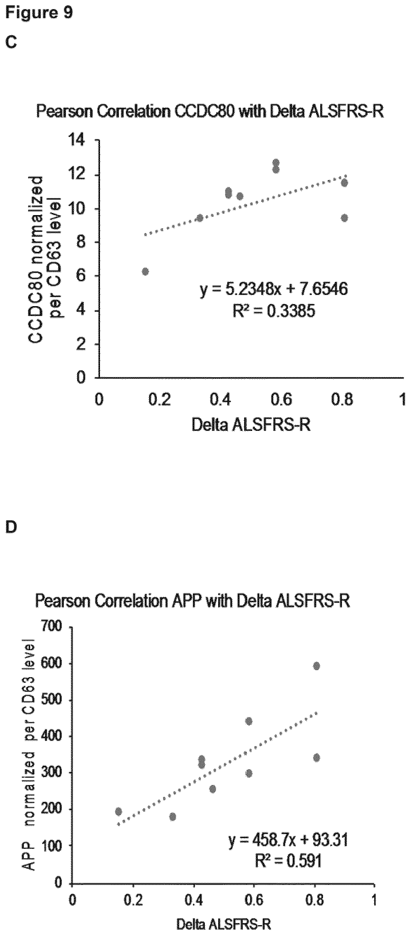

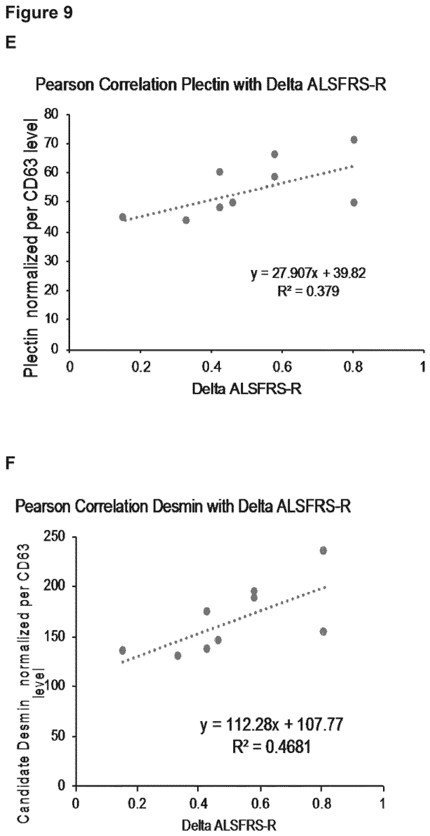

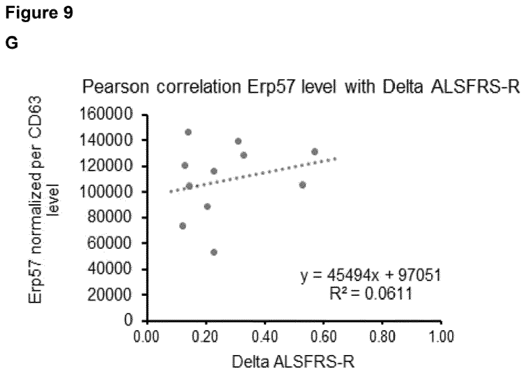

Blood samples were collected from 8 ALS patients, 11 healthy subjects and 9 Parkinson patients. The patient characteristics of the selected patients are presented in Table 4. Exosomes were extracted from serum and used for dot-blot analysis. Levels of DAG1 in exosomes isolated from the blood serum of patients with ALS disease, Parkinson's disease (disease control), or from healthy subjects are presented in Figure 7, and levels of HSPA8 in exosomes isolated from the blood serum of patients with ALS disease, Parkinson's disease (disease control), or from healthy subjects are presented in Figure 8. Levels of each biomarker in exosomes isolated from the blood serum of ALS patients, normalized against the quantity of total blood serum exosome (CD63) were plotted against the rate of progression of the pathology (delta ALSFRS-R) and are presented in Figure 9.

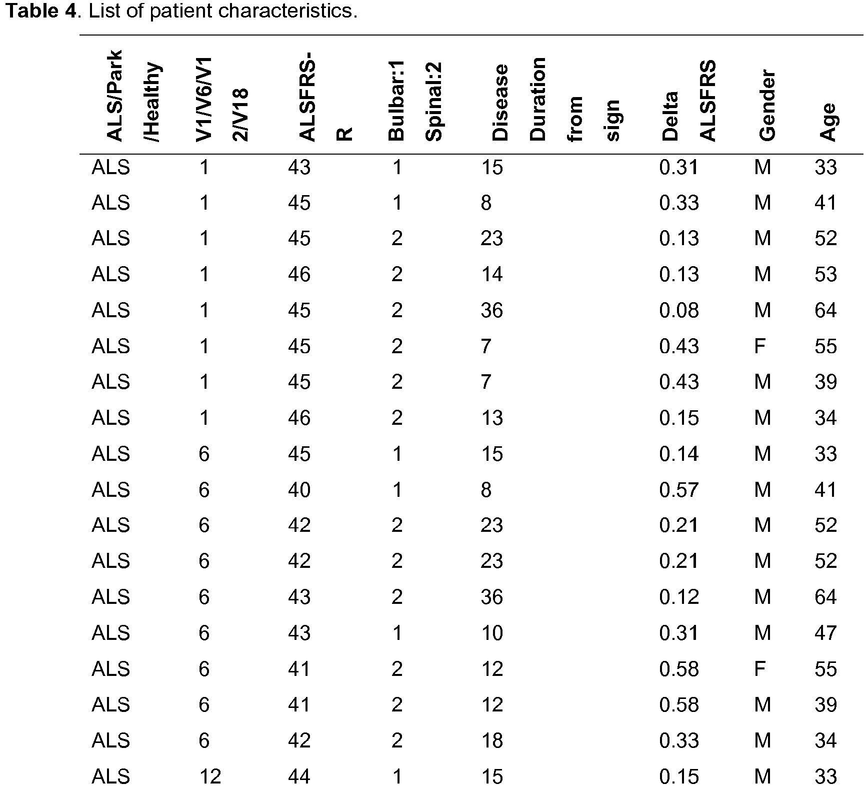

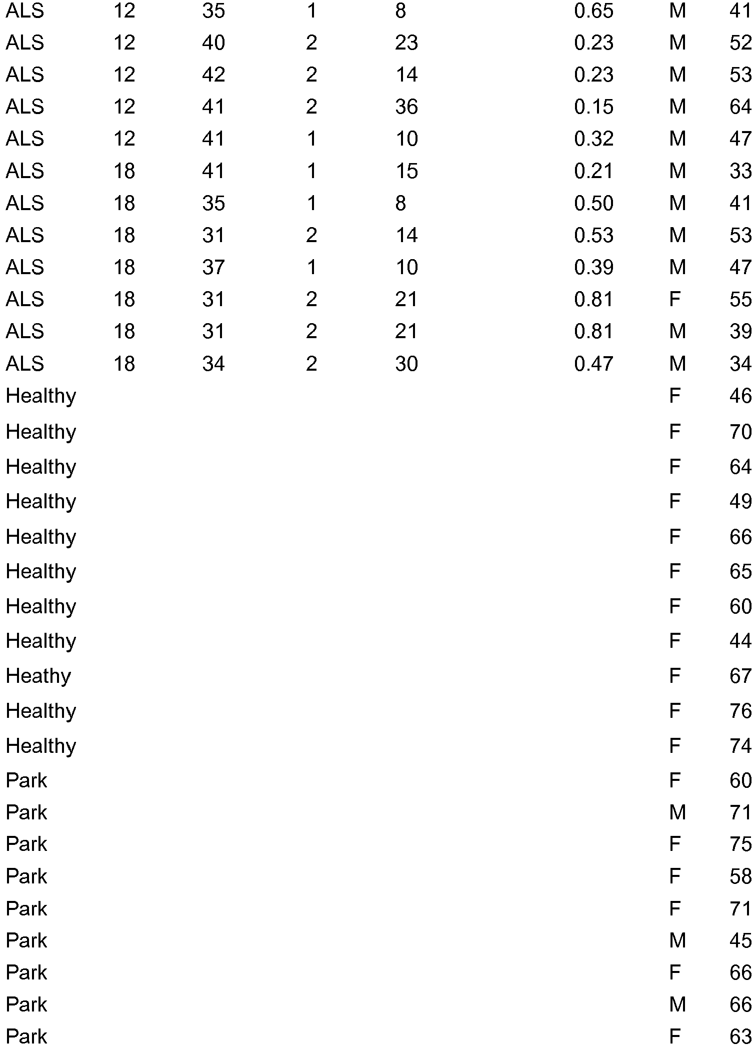

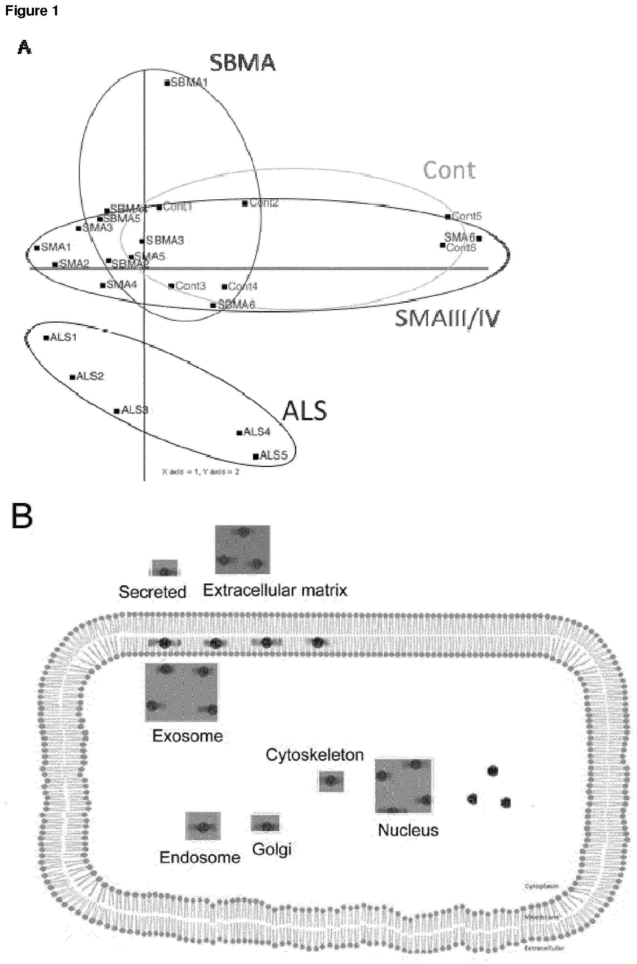

Here provided is a robust ALS gene expression signature and a new driver of toxicity in muscle cells and muscle-motor neuron interactions in ALS patients. By studying differentiated muscle (myotube) cells from muscle biopsies of sporadic ALS patients, the present invention shows: (1) the gene expression profiles of myotubes isolated from ALS patients cluster entirely separately not only from healthy control subjects but also from SBMA and SMA-III/IV disease controls (see Fig. 1A). The 30 genes most strongly contributing to this ALS-specific signature include genes encoding for proteins secreted or localized in exosomes (see Fig. 1B); (2) both intracellular exosomal biogenesis and extracellular exosomal secretion are dramatically increased in ALS myotubes (see Figs. 2A and B), and exosome-rich multi-vesicular bodies are observed by electron microscopy in muscle biopsies of ALS patients (see Fig. 2C); (3) increased exosomal biogenesis and secretion are not observed in myotubes of muscle-denervated human subjects (neither healthy, SBMA, nor SMA-III/IV) or of muscle-denervated healthy murine muscle (see Fig.1, 2A and other data not shown), indicating that exosomal secretion is not a simple downstream consequence of the disease.

## Claims

1. A method of diagnosing or prognosing amyotrophic lateral sclerosis in a subject, the method comprising the steps of:
(a) determining the quantitative or qualitative level of a biomarker in a biological sample from the subject; and
(b) diagnosing or prognosing the amyotrophic lateral sclerosis in the subject based on the quantitative or qualitative level of the biomarker in the biological sample; wherein the biomarker is DAG1.

2. A method according to Claim 1, wherein the biomarker is a gene.

3. A method according to Claim 1, wherein the biomarker is a protein encoded by the gene DAG1.

4. A method according to Claim 3, wherein the biomarker is a protein having a UniProtKB/Swiss-Prot Accession Number Q14118.

5. A method according to any one of Claims 1-4, wherein the diagnosing or prognosing step (b) comprises comparing the quantitative or qualitative level of the biomarker in the biological sample from the subject with the quantitative or qualitative level of the respective biomarker in a normal sample.

6. A method according to Claim 5, wherein a quantitative or qualitative level of the biomarker in the biological sample from the subject greater than the quantitative or qualitative level of the respective biomarker in a normal sample is indicative of the quantitative or qualitative presence of amyotrophic lateral sclerosis.

7. A method according to any one of Claims 1-6, wherein the biological sample is selected from whole blood, serum, plasma, urine, interstitial fluid, peritoneal fluid, cervical swab, tears, saliva, buccal swab, skin, brain tissue, and cerebrospinal fluid.

8. A method according to any one of Claims 1-7, wherein the biological sample is whole blood.

## Patentansprüche

1. Verfahren zum Diagnostizieren oder Prognostizieren von amyotropher Lateralsklerose bei einem Subjekt, wobei das Verfahren die folgenden Schritte umfasst:
(a) Bestimmen des quantitativen oder qualitativen Niveaus eines Biomarkers in einer biologischen Probe des Subjekts; und
(b) Diagnostizieren oder Prognostizieren der amyotrophen Lateralsklerose bei dem Subjekt auf Grundlage des quantitativen oder qualitativen Niveaus des Biomarkers in der biologischen Probe; wobei der Biomarker DAG1 ist.

2. Verfahren nach Anspruch 1, wobei der Biomarker ein Gen ist.

3. Verfahren nach Anspruch 1, wobei der Biomarker ein Protein ist, das durch das Gen DAG1 codiert wird.

4. Verfahren nach Anspruch 3, wobei der Biomarker ein Protein ist, das eine Accession Number Q14118 für UniProtKB/Swiss-Prot aufweist.

5. Verfahren nach einem der Ansprüche 1-4, wobei der Schritt (b) zum Diagnostizieren oder Prognostizieren Vergleichen des quantitativen oder qualitativen Niveaus des Biomarkers in der biologischen Probe des Subjekts mit dem quantitativen oder qualitativen Niveau des jeweiligen Biomarkers in einer normalen Probe umfasst.

6. Verfahren nach Anspruch 5, wobei ein quantitatives oder qualitatives Niveau des Biomarkers in der biologischen Probe des Subjekts, das größer ist als das quantitative oder qualitative Niveau des jeweiligen Biomarkers in einer normalen Probe, ein Hinweis auf das quantitative oder qualitative Vorliegen von amyotropher Lateralsklerose ist.

7. Verfahren nach einem der Ansprüche 1-6, wobei die biologische Probe ausgewählt ist aus Vollblut, Serum, Plasma, Urin, interstitieller Flüssigkeit, Peritonealflüssigkeit, Gebärmutterhalsabstrich, Tränen, Speichel, Wangenabstrich, Haut, Hirngewebe und Zerebrospinalflüssigkeit.

8. Verfahren nach einem der Ansprüche 1-7, wobei die biologische Probe Vollblut ist.

## Revendications

1. Procédé de diagnostic ou de pronostic de la sclérose latérale amyotrophique chez un sujet, le procédé comprenant les étapes de :
(a) détermination du niveau quantitatif ou qualitatif d'un biomarqueur dans un échantillon biologique du sujet ; et
(b) diagnostic ou pronostic de la sclérose latérale amyotrophique chez le sujet sur la base du niveau quantitatif ou qualitatif du biomarqueur dans l'échantillon biologique ; dans lequel le biomarqueur est DAG1.

2. Procédé selon la revendication 1, dans lequel le biomarqueur est un gène.

3. Procédé selon la revendication 1, dans lequel le biomarqueur est une protéine codée par le gène DAG1.

4. Procédé selon la revendication 3, dans lequel le biomarqueur est une protéine ayant un numéro d'accès UniProtKB/Swiss-Prot Q14118.

5. Procédé selon l'une quelconque des revendications 1 à 4, dans lequel l'étape de diagnostic ou de pronostic (b) comprend la comparaison du niveau quantitatif ou qualitatif du biomarqueur dans l'échantillon biologique du sujet avec le niveau quantitatif ou qualitatif du biomarqueur respectif dans un échantillon normal.

6. Procédé selon la revendication 5, dans lequel un niveau quantitatif ou qualitatif du biomarqueur dans l'échantillon biologique du sujet supérieur au niveau quantitatif ou qualitatif du biomarqueur respectif dans un échantillon normal indique la présence quantitative ou qualitative de sclérose latérale amyotrophique.

7. Procédé selon l'une quelconque des revendications 1 à 6, dans lequel l'échantillon biologique est choisi parmi le sang total, le sérum, le plasma, l'urine, le liquide interstitiel, le liquide péritonéal, l'écouvillon cervical, les larmes, la salive, l'écouvillon buccal, la peau, le tissu cérébral et le liquide céphalo-rachidien.

8. Procédé selon l'une quelconque des revendications 1 à 7, dans lequel l'échantillon biologique est du sang total.


### SEQUENCE LISTING

<110> UNIVERSITY OF ULSTER
<120> A method of diagnosing or prognosing a neurological disorder in a subject
<130> P122497PC00
<150> GB1807178.7
   <151> 2018-05-01
<160> 122
<170> PatentIn version 3.5
<210> 1
   <211> 505
   <212> PRT
   <213> Homo sapiens
<400> 1
   Met Arg Leu Arg Arg Leu Ala Leu Phe Pro Gly Val Ala Leu Leu Leu
   1               5                   10                  15
   Ala Ala Ala Arg Leu Ala Ala Ala Ser Asp Val Leu Glu Leu Thr Asp
               20                  25                  30
   Asp Asn Phe Glu Ser Arg Ile Ser Asp Thr Gly Ser Ala Gly Leu Met
           35                  40                  45
   Leu Val Glu Phe Phe Ala Pro Trp Cys Gly His Cys Lys Arg Leu Ala
       50                  55                  60
   Pro Glu Tyr Glu Ala Ala Ala Thr Arg Leu Lys Gly Ile Val Pro Leu
   65                  70                  75                  80
   Ala Lys Val Asp Cys Thr Ala Asn Thr Asn Thr Cys Asn Lys Tyr Gly
                   85                  90                  95
   Val Ser Gly Tyr Pro Thr Leu Lys Ile Phe Arg Asp Gly Glu Glu Ala
               100                 105                 110
   Gly Ala Tyr Asp Gly Pro Arg Thr Ala Asp Gly Ile Val Ser His Leu
           115                 120                 125
   Lys Lys Gln Ala Gly Pro Ala Ser Val Pro Leu Arg Thr Glu Glu Glu
       130                 135                 140
   Phe Lys Lys Phe Ile Ser Asp Lys Asp Ala Ser Ile Val Gly Phe Phe
   145                 150                 155                 160
   Asp Asp Ser Phe Ser Glu Ala His Ser Glu Phe Leu Lys Ala Ala Ser
                   165                 170                 175
   Asn Leu Arg Asp Asn Tyr Arg Phe Ala His Thr Asn Val Glu Ser Leu
               180                 185                 190
   Val Asn Glu Tyr Asp Asp Asn Gly Glu Gly Ile Ile Leu Phe Arg Pro
           195                 200                 205
   Ser His Leu Thr Asn Lys Phe Glu Asp Lys Thr Val Ala Tyr Thr Glu
       210                 215                 220
   Gln Lys Met Thr Ser Gly Lys Ile Lys Lys Phe Ile Gln Glu Asn Ile
   225                 230                 235                 240
   Phe Gly Ile Cys Pro His Met Thr Glu Asp Asn Lys Asp Leu Ile Gln
                   245                 250                 255
   Gly Lys Asp Leu Leu Ile Ala Tyr Tyr Asp Val Asp Tyr Glu Lys Asn
               260                 265                 270
   Ala Lys Gly Ser Asn Tyr Trp Arg Asn Arg Val Met Met Val Ala Lys
           275                 280                 285
   Lys Phe Leu Asp Ala Gly His Lys Leu Asn Phe Ala Val Ala Ser Arg
       290                 295                 300
   Lys Thr Phe Ser His Glu Leu Ser Asp Phe Gly Leu Glu Ser Thr Ala
   305                 310                 315                 320
   Gly Glu Ile Pro Val Val Ala Ile Arg Thr Ala Lys Gly Glu Lys Phe
                   325                 330                 335
   Val Met Gln Glu Glu Phe Ser Arg Asp Gly Lys Ala Leu Glu Arg Phe
               340                 345                 350
   Leu Gln Asp Tyr Phe Asp Gly Asn Leu Lys Arg Tyr Leu Lys Ser Glu
           355                 360                 365
   Pro Ile Pro Glu Ser Asn Asp Gly Pro Val Lys Val Val Val Ala Glu
       370                 375                 380
   Asn Phe Asp Glu Ile Val Asn Asn Glu Asn Lys Asp Val Leu Ile Glu
   385                 390                 395                 400
   Phe Tyr Ala Pro Trp Cys Gly His Cys Lys Asn Leu Glu Pro Lys Tyr
                   405                 410                 415
   Lys Glu Leu Gly Glu Lys Leu Ser Lys Asp Pro Asn Ile Val Ile Ala
               420                 425                 430
   Lys Met Asp Ala Thr Ala Asn Asp Val Pro Ser Pro Tyr Glu Val Arg
           435                 440                 445
   Gly Phe Pro Thr Ile Tyr Phe Ser Pro Ala Asn Lys Lys Leu Asn Pro
       450                 455                 460
   Lys Lys Tyr Glu Gly Gly Arg Glu Leu Ser Asp Phe Ile Ser Tyr Leu
   465                 470                 475                 480
   Gln Arg Glu Ala Thr Asn Pro Pro Val Ile Gln Glu Glu Lys Pro Lys
                   485                 490                 495
   Lys Lys Lys Lys Ala Gln Glu Asp Leu
               500                 505
<210> 2
   <211> 950
   <212> PRT
   <213> Homo sapiens
<400> 2
   Met Thr Trp Arg Met Gly Pro Arg Phe Thr Met Leu Leu Ala Met Trp
   1               5                   10                  15
   Leu Val Cys Gly Ser Glu Pro His Pro His Ala Thr Ile Arg Gly Ser
               20                  25                  30
   His Gly Gly Arg Lys Val Pro Leu Val Ser Pro Asp Ser Ser Arg Pro
           35                  40                  45
   Ala Arg Phe Leu Arg His Thr Gly Arg Ser Arg Gly Ile Glu Arg Ser
       50                  55                  60
   Thr Leu Glu Glu Pro Asn Leu Gln Pro Leu Gln Arg Arg Arg Ser Val
   65                  70                  75                  80
   Pro Val Leu Arg Leu Ala Arg Pro Thr Glu Pro Pro Ala Arg Ser Asp
                   85                  90                  95
   Ile Asn Gly Ala Ala Val Arg Pro Glu Gln Arg Pro Ala Ala Arg Gly
               100                 105                 110
   Ser Pro Arg Glu Met Ile Arg Asp Glu Gly Ser Ser Ala Arg Ser Arg
           115                 120                 125
   Met Leu Arg Phe Pro Ser Gly Ser Ser Ser Pro Asn Ile Leu Ala Ser
       130                 135                 140
   Phe Ala Gly Lys Asn Arg Val Trp Val Ile Ser Ala Pro His Ala Ser
   145                 150                 155                 160
   Glu Gly Tyr Tyr Arg Leu Met Met Ser Leu Leu Lys Asp Asp Val Tyr
                   165                 170                 175
   Cys Glu Leu Ala Glu Arg His Ile Gln Gln Ile Val Leu Phe His Gln
               180                 185                 190
   Ala Gly Glu Glu Gly Gly Lys Val Arg Arg Ile Thr Ser Glu Gly Gln
           195                 200                 205
   Ile Leu Glu Gln Pro Leu Asp Pro Ser Leu Ile Pro Lys Leu Met Ser
       210                 215                 220
   Phe Leu Lys Leu Glu Lys Gly Lys Phe Gly Met Val Leu Leu Lys Lys
   225                 230                 235                 240
   Thr Leu Gln Val Glu Glu Arg Tyr Pro Tyr Pro Val Arg Leu Glu Ala
                   245                 250                 255
   Met Tyr Glu Val Ile Asp Gln Gly Pro Ile Arg Arg Ile Glu Lys Ile
               260                 265                 270
   Arg Gln Lys Gly Phe Val Gln Lys Cys Lys Ala Ser Gly Val Glu Gly
           275                 280                 285
   Gln Val Val Ala Glu Gly Asn Asp Gly Gly Gly Gly Ala Gly Arg Pro
       290                 295                 300
   Ser Leu Gly Ser Glu Lys Lys Lys Glu Asp Pro Arg Arg Ala Gln Val
   305                 310                 315                 320
   Pro Pro Thr Arg Glu Ser Arg Val Lys Val Leu Arg Lys Leu Ala Ala
                   325                 330                 335
   Thr Ala Pro Ala Leu Pro Gln Pro Pro Ser Thr Pro Arg Ala Thr Thr
               340                 345                 350
   Leu Pro Pro Ala Pro Ala Thr Thr Val Thr Arg Ser Thr Ser Arg Ala
           355                 360                 365
   Val Thr Val Ala Ala Arg Pro Met Thr Thr Thr Ala Phe Pro Thr Thr
       370                 375                 380
   Gln Arg Pro Trp Thr Pro Ser Pro Ser His Arg Pro Pro Thr Thr Thr
   385                 390                 395                 400
   Glu Val Ile Thr Ala Arg Arg Pro Ser Val Ser Glu Asn Leu Tyr Pro
                   405                 410                 415
   Pro Ser Arg Lys Asp Gln His Arg Glu Arg Pro Gln Thr Thr Arg Arg
               420                 425                 430
   Pro Ser Lys Ala Thr Ser Leu Glu Ser Phe Thr Asn Ala Pro Pro Thr
           435                 440                 445
   Thr Ile Ser Glu Pro Ser Thr Arg Ala Ala Gly Pro Gly Arg Phe Arg
       450                 455                 460
   Asp Asn Arg Met Asp Arg Arg Glu His Gly His Arg Asp Pro Asn Val
   465                 470                 475                 480
   Val Pro Gly Pro Pro Lys Pro Ala Lys Glu Lys Pro Pro Lys Lys Lys
                   485                 490                 495
   Ala Gln Asp Lys Ile Leu Ser Asn Glu Tyr Glu Glu Lys Tyr Asp Leu
               500                 505                 510
   Ser Arg Pro Thr Ala Ser Gln Leu Glu Asp Glu Leu Gln Val Gly Asn
           515                 520                 525
   Val Pro Leu Lys Lys Ala Lys Glu Ser Lys Lys His Glu Lys Leu Glu
       530                 535                 540
   Lys Pro Glu Lys Glu Lys Lys Lys Lys Met Lys Asn Glu Asn Ala Asp
   545                 550                 555                 560
   Lys Leu Leu Lys Ser Glu Lys Gln Met Lys Lys Ser Glu Lys Lys Ser
                   565                 570                 575
   Lys Gln Glu Lys Glu Lys Ser Lys Lys Lys Lys Gly Gly Lys Thr Glu
               580                 585                 590
   Gln Asp Gly Tyr Gln Lys Pro Thr Asn Lys His Phe Thr Gln Ser Pro
           595                 600                 605
   Lys Lys Ser Val Ala Asp Leu Leu Gly Ser Phe Glu Gly Lys Arg Arg
       610                 615                 620
   Leu Leu Leu Ile Thr Ala Pro Lys Ala Glu Asn Asn Met Tyr Val Gln
   625 630 635 640
   Gln Arg Asp Glu Tyr Leu Glu Ser Phe Cys Lys Met Ala Thr Arg Lys
   645 650 655
   Ile Ser Val Ile Thr Ile Phe Gly Pro Val Asn Asn Ser Thr Met Lys
   660 665 670
   Ile Asp His Phe Gln Leu Asp Asn Glu Lys Pro Met Arg Val Val Asp
   675 680 685
   Asp Glu Asp Leu Val Asp Gln Arg Leu Ile Ser Glu Leu Arg Lys Glu
   690 695 700
   Tyr Gly Met Thr Tyr Asn Asp Phe Phe Met Val Leu Thr Asp Val Asp
   705 710 715 720
   Leu Arg Val Lys Gln Tyr Tyr Glu Val Pro Ile Thr Met Lys Ser Val
   725 730 735
   Phe Asp Leu Ile Asp Thr Phe Gln Ser Arg Ile Lys Asp Met Glu Lys
   740 745 750
   Gln Lys Lys Glu Gly Ile Val Cys Lys Glu Asp Lys Lys Gln Ser Leu
   755 760 765
   Glu Asn Phe Leu Ser Arg Phe Arg Trp Arg Arg Arg Leu Leu Val Ile
   770 775 780
   Ser Ala Pro Asn Asp Glu Asp Trp Ala Tyr Ser Gln Gln Leu Ser Ala
   785 790 795 800
   Leu Ser Gly Gln Ala Cys Asn Phe Gly Leu Arg His Ile Thr Ile Leu
   805 810 815
   Lys Leu Leu Gly Val Gly Glu Glu Val Gly Gly Val Leu Glu Leu Phe
   820 825 830
   Pro Ile Asn Gly Ser Ser Val Val Glu Arg Glu Asp Val Pro Ala His
   835 840 845
   Leu Val Lys Asp Ile Arg Asn Tyr Phe Gln Val Ser Pro Glu Tyr Phe
   850 855 860
   Ser Met Leu Leu Val Gly Lys Asp Gly Asn Val Lys Ser Trp Tyr Pro
   865 870 875 880
   Ser Pro Met Trp Ser Met Val Ile Val Tyr Asp Leu Ile Asp Ser Met
   885 890 895
   Gln Leu Arg Arg Gln Glu Met Ala Ile Gln Gln Ser Leu Gly Met Arg
   900 905 910
   Cys Pro Glu Asp Glu Tyr Ala Gly Tyr Gly Tyr His Ser Tyr His Gln
   915 920 925
   Gly Tyr Gln Asp Gly Tyr Gln Asp Asp Tyr Arg His His Glu Ser Tyr
   930 935 940
   His His Gly Tyr Pro Tyr
   945 950
<210> 3
   <211> 646
   <212> PRT
   <213> Homo sapiens
<400> 3
   Met Ser Lys Gly Pro Ala Val Gly Ile Asp Leu Gly Thr Thr Tyr Ser
   1 5 10 15
   Cys Val Gly Val Phe Gln His Gly Lys Val Glu Ile Ile Ala Asn Asp
   20 25 30
   Gln Gly Asn Arg Thr Thr Pro Ser Tyr Val Ala Phe Thr Asp Thr Glu
   35 40 45
   Arg Leu Ile Gly Asp Ala Ala Lys Asn Gln Val Ala Met Asn Pro Thr
   50 55 60
   Asn Thr Val Phe Asp Ala Lys Arg Leu Ile Gly Arg Arg Phe Asp Asp
   65 70 75 80
   Ala Val Val Gln Ser Asp Met Lys His Trp Pro Phe Met Val Val Asn
   85 90 95
   Asp Ala Gly Arg Pro Lys Val Gln Val Glu Tyr Lys Gly Glu Thr Lys
   100 105 110
   Ser Phe Tyr Pro Glu Glu Val Ser Ser Met Val Leu Thr Lys Met Lys
   115 120 125
   Glu Ile Ala Glu Ala Tyr Leu Gly Lys Thr Val Thr Asn Ala Val Val
   130 135 140
   Thr Val Pro Ala Tyr Phe Asn Asp Ser Gln Arg Gln Ala Thr Lys Asp
   145                 150                 155                 160
   Ala Gly Thr Ile Ala Gly Leu Asn Val Leu Arg Ile Ile Asn Glu Pro
                   165                 170                 175
   Thr Ala Ala Ala Ile Ala Tyr Gly Leu Asp Lys Lys Val Gly Ala Glu
               180                 185                 190
   Arg Asn Val Leu Ile Phe Asp Leu Gly Gly Gly Thr Phe Asp Val Ser
           195                 200                 205
   Ile Leu Thr Ile Glu Asp Gly Ile Phe Glu Val Lys Ser Thr Ala Gly
       210                 215                 220
   Asp Thr His Leu Gly Gly Glu Asp Phe Asp Asn Arg Met Val Asn His
   225                 230                 235                 240
   Phe Ile Ala Glu Phe Lys Arg Lys His Lys Lys Asp Ile Ser Glu Asn
                   245                 250                 255
   Lys Arg Ala Val Arg Arg Leu Arg Thr Ala Cys Glu Arg Ala Lys Arg
               260                 265                 270
   Thr Leu Ser Ser Ser Thr Gln Ala Ser Ile Glu Ile Asp Ser Leu Tyr
           275                 280                 285
   Glu Gly Ile Asp Phe Tyr Thr Ser Ile Thr Arg Ala Arg Phe Glu Glu
       290                 295                 300
   Leu Asn Ala Asp Leu Phe Arg Gly Thr Leu Asp Pro Val Glu Lys Ala
   305                 310                 315                 320
   Leu Arg Asp Ala Lys Leu Asp Lys Ser Gln Ile His Asp Ile Val Leu
                   325                 330                 335
   Val Gly Gly Ser Thr Arg Ile Pro Lys Ile Gln Lys Leu Leu Gln Asp
               340                 345                 350
   Phe Phe Asn Gly Lys Glu Leu Asn Lys Ser Ile Asn Pro Asp Glu Ala
           355                 360                 365
   Val Ala Tyr Gly Ala Ala Val Gln Ala Ala Ile Leu Ser Gly Asp Lys
       370                 375                 380
   Ser Glu Asn Val Gln Asp Leu Leu Leu Leu Asp Val Thr Pro Leu Ser
   385                 390                 395                 400
   Leu Gly Ile Glu Thr Ala Gly Gly Val Met Thr Val Leu Ile Lys Arg
   405 410 415
   Asn Thr Thr Ile Pro Thr Lys Gln Thr Gln Thr Phe Thr Thr Tyr Ser
   420 425 430
   Asp Asn Gln Pro Gly Val Leu Ile Gln Val Tyr Glu Gly Glu Arg Ala
   435 440 445
   Met Thr Lys Asp Asn Asn Leu Leu Gly Lys Phe Glu Leu Thr Gly Ile
   450 455 460
   Pro Pro Ala Pro Arg Gly Val Pro Gln Ile Glu Val Thr Phe Asp Ile
   465 470 475 480
   Asp Ala Asn Gly Ile Leu Asn Val Ser Ala Val Asp Lys Ser Thr Gly
   485 490 495
   Lys Glu Asn Lys Ile Thr Ile Thr Asn Asp Lys Gly Arg Leu Ser Lys
   500 505 510
   Glu Asp Ile Glu Arg Met Val Gln Glu Ala Glu Lys Tyr Lys Ala Glu
   515 520 525
   Asp Glu Lys Gln Arg Asp Lys Val Ser Ser Lys Asn Ser Leu Glu Ser
   530 535 540
   Tyr Ala Phe Asn Met Lys Ala Thr Val Glu Asp Glu Lys Leu Gln Gly
   545 550 555 560
   Lys Ile Asn Asp Glu Asp Lys Gln Lys Ile Leu Asp Lys Cys Asn Glu
   565 570 575
   Ile Ile Asn Trp Leu Asp Lys Asn Gln Thr Ala Glu Lys Glu Glu Phe
   580 585 590
   Glu His Gln Gln Lys Glu Leu Glu Lys Val Cys Asn Pro Ile Ile Thr
   595 600 605
   Lys Leu Tyr Gln Ser Ala Gly Gly Met Pro Gly Gly Met Pro Gly Gly
   610 615 620
   Phe Pro Gly Gly Gly Ala Pro Pro Ser Gly Gly Ala Ser Ser Gly Pro
   625 630 635 640
   Thr Ile Glu Glu Val Asp
   645
<210> 4
   <211> 398
   <212> PRT
   <213> Homo sapiens
<400> 4
   Met Asn Trp His Leu Pro Leu Phe Leu Leu Ala Ser Val Thr Leu Pro
   1               5                   10                  15
   Ser Ile Cys Ser His Phe Asn Pro Leu Ser Leu Glu Glu Leu Gly Ser
               20                  25                  30
   Asn Thr Gly Ile Gln Val Phe Asn Gln Ile Val Lys Ser Arg Pro His
           35                  40                  45
   Asp Asn Ile Val Ile Ser Pro His Gly Ile Ala Ser Val Leu Gly Met
       50                  55                  60
   Leu Gln Leu Gly Ala Asp Gly Arg Thr Lys Lys Gln Leu Ala Met Val
   65                  70                  75                  80
   Met Arg Tyr Gly Val Asn Gly Val Gly Lys Ile Leu Lys Lys Ile Asn
                   85                  90                  95
   Lys Ala Ile Val Ser Lys Lys Asn Lys Asp Ile Val Thr Val Ala Asn
               100                 105                 110
   Ala Val Phe Val Lys Asn Ala Ser Glu Ile Glu Val Pro Phe Val Thr
           115                 120                 125
   Arg Asn Lys Asp Val Phe Gln Cys Glu Val Arg Asn Val Asn Phe Glu
       130                 135                 140
   Asp Pro Ala Ser Ala Cys Asp Ser Ile Asn Ala Trp Val Lys Asn Glu
   145                 150                 155                 160
   Thr Arg Asp Met Ile Asp Asn Leu Leu Ser Pro Asp Leu Ile Asp Gly
                   165                 170                 175
   Val Leu Thr Arg Leu Val Leu Val Asn Ala Val Tyr Phe Lys Gly Leu
               180                 185                 190
   Trp Lys Ser Arg Phe Gln Pro Glu Asn Thr Lys Lys Arg Thr Phe Val
           195                 200                 205
   Ala Ala Asp Gly Lys Ser Tyr Gln Val Pro Met Leu Ala Gln Leu Ser
       210                 215                 220
   Val Phe Arg Cys Gly Ser Thr Ser Ala Pro Asn Asp Leu Trp Tyr Asn
   225 230 235 240
   Phe Ile Glu Leu Pro Tyr His Gly Glu Ser Ile Ser Met Leu Ile Ala
   245 250 255
   Leu Pro Thr Glu Ser Ser Thr Pro Leu Ser Ala Ile Ile Pro His Ile
   260 265 270
   Ser Thr Lys Thr Ile Asp Ser Trp Met Ser Ile Met Val Pro Lys Arg
   275 280 285
   Val Gln Val Ile Leu Pro Lys Phe Thr Ala Val Ala Gln Thr Asp Leu
   290 295 300
   Lys Glu Pro Leu Lys Val Leu Gly Ile Thr Asp Met Phe Asp Ser Ser
   305 310 315 320
   Lys Ala Asn Phe Ala Lys Ile Thr Thr Gly Ser Glu Asn Leu His Val
   325 330 335
   Ser His Ile Leu Gln Lys Ala Lys Ile Glu Val Ser Glu Asp Gly Thr
   340 345 350
   Lys Ala Ser Ala Ala Thr Thr Ala Ile Leu Ile Ala Arg Ser Ser Pro
   355 360 365
   Pro Trp Phe Ile Val Asp Arg Pro Phe Leu Phe Phe Ile Arg His Asn
   370 375 380
   Pro Thr Gly Ala Val Leu Phe Met Gly Gln Ile Asn Lys Pro
   385 390 395
<210> 5
   <211> 4684
   <212> PRT
   <213> Homo sapiens
<400> 5
   Met Val Ala Gly Met Leu Met Pro Arg Asp Gln Leu Arg Ala Ile Tyr
   1 5 10 15
   Glu Val Leu Phe Arg Glu Gly Val Met Val Ala Lys Lys Asp Arg Arg
   20 25 30
   Pro Arg Ser Leu His Pro His Val Pro Gly Val Thr Asn Leu Gln Val
   35 40 45
   Met Arg Ala Met Ala Ser Leu Arg Ala Arg Gly Leu Val Arg Glu Thr
   50 55 60
   Phe Ala Trp Cys His Phe Tyr Trp Tyr Leu Thr Asn Glu Gly Ile Ala
   65 70 75 80
   His Leu Arg Gln Tyr Leu His Leu Pro Pro Glu Ile Val Pro Ala Ser
   85 90 95
   Leu Gln Arg Val Arg Arg Pro Val Ala Met Val Met Pro Ala Arg Arg
   100 105 110
   Thr Pro His Val Gln Ala Val Gln Gly Pro Leu Gly Ser Pro Pro Lys
   115 120 125
   Arg Gly Pro Leu Pro Thr Glu Glu Gln Arg Val Tyr Arg Arg Lys Glu
   130 135 140
   Leu Glu Glu Val Ser Pro Glu Thr Pro Val Val Pro Ala Thr Thr Gln
   145 150 155 160
   Arg Thr Leu Ala Arg Pro Gly Pro Glu Pro Ala Pro Ala Thr Asp Glu
   165 170 175
   Arg Asp Arg Val Gln Lys Lys Thr Phe Thr Lys Trp Val Asn Lys His
   180 185 190
   Leu Ile Lys Ala Gln Arg His Ile Ser Asp Leu Tyr Glu Asp Leu Arg
   195 200 205
   Asp Gly His Asn Leu Ile Ser Leu Leu Glu Val Leu Ser Gly Asp Ser
   210 215 220
   Leu Pro Arg Glu Lys Gly Arg Met Arg Phe His Lys Leu Gln Asn Val
   225 230 235 240
   Gln Ile Ala Leu Asp Tyr Leu Arg His Arg Gln Val Lys Leu Val Asn
   245 250 255
   Ile Arg Asn Asp Asp Ile Ala Asp Gly Asn Pro Lys Leu Thr Leu Gly
   260 265 270
   Leu Ile Trp Thr Ile Ile Leu His Phe Gln Ile Ser Asp Ile Gln Val
   275 280 285
   Ser Gly Gln Ser Glu Asp Met Thr Ala Lys Glu Lys Leu Leu Leu Trp
       290                 295                 300
   Ser Gln Arg Met Val Glu Gly Tyr Gln Gly Leu Arg Cys Asp Asn Phe
   305                 310                 315                 320
   Thr Ser Ser Trp Arg Asp Gly Arg Leu Phe Asn Ala Ile Ile His Arg
                   325                 330                 335
   His Lys Pro Leu Leu Ile Asp Met Asn Lys Val Tyr Arg Gln Thr Asn
               340                 345                 350
   Leu Glu Asn Leu Asp Gln Ala Phe Ser Val Ala Glu Arg Asp Leu Gly
           355                 360                 365
   Val Thr Arg Leu Leu Asp Pro Glu Asp Val Asp Val Pro Gln Pro Asp
       370                 375                 380
   Glu Lys Ser Ile Ile Thr Tyr Val Ser Ser Leu Tyr Asp Ala Met Pro
   385                 390                 395                 400
   Arg Val Pro Asp Val Gln Asp Gly Val Arg Ala Asn Glu Leu Gln Leu
                   405                 410                 415
   Arg Trp Gln Glu Tyr Arg Glu Leu Val Leu Leu Leu Leu Gln Trp Met
               420                 425                 430
   Arg His His Thr Ala Ala Phe Glu Glu Arg Arg Phe Pro Ser Ser Phe
           435                 440                 445
   Glu Glu Ile Glu Ile Leu Trp Ser Gln Phe Leu Lys Phe Lys Glu Met
       450                 455                 460
   Glu Leu Pro Ala Lys Glu Ala Asp Lys Asn Arg Ser Lys Gly Ile Tyr
   465                 470                 475                 480
   Gln Ser Leu Glu Gly Ala Val Gln Ala Gly Gln Leu Lys Val Pro Pro
                   485                 490                 495
   Gly Tyr His Pro Leu Asp Val Glu Lys Glu Trp Gly Lys Leu His Val
               500                 505                 510
   Ala Ile Leu Glu Arg Glu Lys Gln Leu Arg Ser Glu Phe Glu Arg Leu
           515                 520                 525
   Glu Cys Leu Gln Arg Ile Val Thr Lys Leu Gln Met Glu Ala Gly Leu
       530                 535                 540
   Cys Glu Glu Gln Leu Asn Gln Ala Asp Ala Leu Leu Gln Ser Asp Val
   545                 550                 555                 560
   Arg Leu Leu Ala Ala Gly Lys Val Pro Gln Arg Ala Gly Glu Val Glu
                   565                 570                 575
   Arg Asp Leu Asp Lys Ala Asp Ser Met Ile Arg Leu Leu Phe Asn Asp
               580                 585                 590
   Val Gln Thr Leu Lys Asp Gly Arg His Pro Gln Gly Glu Gln Met Tyr
           595                 600                 605
   Arg Arg Val Tyr Arg Leu His Glu Arg Leu Val Ala Ile Arg Thr Glu
       610                 615                 620
   Tyr Asn Leu Arg Leu Lys Ala Gly Val Ala Ala Pro Ala Thr Gln Val
   625                 630                 635                 640
   Ala Gln Val Thr Leu Gln Ser Val Gln Arg Arg Pro Glu Leu Glu Asp
                   645                 650                 655
   Ser Thr Leu Arg Tyr Leu Gln Asp Leu Leu Ala Trp Val Glu Glu Asn
               660                 665                 670
   Gln His Arg Val Asp Gly Ala Glu Trp Gly Val Asp Leu Pro Ser Val
           675                 680                 685
   Glu Ala Gln Leu Gly Ser His Arg Gly Leu His Gln Ser Ile Glu Glu
       690                 695                 700
   Phe Arg Ala Lys Ile Glu Arg Ala Arg Ser Asp Glu Gly Gln Leu Ser
   705                 710                 715                 720
   Pro Ala Thr Arg Gly Ala Tyr Arg Asp Cys Leu Gly Arg Leu Asp Leu
                   725                 730                 735
   Gln Tyr Ala Lys Leu Leu Asn Ser Ser Lys Ala Arg Leu Arg Ser Leu
               740                 745                 750
   Glu Ser Leu His Ser Phe Val Ala Ala Ala Thr Lys Glu Leu Met Trp
           755                 760                 765
   Leu Asn Glu Lys Glu Glu Glu Glu Val Gly Phe Asp Trp Ser Asp Arg
       770                 775                 780
   Asn Thr Asn Met Thr Ala Lys Lys Glu Ser Tyr Ser Ala Leu Met Arg
   785                 790                 795                 800
   Glu Leu Glu Leu Lys Glu Lys Lys Ile Lys Glu Leu Gln Asn Ala Gly
                   805                 810                 815
   Asp Arg Leu Leu Arg Glu Asp His Pro Ala Arg Pro Thr Val Glu Ser
               820                 825                 830
   Phe Gln Ala Ala Leu Gln Thr Gln Trp Ser Trp Met Leu Gln Leu Cys
           835                 840                 845
   Cys Cys Ile Glu Ala His Leu Lys Glu Asn Ala Ala Tyr Phe Gln Phe
       850                 855                 860
   Phe Ser Asp Val Arg Glu Ala Glu Gly Gln Leu Gln Lys Leu Gln Glu
   865                 870                 875                 880
   Ala Leu Arg Arg Lys Tyr Ser Cys Asp Arg Ser Ala Thr Val Thr Arg
                   885                 890                 895
   Leu Glu Asp Leu Leu Gln Asp Ala Gln Asp Glu Lys Glu Gln Leu Asn
               900                 905                 910
   Glu Tyr Lys Gly His Leu Ser Gly Leu Ala Lys Arg Ala Lys Ala Val
           915                 920                 925
   Val Gln Leu Lys Pro Arg His Pro Ala His Pro Met Arg Gly Arg Leu
       930                 935                 940
   Pro Leu Leu Ala Val Cys Asp Tyr Lys Gln Val Glu Val Thr Val His
   945                 950                 955                 960
   Lys Gly Asp Glu Cys Gln Leu Val Gly Pro Ala Gln Pro Ser His Trp
                   965                 970                 975
   Lys Val Leu Ser Ser Ser Gly Ser Glu Ala Ala Val Pro Ser Val Cys
               980                 985                 990
   Phe Leu Val Pro Pro Pro Asn Gln  Glu Ala Gln Glu Ala  Val Thr Arg
           995                 1000                1005
   Leu Glu  Ala Gln His Gln Ala  Leu Val Thr Leu Trp  His Gln Leu
       1010                1015                1020
   His Val  Asp Met Lys Ser Leu  Leu Ala Trp Gln Ser  Leu Arg Arg
       1025                1030                1035
   Asp Val  Gln Leu Ile Arg Ser  Trp Ser Leu Ala Thr  Phe Arg Thr
       1040                1045                1050
   Leu Lys Pro Glu Glu Gln Arg Gln Ala Leu His Ser Leu Glu Leu
   1055 1060 1065
   His Tyr Gln Ala Phe Leu Arg Asp Ser Gln Asp Ala Gly Gly Phe
   1070 1075 1080
   Gly Pro Glu Asp Arg Leu Met Ala Glu Arg Glu Tyr Gly Ser Cys
   1085 1090 1095
   Ser His His Tyr Gln Gln Leu Leu Gln Ser Leu Glu Gln Gly Ala
   1100 1105 1110
   Gln Glu Glu Ser Arg Cys Gln Arg Cys Ile Ser Glu Leu Lys Asp
   1115 1120 1125
   Ile Arg Leu Gln Leu Glu Ala Cys Glu Thr Arg Thr Val His Arg
   1130 1135 1140
   Leu Arg Leu Pro Leu Asp Lys Glu Pro Ala Arg Glu Cys Ala Gln
   1145 1150 1155
   Arg Ile Ala Glu Gln Gln Lys Ala Gln Ala Glu Val Glu Gly Leu
   1160 1165 1170
   Gly Lys Gly Val Ala Arg Leu Ser Ala Glu Ala Glu Lys Val Leu
   1175 1180 1185
   Ala Leu Pro Glu Pro Ser Pro Ala Ala Pro Thr Leu Arg Ser Glu
   1190 1195 1200
   Leu Glu Leu Thr Leu Gly Lys Leu Glu Gln Val Arg Ser Leu Ser
   1205 1210 1215
   Ala Ile Tyr Leu Glu Lys Leu Lys Thr Ile Ser Leu Val Ile Arg
   1220 1225 1230
   Gly Thr Gln Gly Ala Glu Glu Val Leu Arg Ala His Glu Glu Gln
   1235 1240 1245
   Leu Lys Glu Ala Gln Ala Val Pro Ala Thr Leu Pro Glu Leu Glu
   1250 1255 1260
   Ala Thr Lys Ala Ser Leu Lys Lys Leu Arg Ala Gln Ala Glu Ala
   1265 1270 1275
   Gln Gln Pro Thr Phe Asp Ala Leu Arg Asp Glu Leu Arg Gly Ala
   1280 1285 1290
   Gln Glu Val Gly Glu Arg Leu Gln Gln Arg His Gly Glu Arg Asp
   1295 1300 1305
   Val Glu Val Glu Arg Trp Arg Glu Arg Val Ala Gln Leu Leu Glu
   1310 1315 1320
   Arg Trp Gln Ala Val Leu Ala Gln Thr Asp Val Arg Gln Arg Glu
   1325 1330 1335
   Leu Glu Gln Leu Gly Arg Gln Leu Arg Tyr Tyr Arg Glu Ser Ala
   1340 1345 1350
   Asp Pro Leu Gly Ala Trp Leu Gln Asp Ala Arg Arg Arg Gln Glu
   1355 1360 1365
   Gln Ile Gln Ala Met Pro Leu Ala Asp Ser Gln Ala Val Arg Glu
   1370 1375 1380
   Gln Leu Arg Gln Glu Gln Ala Leu Leu Glu Glu Ile Glu Arg His
   1385 1390 1395
   Gly Glu Lys Val Glu Glu Cys Gln Arg Phe Ala Lys Gln Tyr Ile
   1400 1405 1410
   Asn Ala Ile Lys Asp Tyr Glu Leu Gln Leu Val Thr Tyr Lys Ala
   1415 1420 1425
   Gln Leu Glu Pro Val Ala Ser Pro Ala Lys Lys Pro Lys Val Gln
   1430 1435 1440
   Ser Gly Ser Glu Ser Val Ile Gln Glu Tyr Val Asp Leu Arg Thr
   1445 1450 1455
   His Tyr Ser Glu Leu Thr Thr Leu Thr Ser Gln Tyr Ile Lys Phe
   1460 1465 1470
   Ile Ser Glu Thr Leu Arg Arg Met Glu Glu Glu Glu Arg Leu Ala
   1475 1480 1485
   Glu Gln Gln Arg Ala Glu Glu Arg Glu Arg Leu Ala Glu Val Glu
   1490 1495 1500
   Ala Ala Leu Glu Lys Gln Arg Gln Leu Ala Glu Ala His Ala Gln
   1505 1510 1515
   Ala Lys Ala Gln Ala Glu Arg Glu Ala Lys Glu Leu Gln Gln Arg
   1520 1525 1530
   Met Gln Glu Glu Val Val Arg Arg Glu Glu Ala Ala Val Asp Ala
   1535 1540 1545
   Gln Gln Gln Lys Arg Ser Ile Gln Glu Glu Leu Gln Gln Leu Arg
   1550 1555 1560
   Gln Ser Ser Glu Ala Glu Ile Gln Ala Lys Ala Arg Gln Ala Glu
   1565 1570 1575
   Ala Ala Glu Arg Ser Arg Leu Arg Ile Glu Glu Glu Ile Arg Val
   1580 1585 1590
   Val Arg Leu Gln Leu Glu Ala Thr Glu Arg Gln Arg Gly Gly Ala
   1595 1600 1605
   Glu Gly Glu Leu Gln Ala Leu Arg Ala Arg Ala Glu Glu Ala Glu
   1610 1615 1620
   Ala Gln Lys Arg Gln Ala Gln Glu Glu Ala Glu Arg Leu Arg Arg
   1625 1630 1635
   Gln Val Gln Asp Glu Ser Gln Arg Lys Arg Gln Ala Glu Val Glu
   1640 1645 1650
   Leu Ala Ser Arg Val Lys Ala Glu Ala Glu Ala Ala Arg Glu Lys
   1655 1660 1665
   Gln Arg Ala Leu Gln Ala Leu Glu Glu Leu Arg Leu Gln Ala Glu
   1670 1675 1680
   Glu Ala Glu Arg Arg Leu Arg Gln Ala Glu Val Glu Arg Ala Arg
   1685 1690 1695
   Gln Val Gln Val Ala Leu Glu Thr Ala Gln Arg Ser Ala Glu Ala
   1700 1705 1710
   Glu Leu Gln Ser Lys Arg Ala Ser Phe Ala Glu Lys Thr Ala Gln
   1715 1720 1725
   Leu Glu Arg Ser Leu Gln Glu Glu His Val Ala Val Ala Gln Leu
   1730 1735 1740
   Arg Glu Glu Ala Glu Arg Arg Ala Gln Gln Gln Ala Glu Ala Glu
   1745 1750 1755
   Arg Ala  Arg Glu Glu Ala Glu  Arg Glu Leu Glu Arg  Trp Gln Leu
       1760                 1765                 1770
   Lys Ala  Asn Glu Ala Leu Arg  Leu Arg Leu Gln Ala  Glu Glu Val
       1775                 1780                 1785
   Ala Gln  Gln Lys Ser Leu Ala  Gln Ala Glu Ala Glu  Lys Gln Lys
       1790                 1795                 1800
   Glu Glu  Ala Glu Arg Glu Ala  Arg Arg Arg Gly Lys  Ala Glu Glu
       1805                 1810                 1815
   Gln Ala  Val Arg Gln Arg Glu  Leu Ala Glu Gln Glu  Leu Glu Lys
       1820                 1825                 1830
   Gln Arg  Gln Leu Ala Glu Gly  Thr Ala Gln Gln Arg  Leu Ala Ala
       1835                 1840                 1845
   Glu Gln  Glu Leu Ile Arg Leu  Arg Ala Glu Thr Glu  Gln Gly Glu
       1850                 1855                 1860
   Gln Gln  Arg Gln Leu Leu Glu  Glu Glu Leu Ala Arg  Leu Gln Arg
       1865                 1870                 1875
   Glu Ala  Ala Ala Ala Thr Gln  Lys Arg Gln Glu Leu  Glu Ala Glu
       1880                 1885                 1890
   Leu Ala  Lys Val Arg Ala Glu  Met Glu Val Leu Leu  Ala Ser Lys
       1895                 1900                 1905
   Ala Arg  Ala Glu Glu Glu Ser  Arg Ser Thr Ser Glu  Lys Ser Lys
       1910                 1915                 1920
   Gln Arg  Leu Glu Ala Glu Ala  Gly Arg Phe Arg Glu  Leu Ala Glu
       1925                 1930                 1935
   Glu Ala  Ala Arg Leu Arg Ala  Leu Ala Glu Glu Ala  Lys Arg Gln
       1940                 1945                 1950
   Arg Gln  Leu Ala Glu Glu Asp  Ala Ala Arg Gln Arg  Ala Glu Ala
       1955                 1960                 1965
   Glu Arg  Val Leu Ala Glu Lys  Leu Ala Ala Ile Gly  Glu Ala Thr
       1970                 1975                 1980
   Arg Leu  Lys Thr Glu Ala Glu  Ile Ala Leu Lys Glu  Lys Glu Ala
       1985                 1990                 1995
   Glu Asn Glu Arg Leu Arg Arg Leu Ala Glu Asp Glu Ala Phe Gln
   2000 2005 2010
   Arg Arg Arg Leu Glu Glu Gln Ala Ala Gln His Lys Ala Asp Ile
   2015 2020 2025
   Glu Glu Arg Leu Ala Gln Leu Arg Lys Ala Ser Asp Ser Glu Leu
   2030 2035 2040
   Glu Arg Gln Lys Gly Leu Val Glu Asp Thr Leu Arg Gln Arg Arg
   2045 2050 2055
   Gln Val Glu Glu Glu Ile Leu Ala Leu Lys Ala Ser Phe Glu Lys
   2060 2065 2070
   Ala Ala Ala Gly Lys Ala Glu Leu Glu Leu Glu Leu Gly Arg Ile
   2075 2080 2085
   Arg Ser Asn Ala Glu Asp Thr Leu Arg Ser Lys Glu Gln Ala Glu
   2090 2095 2100
   Leu Glu Ala Ala Arg Gln Arg Gln Leu Ala Ala Glu Glu Glu Arg
   2105 2110 2115
   Arg Arg Arg Glu Ala Glu Glu Arg Val Gln Lys Ser Leu Ala Ala
   2120 2125 2130
   Glu Glu Glu Ala Ala Arg Gln Arg Lys Ala Ala Leu Glu Glu Val
   2135 2140 2145
   Glu Arg Leu Lys Ala Lys Val Glu Glu Ala Arg Arg Leu Arg Glu
   2150 2155 2160
   Arg Ala Glu Gln Glu Ser Ala Arg Gln Leu Gln Leu Ala Gln Glu
   2165 2170 2175
   Ala Ala Gln Lys Arg Leu Gln Ala Glu Glu Lys Ala His Ala Phe
   2180 2185 2190
   Ala Val Gln Gln Lys Glu Gln Glu Leu Gln Gln Thr Leu Gln Gln
   2195 2200 2205
   Glu Gln Ser Val Leu Asp Gln Leu Arg Gly Glu Ala Glu Ala Ala
   2210 2215 2220
   Arg Arg Ala Ala Glu Glu Ala Glu Glu Ala Arg Val Gln Ala Glu
   2225 2230 2235
   Arg Glu Ala Ala Gln Ser Arg Arg Gln Val Glu Glu Ala Glu Arg
   2240 2245 2250
   Leu Lys Gln Ser Ala Glu Glu Gln Ala Gln Ala Arg Ala Gln Ala
   2255 2260 2265
   Gln Ala Ala Ala Glu Lys Leu Arg Lys Glu Ala Glu Gln Glu Ala
   2270 2275 2280
   Ala Arg Arg Ala Gln Ala Glu Gln Ala Ala Leu Arg Gln Lys Gln
   2285 2290 2295
   Ala Ala Asp Ala Glu Met Glu Lys His Lys Lys Phe Ala Glu Gln
   2300 2305 2310
   Thr Leu Arg Gln Lys Ala Gln Val Glu Gln Glu Leu Thr Thr Leu
   2315 2320 2325
   Arg Leu Gln Leu Glu Glu Thr Asp His Gln Lys Asn Leu Leu Asp
   2330 2335 2340
   Glu Glu Leu Gln Arg Leu Lys Ala Glu Ala Thr Glu Ala Ala Arg
   2345 2350 2355
   Gln Arg Ser Gln Val Glu Glu Glu Leu Phe Ser Val Arg Val Gln
   2360 2365 2370
   Met Glu Glu Leu Ser Lys Leu Lys Ala Arg Ile Glu Ala Glu Asn
   2375 2380 2385
   Arg Ala Leu Ile Leu Arg Asp Lys Asp Asn Thr Gln Arg Phe Leu
   2390 2395 2400
   Gln Glu Glu Ala Glu Lys Met Lys Gln Val Ala Glu Glu Ala Ala
   2405 2410 2415
   Arg Leu Ser Val Ala Ala Gln Glu Ala Ala Arg Leu Arg Gln Leu
   2420 2425 2430
   Ala Glu Glu Asp Leu Ala Gln Gln Arg Ala Leu Ala Glu Lys Met
   2435 2440 2445
   Leu Lys Glu Lys Met Gln Ala Val Gln Glu Ala Thr Arg Leu Lys
   2450 2455 2460
   Ala Glu Ala Glu Leu Leu Gln Gln Gln Lys Glu Leu Ala Gln Glu
   2465 2470 2475
   Gln Ala Arg Arg Leu Gln Glu Asp Lys Glu Gln Met Ala Gln Gln
   2480 2485 2490
   Leu Ala Glu Glu Thr Gln Gly Phe Gln Arg Thr Leu Glu Ala Glu
   2495 2500 2505
   Arg Gln Arg Gln Leu Glu Met Ser Ala Glu Ala Glu Arg Leu Lys
   2510 2515 2520
   Leu Arg Val Ala Glu Met Ser Arg Ala Gln Ala Arg Ala Glu Glu
   2525 2530 2535
   Asp Ala Gln Arg Phe Arg Lys Gln Ala Glu Glu Ile Gly Glu Lys
   2540 2545 2550
   Leu His Arg Thr Glu Leu Ala Thr Gln Glu Lys Val Thr Leu Val
   2555 2560 2565
   Gln Thr Leu Glu Ile Gln Arg Gln Gln Ser Asp His Asp Ala Glu
   2570 2575 2580
   Arg Leu Arg Glu Ala Ile Ala Glu Leu Glu Arg Glu Lys Glu Lys
   2585 2590 2595
   Leu Gln Gln Glu Ala Lys Leu Leu Gln Leu Lys Ser Glu Glu Met
   2600 2605 2610
   Gln Thr Val Gln Gln Glu Gln Leu Leu Gln Glu Thr Gln Ala Leu
   2615 2620 2625
   Gln Gln Ser Phe Leu Ser Glu Lys Asp Ser Leu Leu Gln Arg Glu
   2630 2635 2640
   Arg Phe Ile Glu Gln Glu Lys Ala Lys Leu Glu Gln Leu Phe Gln
   2645 2650 2655
   Asp Glu Val Ala Lys Ala Gln Gln Leu Arg Glu Glu Gln Gln Arg
   2660 2665 2670
   Gln Gln Gln Gln Met Glu Gln Glu Arg Gln Arg Leu Val Ala Ser
   2675 2680 2685
   Met Glu Glu Ala Arg Arg Arg Gln His Glu Ala Glu Glu Gly Val
   2690 2695 2700
   Arg Arg Lys Gln Glu Glu Leu Gln Gln Leu Glu Gln Gln Arg Arg
   2705 2710 2715
   Gln Gln Glu Glu Leu Leu Ala Glu Glu Asn Gln Arg Leu Arg Glu
   2720 2725 2730
   Gln Leu Gln Leu Leu Glu Glu Gln His Arg Ala Ala Leu Ala His
   2735 2740 2745
   Ser Glu Glu Val Thr Ala Ser Gln Val Ala Ala Thr Lys Thr Leu
   2750 2755 2760
   Pro Asn Gly Arg Asp Ala Leu Asp Gly Pro Ala Ala Glu Ala Glu
   2765 2770 2775
   Pro Glu His Ser Phe Asp Gly Leu Arg Arg Lys Val Ser Ala Gln
   2780 2785 2790
   Arg Leu Gln Glu Ala Gly Ile Leu Ser Ala Glu Glu Leu Gln Arg
   2795 2800 2805
   Leu Ala Gln Gly His Thr Thr Val Asp Glu Leu Ala Arg Arg Glu
   2810 2815 2820
   Asp Val Arg His Tyr Leu Gln Gly Arg Ser Ser Ile Ala Gly Leu
   2825 2830 2835
   Leu Leu Lys Ala Thr Asn Glu Lys Leu Ser Val Tyr Ala Ala Leu
   2840 2845 2850
   Gln Arg Gln Leu Leu Ser Pro Gly Thr Ala Leu Ile Leu Leu Glu
   2855 2860 2865
   Ala Gln Ala Ala Ser Gly Phe Leu Leu Asp Pro Val Arg Asn Arg
   2870 2875 2880
   Arg Leu Thr Val Asn Glu Ala Val Lys Glu Gly Val Val Gly Pro
   2885 2890 2895
   Glu Leu His His Lys Leu Leu Ser Ala Glu Arg Ala Val Thr Gly
   2900 2905 2910
   Tyr Lys Asp Pro Tyr Thr Gly Gln Gln Ile Ser Leu Phe Gln Ala
   2915 2920 2925
   Met Gln Lys Gly Leu Ile Val Arg Glu His Gly Ile Arg Leu Leu
   2930 2935 2940
   Glu Ala Gln Ile Ala Thr Gly Gly Val Ile Asp Pro Val His Ser
   2945 2950 2955
   His Arg Val Pro Val Asp Val Ala Tyr Arg Arg Gly Tyr Phe Asp
   2960 2965 2970
   Glu Glu Met Asn Arg Val Leu Ala Asp Pro Ser Asp Asp Thr Lys
   2975 2980 2985
   Gly Phe Phe Asp Pro Asn Thr His Glu Asn Leu Thr Tyr Leu Gln
   2990 2995 3000
   Leu Leu Glu Arg Cys Val Glu Asp Pro Glu Thr Gly Leu Cys Leu
   3005 3010 3015
   Leu Pro Leu Thr Asp Lys Ala Ala Lys Gly Gly Glu Leu Val Tyr
   3020 3025 3030
   Thr Asp Ser Glu Ala Arg Asp Val Phe Glu Lys Ala Thr Val Ser
   3035 3040 3045
   Ala Pro Phe Gly Lys Phe Gln Gly Lys Thr Val Thr Ile Trp Glu
   3050 3055 3060
   Ile Ile Asn Ser Glu Tyr Phe Thr Ala Glu Gln Arg Arg Asp Leu
   3065 3070 3075
   Leu Arg Gln Phe Arg Thr Gly Arg Ile Thr Val Glu Lys Ile Ile
   3080 3085 3090
   Lys Ile Ile Ile Thr Val Val Glu Glu Gln Glu Gln Lys Gly Arg
   3095 3100 3105
   Leu Cys Phe Glu Gly Leu Arg Ser Leu Val Pro Ala Ala Glu Leu
   3110 3115 3120
   Leu Glu Ser Arg Val Ile Asp Arg Glu Leu Tyr Gln Gln Leu Gln
   3125 3130 3135
   Arg Gly Glu Arg Ser Val Arg Asp Val Ala Glu Val Asp Thr Val
   3140 3145 3150
   Arg Arg Ala Leu Arg Gly Ala Asn Val Ile Ala Gly Val Trp Leu
   3155 3160 3165
   Glu Glu Ala Gly Gln Lys Leu Ser Ile Tyr Asn Ala Leu Lys Lys
   3170 3175 3180
   Asp Leu Leu Pro Ser Asp Met Ala Val Ala Leu Leu Glu Ala Gln
   3185 3190 3195
   Ala Gly Thr Gly His Ile Ile Asp Pro Ala Thr Ser Ala Arg Leu
   3200 3205 3210
   Thr Val Asp Glu Ala Val Arg Ala Gly Leu Val Gly Pro Glu Phe
   3215 3220 3225
   His Glu Lys Leu Leu Ser Ala Glu Lys Ala Val Thr Gly Tyr Arg
   3230 3235 3240
   Asp Pro Tyr Thr Gly Gln Ser Val Ser Leu Phe Gln Ala Leu Lys
   3245 3250 3255
   Lys Gly Leu Ile Pro Arg Glu Gln Gly Leu Arg Leu Leu Asp Ala
   3260 3265 3270
   Gln Leu Ser Thr Gly Gly Ile Val Asp Pro Ser Lys Ser His Arg
   3275 3280 3285
   Val Pro Leu Asp Val Ala Cys Ala Arg Gly Cys Leu Asp Glu Glu
   3290 3295 3300
   Thr Ser Arg Ala Leu Ser Ala Pro Arg Ala Asp Ala Lys Ala Tyr
   3305 3310 3315
   Ser Asp Pro Ser Thr Gly Glu Pro Ala Thr Tyr Gly Glu Leu Gln
   3320 3325 3330
   Gln Arg Cys Arg Pro Asp Gln Leu Thr Gly Leu Ser Leu Leu Pro
   3335 3340 3345
   Leu Ser Glu Lys Ala Ala Arg Ala Arg Gln Glu Glu Leu Tyr Ser
   3350 3355 3360
   Glu Leu Gln Ala Arg Glu Thr Phe Glu Lys Thr Pro Val Glu Val
   3365 3370 3375
   Pro Val Gly Gly Phe Lys Gly Arg Thr Val Thr Val Trp Glu Leu
   3380 3385 3390
   Ile Ser Ser Glu Tyr Phe Thr Ala Glu Gln Arg Gln Glu Leu Leu
   3395 3400 3405
   Arg Gln Phe Arg Thr Gly Lys Val Thr Val Glu Lys Val Ile Lys
   3410 3415 3420
   Ile Leu Ile Thr Ile Val Glu Glu Val Glu Thr Leu Arg Gln Glu
   3425 3430 3435
   Arg Leu Ser Phe Ser Gly Leu Arg Ala Pro Val Pro Ala Ser Glu
   3440 3445 3450
   Leu Leu Ala Ser Gly Val Leu Ser Arg Ala Gln Phe Glu Gln Leu
   3455 3460 3465
   Lys Asp Gly Lys Thr Thr Val Lys Asp Leu Ser Glu Leu Gly Ser
   3470 3475 3480
   Val Arg Thr Leu Leu Gln Gly Ser Gly Cys Leu Ala Gly Ile Tyr
   3485 3490 3495
   Leu Glu Asp Thr Lys Glu Lys Val Ser Ile Tyr Glu Ala Met Arg
   3500 3505 3510
   Arg Gly Leu Leu Arg Ala Thr Thr Ala Ala Leu Leu Leu Glu Ala
   3515 3520 3525
   Gln Ala Ala Thr Gly Phe Leu Val Asp Pro Val Arg Asn Gln Arg
   3530 3535 3540
   Leu Tyr Val His Glu Ala Val Lys Ala Gly Val Val Gly Pro Glu
   3545 3550 3555
   Leu His Glu Gln Leu Leu Ser Ala Glu Lys Ala Val Thr Gly Tyr
   3560 3565 3570
   Arg Asp Pro Tyr Ser Gly Ser Thr Ile Ser Leu Phe Gln Ala Met
   3575 3580 3585
   Gln Lys Gly Leu Val Leu Arg Gln His Gly Ile Arg Leu Leu Glu
   3590 3595 3600
   Ala Gln Ile Ala Thr Gly Gly Ile Ile Asp Pro Val His Ser His
   3605 3610 3615
   Arg Val Pro Val Asp Val Ala Tyr Gln Arg Gly Tyr Phe Ser Glu
   3620 3625 3630
   Glu Met Asn Arg Val Leu Ala Asp Pro Ser Asp Asp Thr Lys Gly
   3635 3640 3645
   Phe Phe  Asp Pro Asn Thr His  Glu Asn Leu Thr Tyr  Arg Gln Leu
       3650                 3655                 3660
   Leu Glu  Arg Cys Val Glu Asp  Pro Glu Thr Gly Leu  Arg Leu Leu
       3665                 3670                 3675
   Pro Leu  Lys Gly Ala Glu Lys  Ala Glu Val Val Glu  Thr Thr Gln
       3680                 3685                 3690
   Val Tyr  Thr Glu Glu Glu Thr  Arg Arg Ala Phe Glu  Glu Thr Gln
       3695                 3700                 3705
   Ile Asp  Ile Pro Gly Gly Gly  Ser His Gly Gly Ser  Thr Met Ser
       3710                 3715                 3720
   Leu Trp  Glu Val Met Gln Ser  Asp Leu Ile Pro Glu  Glu Gln Arg
       3725                 3730                 3735
   Ala Gln  Leu Met Ala Asp Phe  Gln Ala Gly Arg Val  Thr Lys Glu
       3740                 3745                 3750
   Arg Met  Ile Ile Ile Ile Ile  Glu Ile Ile Glu Lys  Thr Glu Ile
       3755                 3760                 3765
   Ile Arg  Gln Gln Gly Leu Ala  Ser Tyr Asp Tyr Val  Arg Arg Arg
       3770                 3775                 3780
   Leu Thr  Ala Glu Asp Leu Phe  Glu Ala Arg Ile Ile  Ser Leu Glu
       3785                 3790                 3795
   Thr Tyr  Asn Leu Leu Arg Glu  Gly Thr Arg Ser Leu  Arg Glu Ala
       3800                 3805                 3810
   Leu Glu  Ala Glu Ser Ala Trp  Cys Tyr Leu Tyr Gly  Thr Gly Ser
       3815                 3820                 3825
   Val Ala  Gly Val Tyr Leu Pro  Gly Ser Arg Gln Thr  Leu Ser Ile
       3830                 3835                 3840
   Tyr Gln  Ala Leu Lys Lys Gly  Leu Leu Ser Ala Glu  Val Ala Arg
       3845                 3850                 3855
   Leu Leu  Leu Glu Ala Gln Ala  Ala Thr Gly Phe Leu  Leu Asp Pro
       3860                 3865                 3870
   Val Lys  Gly Glu Arg Leu Thr  Val Asp Glu Ala Val  Arg Lys Gly
       3875                 3880                 3885
   Leu Val Gly Pro Glu Leu His Asp Arg Leu Leu Ser Ala Glu Arg
   3890 3895 3900
   Ala Val Thr Gly Tyr Arg Asp Pro Tyr Thr Glu Gln Thr Ile Ser
   3905 3910 3915
   Leu Phe Gln Ala Met Lys Lys Glu Leu Ile Pro Thr Glu Glu Ala
   3920 3925 3930
   Leu Arg Leu Leu Asp Ala Gln Leu Ala Thr Gly Gly Ile Val Asp
   3935 3940 3945
   Pro Arg Leu Gly Phe His Leu Pro Leu Glu Val Ala Tyr Gln Arg
   3950 3955 3960
   Gly Tyr Leu Asn Lys Asp Thr His Asp Gln Leu Ser Glu Pro Ser
   3965 3970 3975
   Glu Val Arg Ser Tyr Val Asp Pro Ser Thr Asp Glu Arg Leu Ser
   3980 3985 3990
   Tyr Thr Gln Leu Leu Arg Arg Cys Arg Arg Asp Asp Gly Thr Gly
   3995 4000 4005
   Gln Leu Leu Leu Pro Leu Ser Asp Ala Arg Lys Leu Thr Phe Arg
   4010 4015 4020
   Gly Leu Arg Lys Gln Ile Thr Met Glu Glu Leu Val Arg Ser Gln
   4025 4030 4035
   Val Met Asp Glu Ala Thr Ala Leu Gln Leu Arg Glu Gly Leu Thr
   4040 4045 4050
   Ser Ile Glu Glu Val Thr Lys Asn Leu Gln Lys Phe Leu Glu Gly
   4055 4060 4065
   Thr Ser Cys Ile Ala Gly Val Phe Val Asp Ala Thr Lys Glu Arg
   4070 4075 4080
   Leu Ser Val Tyr Gln Ala Met Lys Lys Gly Ile Ile Arg Pro Gly
   4085 4090 4095
   Thr Ala Phe Glu Leu Leu Glu Ala Gln Ala Ala Thr Gly Tyr Val
   4100 4105 4110
   Ile Asp Pro Ile Lys Gly Leu Lys Leu Thr Val Glu Glu Ala Val
   4115 4120 4125
   Arg Met Gly Ile Val Gly Pro Glu Phe Lys Asp Lys Leu Leu Ser
   4130 4135 4140
   Ala Glu Arg Ala Val Thr Gly Tyr Lys Asp Pro Tyr Ser Gly Lys
   4145 4150 4155
   Leu Ile Ser Leu Phe Gln Ala Met Lys Lys Gly Leu Ile Leu Lys
   4160 4165 4170
   Asp His Gly Ile Arg Leu Leu Glu Ala Gln Ile Ala Thr Gly Gly
   4175 4180 4185
   Ile Ile Asp Pro Glu Glu Ser His Arg Leu Pro Val Glu Val Ala
   4190 4195 4200
   Tyr Lys Arg Gly Leu Phe Asp Glu Glu Met Asn Glu Ile Leu Thr
   4205 4210 4215
   Asp Pro Ser Asp Asp Thr Lys Gly Phe Phe Asp Pro Asn Thr Glu
   4220 4225 4230
   Glu Asn Leu Thr Tyr Leu Gln Leu Met Glu Arg Cys Ile Thr Asp
   4235 4240 4245
   Pro Gln Thr Gly Leu Cys Leu Leu Pro Leu Lys Glu Lys Lys Arg
   4250 4255 4260
   Glu Arg Lys Thr Ser Ser Lys Ser Ser Val Arg Lys Arg Arg Val
   4265 4270 4275
   Val Ile Val Asp Pro Glu Thr Gly Lys Glu Met Ser Val Tyr Glu
   4280 4285 4290
   Ala Tyr Arg Lys Gly Leu Ile Asp His Gln Thr Tyr Leu Glu Leu
   4295 4300 4305
   Ser Glu Gln Glu Cys Glu Trp Glu Glu Ile Thr Ile Ser Ser Ser
   4310 4315 4320
   Asp Gly Val Val Lys Ser Met Ile Ile Asp Arg Arg Ser Gly Arg
   4325 4330 4335
   Gln Tyr Asp Ile Asp Asp Ala Ile Ala Lys Asn Leu Ile Asp Arg
   4340 4345 4350
   Ser Ala Leu Asp Gln Tyr Arg Ala Gly Thr Leu Ser Ile Thr Glu
   4355 4360 4365
   Phe Ala Asp Met Leu Ser Gly Asn Ala Gly Gly Phe Arg Ser Arg
   4370 4375 4380
   Ser Ser Ser Val Gly Ser Ser Ser Ser Tyr Pro Ile Ser Pro Ala
   4385 4390 4395
   Val Ser Arg Thr Gln Leu Ala Ser Trp Ser Asp Pro Thr Glu Glu
   4400 4405 4410
   Thr Gly Pro Val Ala Gly Ile Leu Asp Thr Glu Thr Leu Glu Lys
   4415 4420 4425
   Val Ser Ile Thr Glu Ala Met His Arg Asn Leu Val Asp Asn Ile
   4430 4435 4440
   Thr Gly Gln Arg Leu Leu Glu Ala Gln Ala Cys Thr Gly Gly Ile
   4445 4450 4455
   Ile Asp Pro Ser Thr Gly Glu Arg Phe Pro Val Thr Asp Ala Val
   4460 4465 4470
   Asn Lys Gly Leu Val Asp Lys Ile Met Val Asp Arg Ile Asn Leu
   4475 4480 4485
   Ala Gln Lys Ala Phe Cys Gly Phe Glu Asp Pro Arg Thr Lys Thr
   4490 4495 4500
   Lys Met Ser Ala Ala Gln Ala Leu Lys Lys Gly Trp Leu Tyr Tyr
   4505 4510 4515
   Glu Ala Gly Gln Arg Phe Leu Glu Val Gln Tyr Leu Thr Gly Gly
   4520 4525 4530
   Leu Ile Glu Pro Asp Thr Pro Gly Arg Val Pro Leu Asp Glu Ala
   4535 4540 4545
   Leu Gln Arg Gly Thr Val Asp Ala Arg Thr Ala Gln Lys Leu Arg
   4550 4555 4560
   Asp Val Gly Ala Tyr Ser Lys Tyr Leu Thr Cys Pro Lys Thr Lys
   4565 4570 4575
   Leu Lys Ile Ser Tyr Lys Asp Ala Leu Asp Arg Ser Met Val Glu
   4580 4585 4590
   Glu Gly  Thr Gly Leu Arg Leu  Leu Glu Ala Ala Ala  Gln Ser Thr
       4595                 4600                 4605
   Lys Gly  Tyr Tyr Ser Pro Tyr  Ser Val Ser Gly Ser  Gly Ser Thr
       4610                 4615                 4620
   Ala Gly  Ser Arg Thr Gly Ser  Arg Thr Gly Ser Arg  Ala Gly Ser
       4625                 4630                 4635
   Arg Arg  Gly Ser Phe Asp Ala  Thr Gly Ser Gly Phe  Ser Met Thr
       4640                 4645                 4650
   Phe Ser  Ser Ser Ser Tyr Ser  Ser Ser Gly Tyr Gly  Arg Arg Tyr
       4655                 4660                 4665
   Ala Ser  Gly Ser Ser Ala Ser  Leu Gly Gly Pro Glu  Ser Ala Val
       4670                 4675                 4680
   Ala
<210> 6
   <211> 654
   <212> PRT
   <213> Homo sapiens
<400> 6
   Met Lys Leu Ser Leu Val Ala Ala Met Leu Leu Leu Leu Ser Ala Ala
   1               5                   10                  15
   Arg Ala Glu Glu Glu Asp Lys Lys Glu Asp Val Gly Thr Val Val Gly
               20                  25                  30
   Ile Asp Leu Gly Thr Thr Tyr Ser Cys Val Gly Val Phe Lys Asn Gly
           35                  40                  45
   Arg Val Glu Ile Ile Ala Asn Asp Gln Gly Asn Arg Ile Thr Pro Ser
       50                  55                  60
   Tyr Val Ala Phe Thr Pro Glu Gly Glu Arg Leu Ile Gly Asp Ala Ala
   65                  70                  75                  80
   Lys Asn Gln Leu Thr Ser Asn Pro Glu Asn Thr Val Phe Asp Ala Lys
                   85                  90                  95
   Arg Leu Ile Gly Arg Thr Trp Asn Asp Pro Ser Val Gln Gln Asp Ile
               100                 105                 110
   Lys Phe Leu Pro Phe Lys Val Val Glu Lys Lys Thr Lys Pro Tyr Ile
           115                 120                 125
   Gln Val Asp Ile Gly Gly Gly Gln Thr Lys Thr Phe Ala Pro Glu Glu
       130                 135                 140
   Ile Ser Ala Met Val Leu Thr Lys Met Lys Glu Thr Ala Glu Ala Tyr
   145                 150                 155                 160
   Leu Gly Lys Lys Val Thr His Ala Val Val Thr Val Pro Ala Tyr Phe
                   165                 170                 175
   Asn Asp Ala Gln Arg Gln Ala Thr Lys Asp Ala Gly Thr Ile Ala Gly
               180                 185                 190
   Leu Asn Val Met Arg Ile Ile Asn Glu Pro Thr Ala Ala Ala Ile Ala
           195                 200                 205
   Tyr Gly Leu Asp Lys Arg Glu Gly Glu Lys Asn Ile Leu Val Phe Asp
       210                 215                 220
   Leu Gly Gly Gly Thr Phe Asp Val Ser Leu Leu Thr Ile Asp Asn Gly
   225                 230                 235                 240
   Val Phe Glu Val Val Ala Thr Asn Gly Asp Thr His Leu Gly Gly Glu
                   245                 250                 255
   Asp Phe Asp Gln Arg Val Met Glu His Phe Ile Lys Leu Tyr Lys Lys
               260                 265                 270
   Lys Thr Gly Lys Asp Val Arg Lys Asp Asn Arg Ala Val Gln Lys Leu
           275                 280                 285
   Arg Arg Glu Val Glu Lys Ala Lys Arg Ala Leu Ser Ser Gln His Gln
       290                 295                 300
   Ala Arg Ile Glu Ile Glu Ser Phe Tyr Glu Gly Glu Asp Phe Ser Glu
   305                 310                 315                 320
   Thr Leu Thr Arg Ala Lys Phe Glu Glu Leu Asn Met Asp Leu Phe Arg
                   325                 330                 335
   Ser Thr Met Lys Pro Val Gln Lys Val Leu Glu Asp Ser Asp Leu Lys
               340                 345                 350
   Lys Ser Asp Ile Asp Glu Ile Val Leu Val Gly Gly Ser Thr Arg Ile
           355                 360                 365
   Pro Lys Ile Gln Gln Leu Val Lys Glu Phe Phe Asn Gly Lys Glu Pro
       370                 375                 380
   Ser Arg Gly Ile Asn Pro Asp Glu Ala Val Ala Tyr Gly Ala Ala Val
   385                 390                 395                 400
   Gln Ala Gly Val Leu Ser Gly Asp Gln Asp Thr Gly Asp Leu Val Leu
                   405                 410                 415
   Leu Asp Val Cys Pro Leu Thr Leu Gly Ile Glu Thr Val Gly Gly Val
               420                 425                 430
   Met Thr Lys Leu Ile Pro Arg Asn Thr Val Val Pro Thr Lys Lys Ser
           435                 440                 445
   Gln Ile Phe Ser Thr Ala Ser Asp Asn Gln Pro Thr Val Thr Ile Lys
       450                 455                 460
   Val Tyr Glu Gly Glu Arg Pro Leu Thr Lys Asp Asn His Leu Leu Gly
   465                 470                 475                 480
   Thr Phe Asp Leu Thr Gly Ile Pro Pro Ala Pro Arg Gly Val Pro Gln
                   485                 490                 495
   Ile Glu Val Thr Phe Glu Ile Asp Val Asn Gly Ile Leu Arg Val Thr
               500                 505                 510
   Ala Glu Asp Lys Gly Thr Gly Asn Lys Asn Lys Ile Thr Ile Thr Asn
           515                 520                 525
   Asp Gln Asn Arg Leu Thr Pro Glu Glu Ile Glu Arg Met Val Asn Asp
       530                 535                 540
   Ala Glu Lys Phe Ala Glu Glu Asp Lys Lys Leu Lys Glu Arg Ile Asp
   545                 550                 555                 560
   Thr Arg Asn Glu Leu Glu Ser Tyr Ala Tyr Ser Leu Lys Asn Gln Ile
                   565                 570                 575
   Gly Asp Lys Glu Lys Leu Gly Gly Lys Leu Ser Ser Glu Asp Lys Glu
               580                 585                 590
   Thr Met Glu Lys Ala Val Glu Glu Lys Ile Glu Trp Leu Glu Ser His
           595                 600                 605
   Gln Asp Ala Asp Ile Glu Asp Phe Lys Ala Lys Lys Lys Glu Leu Glu
   610 615 620
   Glu Ile Val Gln Pro Ile Ile Ser Lys Leu Tyr Gly Ser Ala Gly Pro
   625 630 635 640
   Pro Pro Thr Gly Glu Glu Asp Thr Ala Glu Lys Asp Glu Leu
   645 650
<210> 7
   <211> 710
   <212> PRT
   <213> Homo sapiens
<400> 7
   Met Val Lys Leu Ala Lys Ala Gly Lys Asn Gln Gly Asp Pro Lys Lys
   1               5                   10                  15
   Met Ala Pro Pro Pro Lys Glu Val Glu Glu Asp Ser Glu Asp Glu Glu
               20                  25                  30
   Met Ser Glu Asp Glu Glu Asp Asp Ser Ser Gly Glu Glu Val Val Ile
           35                  40                  45
   Pro Gln Lys Lys Gly Lys Lys Ala Ala Ala Thr Ser Ala Lys Lys Val
       50                  55                  60
   Val Val Ser Pro Thr Lys Lys Val Ala Val Ala Thr Pro Ala Lys Lys
   65                  70                  75                  80
   Ala Ala Val Thr Pro Gly Lys Lys Ala Ala Ala Thr Pro Ala Lys Lys
                   85                  90                  95
   Thr Val Thr Pro Ala Lys Ala Val Thr Thr Pro Gly Lys Lys Gly Ala
               100                 105                 110
   Thr Pro Gly Lys Ala Leu Val Ala Thr Pro Gly Lys Lys Gly Ala Ala
           115                 120                 125
   Ile Pro Ala Lys Gly Ala Lys Asn Gly Lys Asn Ala Lys Lys Glu Asp
       130                 135                 140
   Ser Asp Glu Glu Glu Asp Asp Asp Ser Glu Glu Asp Glu Glu Asp Asp
   145                 150                 155                 160
   Glu Asp Glu Asp Glu Asp Glu Asp Glu Ile Glu Pro Ala Ala Met Lys
                   165                 170                 175
   Ala Ala Ala Ala Ala Pro Ala Ser Glu Asp Glu Asp Asp Glu Asp Asp
               180                 185                 190
   Glu Asp Asp Glu Asp Asp Asp Asp Asp Glu Glu Asp Asp Ser Glu Glu
           195                 200                 205
   Glu Ala Met Glu Thr Thr Pro Ala Lys Gly Lys Lys Ala Ala Lys Val
       210                 215                 220
   Val Pro Val Lys Ala Lys Asn Val Ala Glu Asp Glu Asp Glu Glu Glu
   225                 230                 235                 240
   Asp Asp Glu Asp Glu Asp Asp Asp Asp Asp Glu Asp Asp Glu Asp Asp
                   245                 250                 255
   Asp Asp Glu Asp Asp Glu Glu Glu Glu Glu Glu Glu Glu Glu Glu Pro
               260                 265                 270
   Val Lys Glu Ala Pro Gly Lys Arg Lys Lys Glu Met Ala Lys Gln Lys
           275                 280                 285
   Ala Ala Pro Glu Ala Lys Lys Gln Lys Val Glu Gly Thr Glu Pro Thr
       290                 295                 300
   Thr Ala Phe Asn Leu Phe Val Gly Asn Leu Asn Phe Asn Lys Ser Ala
   305                 310                 315                 320
   Pro Glu Leu Lys Thr Gly Ile Ser Asp Val Phe Ala Lys Asn Asp Leu
                   325                 330                 335
   Ala Val Val Asp Val Arg Ile Gly Met Thr Arg Lys Phe Gly Tyr Val
               340                 345                 350
   Asp Phe Glu Ser Ala Glu Asp Leu Glu Lys Ala Leu Glu Leu Thr Gly
           355                 360                 365
   Leu Lys Val Phe Gly Asn Glu Ile Lys Leu Glu Lys Pro Lys Gly Lys
       370                 375                 380
   Asp Ser Lys Lys Glu Arg Asp Ala Arg Thr Leu Leu Ala Lys Asn Leu
   385                 390                 395                 400
   Pro Tyr Lys Val Thr Gln Asp Glu Leu Lys Glu Val Phe Glu Asp Ala
                   405                 410                 415
   Ala Glu Ile Arg Leu Val Ser Lys Asp Gly Lys Ser Lys Gly Ile Ala
               420                 425                 430
   Tyr Ile Glu Phe Lys Thr Glu Ala Asp Ala Glu Lys Thr Phe Glu Glu
   435 440 445
   Lys Gln Gly Thr Glu Ile Asp Gly Arg Ser Ile Ser Leu Tyr Tyr Thr
   450 455 460
   Gly Glu Lys Gly Gln Asn Gln Asp Tyr Arg Gly Gly Lys Asn Ser Thr
   465 470 475 480
   Trp Ser Gly Glu Ser Lys Thr Leu Val Leu Ser Asn Leu Ser Tyr Ser
   485 490 495
   Ala Thr Glu Glu Thr Leu Gln Glu Val Phe Glu Lys Ala Thr Phe Ile
   500 505 510
   Lys Val Pro Gln Asn Gln Asn Gly Lys Ser Lys Gly Tyr Ala Phe Ile
   515 520 525
   Glu Phe Ala Ser Phe Glu Asp Ala Lys Glu Ala Leu Asn Ser Cys Asn
   530 535 540
   Lys Arg Glu Ile Glu Gly Arg Ala Ile Arg Leu Glu Leu Gln Gly Pro
   545 550 555 560
   Arg Gly Ser Pro Asn Ala Arg Ser Gln Pro Ser Lys Thr Leu Phe Val
   565 570 575
   Lys Gly Leu Ser Glu Asp Thr Thr Glu Glu Thr Leu Lys Glu Ser Phe
   580 585 590
   Asp Gly Ser Val Arg Ala Arg Ile Val Thr Asp Arg Glu Thr Gly Ser
   595 600 605
   Ser Lys Gly Phe Gly Phe Val Asp Phe Asn Ser Glu Glu Asp Ala Lys
   610 615 620
   Ala Ala Lys Glu Ala Met Glu Asp Gly Glu Ile Asp Gly Asn Lys Val
   625 630 635 640
   Thr Leu Asp Trp Ala Lys Pro Lys Gly Glu Gly Gly Phe Gly Gly Arg
   645 650 655
   Gly Gly Gly Arg Gly Gly Phe Gly Gly Arg Gly Gly Gly Arg Gly Gly
   660 665 670
   Arg Gly Gly Phe Gly Gly Arg Gly Arg Gly Gly Phe Gly Gly Arg Gly
   675 680 685
   Gly Phe Arg Gly Gly Arg Gly Gly Gly Gly Asp His Lys Pro Gln Gly
   690 695 700
   Lys Lys Thr Lys Phe Glu
   705 710
<210> 8
   <211> 284
   <212> PRT
   <213> Homo sapiens
<400> 8
<210> 9
   <211> 2201
   <212> PRT
   <213> Homo sapiens
<400> 9
<210> 10
   <211> 291
   <212> PRT
   <213> Homo sapiens
<400> 10
   Met Gln Arg Ala Arg Pro Thr Leu Trp Ala Ala Ala Leu Thr Leu Leu
   1 5 10 15
   Val Leu Leu Arg Gly Pro Pro Val Ala Arg Ala Gly Ala Ser Ser Ala
   20 25 30
   Gly Leu Gly Pro Val Val Arg Cys Glu Pro Cys Asp Ala Arg Ala Leu
   35 40 45
   Ala Gln Cys Ala Pro Pro Pro Ala Val Cys Ala Glu Leu Val Arg Glu
   50 55 60
   Pro Gly Cys Gly Cys Cys Leu Thr Cys Ala Leu Ser Glu Gly Gln Pro
   65 70 75 80
   Cys Gly Ile Tyr Thr Glu Arg Cys Gly Ser Gly Leu Arg Cys Gln Pro
                   85                  90                  95
   Ser Pro Asp Glu Ala Arg Pro Leu Gln Ala Leu Leu Asp Gly Arg Gly
               100                 105                 110
   Leu Cys Val Asn Ala Ser Ala Val Ser Arg Leu Arg Ala Tyr Leu Leu
           115                 120                 125
   Pro Ala Pro Pro Ala Pro Gly Asn Ala Ser Glu Ser Glu Glu Asp Arg
       130                 135                 140
   Ser Ala Gly Ser Val Glu Ser Pro Ser Val Ser Ser Thr His Arg Val
   145                 150                 155                 160
   Ser Asp Pro Lys Phe His Pro Leu His Ser Lys Ile Ile Ile Ile Lys
                   165                 170                 175
   Lys Gly His Ala Lys Asp Ser Gln Arg Tyr Lys Val Asp Tyr Glu Ser
               180                 185                 190
   Gln Ser Thr Asp Thr Gln Asn Phe Ser Ser Glu Ser Lys Arg Glu Thr
           195                 200                 205
   Glu Tyr Gly Pro Cys Arg Arg Glu Met Glu Asp Thr Leu Asn His Leu
       210                 215                 220
   Lys Phe Leu Asn Val Leu Ser Pro Arg Gly Val His Ile Pro Asn Cys
   225                 230                 235                 240
   Asp Lys Lys Gly Phe Tyr Lys Lys Lys Gln Cys Arg Pro Ser Lys Gly
                   245                 250                 255
   Arg Lys Arg Gly Phe Cys Trp Cys Val Asp Lys Tyr Gly Gln Pro Leu
               260                 265                 270
   Pro Gly Tyr Thr Thr Lys Gly Lys Glu Asp Val His Cys Tyr Ser Met
           275                 280                 285
   Gln Ser Lys
       290
<210> 11
   <211> 1838
   <212> PRT
   <213> Homo sapiens
<400> 11
<210> 12
   <211> 323
   <212> PRT
   <213> Homo sapiens
<400> 12
   Met Gln Ala Ala Trp Leu Leu Gly Ala Leu Val Val Pro Gln Leu Leu
   1 5 10 15
   Gly Phe Gly His Gly Ala Arg Gly Ala Glu Arg Glu Trp Glu Gly Gly
   20 25 30
   Trp Gly Gly Ala Gln Glu Glu Glu Arg Glu Arg Glu Ala Leu Met Leu
   35 40 45
   Lys His Leu Gln Glu Ala Leu Gly Leu Pro Ala Gly Arg Gly Asp Glu
   50 55 60
   Asn Pro Ala Gly Thr Val Glu Gly Lys Glu Asp Trp Glu Met Glu Glu
   65 70 75 80
   Asp Gln Gly Glu Glu Glu Glu Glu Glu Ala Thr Pro Thr Pro Ser Ser
   85 90 95
   Gly Pro Ser Pro Ser Pro Thr Pro Glu Asp Ile Val Thr Tyr Ile Leu
   100 105 110
   Gly Arg Leu Ala Gly Leu Asp Ala Gly Leu His Gln Leu His Val Arg
   115 120 125
   Leu His Ala Leu Asp Thr Arg Val Val Glu Leu Thr Gln Gly Leu Arg
   130 135 140
   Gln Leu Arg Asn Ala Ala Gly Asp Thr Arg Asp Ala Val Gln Ala Leu
   145 150 155 160
   Gln Glu Ala Gln Gly Arg Ala Glu Arg Glu His Gly Arg Leu Glu Gly
   165 170 175
   Cys Leu Lys Gly Leu Arg Leu Gly His Lys Cys Phe Leu Leu Ser Arg
   180 185 190
   Asp Phe Glu Ala Gln Ala Ala Ala Gln Ala Arg Cys Thr Ala Arg Gly
   195 200 205
   Gly Ser Leu Ala Gln Pro Ala Asp Arg Gln Gln Met Glu Ala Leu Thr
   210 215 220
   Arg Tyr Leu Arg Ala Ala Leu Ala Pro Tyr Asn Trp Pro Val Trp Leu
   225 230 235 240
   Gly Val His Asp Arg Arg Ala Glu Gly Leu Tyr Leu Phe Glu Asn Gly
   245 250 255
   Gln Arg Val Ser Phe Phe Ala Trp His Arg Ser Pro Arg Pro Glu Leu
   260 265 270
   Gly Ala Gln Pro Ser Ala Ser Pro His Pro Leu Ser Pro Asp Gln Pro
   275 280 285
   Asn Gly Gly Thr Leu Glu Asn Cys Val Ala Gln Ala Ser Asp Asp Gly
   290 295 300
   Ser Trp Trp Asp His Asp Cys Gln Arg Arg Leu Tyr Tyr Val Cys Glu
   305 310 315 320
   Phe Pro Phe
<210> 13
   <211> 2468
   <212> PRT
   <213> Homo sapiens
<400> 13
   Met Ala Thr Val Val Val Glu Ala Thr Glu Pro Glu Pro Ser Gly Ser
   1               5                   10                  15
   Ile Ala Asn Pro Ala Ala Ser Thr Ser Pro Ser Leu Ser His Arg Phe
               20                  25                  30
   Leu Asp Ser Lys Phe Tyr Leu Leu Val Val Val Gly Glu Ile Val Thr
           35                  40                  45
   Glu Glu His Leu Arg Arg Ala Ile Gly Asn Ile Glu Leu Gly Ile Arg
       50                  55                  60
   Ser Trp Asp Thr Asn Leu Ile Glu Cys Asn Leu Asp Gln Glu Leu Lys
   65                  70                  75                  80
   Leu Phe Val Ser Arg His Ser Ala Arg Phe Ser Pro Glu Val Pro Gly
                   85                  90                  95
   Gln Lys Ile Leu His His Arg Ser Asp Val Leu Glu Thr Val Val Leu
               100                 105                 110
   Ile Asn Pro Ser Asp Glu Ala Val Ser Thr Glu Val Arg Leu Met Ile
           115                 120                 125
   Thr Asp Ala Ala Arg His Lys Leu Leu Val Leu Thr Gly Gln Cys Phe
       130                 135                 140
   Glu Asn Thr Gly Glu Leu Ile Leu Gln Ser Gly Ser Phe Ser Phe Gln
   145                 150                 155                 160
   Asn Phe Ile Glu Ile Phe Thr Asp Gln Glu Ile Gly Glu Leu Leu Ser
                   165                 170                 175
   Thr Thr His Pro Ala Asn Lys Ala Ser Leu Thr Leu Phe Cys Pro Glu
               180                 185                 190
   Glu Gly Asp Trp Lys Asn Ser Asn Leu Asp Arg His Asn Leu Gln Asp
           195                 200                 205
   Phe Ile Asn Ile Lys Leu Asn Ser Ala Ser Ile Leu Pro Glu Met Glu
       210                 215                 220
   Gly Leu Ser Glu Phe Thr Glu Tyr Leu Ser Glu Ser Val Glu Val Pro
   225                 230                 235                 240
   Ser Pro Phe Asp Ile Leu Glu Pro Pro Thr Ser Gly Gly Phe Leu Lys
                   245                 250                 255
   Leu Ser Lys Pro Cys Cys Tyr Ile Phe Pro Gly Gly Arg Gly Asp Ser
               260                 265                 270
   Ala Leu Phe Ala Val Asn Gly Phe Asn Met Leu Ile Asn Gly Gly Ser
           275                 280                 285
   Glu Arg Lys Ser Cys Phe Trp Lys Leu Ile Arg His Leu Asp Arg Val
       290                 295                 300
   Asp Ser Ile Leu Leu Thr His Ile Gly Asp Asp Asn Leu Pro Gly Ile
   305                 310                 315                 320
   Asn Ser Met Leu Gln Arg Lys Ile Ala Glu Leu Glu Glu Glu Gln Ser
                   325                 330                 335
   Gln Gly Ser Thr Thr Asn Ser Asp Trp Met Lys Asn Leu Ile Ser Pro
               340                 345                 350
   Asp Leu Gly Val Val Phe Leu Asn Val Pro Glu Asn Leu Lys Asn Pro
           355                 360                 365
   Glu Pro Asn Ile Lys Met Lys Arg Ser Ile Glu Glu Ala Cys Phe Thr
       370                 375                 380
   Leu Gln Tyr Leu Asn Lys Leu Ser Met Lys Pro Glu Pro Leu Phe Arg
   385                 390                 395                 400
   Ser Val Gly Asn Thr Ile Asp Pro Val Ile Leu Phe Gln Lys Met Gly
                   405                 410                 415
   Val Gly Lys Leu Glu Met Tyr Val Leu Asn Pro Val Lys Ser Ser Lys
               420                 425                 430
   Glu Met Gln Tyr Phe Met Gln Gln Trp Thr Gly Thr Asn Lys Asp Lys
           435                 440                 445
   Ala Glu Phe Ile Leu Pro Asn Gly Gln Glu Val Asp Leu Pro Ile Ser
       450                 455                 460
   Tyr Leu Thr Ser Val Ser Ser Leu Ile Val Trp His Pro Ala Asn Pro
   465                 470                 475                 480
   Ala Glu Lys Ile Ile Arg Val Leu Phe Pro Gly Asn Ser Thr Gln Tyr
   485 490 495
   Asn Ile Leu Glu Gly Leu Glu Lys Leu Lys His Leu Asp Phe Leu Lys
   500 505 510
   Gln Pro Leu Ala Thr Gln Lys Asp Leu Thr Gly Gln Val Pro Thr Pro
   515 520 525
   Val Val Lys Gln Thr Lys Leu Lys Gln Arg Ala Asp Ser Arg Glu Ser
   530 535 540
   Leu Lys Pro Ala Ala Lys Pro Leu Pro Ser Lys Ser Val Arg Lys Glu
   545 550 555 560
   Ser Lys Glu Glu Thr Pro Glu Val Thr Lys Val Asn His Val Glu Lys
   565 570 575
   Pro Pro Lys Val Glu Ser Lys Glu Lys Val Met Val Lys Lys Asp Lys
   580 585 590
   Pro Ile Lys Thr Glu Thr Lys Pro Ser Val Thr Glu Lys Glu Val Pro
   595 600 605
   Ser Lys Glu Glu Pro Ser Pro Val Lys Ala Glu Val Ala Glu Lys Gln
   610 615 620
   Ala Thr Asp Val Lys Pro Lys Ala Ala Lys Glu Lys Thr Val Lys Lys
   625 630 635 640
   Glu Thr Lys Val Lys Pro Glu Asp Lys Lys Glu Glu Lys Glu Lys Pro
   645 650 655
   Lys Lys Glu Val Ala Lys Lys Glu Asp Lys Thr Pro Ile Lys Lys Glu
   660 665 670
   Glu Lys Pro Lys Lys Glu Glu Val Lys Lys Glu Val Lys Lys Glu Ile
   675 680 685
   Lys Lys Glu Glu Lys Lys Glu Pro Lys Lys Glu Val Lys Lys Glu Thr
   690 695 700
   Pro Pro Lys Glu Val Lys Lys Glu Val Lys Lys Glu Glu Lys Lys Glu
   705 710 715 720
   Val Lys Lys Glu Glu Lys Glu Pro Lys Lys Glu Ile Lys Lys Leu Pro
   725 730 735
   Lys Asp Ala Lys Lys Ser Ser Thr Pro Leu Ser Glu Ala Lys Lys Pro
   740 745 750
   Ala Ala Leu Lys Pro Lys Val Pro Lys Lys Glu Glu Ser Val Lys Lys
   755 760 765
   Asp Ser Val Ala Ala Gly Lys Pro Lys Glu Lys Gly Lys Ile Lys Val
   770 775 780
   Ile Lys Lys Glu Gly Lys Ala Ala Glu Ala Val Ala Ala Ala Val Gly
   785 790 795 800
   Thr Gly Ala Thr Thr Ala Ala Val Met Ala Ala Ala Gly Ile Ala Ala
   805 810 815
   Ile Gly Pro Ala Lys Glu Leu Glu Ala Glu Arg Ser Leu Met Ser Ser
   820 825 830
   Pro Glu Asp Leu Thr Lys Asp Phe Glu Glu Leu Lys Ala Glu Glu Val
   835 840 845
   Asp Val Thr Lys Asp Ile Lys Pro Gln Leu Glu Leu Ile Glu Asp Glu
   850 855 860
   Glu Lys Leu Lys Glu Thr Glu Pro Val Glu Ala Tyr Val Ile Gln Lys
   865 870 875 880
   Glu Arg Glu Val Thr Lys Gly Pro Ala Glu Ser Pro Asp Glu Gly Ile
   885 890 895
   Thr Thr Thr Glu Gly Glu Gly Glu Cys Glu Gln Thr Pro Glu Glu Leu
   900 905 910
   Glu Pro Val Glu Lys Gln Gly Val Asp Asp Ile Glu Lys Phe Glu Asp
   915 920 925
   Glu Gly Ala Gly Phe Glu Glu Ser Ser Glu Thr Gly Asp Tyr Glu Glu
   930 935 940
   Lys Ala Glu Thr Glu Glu Ala Glu Glu Pro Glu Glu Asp Gly Glu Glu
   945 950 955 960
   His Val Cys Val Ser Ala Ser Lys His Ser Pro Thr Glu Asp Glu Glu
   965 970 975
   Ser Ala Lys Ala Glu Ala Asp Ala Tyr Ile Arg Glu Lys Arg Glu Ser
               980                 985                 990
   Val Ala Ser Gly Asp Asp Arg Ala  Glu Glu Asp Met Asp  Glu Ala Ile
           995                 1000                 1005
   Glu Lys  Gly Glu Ala Glu Gln  Ser Glu Glu Glu Ala  Asp Glu Glu
       1010                 1015                 1020
   Asp Lys  Ala Glu Asp Ala Arg  Glu Glu Glu Tyr Glu  Pro Glu Lys
       1025                 1030                 1035
   Met Glu  Ala Glu Asp Tyr Val  Met Ala Val Val Asp  Lys Ala Ala
       1040                 1045                 1050
   Glu Ala  Gly Gly Ala Glu Glu  Gln Tyr Gly Phe Leu  Thr Thr Pro
       1055                 1060                 1065
   Thr Lys  Gln Leu Gly Ala Gln  Ser Pro Gly Arg Glu  Pro Ala Ser
       1070                 1075                 1080
   Ser Ile  His Asp Glu Thr Leu  Pro Gly Gly Ser Glu  Ser Glu Ala
       1085                 1090                 1095
   Thr Ala  Ser Asp Glu Glu Asn  Arg Glu Asp Gln Pro  Glu Glu Phe
       1100                 1105                 1110
   Thr Ala  Thr Ser Gly Tyr Thr  Gln Ser Thr Ile Glu  Ile Ser Ser
       1115                 1120                 1125
   Glu Pro  Thr Pro Met Asp Glu  Met Ser Thr Pro Arg  Asp Val Met
       1130                 1135                 1140
   Ser Asp  Glu Thr Asn Asn Glu  Glu Thr Glu Ser Pro  Ser Gln Glu
       1145                 1150                 1155
   Phe Val  Asn Ile Thr Lys Tyr  Glu Ser Ser Leu Tyr  Ser Gln Glu
       1160                 1165                 1170
   Tyr Ser  Lys Pro Ala Asp Val  Thr Pro Leu Asn Gly  Phe Ser Glu
       1175                 1180                 1185
   Gly Ser  Lys Thr Asp Ala Thr  Asp Gly Lys Asp Tyr  Asn Ala Ser
       1190                 1195                 1200
   Ala Ser  Thr Ile Ser Pro Pro  Ser Ser Met Glu Glu  Asp Lys Phe
       1205                 1210                 1215
   Ser Arg Ser Ala Leu Arg Asp Ala Tyr Cys Ser Glu Val Lys Ala
   1220 1225 1230
   Ser Thr Thr Leu Asp Ile Lys Asp Ser Ile Ser Ala Val Ser Ser
   1235 1240 1245
   Glu Lys Val Ser Pro Ser Lys Ser Pro Ser Leu Ser Pro Ser Pro
   1250 1255 1260
   Pro Ser Pro Leu Glu Lys Thr Pro Leu Gly Glu Arg Ser Val Asn
   1265 1270 1275
   Phe Ser Leu Thr Pro Asn Glu Ile Lys Val Ser Ala Glu Ala Glu
   1280 1285 1290
   Val Ala Pro Val Ser Pro Glu Val Thr Gln Glu Val Val Glu Glu
   1295 1300 1305
   His Cys Ala Ser Pro Glu Asp Lys Thr Leu Glu Val Val Ser Pro
   1310 1315 1320
   Ser Gln Ser Val Thr Gly Ser Ala Gly His Thr Pro Tyr Tyr Gln
   1325 1330 1335
   Ser Pro Thr Asp Glu Lys Ser Ser His Leu Pro Thr Glu Val Ile
   1340 1345 1350
   Glu Lys Pro Pro Ala Val Pro Val Ser Phe Glu Phe Ser Asp Ala
   1355 1360 1365
   Lys Asp Glu Asn Glu Arg Ala Ser Val Ser Pro Met Asp Glu Pro
   1370 1375 1380
   Val Pro Asp Ser Glu Ser Pro Ile Glu Lys Val Leu Ser Pro Leu
   1385 1390 1395
   Arg Ser Pro Pro Leu Ile Gly Ser Glu Ser Ala Tyr Glu Ser Phe
   1400 1405 1410
   Leu Ser Ala Asp Asp Lys Ala Ser Gly Arg Gly Ala Glu Ser Pro
   1415 1420 1425
   Phe Glu Glu Lys Ser Gly Lys Gln Gly Ser Pro Asp Gln Val Ser
   1430 1435 1440
   Pro Val Ser Glu Met Thr Ser Thr Ser Leu Tyr Gln Asp Lys Gln
   1445 1450 1455
   Glu Gly Lys Ser Thr Asp Phe Ala Pro Ile Lys Glu Asp Phe Gly
   1460 1465 1470
   Gln Glu Lys Lys Thr Asp Asp Val Glu Ala Met Ser Ser Gln Pro
   1475 1480 1485
   Ala Leu Ala Leu Asp Glu Arg Lys Leu Gly Asp Val Ser Pro Thr
   1490 1495 1500
   Gln Ile Asp Val Ser Gln Phe Gly Ser Phe Lys Glu Asp Thr Lys
   1505 1510 1515
   Met Ser Ile Ser Glu Gly Thr Val Ser Asp Lys Ser Ala Thr Pro
   1520 1525 1530
   Val Asp Glu Gly Val Ala Glu Asp Thr Tyr Ser His Met Glu Gly
   1535 1540 1545
   Val Ala Ser Val Ser Thr Ala Ser Val Ala Thr Ser Ser Phe Pro
   1550 1555 1560
   Glu Pro Thr Thr Asp Asp Val Ser Pro Ser Leu His Ala Glu Val
   1565 1570 1575
   Gly Ser Pro His Ser Thr Glu Val Asp Asp Ser Leu Ser Val Ser
   1580 1585 1590
   Val Val Gln Thr Pro Thr Thr Phe Gln Glu Thr Glu Met Ser Pro
   1595 1600 1605
   Ser Lys Glu Glu Cys Pro Arg Pro Met Ser Ile Ser Pro Pro Asp
   1610 1615 1620
   Phe Ser Pro Lys Thr Ala Lys Ser Arg Thr Pro Val Gln Asp His
   1625 1630 1635
   Arg Ser Glu Gln Ser Ser Met Ser Ile Glu Phe Gly Gln Glu Ser
   1640 1645 1650
   Pro Glu Gln Ser Leu Ala Met Asp Phe Ser Arg Gln Ser Pro Asp
   1655 1660 1665
   His Pro Thr Val Gly Ala Gly Val Leu His Ile Thr Glu Asn Gly
   1670 1675 1680
   Pro Thr Glu Val Asp Tyr Ser Pro Ser Asp Met Gln Asp Ser Ser
   1685 1690 1695
   Leu Ser His Lys Ile Pro Pro Met Glu Glu Pro Ser Tyr Thr Gln
   1700 1705 1710
   Asp Asn Asp Leu Ser Glu Leu Ile Ser Val Ser Gln Val Glu Ala
   1715 1720 1725
   Ser Pro Ser Thr Ser Ser Ala His Thr Pro Ser Gln Ile Ala Ser
   1730 1735 1740
   Pro Leu Gln Glu Asp Thr Leu Ser Asp Val Ala Pro Pro Arg Asp
   1745 1750 1755
   Met Ser Leu Tyr Ala Ser Leu Thr Ser Glu Lys Val Gln Ser Leu
   1760 1765 1770
   Glu Gly Glu Lys Leu Ser Pro Lys Ser Asp Ile Ser Pro Leu Thr
   1775 1780 1785
   Pro Arg Glu Ser Ser Pro Leu Tyr Ser Pro Thr Phe Ser Asp Ser
   1790 1795 1800
   Thr Ser Ala Val Lys Glu Lys Thr Ala Thr Cys His Ser Ser Ser
   1805 1810 1815
   Ser Pro Pro Ile Asp Ala Ala Ser Ala Glu Pro Tyr Gly Phe Arg
   1820 1825 1830
   Ala Ser Val Leu Phe Asp Thr Met Gln His His Leu Ala Leu Asn
   1835 1840 1845
   Arg Asp Leu Ser Thr Pro Gly Leu Glu Lys Asp Ser Gly Gly Lys
   1850 1855 1860
   Thr Pro Gly Asp Phe Ser Tyr Ala Tyr Gln Lys Pro Glu Glu Thr
   1865 1870 1875
   Thr Arg Ser Pro Asp Glu Glu Asp Tyr Asp Tyr Glu Ser Tyr Glu
   1880 1885 1890
   Lys Thr Thr Arg Thr Ser Asp Val Gly Gly Tyr Tyr Tyr Glu Lys
   1895 1900 1905
   Ile Glu Arg Thr Thr Lys Ser Pro Ser Asp Ser Gly Tyr Ser Tyr
   1910 1915 1920
   Glu Thr  Ile Gly Lys Thr Thr  Lys Thr Pro Glu Asp  Gly Asp Tyr
       1925                 1930                 1935
   Ser Tyr  Glu Ile Ile Glu Lys  Thr Thr Arg Thr Pro  Glu Glu Gly
       1940                 1945                 1950
   Gly Tyr  Ser Tyr Asp Ile Ser  Glu Lys Thr Thr Ser  Pro Pro Glu
       1955                 1960                 1965
   Val Ser  Gly Tyr Ser Tyr Glu  Lys Thr Glu Arg Ser  Arg Arg Leu
       1970                 1975                 1980
   Leu Asp  Asp Ile Ser Asn Gly  Tyr Asp Asp Ser Glu  Asp Gly Gly
       1985                 1990                 1995
   His Thr  Leu Gly Asp Pro Ser  Tyr Ser Tyr Glu Thr  Thr Glu Lys
       2000                 2005                 2010
   Ile Thr  Ser Phe Pro Glu Ser  Glu Gly Tyr Ser Tyr  Glu Thr Ser
       2015                 2020                 2025
   Thr Lys  Thr Thr Arg Thr Pro  Asp Thr Ser Thr Tyr  Cys Tyr Glu
       2030                 2035                 2040
   Thr Ala  Glu Lys Ile Thr Arg  Thr Pro Gln Ala Ser  Thr Tyr Ser
       2045                 2050                 2055
   Tyr Glu  Thr Ser Asp Leu Cys  Tyr Thr Ala Glu Lys  Lys Ser Pro
       2060                 2065                 2070
   Ser Glu  Ala Arg Gln Asp Val  Asp Leu Cys Leu Val  Ser Ser Cys
       2075                 2080                 2085
   Glu Tyr  Lys His Pro Lys Thr  Glu Leu Ser Pro Ser  Phe Ile Asn
       2090                 2095                 2100
   Pro Asn  Pro Leu Glu Trp Phe  Ala Ser Glu Glu Pro  Thr Glu Glu
       2105                 2110                 2115
   Ser Glu  Lys Pro Leu Thr Gln  Ser Gly Gly Ala Pro  Pro Pro Pro
       2120                 2125                 2130
   Gly Gly  Lys Gln Gln Gly Arg  Gln Cys Asp Glu Thr  Pro Pro Thr
       2135                 2140                 2145
   Ser Val  Ser Glu Ser Ala Pro  Ser Gln Thr Asp Ser  Asp Val Pro
       2150                 2155                 2160
   Pro Glu  Thr Glu Glu Cys Pro  Ser Ile Thr Ala Asp  Ala Asn Ile
       2165                 2170                 2175
   Asp Ser  Glu Asp Glu Ser Glu  Thr Ile Pro Thr Asp  Lys Thr Val
       2180                 2185                 2190
   Thr Tyr  Lys His Met Asp Pro  Pro Pro Ala Pro Val  Gln Asp Arg
       2195                 2200                 2205
   Ser Pro  Ser Pro Arg His Pro  Asp Val Ser Met Val  Asp Pro Glu
       2210                 2215                 2220
   Ala Leu  Ala Ile Glu Gln Asn  Leu Gly Lys Ala Leu  Lys Lys Asp
       2225                 2230                 2235
   Leu Lys  Glu Lys Thr Lys Thr  Lys Lys Pro Gly Thr  Lys Thr Lys
       2240                 2245                 2250
   Ser Ser  Ser Pro Val Lys Lys  Ser Asp Gly Lys Ser  Lys Pro Leu
       2255                 2260                 2265
   Ala Ala  Ser Pro Lys Pro Ala  Gly Leu Lys Glu Ser  Ser Asp Lys
       2270                 2275                 2280
   Val Ser  Arg Val Ala Ser Pro  Lys Lys Lys Glu Ser  Val Glu Lys
       2285                 2290                 2295
   Ala Ala  Lys Pro Thr Thr Thr  Pro Glu Val Lys Ala  Ala Arg Gly
       2300                 2305                 2310
   Glu Glu  Lys Asp Lys Glu Thr  Lys Asn Ala Ala Asn  Ala Ser Ala
       2315                 2320                 2325
   Ser Lys  Ser Ala Lys Thr Ala  Thr Ala Gly Pro Gly  Thr Thr Lys
       2330                 2335                 2340
   Thr Thr  Lys Ser Ser Ala Val  Pro Pro Gly Leu Pro  Val Tyr Leu
       2345                 2350                 2355
   Asp Leu  Cys Tyr Ile Pro Asn  His Ser Asn Ser Lys  Asn Val Asp
       2360                 2365                 2370
   Val Glu  Phe Phe Lys Arg Val  Arg Ser Ser Tyr Tyr  Val Val Ser
       2375                 2380                 2385
   Gly Asn  Asp Pro Ala Ala Glu  Glu Pro Ser Arg Ala  Val Leu Asp
       2390                 2395                 2400
   Ala Leu  Leu Glu Gly Lys Ala  Gln Trp Gly Ser Asn  Met Gln Val
       2405                 2410                 2415
   Thr Leu  Ile Pro Thr His Asp  Ser Glu Val Met Arg  Glu Trp Tyr
       2420                 2425                 2430
   Gln Glu  Thr His Glu Lys Gln  Gln Asp Leu Asn Ile  Met Val Leu
       2435                 2440                 2445
   Ala Ser  Ser Ser Thr Val Val  Met Gln Asp Glu Ser  Phe Pro Ala
       2450                 2455                 2460
   Cys Lys  Ile Glu Leu
       2465
<210> 14
   <211> 215
   <212> PRT
   <213> Homo sapiens
<400> 14
   Met Pro Gly Glu Ala Thr Glu Thr Val Pro Ala Thr Glu Gln Glu Leu
   1               5                   10                  15
   Pro Gln Pro Gln Ala Glu Thr Gly Ser Gly Thr Glu Ser Asp Ser Asp
               20                  25                  30
   Glu Ser Val Pro Glu Leu Glu Glu Gln Asp Ser Thr Gln Ala Thr Thr
           35                  40                  45
   Gln Gln Ala Gln Leu Ala Ala Ala Ala Glu Ile Asp Glu Glu Pro Val
       50                  55                  60
   Ser Lys Ala Lys Gln Ser Arg Ser Glu Lys Lys Ala Arg Lys Ala Met
   65                  70                  75                  80
   Ser Lys Leu Gly Leu Arg Gln Val Thr Gly Val Thr Arg Val Thr Ile
                   85                  90                  95
   Arg Lys Ser Lys Asn Ile Leu Phe Val Ile Thr Lys Pro Asp Val Tyr
               100                 105                 110
   Lys Ser Pro Ala Ser Asp Thr Tyr Ile Val Phe Gly Glu Ala Lys Ile
           115                 120                 125
   Glu Asp Leu Ser Gln Gln Ala Gln Leu Ala Ala Ala Glu Lys Phe Lys
       130                 135                 140
   Val Gln Gly Glu Ala Val Ser Asn Ile Gln Glu Asn Thr Gln Thr Pro
   145 150 155 160
   Thr Val Gln Glu Glu Ser Glu Glu Glu Glu Val Asp Glu Thr Gly Val
   165 170 175
   Glu Val Lys Asp Ile Glu Leu Val Met Ser Gln Ala Asn Val Ser Arg
   180 185 190
   Ala Lys Ala Val Arg Ala Leu Lys Asn Asn Ser Asn Asp Ile Val Asn
   195 200 205
   Ala Ile Met Glu Leu Thr Met
   210 215
<210> 15
   <211> 234
   <212> PRT
   <213> Homo sapiens
<400> 15
   Met Ala Glu Arg Gly Tyr Ser Phe Ser Leu Thr Thr Phe Ser Pro Ser
   1 5 10 15
   Gly Lys Leu Val Gln Ile Glu Tyr Ala Leu Ala Ala Val Ala Gly Gly
   20 25 30
   Ala Pro Ser Val Gly Ile Lys Ala Ala Asn Gly Val Val Leu Ala Thr
   35 40 45
   Glu Lys Lys Gln Lys Ser Ile Leu Tyr Asp Glu Arg Ser Val His Lys
   50 55 60
   Val Glu Pro Ile Thr Lys His Ile Gly Leu Val Tyr Ser Gly Met Gly
   65 70 75 80
   Pro Asp Tyr Arg Val Leu Val His Arg Ala Arg Lys Leu Ala Gln Gln
   85 90 95
   Tyr Tyr Leu Val Tyr Gln Glu Pro Ile Pro Thr Ala Gln Leu Val Gln
   100 105 110
   Arg Val Ala Ser Val Met Gln Glu Tyr Thr Gln Ser Gly Gly Val Arg
   115 120 125
   Pro Phe Gly Val Ser Leu Leu Ile Cys Gly Trp Asn Glu Gly Arg Pro
   130 135 140
   Tyr Leu Phe Gln Ser Asp Pro Ser Gly Ala Tyr Phe Ala Trp Lys Ala
   145                 150                 155                 160
   Thr Ala Met Gly Lys Asn Tyr Val Asn Gly Lys Thr Phe Leu Glu Lys
                   165                 170                 175
   Arg Tyr Asn Glu Asp Leu Glu Leu Glu Asp Ala Ile His Thr Ala Ile
               180                 185                 190
   Leu Thr Leu Lys Glu Ser Phe Glu Gly Gln Met Thr Glu Asp Asn Ile
           195                 200                 205
   Glu Val Gly Ile Cys Asn Glu Ala Gly Phe Arg Arg Leu Thr Pro Thr
       210                 215                 220
   Glu Val Lys Asp Tyr Leu Ala Ala Ile Ala
   225                 230
<210> 16
   <211> 297
   <212> PRT
   <213> Homo sapiens
<400> 16
<210> 17
   <211> 151
   <212> PRT
   <213> Homo sapiens
<400> 17
   Met Cys Asp Phe Thr Glu Asp Gln Thr Ala Glu Phe Lys Glu Ala Phe
   1 5 10 15
   Gln Leu Phe Asp Arg Thr Gly Asp Gly Lys Ile Leu Tyr Ser Gln Cys
   20 25 30
   Gly Asp Val Met Arg Ala Leu Gly Gln Asn Pro Thr Asn Ala Glu Val
   35 40 45
   Leu Lys Val Leu Gly Asn Pro Lys Ser Asp Glu Met Asn Val Lys Val
   50 55 60
   Leu Asp Phe Glu His Phe Leu Pro Met Leu Gln Thr Val Ala Lys Asn
   65 70 75 80
   Lys Asp Gln Gly Thr Tyr Glu Asp Tyr Val Glu Gly Leu Arg Val Phe
   85 90 95
   Asp Lys Glu Gly Asn Gly Thr Val Met Gly Ala Glu Ile Arg His Val
   100 105 110
   Leu Val Thr Leu Gly Glu Lys Met Thr Glu Glu Glu Val Glu Met Leu
   115 120 125
   Val Ala Gly His Glu Asp Ser Asn Gly Cys Ile Asn Tyr Glu Ala Phe
   130 135 140
   Val Arg His Ile Leu Ser Gly
   145 150
<210> 18
   <211> 339
   <212> PRT
   <213> Homo sapiens
<400> 18
   Met Trp Gln Leu Trp Ala Ser Leu Cys Cys Leu Leu Val Leu Ala Asn
   1 5 10 15
   Ala Arg Ser Arg Pro Ser Phe His Pro Leu Ser Asp Glu Leu Val Asn
   20 25 30
   Tyr Val Asn Lys Arg Asn Thr Thr Trp Gln Ala Gly His Asn Phe Tyr
   35 40 45
   Asn Val Asp Met Ser Tyr Leu Lys Arg Leu Cys Gly Thr Phe Leu Gly
   50 55 60
   Gly Pro Lys Pro Pro Gln Arg Val Met Phe Thr Glu Asp Leu Lys Leu
   65 70 75 80
   Pro Ala Ser Phe Asp Ala Arg Glu Gln Trp Pro Gln Cys Pro Thr Ile
   85 90 95
   Lys Glu Ile Arg Asp Gln Gly Ser Cys Gly Ser Cys Trp Ala Phe Gly
   100 105 110
   Ala Val Glu Ala Ile Ser Asp Arg Ile Cys Ile His Thr Asn Ala His
           115                 120                 125
   Val Ser Val Glu Val Ser Ala Glu Asp Leu Leu Thr Cys Cys Gly Ser
       130                 135                 140
   Met Cys Gly Asp Gly Cys Asn Gly Gly Tyr Pro Ala Glu Ala Trp Asn
   145                 150                 155                 160
   Phe Trp Thr Arg Lys Gly Leu Val Ser Gly Gly Leu Tyr Glu Ser His
                   165                 170                 175
   Val Gly Cys Arg Pro Tyr Ser Ile Pro Pro Cys Glu His His Val Asn
               180                 185                 190
   Gly Ser Arg Pro Pro Cys Thr Gly Glu Gly Asp Thr Pro Lys Cys Ser
           195                 200                 205
   Lys Ile Cys Glu Pro Gly Tyr Ser Pro Thr Tyr Lys Gln Asp Lys His
       210                 215                 220
   Tyr Gly Tyr Asn Ser Tyr Ser Val Ser Asn Ser Glu Lys Asp Ile Met
   225                 230                 235                 240
   Ala Glu Ile Tyr Lys Asn Gly Pro Val Glu Gly Ala Phe Ser Val Tyr
                   245                 250                 255
   Ser Asp Phe Leu Leu Tyr Lys Ser Gly Val Tyr Gln His Val Thr Gly
               260                 265                 270
   Glu Met Met Gly Gly His Ala Ile Arg Ile Leu Gly Trp Gly Val Glu
           275                 280                 285
   Asn Gly Thr Pro Tyr Trp Leu Val Ala Asn Ser Trp Asn Thr Asp Trp
       290                 295                 300
   Gly Asp Asn Gly Phe Phe Lys Ile Leu Arg Gly Gln Asp His Cys Gly
   305                 310                 315                 320
   Ile Glu Ser Glu Val Val Ala Gly Ile Pro Arg Thr Asp Gln Tyr Trp
                   325                 330                 335
   Glu Lys Ile
<210> 19
   <211> 573
   <212> PRT
   <213> Homo sapiens
<400> 19
<210> 20
   <211> 390
   <212> PRT
   <213> Homo sapiens
<400> 20
   Met Glu Asp Pro Thr Leu Tyr Ile Val Glu Arg Pro Leu Pro Gly Tyr
   1 5 10 15
   Pro Asp Ala Glu Ala Pro Glu Pro Ser Ser Ala Gly Ala Gln Ala Ala
   20 25 30
   Glu Glu Pro Ser Gly Ala Gly Ser Glu Glu Leu Ile Lys Ser Asp Gln
   35 40 45
   Val Asn Gly Val Leu Val Leu Ser Leu Leu Asp Lys Ile Ile Gly Ala
   50 55 60
   Val Asp Gln Ile Gln Leu Thr Gln Ala Gln Leu Glu Glu Arg Gln Ala
   65 70 75 80
   Glu Met Glu Gly Ala Val Gln Ser Ile Gln Gly Glu Leu Ser Lys Leu
   85 90 95
   Gly Lys Ala His Ala Thr Thr Ser Asn Thr Val Ser Lys Leu Leu Glu
   100 105 110
   Lys Val Arg Lys Val Ser Val Asn Val Lys Thr Val Arg Gly Ser Leu
   115 120 125
   Glu Arg Gln Ala Gly Gln Ile Lys Lys Leu Glu Val Asn Glu Ala Glu
   130 135 140
   Leu Leu Arg Arg Arg Asn Phe Lys Val Met Ile Tyr Gln Asp Glu Val
   145 150 155 160
   Lys Leu Pro Ala Lys Leu Ser Ile Ser Lys Ser Leu Lys Glu Ser Glu
   165 170 175
   Ala Leu Pro Glu Lys Glu Gly Glu Glu Leu Gly Glu Gly Glu Arg Pro
   180 185 190
   Glu Glu Asp Ala Ala Ala Leu Glu Leu Ser Ser Asp Glu Ala Val Glu
   195 200 205
   Val Glu Glu Val Ile Glu Glu Ser Arg Ala Glu Arg Ile Lys Arg Ser
   210 215 220
   Gly Leu Arg Arg Val Asp Asp Phe Lys Lys Ala Phe Ser Lys Glu Lys
   225 230 235 240
   Met Glu Lys Thr Lys Val Arg Thr Arg Glu Asn Leu Glu Lys Thr Arg
   245 250 255
   Leu Lys Thr Lys Glu Asn Leu Glu Lys Thr Arg His Thr Leu Glu Lys
   260 265 270
   Arg Met Asn Lys Leu Gly Thr Arg Leu Val Pro Ala Glu Arg Arg Glu
   275 280 285
   Lys Leu Lys Thr Ser Arg Asp Lys Leu Arg Lys Ser Phe Thr Pro Asp
   290 295 300
   His Val Val Tyr Ala Arg Ser Lys Thr Ala Val Tyr Lys Val Pro Pro
   305 310 315 320
   Phe Thr Phe His Val Lys Lys Ile Arg Glu Gly Gln Val Glu Val Leu
   325 330 335
   Lys Ala Thr Glu Met Val Glu Val Gly Ala Asp Asp Asp Glu Gly Gly
   340 345 350
   Ala Glu Arg Gly Glu Ala Gly Asp Leu Arg Arg Gly Ser Ser Pro Asp
   355 360 365
   Val His Ala Leu Leu Glu Ile Thr Glu Glu Ser Asp Ala Val Leu Val
   370 375 380
   Asp Lys Ser Asp Ser Asp
   385 390
<210> 21
   <211> 461
   <212> PRT
   <213> Homo sapiens
<400> 21
   Met Pro Pro Ser Gly Pro Arg Gly Thr Leu Leu Leu Leu Pro Leu Leu
   1 5 10 15
   Leu Leu Leu Leu Leu Arg Ala Val Leu Ala Val Pro Leu Glu Arg Gly
   20 25 30
   Ala Pro Asn Lys Glu Glu Thr Pro Ala Thr Glu Ser Pro Asp Thr Gly
   35 40 45
   Leu Tyr Tyr His Arg Tyr Leu Gln Glu Val Ile Asp Val Leu Glu Thr
   50 55 60
   Asp Gly His Phe Arg Glu Lys Leu Gln Ala Ala Asn Ala Glu Asp Ile
   65 70 75 80
   Lys Ser Gly Lys Leu Ser Arg Glu Leu Asp Phe Val Ser His His Val
   85 90 95
   Arg Thr Lys Leu Asp Glu Leu Lys Arg Gln Glu Val Ser Arg Leu Arg
   100 105 110
   Met Leu Leu Lys Ala Lys Met Asp Ala Glu Gln Asp Pro Asn Val Gln
   115 120 125
   Val Asp His Leu Asn Leu Leu Lys Gln Phe Glu His Leu Asp Pro Gln
   130 135 140
   Asn Gln His Thr Phe Glu Ala Arg Asp Leu Glu Leu Leu Ile Gln Thr
   145 150 155 160
   Ala Thr Arg Asp Leu Ala Gln Tyr Asp Ala Ala His His Glu Glu Phe
   165 170 175
   Lys Arg Tyr Glu Met Leu Lys Glu His Glu Arg Arg Arg Tyr Leu Glu
   180 185 190
   Ser Leu Gly Glu Glu Gln Arg Lys Glu Ala Glu Arg Lys Leu Glu Glu
   195 200 205
   Gln Gln Arg Arg His Arg Glu His Pro Lys Val Asn Val Pro Gly Ser
       210                 215                 220
   Gln Ala Gln Leu Lys Glu Val Trp Glu Glu Leu Asp Gly Leu Asp Pro
   225                 230                 235                 240
   Asn Arg Phe Asn Pro Lys Thr Phe Phe Ile Leu His Asp Ile Asn Ser
                   245                 250                 255
   Asp Gly Val Leu Asp Glu Gln Glu Leu Glu Ala Leu Phe Thr Lys Glu
               260                 265                 270
   Leu Glu Lys Val Tyr Asp Pro Lys Asn Glu Glu Asp Asp Met Arg Glu
           275                 280                 285
   Met Glu Glu Glu Arg Leu Arg Met Arg Glu His Val Met Lys Asn Val
       290                 295                 300
   Asp Thr Asn Gln Asp Arg Leu Val Thr Leu Glu Glu Phe Leu Ala Ser
   305                 310                 315                 320
   Thr Gln Arg Lys Glu Phe Gly Asp Thr Gly Glu Gly Trp Glu Thr Val
                   325                 330                 335
   Glu Met His Pro Ala Tyr Thr Glu Glu Glu Leu Arg Arg Phe Glu Glu
               340                 345                 350
   Glu Leu Ala Ala Arg Glu Ala Glu Leu Asn Ala Lys Ala Gln Arg Leu
           355                 360                 365
   Ser Gln Glu Thr Glu Ala Leu Gly Arg Ser Gln Gly Arg Leu Glu Ala
       370                 375                 380
   Gln Lys Arg Glu Leu Gln Gln Ala Val Leu His Met Glu Gln Arg Lys
   385                 390                 395                 400
   Gln Gln Gln Gln Gln Gln Gln Gly His Lys Ala Pro Ala Ala His Pro
                   405                 410                 415
   Glu Gly Gln Leu Lys Phe His Pro Asp Thr Asp Asp Val Pro Val Pro
               420                 425                 430
   Ala Pro Ala Gly Asp Gln Lys Glu Val Asp Thr Ser Glu Lys Lys Leu
           435                 440                 445
   Leu Glu Arg Leu Pro Glu Val Glu Val Pro Gln His Leu
       450                 455                 460
<210> 22
   <211> 645
   <212> PRT
   <213> Homo sapiens
<400> 22
   Met Arg Pro Arg Lys Ala Phe Leu Leu Leu Leu Leu Leu Gly Leu Val
   1               5                   10                  15
   Gln Leu Leu Ala Val Ala Gly Ala Glu Gly Pro Asp Glu Asp Ser Ser
               20                  25                  30
   Asn Arg Glu Asn Ala Ile Glu Asp Glu Glu Glu Glu Glu Glu Glu Asp
           35                  40                  45
   Asp Asp Glu Glu Glu Asp Asp Leu Glu Val Lys Glu Glu Asn Gly Val
       50                  55                  60
   Leu Val Leu Asn Asp Ala Asn Phe Asp Asn Phe Val Ala Asp Lys Asp
   65                  70                  75                  80
   Thr Val Leu Leu Glu Phe Tyr Ala Pro Trp Cys Gly His Cys Lys Gln
                   85                  90                  95
   Phe Ala Pro Glu Tyr Glu Lys Ile Ala Asn Ile Leu Lys Asp Lys Asp
               100                 105                 110
   Pro Pro Ile Pro Val Ala Lys Ile Asp Ala Thr Ser Ala Ser Val Leu
           115                 120                 125
   Ala Ser Arg Phe Asp Val Ser Gly Tyr Pro Thr Ile Lys Ile Leu Lys
       130                 135                 140
   Lys Gly Gln Ala Val Asp Tyr Glu Gly Ser Arg Thr Gln Glu Glu Ile
   145                 150                 155                 160
   Val Ala Lys Val Arg Glu Val Ser Gln Pro Asp Trp Thr Pro Pro Pro
                   165                 170                 175
   Glu Val Thr Leu Val Leu Thr Lys Glu Asn Phe Asp Glu Val Val Asn
               180                 185                 190
   Asp Ala Asp Ile Ile Leu Val Glu Phe Tyr Ala Pro Trp Cys Gly His
           195                 200                 205
   Cys Lys Lys Leu Ala Pro Glu Tyr Glu Lys Ala Ala Lys Glu Leu Ser
       210                 215                 220
   Lys Arg Ser Pro Pro Ile Pro Leu Ala Lys Val Asp Ala Thr Ala Glu
   225 230 235 240
   Thr Asp Leu Ala Lys Arg Phe Asp Val Ser Gly Tyr Pro Thr Leu Lys
   245 250 255
   Ile Phe Arg Lys Gly Arg Pro Tyr Asp Tyr Asn Gly Pro Arg Glu Lys
   260 265 270
   Tyr Gly Ile Val Asp Tyr Met Ile Glu Gln Ser Gly Pro Pro Ser Lys
   275 280 285
   Glu Ile Leu Thr Leu Lys Gln Val Gln Glu Phe Leu Lys Asp Gly Asp
   290 295 300
   Asp Val Ile Ile Ile Gly Val Phe Lys Gly Glu Ser Asp Pro Ala Tyr
   305 310 315 320
   Gln Gln Tyr Gln Asp Ala Ala Asn Asn Leu Arg Glu Asp Tyr Lys Phe
   325 330 335
   His His Thr Phe Ser Thr Glu Ile Ala Lys Phe Leu Lys Val Ser Gln
   340 345 350
   Gly Gln Leu Val Val Met Gln Pro Glu Lys Phe Gln Ser Lys Tyr Glu
   355 360 365
   Pro Arg Ser His Met Met Asp Val Gln Gly Ser Thr Gln Asp Ser Ala
   370 375 380
   Ile Lys Asp Phe Val Leu Lys Tyr Ala Leu Pro Leu Val Gly His Arg
   385 390 395 400
   Lys Val Ser Asn Asp Ala Lys Arg Tyr Thr Arg Arg Pro Leu Val Val
   405 410 415
   Val Tyr Tyr Ser Val Asp Phe Ser Phe Asp Tyr Arg Ala Ala Thr Gln
   420 425 430
   Phe Trp Arg Ser Lys Val Leu Glu Val Ala Lys Asp Phe Pro Glu Tyr
   435 440 445
   Thr Phe Ala Ile Ala Asp Glu Glu Asp Tyr Ala Gly Glu Val Lys Asp
   450 455 460
   Leu Gly Leu Ser Glu Ser Gly Glu Asp Val Asn Ala Ala Ile Leu Asp
   465 470 475 480
   Glu Ser Gly Lys Lys Phe Ala Met Glu Pro Glu Glu Phe Asp Ser Asp
   485 490 495
   Thr Leu Arg Glu Phe Val Thr Ala Phe Lys Lys Gly Lys Leu Lys Pro
   500 505 510
   Val Ile Lys Ser Gln Pro Val Pro Lys Asn Asn Lys Gly Pro Val Lys
   515 520 525
   Val Val Val Gly Lys Thr Phe Asp Ser Ile Val Met Asp Pro Lys Lys
   530 535 540
   Asp Val Leu Ile Glu Phe Tyr Ala Pro Trp Cys Gly His Cys Lys Gln
   545 550 555 560
   Leu Glu Pro Val Tyr Asn Ser Leu Ala Lys Lys Tyr Lys Gly Gln Lys
   565 570 575
   Gly Leu Val Ile Ala Lys Met Asp Ala Thr Ala Asn Asp Val Pro Ser
   580 585 590
   Asp Arg Tyr Lys Val Glu Gly Phe Pro Thr Ile Tyr Phe Ala Pro Ser
   595 600 605
   Gly Asp Lys Lys Asn Pro Val Lys Phe Glu Gly Gly Asp Arg Asp Leu
   610 615 620
   Glu His Leu Ser Lys Phe Ile Glu Glu His Ala Thr Lys Leu Ser Arg
   625 630 635 640
   Thr Lys Glu Glu Leu
   645
<210> 23
   <211> 508
   <212> PRT
   <213> Homo sapiens
<400> 23
<210> 24
   <211> 911
   <212> PRT
   <213> Homo sapiens
<400> 24
<210> 25
   <211> 281
   <212> PRT
   <213> Homo sapiens
<400> 25
   Met Ala Thr Asn Phe Leu Ala His Glu Lys Ile Trp Phe Asp Lys Phe
   1               5                   10                  15
   Lys Tyr Asp Asp Ala Glu Arg Arg Phe Tyr Glu Gln Met Asn Gly Pro
               20                  25                  30
   Val Ala Gly Ala Ser Arg Gln Glu Asn Gly Ala Ser Val Ile Leu Arg
           35                  40                  45
   Asp Ile Ala Arg Ala Arg Glu Asn Ile Gln Lys Ser Leu Ala Gly Ser
       50                  55                  60
   Ser Gly Pro Gly Ala Ser Ser Gly Thr Ser Gly Asp His Gly Glu Leu
   65                  70                  75                  80
   Val Val Arg Ile Ala Ser Leu Glu Val Glu Asn Gln Ser Leu Arg Gly
                   85                  90                  95
   Val Val Gln Glu Leu Gln Gln Ala Ile Ser Lys Leu Glu Ala Arg Leu
               100                 105                 110
   Asn Val Leu Glu Lys Ser Ser Pro Gly His Arg Ala Thr Ala Pro Gln
           115                 120                 125
   Thr Gln His Val Ser Pro Met Arg Gln Val Glu Pro Pro Ala Lys Lys
       130                 135                 140
   Pro Ala Thr Pro Ala Glu Asp Asp Glu Asp Asp Asp Ile Asp Leu Phe
   145                 150                 155                 160
   Gly Ser Asp Asn Glu Glu Glu Asp Lys Glu Ala Ala Gln Leu Arg Glu
                   165                 170                 175
   Glu Arg Leu Arg Gln Tyr Ala Glu Lys Lys Ala Lys Lys Pro Ala Leu
               180                 185                 190
   Val Ala Lys Ser Ser Ile Leu Leu Asp Val Lys Pro Trp Asp Asp Glu
           195                 200                 205
   Thr Asp Met Ala Gln Leu Glu Ala Cys Val Arg Ser Ile Gln Leu Asp
       210                 215                 220
   Gly Leu Val Trp Gly Ala Ser Lys Leu Val Pro Val Gly Tyr Gly Ile
   225                 230                 235                 240
   Arg Lys Leu Gln Ile Gln Cys Val Val Glu Asp Asp Lys Val Gly Thr
                   245                 250                 255
   Asp Leu Leu Glu Glu Glu Ile Thr Lys Phe Glu Glu His Val Gln Ser
               260                 265                 270
   Val Asp Ile Ala Ala Phe Asn Lys Ile
           275                 280
<210> 26
   <211> 286
   <212> PRT
   <213> Homo sapiens
<400> 26
   Met Ala Glu Asp Gly Glu Glu Ala Glu Phe His Phe Ala Ala Leu Tyr
   1               5                   10                  15
   Ile Ser Gly Gln Trp Pro Arg Leu Arg Ala Asp Thr Asp Leu Gln Arg
               20                  25                  30
   Leu Gly Ser Ser Ala Met Ala Pro Ser Arg Lys Phe Phe Val Gly Gly
           35                  40                  45
   Asn Trp Lys Met Asn Gly Arg Lys Gln Ser Leu Gly Glu Leu Ile Gly
       50                  55                  60
   Thr Leu Asn Ala Ala Lys Val Pro Ala Asp Thr Glu Val Val Cys Ala
   65                  70                  75                  80
   Pro Pro Thr Ala Tyr Ile Asp Phe Ala Arg Gln Lys Leu Asp Pro Lys
                   85                  90                  95
   Ile Ala Val Ala Ala Gln Asn Cys Tyr Lys Val Thr Asn Gly Ala Phe
               100                 105                 110
   Thr Gly Glu Ile Ser Pro Gly Met Ile Lys Asp Cys Gly Ala Thr Trp
           115                 120                 125
   Val Val Leu Gly His Ser Glu Arg Arg His Val Phe Gly Glu Ser Asp
       130                 135                 140
   Glu Leu Ile Gly Gln Lys Val Ala His Ala Leu Ala Glu Gly Leu Gly
   145                 150                 155                 160
   Val Ile Ala Cys Ile Gly Glu Lys Leu Asp Glu Arg Glu Ala Gly Ile
                   165                 170                 175
   Thr Glu Lys Val Val Phe Glu Gln Thr Lys Val Ile Ala Asp Asn Val
               180                 185                 190
   Lys Asp Trp Ser Lys Val Val Leu Ala Tyr Glu Pro Val Trp Ala Ile
           195                 200                 205
   Gly Thr Gly Lys Thr Ala Thr Pro Gln Gln Ala Gln Glu Val His Glu
       210                 215                 220
   Lys Leu Arg Gly Trp Leu Lys Ser Asn Val Ser Asp Ala Val Ala Gln
   225                 230                 235                 240
   Ser Thr Arg Ile Ile Tyr Gly Gly Ser Val Thr Gly Ala Thr Cys Lys
                   245                 250                 255
   Glu Leu Ala Ser Gln Pro Asp Val Asp Gly Phe Leu Val Gly Gly Ala
   260 265 270
   Ser Leu Lys Pro Glu Phe Val Asp Ile Ile Asn Ala Lys Gln
   275 280 285
<210> 27
   <211> 148
   <212> PRT
   <213> Homo sapiens
<400> 27
<210> 28
   <211> 276
   <212> PRT
   <213> Homo sapiens
<400> 28
<210> 29
   <211> 524
   <212> PRT
   <213> Homo sapiens
<400> 29
<210> 30
   <211> 277
   <212> PRT
   <213> Homo sapiens
<400> 30
   Met Ala Ala Val Ser Val Tyr Ala Pro Pro Val Gly Gly Phe Ser Phe
   1               5                   10                  15
   Asp Asn Cys Arg Arg Asn Ala Val Leu Glu Ala Asp Phe Ala Lys Arg
               20                  25                  30
   Gly Tyr Lys Leu Pro Lys Val Arg Lys Thr Gly Thr Thr Ile Ala Gly
           35                  40                  45
   Val Val Tyr Lys Asp Gly Ile Val Leu Gly Ala Asp Thr Arg Ala Thr
       50                  55                  60
   Glu Gly Met Val Val Ala Asp Lys Asn Cys Ser Lys Ile His Phe Ile
   65                  70                  75                  80
   Ser Pro Asn Ile Tyr Cys Cys Gly Ala Gly Thr Ala Ala Asp Thr Asp
                   85                  90                  95
   Met Thr Thr Gln Leu Ile Ser Ser Asn Leu Glu Leu His Ser Leu Ser
               100                 105                 110
   Thr Gly Arg Leu Pro Arg Val Val Thr Ala Asn Arg Met Leu Lys Gln
           115                 120                 125
   Met Leu Phe Arg Tyr Gln Gly Tyr Ile Gly Ala Ala Leu Val Leu Gly
       130                 135                 140
   Gly Val Asp Val Thr Gly Pro His Leu Tyr Ser Ile Tyr Pro His Gly
   145 150 155 160
   Ser Thr Asp Lys Leu Pro Tyr Val Thr Met Gly Ser Gly Ser Leu Ala
   165 170 175
   Ala Met Ala Val Phe Glu Asp Lys Phe Arg Pro Asp Met Glu Glu Glu
   180 185 190
   Glu Ala Lys Asn Leu Val Ser Glu Ala Ile Ala Ala Gly Ile Phe Asn
   195 200 205
   Asp Leu Gly Ser Gly Ser Asn Ile Asp Leu Cys Val Ile Ser Lys Asn
   210 215 220
   Lys Leu Asp Phe Leu Arg Pro Tyr Thr Val Pro Asn Lys Lys Gly Thr
   225 230 235 240
   Arg Leu Gly Arg Tyr Arg Cys Glu Lys Gly Thr Thr Ala Val Leu Thr
   245 250 255
   Glu Lys Ile Thr Pro Leu Glu Ile Glu Val Leu Glu Glu Thr Val Gln
   260 265 270
   Thr Met Asp Thr Ser
   275
<210> 31
   <211> 286
   <212> PRT
   <213> Homo sapiens
<400> 31
   Met Ala Asp Phe Asp Asp Arg Val Ser Asp Glu Glu Lys Val Arg Ile
   1 5 10 15
   Ala Ala Lys Phe Ile Thr His Ala Pro Pro Gly Glu Phe Asn Glu Val
   20 25 30
   Phe Asn Asp Val Arg Leu Leu Leu Asn Asn Asp Asn Leu Leu Arg Glu
   35 40 45
   Gly Ala Ala His Ala Phe Ala Gln Tyr Asn Met Asp Gln Phe Thr Pro
   50 55 60
   Val Lys Ile Glu Gly Tyr Glu Asp Gln Val Leu Ile Thr Glu His Gly
   65 70 75 80
   Asp Leu Gly Asn Ser Arg Phe Leu Asp Pro Arg Asn Lys Ile Ser Phe
   85 90 95
   Lys Phe Asp His Leu Arg Lys Glu Ala Ser Asp Pro Gln Pro Glu Glu
   100 105 110
   Ala Asp Gly Gly Leu Lys Ser Trp Arg Glu Ser Cys Asp Ser Ala Leu
   115 120 125
   Arg Ala Tyr Val Lys Asp His Tyr Ser Asn Gly Phe Cys Thr Val Tyr
   130 135 140
   Ala Lys Thr Ile Asp Gly Gln Gln Thr Ile Ile Ala Cys Ile Glu Ser
   145 150 155 160
   His Gln Phe Gln Pro Lys Asn Phe Trp Asn Gly Arg Trp Arg Ser Glu
   165 170 175
   Trp Lys Phe Thr Ile Thr Pro Pro Thr Ala Gln Val Val Gly Val Leu
   180 185 190
   Lys Ile Gln Val His Tyr Tyr Glu Asp Gly Asn Val Gln Leu Val Ser
   195 200 205
   His Lys Asp Val Gln Asp Ser Leu Thr Val Ser Asn Glu Ala Gln Thr
   210 215 220
   Ala Lys Glu Phe Ile Lys Ile Ile Glu Asn Ala Glu Asn Glu Tyr Gln
   225 230 235 240
   Thr Ala Ile Ser Glu Asn Tyr Gln Thr Met Ser Asp Thr Thr Phe Lys
   245 250 255
   Ala Leu Arg Arg Gln Leu Pro Val Thr Arg Thr Lys Ile Asp Trp Asn
   260 265 270
   Lys Ile Leu Ser Tyr Lys Ile Gly Lys Glu Met Gln Asn Ala
   275 280 285
<210> 32
   <211> 199
   <212> PRT
   <213> Homo sapiens
<400> 32
   Met Ser Ser Gly Asn Ala Lys Ile Gly His Pro Ala Pro Asn Phe Lys
   1 5 10 15
   Ala Thr Ala Val Met Pro Asp Gly Gln Phe Lys Asp Ile Ser Leu Ser
               20                  25                  30
   Asp Tyr Lys Gly Lys Tyr Val Val Phe Phe Phe Tyr Pro Leu Asp Phe
           35                  40                  45
   Thr Phe Val Cys Pro Thr Glu Ile Ile Ala Phe Ser Asp Arg Ala Glu
       50                  55                  60
   Glu Phe Lys Lys Leu Asn Cys Gln Val Ile Gly Ala Ser Val Asp Ser
   65                  70                  75                  80
   His Phe Cys His Leu Ala Trp Val Asn Thr Pro Lys Lys Gln Gly Gly
                   85                  90                  95
   Leu Gly Pro Met Asn Ile Pro Leu Val Ser Asp Pro Lys Arg Thr Ile
               100                 105                 110
   Ala Gln Asp Tyr Gly Val Leu Lys Ala Asp Glu Gly Ile Ser Phe Arg
           115                 120                 125
   Gly Leu Phe Ile Ile Asp Asp Lys Gly Ile Leu Arg Gln Ile Thr Val
       130                 135                 140
   Asn Asp Leu Pro Val Gly Arg Ser Val Asp Glu Thr Leu Arg Leu Val
   145                 150                 155                 160
   Gln Ala Phe Gln Phe Thr Asp Lys His Gly Glu Val Cys Pro Ala Gly
                   165                 170                 175
   Trp Lys Pro Gly Ser Asp Thr Ile Lys Pro Asp Val Gln Lys Ser Lys
               180                 185                 190
   Glu Tyr Phe Ser Lys Gln Lys
           195
<210> 33
   <211> 613
   <212> PRT
   <213> Homo sapiens
<400> 33
   Met Ala Gln Ser Arg Val Leu Leu Leu Leu Leu Leu Leu Pro Pro Gln
   1               5                   10                  15
   Leu His Leu Gly Pro Val Leu Ala Val Arg Ala Pro Gly Phe Gly Arg
               20                  25                  30
   Ser Gly Gly His Ser Leu Ser Pro Glu Glu Asn Glu Phe Ala Glu Glu
   35 40 45
   Glu Pro Val Leu Val Leu Ser Pro Glu Glu Pro Gly Pro Gly Pro Ala
   50 55 60
   Ala Val Ser Cys Pro Arg Asp Cys Ala Cys Ser Gln Glu Gly Val Val
   65 70 75 80
   Asp Cys Gly Gly Ile Asp Leu Arg Glu Phe Pro Gly Asp Leu Pro Glu
   85 90 95
   His Thr Asn His Leu Ser Leu Gln Asn Asn Gln Leu Glu Lys Ile Tyr
   100 105 110
   Pro Glu Glu Leu Ser Arg Leu His Arg Leu Glu Thr Leu Asn Leu Gln
   115 120 125
   Asn Asn Arg Leu Thr Ser Arg Gly Leu Pro Glu Lys Ala Phe Glu His
   130 135 140
   Leu Thr Asn Leu Asn Tyr Leu Tyr Leu Ala Asn Asn Lys Leu Thr Leu
   145 150 155 160
   Ala Pro Arg Phe Leu Pro Asn Ala Leu Ile Ser Val Asp Phe Ala Ala
   165 170 175
   Asn Tyr Leu Thr Lys Ile Tyr Gly Leu Thr Phe Gly Gln Lys Pro Asn
   180 185 190
   Leu Arg Ser Val Tyr Leu His Asn Asn Lys Leu Ala Asp Ala Gly Leu
   195 200 205
   Pro Asp Asn Met Phe Asn Gly Ser Ser Asn Val Glu Val Leu Ile Leu
   210 215 220
   Ser Ser Asn Phe Leu Arg His Val Pro Lys His Leu Pro Pro Ala Leu
   225 230 235 240
   Tyr Lys Leu His Leu Lys Asn Asn Lys Leu Glu Lys Ile Pro Pro Gly
   245 250 255
   Ala Phe Ser Glu Leu Ser Ser Leu Arg Glu Leu Tyr Leu Gln Asn Asn
   260 265 270
   Tyr Leu Thr Asp Glu Gly Leu Asp Asn Glu Thr Phe Trp Lys Leu Ser
   275 280 285
   Ser Leu Glu Tyr Leu Asp Leu Ser Ser Asn Asn Leu Ser Arg Val Pro
   290 295 300
   Ala Gly Leu Pro Arg Ser Leu Val Leu Leu His Leu Glu Lys Asn Ala
   305 310 315 320
   Ile Arg Ser Val Asp Ala Asn Val Leu Thr Pro Ile Arg Ser Leu Glu
   325 330 335
   Tyr Leu Leu Leu His Ser Asn Gln Leu Arg Glu Gln Gly Ile His Pro
   340 345 350
   Leu Ala Phe Gln Gly Leu Lys Arg Leu His Thr Val His Leu Tyr Asn
   355 360 365
   Asn Ala Leu Glu Arg Val Pro Ser Gly Leu Pro Arg Arg Val Arg Thr
   370 375 380
   Leu Met Ile Leu His Asn Gln Ile Thr Gly Ile Gly Arg Glu Asp Phe
   385 390 395 400
   Ala Thr Thr Tyr Phe Leu Glu Glu Leu Asn Leu Ser Tyr Asn Arg Ile
   405 410 415
   Thr Ser Pro Gln Val His Arg Asp Ala Phe Arg Lys Leu Arg Leu Leu
   420 425 430
   Arg Ser Leu Asp Leu Ser Gly Asn Arg Leu His Thr Leu Pro Pro Gly
   435 440 445
   Leu Pro Arg Asn Val His Val Leu Lys Val Lys Arg Asn Glu Leu Ala
   450 455 460
   Ala Leu Ala Arg Gly Ala Leu Val Gly Met Ala Gln Leu Arg Glu Leu
   465 470 475 480
   Tyr Leu Thr Ser Asn Arg Leu Arg Ser Arg Ala Leu Gly Pro Arg Ala
   485 490 495
   Trp Val Asp Leu Ala His Leu Gln Leu Leu Asp Ile Ala Gly Asn Gln
   500 505 510
   Leu Thr Glu Ile Pro Glu Gly Leu Pro Glu Ser Leu Glu Tyr Leu Tyr
   515 520 525
   Leu Gln Asn Asn Lys Ile Ser Ala Val Pro Ala Asn Ala Phe Asp Ser
   530 535 540
   Thr Pro Asn Leu Lys Gly Ile Phe Leu Arg Phe Asn Lys Leu Ala Val
   545 550 555 560
   Gly Ser Val Val Asp Ser Ala Phe Arg Arg Leu Lys His Leu Gln Val
   565 570 575
   Leu Asp Ile Glu Gly Asn Leu Glu Phe Gly Asp Ile Ser Lys Asp Arg
   580 585 590
   Gly Arg Leu Gly Lys Glu Lys Glu Glu Glu Glu Glu Glu Glu Glu Glu
   595 600 605
   Glu Glu Glu Thr Arg
   610
<210> 34
   <211> 469
   <212> PRT
   <213> Homo sapiens
<400> 34
   Met His Ser Phe Pro Pro Leu Leu Leu Leu Leu Phe Trp Gly Val Val
   1               5                   10                  15
   Ser His Ser Phe Pro Ala Thr Leu Glu Thr Gln Glu Gln Asp Val Asp
               20                  25                  30
   Leu Val Gln Lys Tyr Leu Glu Lys Tyr Tyr Asn Leu Lys Asn Asp Gly
           35                  40                  45
   Arg Gln Val Glu Lys Arg Arg Asn Ser Gly Pro Val Val Glu Lys Leu
       50                  55                  60
   Lys Gln Met Gln Glu Phe Phe Gly Leu Lys Val Thr Gly Lys Pro Asp
   65                  70                  75                  80
   Ala Glu Thr Leu Lys Val Met Lys Gln Pro Arg Cys Gly Val Pro Asp
                   85                  90                  95
   Val Ala Gln Phe Val Leu Thr Glu Gly Asn Pro Arg Trp Glu Gln Thr
               100                 105                 110
   His Leu Thr Tyr Arg Ile Glu Asn Tyr Thr Pro Asp Leu Pro Arg Ala
           115                 120                 125
   Asp Val Asp His Ala Ile Glu Lys Ala Phe Gln Leu Trp Ser Asn Val
   130 135 140
   Thr Pro Leu Thr Phe Thr Lys Val Ser Glu Gly Gln Ala Asp Ile Met
   145 150 155 160
   Ile Ser Phe Val Arg Gly Asp His Arg Asp Asn Ser Pro Phe Asp Gly
   165 170 175
   Pro Gly Gly Asn Leu Ala His Ala Phe Gln Pro Gly Pro Gly Ile Gly
   180 185 190
   Gly Asp Ala His Phe Asp Glu Asp Glu Arg Trp Thr Asn Asn Phe Arg
   195 200 205
   Glu Tyr Asn Leu His Arg Val Ala Ala His Glu Leu Gly His Ser Leu
   210 215 220
   Gly Leu Ser His Ser Thr Asp Ile Gly Ala Leu Met Tyr Pro Ser Tyr
   225 230 235 240
   Thr Phe Ser Gly Asp Val Gln Leu Ala Gln Asp Asp Ile Asp Gly Ile
   245 250 255
   Gln Ala Ile Tyr Gly Arg Ser Gln Asn Pro Val Gln Pro Ile Gly Pro
   260 265 270
   Gln Thr Pro Lys Ala Cys Asp Ser Lys Leu Thr Phe Asp Ala Ile Thr
   275 280 285
   Thr Ile Arg Gly Glu Val Met Phe Phe Lys Asp Arg Phe Tyr Met Arg
   290 295 300
   Thr Asn Pro Phe Tyr Pro Glu Val Glu Leu Asn Phe Ile Ser Val Phe
   305 310 315 320
   Trp Pro Gln Leu Pro Asn Gly Leu Glu Ala Ala Tyr Glu Phe Ala Asp
   325 330 335
   Arg Asp Glu Val Arg Phe Phe Lys Gly Asn Lys Tyr Trp Ala Val Gln
   340 345 350
   Gly Gln Asn Val Leu His Gly Tyr Pro Lys Asp Ile Tyr Ser Ser Phe
   355 360 365
   Gly Phe Pro Arg Thr Val Lys His Ile Asp Ala Ala Leu Ser Glu Glu
   370 375 380
   Asn Thr Gly Lys Thr Tyr Phe Phe Val Ala Asn Lys Tyr Trp Arg Tyr
   385 390 395 400
   Asp Glu Tyr Lys Arg Ser Met Asp Pro Gly Tyr Pro Lys Met Ile Ala
   405 410 415
   His Asp Phe Pro Gly Ile Gly His Lys Val Asp Ala Val Phe Met Lys
   420 425 430
   Asp Gly Phe Phe Tyr Phe Phe His Gly Thr Arg Gln Tyr Lys Phe Asp
   435 440 445
   Pro Lys Thr Lys Arg Ile Leu Thr Leu Gln Lys Ala Asn Ser Trp Phe
   450 455 460
   Asn Cys Arg Lys Asn
   465
<210> 35
   <211> 420
   <212> PRT
   <213> Homo sapiens
<400> 35
   Met Arg Trp Arg Thr Ile Leu Leu Gln Tyr Cys Phe Leu Leu Ile Thr
   1 5 10 15
   Cys Leu Leu Thr Ala Leu Glu Ala Val Pro Ile Asp Ile Asp Lys Thr
   20 25 30
   Lys Val Gln Asn Ile His Pro Val Glu Ser Ala Lys Ile Glu Pro Pro
   35 40 45
   Asp Thr Gly Leu Tyr Tyr Asp Glu Tyr Leu Lys Gln Val Ile Asp Val
   50 55 60
   Leu Glu Thr Asp Lys His Phe Arg Glu Lys Leu Gln Lys Ala Asp Ile
   65 70 75 80
   Glu Glu Ile Lys Ser Gly Arg Leu Ser Lys Glu Leu Asp Leu Val Ser
   85 90 95
   His His Val Arg Thr Lys Leu Asp Glu Leu Lys Arg Gln Glu Val Gly
   100 105 110
   Arg Leu Arg Met Leu Ile Lys Ala Lys Leu Asp Ser Leu Gln Asp Ile
   115 120 125
   Gly Met Asp His Gln Ala Leu Leu Lys Gln Phe Asp His Leu Asn His
       130                 135                 140
   Leu Asn Pro Asp Lys Phe Glu Ser Thr Asp Leu Asp Met Leu Ile Lys
   145                 150                 155                 160
   Ala Ala Thr Ser Asp Leu Glu His Tyr Asp Lys Thr Arg His Glu Glu
                   165                 170                 175
   Phe Lys Lys Tyr Glu Met Met Lys Glu His Glu Arg Arg Glu Tyr Leu
               180                 185                 190
   Lys Thr Leu Asn Glu Glu Lys Arg Lys Glu Glu Glu Ser Lys Phe Glu
           195                 200                 205
   Glu Met Lys Lys Lys His Glu Asn His Pro Lys Val Asn His Pro Gly
       210                 215                 220
   Ser Lys Asp Gln Leu Lys Glu Val Trp Glu Glu Thr Asp Gly Leu Asp
   225                 230                 235                 240
   Pro Asn Asp Phe Asp Pro Lys Thr Phe Phe Lys Leu His Asp Val Asn
                   245                 250                 255
   Ser Asp Gly Phe Leu Asp Glu Gln Glu Leu Glu Ala Leu Phe Thr Lys
               260                 265                 270
   Glu Leu Glu Lys Val Tyr Asp Pro Lys Asn Glu Glu Asp Asp Met Val
           275                 280                 285
   Glu Met Glu Glu Glu Arg Leu Arg Met Arg Glu His Val Met Ser Glu
       290                 295                 300
   Val Asp Thr Asn Lys Asp Arg Leu Val Thr Leu Glu Glu Phe Leu Lys
   305                 310                 315                 320
   Ala Thr Glu Lys Lys Glu Phe Leu Glu Pro Asp Ser Trp Glu Thr Leu
                   325                 330                 335
   Asp Gln Gln Gln Phe Phe Thr Glu Glu Glu Leu Lys Glu Tyr Glu Asn
               340                 345                 350
   Ile Ile Ala Leu Gln Glu Asn Glu Leu Lys Lys Lys Ala Asp Glu Leu
           355                 360                 365
   Gln Lys Gln Lys Glu Glu Leu Gln Arg Gln His Asp Gln Leu Glu Ala
       370                 375                 380
   Gln Lys Leu Glu Tyr His Gln Val Ile Gln Gln Met Glu Gln Lys Lys
   385                 390                 395                 400
   Leu Gln Gln Gly Ile Pro Pro Ser Gly Pro Ala Gly Glu Leu Lys Phe
                   405                 410                 415
   Glu Pro His Ile
               420
<210> 36
   <211> 215
   <212> PRT
   <213> Homo sapiens
<400> 36
   Met Gly Lys Gly Asp Pro Lys Lys Pro Arg Gly Lys Met Ser Ser Tyr
   1               5                   10                  15
   Ala Phe Phe Val Gln Thr Cys Arg Glu Glu His Lys Lys Lys His Pro
               20                  25                  30
   Asp Ala Ser Val Asn Phe Ser Glu Phe Ser Lys Lys Cys Ser Glu Arg
           35                  40                  45
   Trp Lys Thr Met Ser Ala Lys Glu Lys Gly Lys Phe Glu Asp Met Ala
       50                  55                  60
   Lys Ala Asp Lys Ala Arg Tyr Glu Arg Glu Met Lys Thr Tyr Ile Pro
   65                  70                  75                  80
   Pro Lys Gly Glu Thr Lys Lys Lys Phe Lys Asp Pro Asn Ala Pro Lys
                   85                  90                  95
   Arg Pro Pro Ser Ala Phe Phe Leu Phe Cys Ser Glu Tyr Arg Pro Lys
               100                 105                 110
   Ile Lys Gly Glu His Pro Gly Leu Ser Ile Gly Asp Val Ala Lys Lys
           115                 120                 125
   Leu Gly Glu Met Trp Asn Asn Thr Ala Ala Asp Asp Lys Gln Pro Tyr
       130                 135                 140
   Glu Lys Lys Ala Ala Lys Leu Lys Glu Lys Tyr Glu Lys Asp Ile Ala
   145                 150                 155                 160
   Ala Tyr Arg Ala Lys Gly Lys Pro Asp Ala Ala Lys Lys Gly Val Val
                   165                 170                 175
   Lys Ala Glu Lys Ser Lys Lys Lys Lys Glu Glu Glu Glu Asp Glu Glu
   180 185 190
   Asp Glu Glu Asp Glu Glu Glu Glu Glu Asp Glu Glu Asp Glu Asp Glu
   195 200 205
   Glu Glu Asp Asp Asp Asp Glu
   210 215
<210> 37
   <211> 5890
   <212> PRT
   <213> Homo sapiens
<400> 37
   Met Glu Lys Glu Glu Thr Thr Arg Glu Leu Leu Leu Pro Asn Trp Gln
   1 5 10 15
   Gly Ser Gly Ser His Gly Leu Thr Ile Ala Gln Arg Asp Asp Gly Val
   20 25 30
   Phe Val Gln Glu Val Thr Gln Asn Ser Pro Ala Ala Arg Thr Gly Val
   35 40 45
   Val Lys Glu Gly Asp Gln Ile Val Gly Ala Thr Ile Tyr Phe Asp Asn
   50 55 60
   Leu Gln Ser Gly Glu Val Thr Gln Leu Leu Asn Thr Met Gly His His
   65 70 75 80
   Thr Val Gly Leu Lys Leu His Arg Lys Gly Asp Arg Ser Pro Glu Pro
   85 90 95
   Gly Gln Thr Trp Thr Arg Glu Val Phe Ser Ser Cys Ser Ser Glu Val
   100 105 110
   Val Leu Ser Gly Asp Asp Glu Glu Tyr Gln Arg Ile Tyr Thr Thr Lys
   115 120 125
   Ile Lys Pro Arg Leu Lys Ser Glu Asp Gly Val Glu Gly Asp Leu Gly
   130 135 140
   Glu Thr Gln Ser Arg Thr Ile Thr Val Thr Arg Arg Val Thr Ala Tyr
   145 150 155 160
   Thr Val Asp Val Thr Gly Arg Glu Gly Ala Lys Asp Ile Asp Ile Ser
   165 170 175
   Ser Pro Glu Phe Lys Ile Lys Ile Pro Arg His Glu Leu Thr Glu Ile
   180 185 190
   Ser Asn Val Asp Val Glu Thr Gln Ser Gly Lys Thr Val Ile Arg Leu
   195 200 205
   Pro Ser Gly Ser Gly Ala Ala Ser Pro Thr Gly Ser Ala Val Asp Ile
   210 215 220
   Arg Ala Gly Ala Ile Ser Ala Ser Gly Pro Glu Leu Gln Gly Ala Gly
   225 230 235 240
   His Ser Lys Leu Gln Val Thr Met Pro Gly Ile Lys Val Gly Gly Ser
   245 250 255
   Gly Val Asn Val Asn Ala Lys Gly Leu Asp Leu Gly Gly Arg Gly Gly
   260 265 270
   Val Gln Val Pro Ala Val Asp Ile Ser Ser Ser Leu Gly Gly Arg Ala
   275 280 285
   Val Glu Val Gln Gly Pro Ser Leu Glu Ser Gly Asp His Gly Lys Ile
   290 295 300
   Lys Phe Pro Thr Met Lys Val Pro Lys Phe Gly Val Ser Thr Gly Arg
   305 310 315 320
   Glu Gly Gln Thr Pro Lys Ala Gly Leu Arg Val Ser Ala Pro Glu Val
   325 330 335
   Ser Val Gly His Lys Gly Gly Lys Pro Gly Leu Thr Ile Gln Ala Pro
   340 345 350
   Gln Leu Glu Val Ser Val Pro Ser Ala Asn Ile Glu Gly Leu Glu Gly
   355 360 365
   Lys Leu Lys Gly Pro Gln Ile Thr Gly Pro Ser Leu Glu Gly Asp Leu
   370 375 380
   Gly Leu Lys Gly Ala Lys Pro Gln Gly His Ile Gly Val Asp Ala Ser
   385 390 395 400
   Ala Pro Gln Ile Gly Gly Ser Ile Thr Gly Pro Ser Val Glu Val Gln
   405 410 415
   Ala Pro Asp Ile Asp Val Gln Gly Pro Gly Ser Lys Leu Asn Val Pro
   420 425 430
   Lys Met Lys Val Pro Lys Phe Ser Val Ser Gly Ala Lys Gly Glu Glu
   435 440 445
   Thr Gly Ile Asp Val Thr Leu Pro Thr Gly Glu Val Thr Val Pro Gly
   450 455 460
   Val Ser Gly Asp Val Ser Leu Pro Glu Ile Ala Thr Gly Gly Leu Glu
   465 470 475 480
   Gly Lys Met Lys Gly Thr Lys Val Lys Thr Pro Glu Met Ile Ile Gln
   485 490 495
   Lys Pro Lys Ile Ser Met Gln Asp Val Asp Leu Ser Leu Gly Ser Pro
   500 505 510
   Lys Leu Lys Gly Asp Ile Lys Val Ser Ala Pro Gly Val Gln Gly Asp
   515 520 525
   Val Lys Gly Pro Gln Val Ala Leu Lys Gly Ser Arg Val Asp Ile Glu
   530 535 540
   Thr Pro Asn Leu Glu Gly Thr Leu Thr Gly Pro Arg Leu Gly Ser Pro
   545 550 555 560
   Ser Gly Lys Thr Gly Thr Cys Arg Ile Ser Met Ser Glu Val Asp Leu
   565 570 575
   Asn Val Ala Ala Pro Lys Val Lys Gly Gly Val Asp Val Thr Leu Pro
   580 585 590
   Arg Val Glu Gly Lys Val Lys Val Pro Glu Val Asp Val Arg Gly Pro
   595 600 605
   Lys Val Asp Val Ser Ala Pro Asp Val Glu Ala His Gly Pro Glu Trp
   610 615 620
   Asn Leu Lys Met Pro Lys Met Lys Met Pro Thr Phe Ser Thr Pro Gly
   625 630 635 640
   Ala Lys Gly Glu Gly Pro Asp Val His Met Thr Leu Pro Lys Gly Asp
   645 650 655
   Ile Ser Ile Ser Gly Pro Lys Val Asn Val Glu Ala Pro Asp Val Asn
   660 665 670
   Leu Glu Gly Leu Gly Gly Lys Leu Lys Gly Pro Asp Val Lys Leu Pro
   675 680 685
   Asp Met Ser Val Lys Thr Pro Lys Ile Ser Met Pro Asp Val Asp Leu
   690 695 700
   His Val Lys Gly Thr Lys Val Lys Gly Glu Tyr Asp Val Thr Val Pro
   705 710 715 720
   Lys Leu Glu Gly Glu Leu Lys Gly Pro Lys Val Asp Ile Asp Ala Pro
   725 730 735
   Asp Val Asp Val His Gly Pro Asp Trp His Leu Lys Met Pro Lys Met
   740 745 750
   Lys Met Pro Lys Phe Ser Val Pro Gly Phe Lys Ala Glu Gly Pro Glu
   755 760 765
   Val Asp Val Asn Leu Pro Lys Ala Asp Val Asp Ile Ser Gly Pro Lys
   770 775 780
   Ile Asp Val Thr Ala Pro Asp Val Ser Ile Glu Glu Pro Glu Gly Lys
   785 790 795 800
   Leu Lys Gly Pro Lys Phe Lys Met Pro Glu Met Asn Ile Lys Val Pro
   805 810 815
   Lys Ile Ser Met Pro Asp Val Asp Leu His Leu Lys Gly Pro Asn Val
   820 825 830
   Lys Gly Glu Tyr Asp Val Thr Met Pro Lys Val Glu Ser Glu Ile Lys
   835 840 845
   Val Pro Asp Val Glu Leu Lys Ser Ala Lys Met Asp Ile Asp Val Pro
   850 855 860
   Asp Val Glu Val Gln Gly Pro Asp Trp His Leu Lys Met Pro Lys Met
   865 870 875 880
   Lys Met Pro Lys Phe Ser Met Pro Gly Phe Lys Ala Glu Gly Pro Glu
   885 890 895
   Val Asp Val Asn Leu Pro Lys Ala Asp Val Asp Ile Ser Gly Pro Lys
   900 905 910
   Val Gly Val Glu Val Pro Asp Val Asn Ile Glu Gly Pro Glu Gly Lys
   915 920 925
   Leu Lys Gly Pro Lys Phe Lys Met Pro Glu Met Asn Ile Lys Ala Pro
       930                 935                 940
   Lys Ile Ser Met Pro Asp Val Asp Leu His Met Lys Gly Pro Lys Val
   945                 950                 955                 960
   Lys Gly Glu Tyr Asp Met Thr Val Pro Lys Leu Glu Gly Asp Leu Lys
                   965                 970                 975
   Gly Pro Lys Val Asp Val Ser Ala Pro Asp Val Glu Met Gln Gly Pro
               980                 985                 990
   Asp Trp Asn Leu Lys Met Pro Lys  Ile Lys Met Pro Lys  Phe Ser Met
           995                 1000                 1005
   Pro Ser  Leu Lys Gly Glu Gly  Pro Glu Phe Asp Val  Asn Leu Ser
       1010                 1015                 1020
   Lys Ala  Asn Val Asp Ile Ser  Ala Pro Lys Val Asp  Thr Asn Ala
       1025                 1030                 1035
   Pro Asp  Leu Ser Leu Glu Gly  Pro Glu Gly Lys Leu  Lys Gly Pro
       1040                 1045                 1050
   Lys Phe  Lys Met Pro Glu Met  His Phe Arg Ala Pro  Lys Met Ser
       1055                 1060                 1065
   Leu Pro  Asp Val Asp Leu Asp  Leu Lys Gly Pro Lys  Met Lys Gly
       1070                 1075                 1080
   Asn Val  Asp Ile Ser Ala Pro  Lys Ile Glu Gly Glu  Met Gln Val
       1085                 1090                 1095
   Pro Asp  Val Asp Ile Arg Gly  Pro Lys Val Asp Ile  Lys Ala Pro
       1100                 1105                 1110
   Asp Val  Glu Gly Gln Gly Leu  Asp Trp Ser Leu Lys  Ile Pro Lys
       1115                 1120                 1125
   Met Lys  Met Pro Lys Phe Ser  Met Pro Ser Leu Lys  Gly Glu Gly
       1130                 1135                 1140
   Pro Glu  Val Asp Val Asn Leu  Pro Lys Ala Asp Val  Val Val Ser
       1145                 1150                 1155
   Gly Pro  Lys Val Asp Ile Glu  Ala Pro Asp Val Ser  Leu Glu Gly
       1160                 1165                 1170
   Pro Glu Gly Lys Leu Lys Gly Pro Lys Phe Lys Met Pro Glu Met
   1175 1180 1185
   His Phe Lys Thr Pro Lys Ile Ser Met Pro Asp Val Asp Leu His
   1190 1195 1200
   Leu Lys Gly Pro Lys Val Lys Gly Asp Val Asp Val Ser Val Pro
   1205 1210 1215
   Lys Val Glu Gly Glu Met Lys Val Pro Asp Val Glu Ile Lys Gly
   1220 1225 1230
   Pro Lys Met Asp Ile Asp Ala Pro Asp Val Glu Val Gln Gly Pro
   1235 1240 1245
   Asp Trp His Leu Lys Met Pro Lys Met Lys Met Pro Lys Phe Ser
   1250 1255 1260
   Met Pro Gly Phe Lys Gly Glu Gly Arg Glu Val Asp Val Asn Leu
   1265 1270 1275
   Pro Lys Ala Asp Ile Asp Val Ser Gly Pro Lys Val Asp Val Glu
   1280 1285 1290
   Val Pro Asp Val Ser Leu Glu Gly Pro Glu Gly Lys Leu Lys Gly
   1295 1300 1305
   Pro Lys Phe Lys Met Pro Glu Met His Phe Lys Ala Pro Lys Ile
   1310 1315 1320
   Ser Met Pro Asp Val Asp Leu Asn Leu Lys Gly Pro Lys Leu Lys
   1325 1330 1335
   Gly Asp Val Asp Val Ser Leu Pro Glu Val Glu Gly Glu Met Lys
   1340 1345 1350
   Val Pro Asp Val Asp Ile Lys Gly Pro Lys Val Asp Ile Ser Ala
   1355 1360 1365
   Pro Asp Val Asp Val His Gly Pro Asp Trp His Leu Lys Met Pro
   1370 1375 1380
   Lys Val Lys Met Pro Lys Phe Ser Met Pro Gly Phe Lys Gly Glu
   1385 1390 1395
   Gly Pro Glu Val Asp Val Lys Leu Pro Lys Ala Asp Val Asp Val
   1400 1405 1410
   Ser Gly Pro Lys Met Asp Ala Glu Val Pro Asp Val Asn Ile Glu
   1415 1420 1425
   Gly Pro Asp Ala Lys Leu Lys Gly Pro Lys Phe Lys Met Pro Glu
   1430 1435 1440
   Met Ser Ile Lys Pro Gln Lys Ile Ser Ile Pro Asp Val Gly Leu
   1445 1450 1455
   His Leu Lys Gly Pro Lys Met Lys Gly Asp Tyr Asp Val Thr Val
   1460 1465 1470
   Pro Lys Val Glu Gly Glu Ile Lys Ala Pro Asp Val Asp Ile Lys
   1475 1480 1485
   Gly Pro Lys Val Asp Ile Asn Ala Pro Asp Val Glu Val His Gly
   1490 1495 1500
   Pro Asp Trp His Leu Lys Met Pro Lys Val Lys Met Pro Lys Phe
   1505 1510 1515
   Ser Met Pro Gly Phe Lys Gly Glu Gly Pro Glu Val Asp Met Asn
   1520 1525 1530
   Leu Pro Lys Ala Asp Leu Gly Val Ser Gly Pro Lys Val Asp Ile
   1535 1540 1545
   Asp Val Pro Asp Val Asn Leu Glu Ala Pro Glu Gly Lys Leu Lys
   1550 1555 1560
   Gly Pro Lys Phe Lys Met Pro Ser Met Asn Ile Gln Thr His Lys
   1565 1570 1575
   Ile Ser Met Pro Asp Val Gly Leu Asn Leu Lys Ala Pro Lys Leu
   1580 1585 1590
   Lys Thr Asp Val Asp Val Ser Leu Pro Lys Val Glu Gly Asp Leu
   1595 1600 1605
   Lys Gly Pro Glu Ile Asp Val Lys Ala Pro Lys Met Asp Val Asn
   1610 1615 1620
   Val Gly Asp Ile Asp Ile Glu Gly Pro Glu Gly Lys Leu Lys Gly
   1625 1630 1635
   Pro Lys Phe Lys Met Pro Glu Met His Phe Lys Ala Pro Lys Ile
   1640 1645 1650
   Ser Met Pro Asp Val Asp Leu His Leu Lys Gly Pro Lys Val Lys
   1655 1660 1665
   Gly Asp Met Asp Val Ser Val Pro Lys Val Glu Gly Glu Met Lys
   1670 1675 1680
   Val Pro Asp Val Asp Ile Lys Gly Pro Lys Val Asp Ile Asp Ala
   1685 1690 1695
   Pro Asp Val Glu Val His Asp Pro Asp Trp His Leu Lys Met Pro
   1700 1705 1710
   Lys Met Lys Met Pro Lys Phe Ser Met Pro Gly Phe Lys Ala Glu
   1715 1720 1725
   Gly Pro Glu Val Asp Val Asn Leu Pro Lys Ala Asp Ile Asp Val
   1730 1735 1740
   Ser Gly Pro Ser Val Asp Thr Asp Ala Pro Asp Leu Asp Ile Glu
   1745 1750 1755
   Gly Pro Glu Gly Lys Leu Lys Gly Ser Lys Phe Lys Met Pro Lys
   1760 1765 1770
   Leu Asn Ile Lys Ala Pro Lys Val Ser Met Pro Asp Val Asp Leu
   1775 1780 1785
   Asn Leu Lys Gly Pro Lys Leu Lys Gly Glu Ile Asp Ala Ser Val
   1790 1795 1800
   Pro Glu Leu Glu Gly Asp Leu Arg Gly Pro Gln Val Asp Val Lys
   1805 1810 1815
   Gly Pro Phe Val Glu Ala Glu Val Pro Asp Val Asp Leu Glu Cys
   1820 1825 1830
   Pro Asp Ala Lys Leu Lys Gly Pro Lys Phe Lys Met Pro Glu Met
   1835 1840 1845
   His Phe Lys Ala Pro Lys Ile Ser Met Pro Asp Val Asp Leu His
   1850 1855 1860
   Leu Lys Gly Pro Lys Val Lys Gly Asp Ala Asp Val Ser Val Pro
   1865 1870 1875
   Lys Leu  Glu Gly Asp Leu Thr  Gly Pro Ser Val Gly  Val Glu Val
       1880                 1885                 1890
   Pro Asp  Val Glu Leu Glu Cys  Pro Asp Ala Lys Leu  Lys Gly Pro
       1895                 1900                 1905
   Lys Phe  Lys Met Pro Asp Met  His Phe Lys Ala Pro  Lys Ile Ser
       1910                 1915                 1920
   Met Pro  Asp Val Asp Leu His  Leu Lys Gly Pro Lys  Val Lys Gly
       1925                 1930                 1935
   Asp Val  Asp Val Ser Val Pro  Lys Leu Glu Gly Asp  Leu Thr Gly
       1940                 1945                 1950
   Pro Ser  Val Gly Val Glu Val  Pro Asp Val Glu Leu  Glu Cys Pro
       1955                 1960                 1965
   Asp Ala  Lys Leu Lys Gly Pro  Lys Phe Lys Met Pro  Glu Met His
       1970                 1975                 1980
   Phe Lys  Thr Pro Lys Ile Ser  Met Pro Asp Val Asp  Leu His Leu
       1985                 1990                 1995
   Lys Gly  Pro Lys Val Lys Gly  Asp Met Asp Val Ser  Val Pro Lys
       2000                 2005                 2010
   Val Glu  Gly Glu Met Lys Val  Pro Asp Val Asp Ile  Lys Gly Pro
       2015                 2020                 2025
   Lys Met  Asp Ile Asp Ala Pro  Asp Val Asp Val His  Gly Pro Asp
       2030                 2035                 2040
   Trp His  Leu Lys Met Pro Lys  Met Lys Met Pro Lys  Phe Ser Met
       2045                 2050                 2055
   Pro Gly  Phe Lys Ala Glu Gly  Pro Glu Val Asp Val  Asn Leu Pro
       2060                 2065                 2070
   Lys Ala  Asp Val Val Val Ser  Gly Pro Lys Val Asp  Val Glu Val
       2075                 2080                 2085
   Pro Asp  Val Ser Leu Glu Gly  Pro Glu Gly Lys Leu  Lys Gly Pro
       2090                 2095                 2100
   Lys Leu  Lys Met Pro Glu Met  His Phe Lys Ala Pro  Lys Ile Ser
       2105                 2110                 2115
   Met Pro Asp Val Asp Leu His Leu Lys Gly Pro Lys Val Lys Gly
   2120 2125 2130
   Asp Val Asp Val Ser Leu Pro Lys Leu Glu Gly Asp Leu Thr Gly
   2135 2140 2145
   Pro Ser Val Asp Val Glu Val Pro Asp Val Glu Leu Glu Cys Pro
   2150 2155 2160
   Asp Ala Lys Leu Lys Gly Pro Lys Phe Lys Met Pro Glu Met His
   2165 2170 2175
   Phe Lys Thr Pro Lys Ile Ser Met Pro Asp Val Asn Leu Asn Leu
   2180 2185 2190
   Lys Gly Pro Lys Val Lys Gly Asp Met Asp Val Ser Val Pro Lys
   2195 2200 2205
   Val Glu Gly Glu Met Lys Val Pro Asp Val Asp Ile Arg Gly Pro
   2210 2215 2220
   Lys Val Asp Ile Asp Ala Pro Asp Val Asp Val His Gly Pro Asp
   2225 2230 2235
   Trp His Leu Lys Met Pro Lys Met Lys Met Pro Lys Phe Ser Met
   2240 2245 2250
   Pro Gly Phe Lys Gly Glu Gly Pro Glu Val Asp Val Asn Leu Pro
   2255 2260 2265
   Lys Ala Asp Val Asp Val Ser Gly Pro Lys Val Asp Val Glu Val
   2270 2275 2280
   Pro Asp Val Ser Leu Glu Gly Pro Glu Gly Lys Leu Lys Gly Pro
   2285 2290 2295
   Lys Phe Lys Met Pro Glu Met His Phe Lys Thr Pro Lys Ile Ser
   2300 2305 2310
   Met Pro Asp Val Asp Phe Asn Leu Lys Gly Pro Lys Ile Lys Gly
   2315 2320 2325
   Asp Val Asp Val Ser Ala Pro Lys Leu Glu Gly Glu Leu Lys Gly
   2330 2335 2340
   Pro Glu Leu Asp Val Lys Gly Pro Lys Leu Asp Ala Asp Met Pro
   2345 2350 2355
   Glu Val Ala Val Glu Gly Pro Asn Gly Lys Trp Lys Thr Pro Lys
   2360 2365 2370
   Phe Lys Met Pro Asp Met His Phe Lys Ala Pro Lys Ile Ser Met
   2375 2380 2385
   Pro Asp Leu Asp Leu His Leu Lys Ser Pro Lys Ala Lys Gly Glu
   2390 2395 2400
   Val Asp Val Asp Val Pro Lys Leu Glu Gly Asp Leu Lys Gly Pro
   2405 2410 2415
   His Val Asp Val Ser Gly Pro Asp Ile Asp Ile Glu Gly Pro Glu
   2420 2425 2430
   Gly Lys Leu Lys Gly Pro Lys Phe Lys Met Pro Asp Met His Phe
   2435 2440 2445
   Lys Ala Pro Asn Ile Ser Met Pro Asp Val Asp Leu Asn Leu Lys
   2450 2455 2460
   Gly Pro Lys Ile Lys Gly Asp Val Asp Val Ser Val Pro Glu Val
   2465 2470 2475
   Glu Gly Lys Leu Glu Val Pro Asp Met Asn Ile Arg Gly Pro Lys
   2480 2485 2490
   Val Asp Val Asn Ala Pro Asp Val Gln Ala Pro Asp Trp His Leu
   2495 2500 2505
   Lys Met Pro Lys Met Lys Met Pro Lys Phe Ser Met Pro Gly Phe
   2510 2515 2520
   Lys Ala Glu Gly Pro Glu Val Asp Val Asn Leu Pro Lys Ala Asp
   2525 2530 2535
   Val Asp Ile Ser Gly Pro Lys Val Asp Ile Glu Gly Pro Asp Val
   2540 2545 2550
   Asn Ile Glu Gly Pro Glu Gly Lys Leu Lys Gly Pro Lys Leu Lys
   2555 2560 2565
   Met Pro Glu Met Asn Ile Lys Ala Pro Lys Ile Ser Met Pro Asp
   2570 2575 2580
   Phe Asp Leu His Leu Lys Gly Pro Lys Val Lys Gly Asp Val Asp
   2585 2590 2595
   Val Ser Leu Pro Lys Val Glu Gly Asp Leu Lys Gly Pro Glu Val
   2600 2605 2610
   Asp Ile Lys Gly Pro Lys Val Asp Ile Asn Ala Pro Asp Val Gly
   2615 2620 2625
   Val Gln Gly Pro Asp Trp His Leu Lys Met Pro Lys Val Lys Met
   2630 2635 2640
   Pro Lys Phe Ser Met Pro Gly Phe Lys Gly Glu Gly Pro Asp Gly
   2645 2650 2655
   Asp Val Lys Leu Pro Lys Ala Asp Ile Asp Val Ser Gly Pro Lys
   2660 2665 2670
   Val Asp Ile Glu Gly Pro Asp Val Asn Ile Glu Gly Pro Glu Gly
   2675 2680 2685
   Lys Leu Lys Gly Pro Lys Phe Lys Met Pro Glu Met Asn Ile Lys
   2690 2695 2700
   Ala Pro Lys Ile Ser Met Pro Asp Ile Asp Leu Asn Leu Lys Gly
   2705 2710 2715
   Pro Lys Val Lys Gly Asp Val Asp Val Ser Leu Pro Lys Val Glu
   2720 2725 2730
   Gly Asp Leu Lys Gly Pro Glu Val Asp Ile Lys Gly Pro Lys Val
   2735 2740 2745
   Asp Ile Asp Ala Pro Asp Val Asp Val His Gly Pro Asp Trp His
   2750 2755 2760
   Leu Lys Met Pro Lys Ile Lys Met Pro Lys Ile Ser Met Pro Gly
   2765 2770 2775
   Phe Lys Gly Glu Gly Pro Asp Val Asp Val Asn Leu Pro Lys Ala
   2780 2785 2790
   Asp Ile Asp Val Ser Gly Pro Lys Val Asp Val Glu Cys Pro Asp
   2795 2800 2805
   Val Asn Ile Glu Gly Pro Glu Gly Lys Trp Lys Ser Pro Lys Phe
   2810 2815 2820
   Lys Met Pro Glu Met His Phe Lys Thr Pro Lys Ile Ser Met Pro
   2825 2830 2835
   Asp Ile Asp Leu Asn Leu Thr Gly Pro Lys Ile Lys Gly Asp Val
   2840 2845 2850
   Asp Val Thr Gly Pro Lys Val Glu Gly Asp Leu Lys Gly Pro Glu
   2855 2860 2865
   Val Asp Leu Lys Gly Pro Lys Val Asp Ile Asp Val Pro Asp Val
   2870 2875 2880
   Asn Val Gln Gly Pro Asp Trp His Leu Lys Met Pro Lys Met Lys
   2885 2890 2895
   Met Pro Lys Phe Ser Met Pro Gly Phe Lys Ala Glu Gly Pro Glu
   2900 2905 2910
   Val Asp Val Asn Leu Pro Lys Ala Asp Val Asp Val Ser Gly Pro
   2915 2920 2925
   Lys Val Asp Val Glu Gly Pro Asp Val Asn Ile Glu Gly Pro Glu
   2930 2935 2940
   Gly Lys Leu Lys Gly Pro Lys Phe Lys Met Pro Glu Met Asn Ile
   2945 2950 2955
   Lys Ala Pro Lys Ile Pro Met Pro Asp Phe Asp Leu His Leu Lys
   2960 2965 2970
   Gly Pro Lys Val Lys Gly Asp Val Asp Ile Ser Leu Pro Lys Val
   2975 2980 2985
   Glu Gly Asp Leu Lys Gly Pro Glu Val Asp Ile Arg Gly Pro Gln
   2990 2995 3000
   Val Asp Ile Asp Val Pro Asp Val Gly Val Gln Gly Pro Asp Trp
   3005 3010 3015
   His Leu Lys Met Pro Lys Val Lys Met Pro Lys Phe Ser Met Pro
   3020 3025 3030
   Gly Phe Lys Gly Glu Gly Pro Asp Val Asp Val Asn Leu Pro Lys
   3035 3040 3045
   Ala Asp Leu Asp Val Ser Gly Pro Lys Val Asp Ile Asp Val Pro
   3050 3055 3060
   Asp Val Asn Ile Glu Gly Pro Glu Gly Lys Leu Lys Gly Pro Lys
   3065 3070 3075
   Phe Lys Met Pro Glu Met Asn Ile Lys Ala Pro Lys Ile Ser Met
   3080 3085 3090
   Pro Asp Ile Asp Leu Asn Leu Lys Gly Pro Lys Val Lys Gly Asp
   3095 3100 3105
   Met Asp Val Ser Leu Pro Lys Val Glu Gly Asp Met Lys Val Pro
   3110 3115 3120
   Asp Val Asp Ile Lys Gly Pro Lys Val Asp Ile Asn Ala Pro Asp
   3125 3130 3135
   Val Asp Val Gln Gly Pro Asp Trp His Leu Lys Met Pro Lys Ile
   3140 3145 3150
   Lys Met Pro Lys Ile Ser Met Pro Gly Phe Lys Gly Glu Gly Pro
   3155 3160 3165
   Glu Val Asp Val Asn Leu Pro Lys Ala Asp Leu Asp Val Ser Gly
   3170 3175 3180
   Pro Lys Val Asp Val Asp Val Pro Asp Val Asn Ile Glu Gly Pro
   3185 3190 3195
   Asp Ala Lys Leu Lys Gly Pro Lys Phe Lys Met Pro Glu Met Asn
   3200 3205 3210
   Ile Lys Ala Pro Lys Ile Ser Met Pro Asp Leu Asp Leu Asn Leu
   3215 3220 3225
   Lys Gly Pro Lys Met Lys Gly Glu Val Asp Val Ser Leu Ala Asn
   3230 3235 3240
   Val Glu Gly Asp Leu Lys Gly Pro Ala Leu Asp Ile Lys Gly Pro
   3245 3250 3255
   Lys Ile Asp Val Asp Ala Pro Asp Ile Asp Ile His Gly Pro Asp
   3260 3265 3270
   Ala Lys Leu Lys Gly Pro Lys Leu Lys Met Pro Asp Met His Val
   3275 3280 3285
   Asn Met Pro Lys Ile Ser Met Pro Glu Ile Asp Leu Asn Leu Lys
   3290 3295 3300
   Gly Ser Lys Leu Lys Gly Asp Val Asp Val Ser Gly Pro Lys Leu
   3305 3310 3315
   Glu Gly Asp Ile Lys Ala Pro Ser Leu Asp Ile Lys Gly Pro Glu
   3320 3325 3330
   Val Asp Val Ser Gly Pro Lys Leu Asn Ile Glu Gly Lys Ser Lys
   3335 3340 3345
   Lys Ser Arg Phe Lys Leu Pro Lys Phe Asn Phe Ser Gly Ser Lys
   3350 3355 3360
   Val Gln Thr Pro Glu Val Asp Val Lys Gly Lys Lys Pro Asp Ile
   3365 3370 3375
   Asp Ile Thr Gly Pro Lys Val Asp Ile Asn Ala Pro Asp Val Glu
   3380 3385 3390
   Val Gln Gly Lys Val Lys Gly Ser Lys Phe Lys Met Pro Phe Leu
   3395 3400 3405
   Ser Ile Ser Ser Pro Lys Val Ser Met Pro Asp Val Glu Leu Asn
   3410 3415 3420
   Leu Lys Ser Pro Lys Val Lys Gly Asp Leu Asp Ile Ala Gly Pro
   3425 3430 3435
   Asn Leu Glu Gly Asp Phe Lys Gly Pro Lys Val Asp Ile Lys Ala
   3440 3445 3450
   Pro Glu Val Asn Leu Asn Ala Pro Asp Val Asp Val His Gly Pro
   3455 3460 3465
   Asp Trp Asn Leu Lys Met Pro Lys Met Lys Met Pro Lys Phe Ser
   3470 3475 3480
   Val Ser Gly Leu Lys Ala Glu Gly Pro Asp Val Ala Val Asp Leu
   3485 3490 3495
   Pro Lys Gly Asp Ile Asn Ile Glu Gly Pro Ser Met Asn Ile Glu
   3500 3505 3510
   Gly Pro Asp Leu Asn Val Glu Gly Pro Glu Gly Gly Leu Lys Gly
   3515 3520 3525
   Pro Lys Phe Lys Met Pro Asp Met Asn Ile Lys Ala Pro Lys Ile
   3530 3535 3540
   Ser Met Pro Asp Ile Asp Leu Asn Leu Lys Gly Pro Lys Val Lys
   3545 3550 3555
   Gly Asp Val Asp Ile Ser Leu Pro Lys Leu Glu Gly Asp Leu Lys
   3560 3565 3570
   Gly Pro Glu Val Asp Ile Lys Gly Pro Lys Val Asp Ile Asn Ala
   3575 3580 3585
   Pro Asp Val Asp Val His Gly Pro Asp Trp His Leu Lys Met Pro
   3590 3595 3600
   Lys Val Lys Met Pro Lys Phe Ser Met Pro Gly Phe Lys Gly Glu
   3605 3610 3615
   Gly Pro Glu Val Asp Val Thr Leu Pro Lys Ala Asp Ile Asp Ile
   3620 3625 3630
   Ser Gly Pro Asn Val Asp Val Asp Val Pro Asp Val Asn Ile Glu
   3635 3640 3645
   Gly Pro Asp Ala Lys Leu Lys Gly Pro Lys Phe Lys Met Pro Glu
   3650 3655 3660
   Met Asn Ile Lys Ala Pro Lys Ile Ser Met Pro Asp Phe Asp Leu
   3665 3670 3675
   Asn Leu Lys Gly Pro Lys Met Lys Gly Asp Val Val Val Ser Leu
   3680 3685 3690
   Pro Lys Val Glu Gly Asp Leu Lys Gly Pro Glu Val Asp Ile Lys
   3695 3700 3705
   Gly Pro Lys Val Asp Ile Asp Thr Pro Asp Ile Asn Ile Glu Gly
   3710 3715 3720
   Ser Glu Gly Lys Phe Lys Gly Pro Lys Phe Lys Ile Pro Glu Met
   3725 3730 3735
   His Leu Lys Ala Pro Lys Ile Ser Met Pro Asp Ile Asp Leu Asn
   3740 3745 3750
   Leu Lys Gly Pro Lys Val Lys Gly Asp Val Asp Val Ser Leu Pro
   3755 3760 3765
   Lys Met Glu Gly Asp Leu Lys Gly Pro Glu Val Asp Ile Lys Gly
   3770 3775 3780
   Pro Lys Val Asp Ile Asn Ala Pro Asp Val Asp Val Gln Gly Pro
   3785 3790 3795
   Asp Trp His Leu Lys Met Pro Lys Val Lys Met Pro Lys Phe Ser
   3800 3805 3810
   Met Pro Gly Phe Lys Gly Glu Gly Pro Asp Val Asp Val Asn Leu
   3815 3820 3825
   Pro Lys Ala Asp Leu Asp Val Ser Gly Pro Lys Val Asp Ile Asp
   3830 3835 3840
   Val Pro Asp Val Asn Ile Glu Gly Pro Glu Gly Lys Leu Lys Gly
   3845 3850 3855
   Pro Lys Phe Lys Met Pro Glu Met Asn Ile Lys Ala Pro Lys Ile
   3860 3865 3870
   Ser Met Pro Asp Ile Asp Leu Asn Leu Lys Gly Pro Lys Val Lys
   3875 3880 3885
   Gly Asp Met Asp Val Ser Leu Pro Lys Val Glu Gly Asp Met Gln
   3890 3895 3900
   Val Pro Asp Leu Asp Ile Lys Gly Pro Lys Val Asp Ile Asn Ala
   3905 3910 3915
   Pro Asp Val Asp Val Arg Gly Pro Asp Trp His Leu Lys Met Pro
   3920 3925 3930
   Lys Ile Lys Met Pro Lys Ile Ser Met Pro Gly Phe Lys Gly Glu
   3935 3940 3945
   Gly Pro Glu Val Asp Val Asn Leu Pro Lys Ala Asp Leu Asp Val
   3950 3955 3960
   Ser Gly Pro Lys Val Asp Val Asp Val Pro Asp Val Asn Ile Glu
   3965 3970 3975
   Gly Pro Asp Ala Lys Leu Lys Gly Pro Lys Phe Lys Met Pro Glu
   3980 3985 3990
   Met Asn Ile Lys Ala Pro Lys Ile Ser Met Pro Asp Phe Asp Leu
   3995 4000 4005
   His Leu Lys Gly Pro Lys Val Lys Gly Asp Val Asp Val Ser Leu
   4010 4015 4020
   Pro Lys Met Glu Gly Asp Leu Lys Ala Pro Glu Val Asp Ile Lys
   4025 4030 4035
   Gly Pro Lys Val Asp Ile Asp Ala Pro Asp Val Asp Val His Gly
   4040 4045 4050
   Pro Asp Trp His Leu Lys Met Pro Lys Val Lys Met Pro Lys Phe
   4055 4060 4065
   Ser Met Pro Gly Phe Lys Gly Glu Gly Pro Glu Val Asp Val Asn
   4070 4075 4080
   Leu Pro Lys Ala Asp Ile Asp Val Ser Gly Pro Lys Val Asp Ile
   4085 4090 4095
   Asp Thr Pro Asp Ile Asp Ile His Gly Pro Glu Gly Lys Leu Lys
   4100 4105 4110
   Gly Pro Lys Phe Lys Met Pro Asp Leu His Leu Lys Ala Pro Lys
   4115 4120 4125
   Ile Ser Met Pro Glu Val Asp Leu Asn Leu Lys Gly Pro Lys Met
   4130 4135 4140
   Lys Gly Asp Val Asp Val Ser Leu Pro Lys Val Glu Gly Asp Leu
   4145 4150 4155
   Lys Gly Pro Glu Val Asp Ile Lys Gly Pro Lys Val Asp Ile Asp
   4160 4165 4170
   Val Pro Asp Val Asp Val Gln Gly Pro Asp Trp His Leu Lys Met
   4175 4180 4185
   Pro Lys Val Lys Met Pro Lys Phe Ser Met Pro Gly Phe Lys Gly
   4190 4195 4200
   Glu Gly Pro Asp Val Asp Val Asn Leu Pro Lys Ala Asp Leu Asp
   4205 4210 4215
   Val Ser Gly Pro Lys Val Asp Ile Asp Val Pro Asp Val Asn Ile
   4220 4225 4230
   Glu Gly Pro Asp Ala Lys Leu Lys Gly Pro Lys Phe Lys Met Pro
   4235 4240 4245
   Glu Met Asn Ile Lys Ala Pro Lys Ile Ser Met Pro Asp Phe Asp
   4250 4255 4260
   Leu His Leu Lys Gly Pro Lys Val Lys Gly Asp Val Asp Val Ser
   4265 4270 4275
   Leu Pro Lys Val Glu Gly Asp Leu Lys Gly Pro Glu Val Asp Ile
   4280 4285 4290
   Lys Gly Pro Lys Val Asp Ile Asp Ala Pro Asp Val Asp Val His
   4295 4300 4305
   Gly Pro Asp Trp His Leu Lys Met Pro Lys Val Lys Met Pro Lys
   4310 4315 4320
   Phe Ser Met Pro Gly Phe Lys Gly Glu Gly Pro Asp Val Asp Val
   4325 4330 4335
   Thr Leu Pro Lys Ala Asp Ile Glu Ile Ser Gly Pro Lys Val Asp
   4340 4345 4350
   Ile Asp Ala Pro Asp Val Ser Ile Glu Gly Pro Asp Ala Lys Leu
   4355 4360 4365
   Lys Gly Pro Lys Phe Lys Met Pro Glu Met Asn Ile Lys Ala Pro
   4370 4375 4380
   Lys Ile Ser Met Pro Asp Ile Asp Phe Asn Leu Lys Gly Pro Lys
   4385 4390 4395
   Val Lys Gly Asp Val Asp Val Ser Leu Pro Lys Val Glu Gly Asp
   4400 4405 4410
   Leu Lys Gly Pro Glu Ile Asp Ile Lys Gly Pro Ser Leu Asp Ile
   4415 4420 4425
   Asp Thr Pro Asp Val Asn Ile Glu Gly Pro Glu Gly Lys Leu Lys
   4430 4435 4440
   Gly Pro Lys Phe Lys Met Pro Glu Met Asn Ile Lys Ala Pro Lys
   4445 4450 4455
   Ile Ser Met Pro Asp Phe Asp Leu His Leu Lys Gly Pro Lys Val
   4460 4465 4470
   Lys Gly Asp Val Asp Val Ser Leu Pro Lys Val Glu Ser Asp Leu
   4475 4480 4485
   Lys Gly Pro Glu Val Asp Ile Glu Gly Pro Glu Gly Lys Leu Lys
   4490 4495 4500
   Gly Pro Lys Phe Lys Met Pro Asp Val His Phe Lys Ser Pro Gln
   4505 4510 4515
   Ile Ser Met Ser Asp Ile Asp Leu Asn Leu Lys Gly Pro Lys Ile
   4520 4525 4530
   Lys Gly Asp Met Asp Ile Ser Val Pro Lys Leu Glu Gly Asp Leu
   4535 4540 4545
   Lys Gly Pro Lys Val Asp Val Lys Gly Pro Lys Val Gly Ile Asp
   4550 4555 4560
   Thr Pro Asp Ile Asp Ile His Gly Pro Glu Gly Lys Leu Lys Gly
   4565 4570 4575
   Pro Lys Phe Lys Met Pro Asp Leu His Leu Lys Ala Pro Lys Ile
   4580 4585 4590
   Ser Met Pro Glu Val Asp Leu Asn Leu Lys Gly Pro Lys Val Lys
   4595 4600 4605
   Gly Asp Met Asp Ile Ser Leu Pro Lys Val Glu Gly Asp Leu Lys
   4610 4615 4620
   Gly Pro Glu Val Asp Ile Arg Asp Pro Lys Val Asp Ile Asp Val
   4625 4630 4635
   Pro Asp Val Asp Val Gln Gly Pro Asp Trp His Leu Lys Met Pro
   4640 4645 4650
   Lys Val Lys Met Pro Lys Phe Ser Met Pro Gly Phe Lys Gly Glu
   4655 4660 4665
   Gly Pro Asp Val Asp Val Asn Leu Pro Lys Ala Asp Ile Asp Val
   4670 4675 4680
   Ser Gly Pro Lys Val Asp Val Asp Val Pro Asp Val Asn Ile Glu
   4685 4690 4695
   Gly Pro Asp Ala Lys Leu Lys Gly Pro Lys Phe Lys Met Pro Glu
   4700 4705 4710
   Met Ser Ile Lys Ala Pro Lys Ile Ser Met Pro Asp Ile Asp Leu
   4715 4720 4725
   Asn Leu Lys Gly Pro Lys Val Lys Gly Asp Val Asp Val Thr Leu
   4730 4735 4740
   Pro Lys Val Glu Gly Asp Leu Lys Gly Pro Glu Ala Asp Ile Lys
   4745 4750 4755
   Gly Pro Lys Val Asp Ile Asn Thr Pro Asp Val Asp Val His Gly
   4760 4765 4770
   Pro Asp Trp His Leu Lys Met Pro Lys Val Lys Met Pro Lys Phe
   4775 4780 4785
   Ser Met Pro Gly Phe Lys Gly Glu Gly Pro Asp Val Asp Val Ser
   4790 4795 4800
   Leu Pro Lys Ala Asp Ile Asp Val Ser Gly Pro Lys Val Asp Val
   4805 4810 4815
   Asp Ile Pro Asp Val Asn Ile Glu Gly Pro Asp Ala Lys Leu Lys
   4820 4825 4830
   Gly Pro Lys Phe Lys Met Pro Glu Ile Asn Ile Lys Ala Pro Lys
   4835 4840 4845
   Ile Ser Ile Pro Asp Val Asp Leu Asp Leu Lys Gly Pro Lys Val
   4850 4855 4860
   Lys Gly Asp Phe Asp Val Ser Val Pro Lys Val Glu Gly Thr Leu
   4865 4870 4875
   Lys Gly Pro Glu Val Asp Leu Lys Gly Pro Arg Leu Asp Phe Glu
   4880 4885 4890
   Gly Pro Asp Ala Lys Leu Ser Gly Pro Ser Leu Lys Met Pro Ser
   4895 4900 4905
   Leu Glu Ile Ser Ala Pro Lys Val Thr Ala Pro Asp Val Asp Leu
   4910 4915 4920
   His Leu Lys Ala Pro Lys Ile Gly Phe Ser Gly Pro Lys Leu Glu
   4925 4930 4935
   Gly Gly Glu Val Asp Leu Lys Gly Pro Lys Val Glu Ala Pro Ser
   4940 4945 4950
   Leu Asp Val His Met Asp Ser Pro Asp Ile Asn Ile Glu Gly Pro
   4955 4960 4965
   Asp Val Lys Ile Pro Lys Phe Lys Lys Pro Lys Phe Gly Phe Gly
   4970 4975 4980
   Ala Lys Ser Pro Lys Ala Asp Ile Lys Ser Pro Ser Leu Asp Val
   4985 4990 4995
   Thr Val Pro Glu Ala Glu Leu Asn Leu Glu Thr Pro Glu Ile Ser
   5000 5005 5010
   Val Gly Gly Lys Gly Lys Lys Ser Lys Phe Lys Met Pro Lys Ile
   5015 5020 5025
   His Met Ser Gly Pro Lys Ile Lys Ala Lys Lys Gln Gly Phe Asp
   5030 5035 5040
   Leu Asn Val Pro Gly Gly Glu Ile Asp Ala Ser Leu Lys Ala Pro
   5045 5050 5055
   Asp Val Asp Val Asn Ile Ala Gly Pro Asp Ala Ala Leu Lys Val
   5060 5065 5070
   Asp Val Lys Ser Pro Lys Thr Lys Lys Thr Met Phe Gly Lys Met
   5075 5080 5085
   Tyr Phe Pro Asp Val Glu Phe Asp Ile Lys Ser Pro Lys Phe Lys
   5090 5095 5100
   Ala Glu Ala Pro Leu Pro Ser Pro Lys Leu Glu Gly Glu Leu Gln
   5105 5110 5115
   Ala Pro Asp Leu Glu Leu Ser Leu Pro Ala Ile His Val Glu Gly
   5120 5125 5130
   Leu Asp Ile Lys Ala Lys Ala Pro Lys Val Lys Met Pro Asp Val
   5135 5140 5145
   Asp Ile Ser Val Pro Lys Ile Glu Gly Asp Leu Lys Gly Pro Lys
   5150 5155 5160
   Val Gln Ala Asn Leu Gly Ala Pro Asp Ile Asn Ile Glu Gly Leu
   5165 5170 5175
   Asp Ala Lys Val Lys Thr Pro Ser Phe Gly Ile Ser Ala Pro Gln
   5180 5185 5190
   Val Ser Ile Pro Asp Val Asn Val Asn Leu Lys Gly Pro Lys Ile
   5195 5200 5205
   Lys Gly Asp Val Pro Ser Val Gly Leu Glu Gly Pro Asp Val Asp
   5210 5215 5220
   Leu Gln Gly Pro Glu Ala Lys Ile Lys Phe Pro Lys Phe Ser Met
   5225 5230 5235
   Pro Lys Ile Gly Ile Pro Gly Val Lys Met Glu Gly Gly Gly Ala
   5240 5245 5250
   Glu Val His Ala Gln Leu Pro Ser Leu Glu Gly Asp Leu Arg Gly
   5255 5260 5265
   Pro Asp Val Lys Leu Glu Gly Pro Asp Val Ser Leu Lys Gly Pro
   5270 5275 5280
   Gly Val Asp Leu Pro Ser Val Asn Leu Ser Met Pro Lys Val Ser
   5285 5290 5295
   Gly Pro Asp Leu Asp Leu Asn Leu Lys Gly Pro Ser Leu Lys Gly
   5300 5305 5310
   Asp Leu Asp Ala Ser Val Pro Ser Met Lys Val His Ala Pro Gly
   5315 5320 5325
   Leu Asn Leu Ser Gly Val Gly Gly Lys Met Gln Val Gly Gly Asp
   5330 5335 5340
   Gly Val Lys Val Pro Gly Ile Asp Ala Thr Thr Lys Leu Asn Val
   5345 5350 5355
   Gly Ala Pro Asp Val Thr Leu Arg Gly Pro Ser Leu Gln Gly Asp
   5360 5365 5370
   Leu Ala Val Ser Gly Asp Ile Lys Cys Pro Lys Val Ser Val Gly
   5375 5380 5385
   Ala Pro Asp Leu Ser Leu Glu Ala Ser Glu Gly Ser Ile Lys Leu
   5390 5395 5400
   Pro Lys Met Lys Leu Pro Gln Phe Gly Ile Ser Thr Pro Gly Ser
   5405 5410 5415
   Asp Leu His Val Asn Ala Lys Gly Pro Gln Val Ser Gly Glu Leu
   5420 5425 5430
   Lys Gly Pro Gly Val Asp Val Asn Leu Lys Gly Pro Arg Ile Ser
   5435 5440 5445
   Ala Pro Asn Val Asp Phe Asn Leu Glu Gly Pro Lys Val Lys Gly
   5450 5455 5460
   Ser Leu Gly Ala Thr Gly Glu Ile Lys Gly Pro Thr Val Gly Gly
   5465 5470 5475
   Gly Leu Pro Gly Ile Gly Val Gln Gly Leu Glu Gly Asn Leu Gln
   5480 5485 5490
   Met Pro Gly Ile Lys Ser Ser Gly Cys Asp Val Asn Leu Pro Gly
   5495 5500 5505
   Val Asn Val Lys Leu Pro Thr Gly Gln Ile Ser Gly Pro Glu Ile
   5510 5515 5520
   Lys Gly Gly Leu Lys Gly Ser Glu Val Gly Phe His Gly Ala Ala
   5525 5530 5535
   Pro Asp Ile Ser Val Lys Gly Pro Ala Phe Asn Met Ala Ser Pro
   5540 5545 5550
   Glu Ser Asp Phe Gly Ile Asn Leu Lys Gly Pro Lys Ile Lys Gly
   5555 5560 5565
   Gly Ala Asp Val Ser Gly Gly Val Ser Ala Pro Asp Ile Ser Leu
   5570 5575 5580
   Gly Glu Gly His Leu Ser Val Lys Gly Ser Gly Gly Glu Trp Lys
   5585 5590 5595
   Gly Pro Gln Val Ser Ser Ala Leu Asn Leu Asp Thr Ser Lys Phe
   5600 5605 5610
   Ala Gly Gly Leu His Phe Ser Gly Pro Lys Val Glu Gly Gly Val
   5615 5620 5625
   Lys Gly Gly Gln Ile Gly Leu Gln Ala Pro Gly Leu Ser Val Ser
   5630 5635 5640
   Gly Pro Gln Gly His Leu Glu Ser Gly Ser Gly Lys Val Thr Phe
   5645 5650 5655
   Pro Lys Met Lys Ile Pro Lys Phe Thr Phe Ser Gly Arg Glu Leu
   5660 5665 5670
   Val Gly Arg Glu Met Gly Val Asp Val His Phe Pro Lys Ala Glu
   5675 5680 5685
   Ala Ser Ile Gln Ala Gly Ala Gly Asp Gly Glu Trp Glu Glu Ser
   5690 5695 5700
   Glu Val Lys Leu Lys Lys Ser Lys Ile Lys Met Pro Lys Phe Asn
   5705 5710 5715
   Phe Ser Lys Pro Lys Gly Lys Gly Gly Val Thr Gly Ser Pro Glu
   5720 5725 5730
   Ala Ser Ile Ser Gly Ser Lys Gly Asp Leu Lys Ser Ser Lys Ala
   5735 5740 5745
   Ser Leu Gly Ser Leu Glu Gly Glu Ala Glu Ala Glu Ala Ser Ser
   5750 5755 5760
   Pro Lys Gly Lys Phe Ser Leu Phe Lys Ser Lys Lys Pro Arg His
   5765 5770 5775
   Arg Ser Asn Ser Phe Ser Asp Glu Arg Glu Phe Ser Gly Pro Ser
   5780 5785 5790
   Thr Pro Thr Gly Thr Leu Glu Phe Glu Gly Gly Glu Val Ser Leu
   5795 5800 5805
   Glu Gly Gly Lys Val Lys Gly Lys His Gly Lys Leu Lys Phe Gly
   5810 5815 5820
   Thr Phe Gly Gly Leu Gly Ser Lys Ser Lys Gly His Tyr Glu Val
   5825 5830 5835
   Thr Gly Ser Asp Asp Glu Thr Gly Lys Leu Gln Gly Ser Gly Val
   5840 5845 5850
   Ser Leu Ala Ser Lys Lys Ser Arg Leu Ser Ser Ser Ser Ser Asn
   5855 5860 5865
   Asp Ser Gly Asn Lys Val Gly Ile Gln Leu Pro Glu Val Glu Leu
   5870 5875 5880
   Ser Val Ser Thr Lys Lys Glu
   5885 5890
<210> 38
   <211> 1894
   <212> PRT
   <213> Homo sapiens
<400> 38
   Met Ala Pro Glu Ala Gly Ala Thr Leu Arg Ala Pro Arg Arg Leu Ser
   1               5                   10                  15
   Trp Ala Ala Leu Leu Leu Leu Ala Ala Leu Leu Pro Val Ala Ser Ser
               20                  25                  30
   Ala Ala Ala Ser Val Asp His Pro Leu Lys Pro Arg His Val Lys Leu
           35                  40                  45
   Leu Ser Thr Lys Met Gly Leu Lys Val Thr Trp Asp Pro Pro Lys Asp
       50                  55                  60
   Ala Thr Ser Arg Pro Val Glu His Tyr Asn Ile Ala Tyr Gly Lys Ser
   65                  70                  75                  80
   Leu Lys Ser Leu Lys Tyr Ile Lys Val Asn Ala Glu Thr Tyr Ser Phe
                   85                  90                  95
   Leu Ile Glu Asp Val Glu Pro Gly Val Val Tyr Phe Val Leu Leu Thr
               100                 105                 110
   Ala Glu Asn His Ser Gly Val Ser Arg Pro Val Tyr Arg Ala Glu Ser
           115                 120                 125
   Pro Pro Gly Gly Glu Trp Ile Glu Ile Asp Gly Phe Pro Ile Lys Gly
       130                 135                 140
   Pro Gly Pro Phe Asn Glu Thr Val Thr Glu Lys Glu Val Pro Asn Lys
   145                 150                 155                 160
   Pro Leu Arg Val Arg Val Arg Ser Ser Asp Asp Arg Leu Ser Val Ala
                   165                 170                 175
   Trp Lys Ala Pro Arg Leu Ser Gly Ala Lys Ser Pro Arg Arg Ser Arg
               180                 185                 190
   Gly Phe Leu Leu Gly Tyr Gly Glu Ser Gly Arg Lys Met Asn Tyr Val
           195                 200                 205
   Pro Leu Thr Arg Asp Glu Arg Thr His Glu Ile Lys Lys Leu Ala Ser
       210                 215                 220
   Glu Ser Val Tyr Val Val Ser Leu Gln Ser Met Asn Ser Gln Gly Arg
   225                 230                 235                 240
   Ser Gln Pro Val Tyr Arg Ala Ala Leu Thr Lys Arg Lys Ile Ser Glu
                   245                 250                 255
   Glu Asp Glu Leu Asp Val Pro Asp Asp Ile Ser Val Arg Val Met Ser
               260                 265                 270
   Ser Gln Ser Val Leu Val Ser Trp Val Asp Pro Val Leu Glu Lys Gln
           275                 280                 285
   Lys Lys Val Val Ala Ser Arg Gln Tyr Thr Val Arg Tyr Arg Glu Lys
       290                 295                 300
   Gly Glu Leu Ala Arg Trp Asp Tyr Lys Gln Ile Ala Asn Arg Arg Val
   305                 310                 315                 320
   Leu Ile Glu Asn Leu Ile Pro Asp Thr Val Tyr Glu Phe Ala Val Arg
                   325                 330                 335
   Ile Ser Gln Gly Glu Arg Asp Gly Lys Trp Ser Thr Ser Val Phe Gln
               340                 345                 350
   Arg Thr Pro Glu Ser Ala Pro Thr Thr Ala Pro Glu Asn Leu Asn Val
           355                 360                 365
   Trp Pro Val Asn Gly Lys Pro Thr Val Val Ala Ala Ser Trp Asp Ala
       370                 375                 380
   Leu Pro Glu Thr Glu Gly Lys Val Lys Glu Tyr Ile Leu Ser Tyr Ala
   385                 390                 395                 400
   Pro Ala Leu Lys Pro Phe Gly Ala Lys Ser Leu Thr Tyr Pro Gly Asp
                   405                 410                 415
   Thr Thr Ser Ala Leu Val Asp Gly Leu Gln Pro Gly Glu Arg Tyr Leu
               420                 425                 430
   Phe Lys Ile Arg Ala Thr Asn Arg Arg Gly Leu Gly Pro His Ser Lys
           435                 440                 445
   Ala Phe Ile Val Ala Met Pro Thr Thr Ser Lys Ala Asp Val Glu Gln
       450                 455                 460
   Asn Thr Glu Asp Asn Gly Lys Pro Glu Lys Pro Glu Pro Ser Ser Pro
   465                 470                 475                 480
   Ser Pro Arg Ala Pro Ala Ser Ser Gln His Pro Ser Val Pro Ala Ser
   485 490 495
   Pro Gln Gly Arg Asn Ala Lys Asp Leu Leu Leu Asp Leu Lys Asn Lys
   500 505 510
   Ile Leu Ala Asn Gly Gly Ala Pro Arg Lys Pro Gln Leu Arg Ala Lys
   515 520 525
   Lys Ala Glu Glu Leu Asp Leu Gln Ser Thr Glu Ile Thr Gly Glu Glu
   530 535 540
   Glu Leu Gly Ser Arg Glu Asp Ser Pro Met Ser Pro Ser Asp Thr Gln
   545 550 555 560
   Asp Gln Lys Arg Thr Leu Arg Pro Pro Ser Arg His Gly His Ser Val
   565 570 575
   Val Ala Pro Gly Arg Thr Ala Val Arg Ala Arg Met Pro Ala Leu Pro
   580 585 590
   Arg Arg Glu Gly Val Asp Lys Pro Gly Phe Ser Leu Ala Thr Gln Pro
   595 600 605
   Arg Pro Gly Ala Pro Pro Ser Ala Ser Ala Ser Pro Ala His His Ala
   610 615 620
   Ser Thr Gln Gly Thr Ser His Arg Pro Ser Leu Pro Ala Ser Leu Asn
   625 630 635 640
   Asp Asn Asp Leu Val Asp Ser Asp Glu Asp Glu Arg Ala Val Gly Ser
   645 650 655
   Leu His Pro Lys Gly Ala Phe Ala Gln Pro Arg Pro Ala Leu Ser Pro
   660 665 670
   Ser Arg Gln Ser Pro Ser Ser Val Leu Arg Asp Arg Ser Ser Val His
   675 680 685
   Pro Gly Ala Lys Pro Ala Ser Pro Ala Arg Arg Thr Pro His Ser Gly
   690 695 700
   Ala Ala Glu Glu Asp Ser Ser Ala Ser Ala Pro Pro Ser Arg Leu Ser
   705 710 715 720
   Pro Pro His Gly Gly Ser Ser Arg Leu Leu Pro Thr Gln Pro His Leu
   725 730 735
   Ser Ser Pro Leu Ser Lys Gly Gly Lys Asp Gly Glu Asp Ala Pro Ala
   740 745 750
   Thr Asn Ser Asn Ala Pro Ser Arg Ser Thr Met Ser Ser Ser Val Ser
   755 760 765
   Ser His Leu Ser Ser Arg Thr Gln Val Ser Glu Gly Ala Glu Ala Ser
   770 775 780
   Asp Gly Glu Ser His Gly Asp Gly Asp Arg Glu Asp Gly Gly Arg Gln
   785 790 795 800
   Ala Glu Ala Thr Ala Gln Thr Leu Arg Ala Arg Pro Ala Ser Gly His
   805 810 815
   Phe His Leu Leu Arg His Lys Pro Phe Ala Ala Asn Gly Arg Ser Pro
   820 825 830
   Ser Arg Phe Ser Ile Gly Arg Gly Pro Arg Leu Gln Pro Ser Ser Ser
   835 840 845
   Pro Gln Ser Thr Val Pro Ser Arg Ala His Pro Arg Val Pro Ser His
   850 855 860
   Ser Asp Ser His Pro Lys Leu Ser Ser Gly Ile His Gly Asp Glu Glu
   865 870 875 880
   Asp Glu Lys Pro Leu Pro Ala Thr Val Val Asn Asp His Val Pro Ser
   885 890 895
   Ser Ser Arg Gln Pro Ile Ser Arg Gly Trp Glu Asp Leu Arg Arg Ser
   900 905 910
   Pro Gln Arg Gly Ala Ser Leu His Arg Lys Glu Pro Ile Pro Glu Asn
   915 920 925
   Pro Lys Ser Thr Gly Ala Asp Thr His Pro Gln Gly Lys Tyr Ser Ser
   930 935 940
   Leu Ala Ser Lys Ala Gln Asp Val Gln Gln Ser Thr Asp Ala Asp Thr
   945 950 955 960
   Glu Gly His Ser Pro Lys Ala Gln Pro Gly Ser Thr Asp Arg His Ala
   965 970 975
   Ser Pro Ala Arg Pro Pro Ala Ala Arg Ser Gln Gln His Pro Ser Val
   980 985 990
   Pro Arg Arg Met Thr Pro Gly Arg Ala Pro Gln Gln Gln Pro Pro Pro
   995 1000 1005
   Pro Val Ala Thr Ser Gln His His Pro Gly Pro Gln Ser Arg Asp
   1010 1015 1020
   Ala Gly Arg Ser Pro Ser Gln Pro Arg Leu Ser Leu Thr Gln Ala
   1025 1030 1035
   Gly Arg Pro Arg Pro Thr Ser Gln Gly Arg Ser His Ser Ser Ser
   1040 1045 1050
   Asp Pro Tyr Thr Ala Ser Ser Arg Gly Met Leu Pro Thr Ala Leu
   1055 1060 1065
   Gln Asn Gln Asp Glu Asp Ala Gln Gly Ser Tyr Asp Asp Asp Ser
   1070 1075 1080
   Thr Glu Val Glu Ala Gln Asp Val Arg Ala Pro Ala His Ala Ala
   1085 1090 1095
   Arg Ala Lys Glu Ala Ala Ala Ser Leu Pro Lys His Gln Gln Val
   1100 1105 1110
   Glu Ser Pro Thr Gly Ala Gly Ala Gly Gly Asp His Arg Ser Gln
   1115 1120 1125
   Arg Gly His Ala Ala Ser Pro Ala Arg Pro Ser Arg Pro Gly Gly
   1130 1135 1140
   Pro Gln Ser Arg Ala Arg Val Pro Ser Arg Ala Ala Pro Gly Lys
   1145 1150 1155
   Ser Glu Pro Pro Ser Lys Arg Pro Leu Ser Ser Lys Ser Gln Gln
   1160 1165 1170
   Ser Val Ser Ala Glu Asp Asp Glu Glu Glu Asp Ala Gly Phe Phe
   1175 1180 1185
   Lys Gly Gly Lys Glu Asp Leu Leu Ser Ser Ser Val Pro Lys Trp
   1190 1195 1200
   Pro Ser Ser Ser Thr Pro Arg Gly Gly Lys Asp Ala Asp Gly Ser
   1205 1210 1215
   Leu Ala Lys Glu Glu Arg Glu Pro Ala Ile Ala Leu Ala Pro Arg
   1220 1225 1230
   Gly Gly Ser Leu Ala Pro Val Lys Arg Pro Leu Pro Pro Pro Pro
   1235 1240 1245
   Gly Ser Ser Pro Arg Ala Ser His Val Pro Ser Arg Leu Pro Pro
   1250 1255 1260
   Arg Ser Ala Ala Thr Val Ser Pro Val Ala Gly Thr His Pro Trp
   1265 1270 1275
   Pro Gln Tyr Thr Thr Arg Ala Pro Pro Gly His Phe Ser Thr Thr
   1280 1285 1290
   Pro Met Leu Ser Leu Arg Gln Arg Met Met His Ala Arg Phe Arg
   1295 1300 1305
   Asn Pro Leu Ser Arg Gln Pro Ala Arg Pro Ser Tyr Arg Gln Gly
   1310 1315 1320
   Tyr Asn Gly Arg Pro Asn Val Glu Gly Lys Val Leu Pro Gly Ser
   1325 1330 1335
   Asn Gly Lys Pro Asn Gly Gln Arg Ile Ile Asn Gly Pro Gln Gly
   1340 1345 1350
   Thr Lys Trp Val Val Asp Leu Asp Arg Gly Leu Val Leu Asn Ala
   1355 1360 1365
   Glu Gly Arg Tyr Leu Gln Asp Ser His Gly Asn Pro Leu Arg Ile
   1370 1375 1380
   Lys Leu Gly Gly Asp Gly Arg Thr Ile Val Asp Leu Glu Gly Thr
   1385 1390 1395
   Pro Val Val Ser Pro Asp Gly Leu Pro Leu Phe Gly Gln Gly Arg
   1400 1405 1410
   His Gly Thr Pro Leu Ala Asn Ala Gln Asp Lys Pro Ile Leu Ser
   1415 1420 1425
   Leu Gly Gly Lys Pro Leu Val Gly Leu Glu Val Ile Lys Lys Thr
   1430 1435 1440
   Thr His Pro Pro Thr Thr Thr Met Gln Pro Thr Thr Thr Thr Thr
   1445 1450 1455
   Pro Leu Pro Thr Thr Thr Thr Pro Arg Pro Thr Thr Ala Thr Thr
   1460 1465 1470
   Arg Arg Thr Thr Thr Thr Arg Arg Thr Thr Thr Arg Arg Pro Thr
   1475 1480 1485
   Thr Thr Val Arg Thr Thr Thr Arg Thr Thr Thr Thr Thr Thr Pro
   1490 1495 1500
   Thr Pro Thr Thr Pro Ile Pro Thr Cys Pro Pro Gly Thr Leu Glu
   1505 1510 1515
   Arg His Asp Asp Asp Gly Asn Leu Ile Met Ser Ser Asn Gly Ile
   1520 1525 1530
   Pro Glu Cys Tyr Ala Glu Glu Asp Glu Phe Ser Gly Leu Glu Thr
   1535 1540 1545
   Asp Thr Ala Val Pro Thr Glu Glu Ala Tyr Val Ile Tyr Asp Glu
   1550 1555 1560
   Asp Tyr Glu Phe Glu Thr Ser Arg Pro Pro Thr Thr Thr Glu Pro
   1565 1570 1575
   Ser Thr Thr Ala Thr Thr Pro Arg Val Ile Pro Glu Glu Gly Ala
   1580 1585 1590
   Ile Ser Ser Phe Pro Glu Glu Glu Phe Asp Leu Ala Gly Arg Lys
   1595 1600 1605
   Arg Phe Val Ala Pro Tyr Val Thr Tyr Leu Asn Lys Asp Pro Ser
   1610 1615 1620
   Ala Pro Cys Ser Leu Thr Asp Ala Leu Asp His Phe Gln Val Asp
   1625 1630 1635
   Ser Leu Asp Glu Ile Ile Pro Asn Asp Leu Lys Lys Ser Asp Leu
   1640 1645 1650
   Pro Pro Gln His Ala Pro Arg Asn Ile Thr Val Val Ala Val Glu
   1655 1660 1665
   Gly Cys His Ser Phe Val Ile Val Asp Trp Asp Lys Ala Thr Pro
   1670 1675 1680
   Gly Asp Val Val Thr Gly Tyr Leu Val Tyr Ser Ala Ser Tyr Glu
   1685 1690 1695
   Asp Phe Ile Arg Asn Lys Trp Ser Thr Gln Ala Ser Ser Val Thr
   1700 1705 1710
   His Leu Pro Ile Glu Asn Leu Lys Pro Asn Thr Arg Tyr Tyr Phe
   1715 1720 1725
   Lys Val Gln Ala Gln Asn Pro His Gly Tyr Gly Pro Ile Ser Pro
   1730 1735 1740
   Ser Val Ser Phe Val Thr Glu Ser Asp Asn Pro Leu Leu Val Val
   1745 1750 1755
   Arg Pro Pro Gly Gly Glu Pro Ile Trp Ile Pro Phe Ala Phe Lys
   1760 1765 1770
   His Asp Pro Ser Tyr Thr Asp Cys His Gly Arg Gln Tyr Val Lys
   1775 1780 1785
   Arg Thr Trp Tyr Arg Lys Phe Val Gly Val Val Leu Cys Asn Ser
   1790 1795 1800
   Leu Arg Tyr Lys Ile Tyr Leu Ser Asp Asn Leu Lys Asp Thr Phe
   1805 1810 1815
   Tyr Ser Ile Gly Asp Ser Trp Gly Arg Gly Glu Asp His Cys Gln
   1820 1825 1830
   Phe Val Asp Ser His Leu Asp Gly Arg Thr Gly Pro Gln Ser Tyr
   1835 1840 1845
   Val Glu Ala Leu Pro Thr Ile Gln Gly Tyr Tyr Arg Gln Tyr Arg
   1850 1855 1860
   Gln Glu Pro Val Arg Phe Gly Asn Ile Gly Phe Gly Thr Pro Tyr
   1865 1870 1875
   Tyr Tyr Val Gly Trp Tyr Glu Cys Gly Val Ser Ile Pro Gly Lys
   1880 1885 1890
   Trp
<210> 39
   <211> 198
   <212> PRT
   <213> Homo sapiens
<400> 39
   Met Ala Ala Asp Ile Ser Glu Ser Ser Gly Ala Asp Cys Lys Gly Asp
   1               5                   10                  15
   Pro Arg Asn Ser Ala Lys Leu Asp Ala Asp Tyr Pro Leu Arg Val Leu
               20                  25                  30
   Tyr Cys Gly Val Cys Ser Leu Pro Thr Glu Tyr Cys Glu Tyr Met Pro
           35                  40                  45
   Asp Val Ala Lys Cys Arg Gln Trp Leu Glu Lys Asn Phe Pro Asn Glu
       50                  55                  60
   Phe Ala Lys Leu Thr Val Glu Asn Ser Pro Lys Gln Glu Ala Gly Ile
   65                  70                  75                  80
   Ser Glu Gly Gln Gly Thr Ala Gly Glu Glu Glu Glu Lys Lys Lys Gln
                   85                  90                  95
   Lys Arg Gly Gly Arg Gly Gln Ile Lys Gln Lys Lys Lys Thr Val Pro
               100                 105                 110
   Gln Lys Val Thr Ile Ala Lys Ile Pro Arg Ala Lys Lys Lys Tyr Val
           115                 120                 125
   Thr Arg Val Cys Gly Leu Ala Thr Phe Glu Ile Asp Leu Lys Glu Ala
       130                 135                 140
   Gln Arg Phe Phe Ala Gln Lys Phe Ser Cys Gly Ala Ser Val Thr Gly
   145                 150                 155                 160
   Glu Asp Glu Ile Ile Ile Gln Gly Asp Phe Thr Asp Asp Ile Ile Asp
                   165                 170                 175
   Val Ile Gln Glu Lys Trp Pro Glu Val Asp Asp Asp Ser Ile Glu Asp
               180                 185                 190
   Leu Gly Glu Val Lys Lys
           195
<210> 40
   <211> 267
   <212> PRT
   <213> Homo sapiens
<400> 40
   Met Ala Arg Phe Leu Thr Leu Cys Thr Trp Leu Leu Leu Leu Gly Pro
   1               5                   10                  15
   Gly Leu Leu Ala Thr Val Arg Ala Glu Cys Ser Gln Asp Cys Ala Thr
               20                  25                  30
   Cys Ser Tyr Arg Leu Val Arg Pro Ala Asp Ile Asn Phe Leu Ala Cys
           35                  40                  45
   Val Met Glu Cys Glu Gly Lys Leu Pro Ser Leu Lys Ile Trp Glu Thr
       50                  55                  60
   Cys Lys Glu Leu Leu Gln Leu Ser Lys Pro Glu Leu Pro Gln Asp Gly
   65                  70                  75                  80
   Thr Ser Thr Leu Arg Glu Asn Ser Lys Pro Glu Glu Ser His Leu Leu
                   85                  90                  95
   Ala Lys Arg Tyr Gly Gly Phe Met Lys Arg Tyr Gly Gly Phe Met Lys
               100                 105                 110
   Lys Met Asp Glu Leu Tyr Pro Met Glu Pro Glu Glu Glu Ala Asn Gly
           115                 120                 125
   Ser Glu Ile Leu Ala Lys Arg Tyr Gly Gly Phe Met Lys Lys Asp Ala
       130                 135                 140
   Glu Glu Asp Asp Ser Leu Ala Asn Ser Ser Asp Leu Leu Lys Glu Leu
   145                 150                 155                 160
   Leu Glu Thr Gly Asp Asn Arg Glu Arg Ser His His Gln Asp Gly Ser
                   165                 170                 175
   Asp Asn Glu Glu Glu Val Ser Lys Arg Tyr Gly Gly Phe Met Arg Gly
               180                 185                 190
   Leu Lys Arg Ser Pro Gln Leu Glu Asp Glu Ala Lys Glu Leu Gln Lys
           195                 200                 205
   Arg Tyr Gly Gly Phe Met Arg Arg Val Gly Arg Pro Glu Trp Trp Met
       210                 215                 220
   Asp Tyr Gln Lys Arg Tyr Gly Gly Phe Leu Lys Arg Phe Ala Glu Ala
   225                 230                 235                 240
   Leu Pro Ser Asp Glu Glu Gly Glu Ser Tyr Ser Lys Glu Val Pro Glu
                   245                 250                 255
   Met Glu Lys Arg Tyr Gly Gly Phe Met Arg Phe
   260 265
<210> 41
   <211> 105
   <212> PRT
   <213> Homo sapiens
<400> 41
   Met Ala Lys Ile Ser Ser Pro Thr Glu Thr Glu Arg Cys Ile Glu Ser
   1 5 10 15
   Leu Ile Ala Val Phe Gln Lys Tyr Ala Gly Lys Asp Gly Tyr Asn Tyr
   20 25 30
   Thr Leu Ser Lys Thr Glu Phe Leu Ser Phe Met Asn Thr Glu Leu Ala
   35 40 45
   Ala Phe Thr Lys Asn Gln Lys Asp Pro Gly Val Leu Asp Arg Met Met
   50 55 60
   Lys Lys Leu Asp Thr Asn Ser Asp Gly Gln Leu Asp Phe Ser Glu Phe
   65 70 75 80
   Leu Asn Leu Ile Gly Gly Leu Ala Met Ala Cys His Asp Ser Phe Leu
   85 90 95
   Lys Ala Val Pro Ser Gln Lys Arg Thr
   100 105
<210> 42
   <211> 159
   <212> PRT
   <213> Homo sapiens
<400> 42
<210> 43
   <211> 136
   <212> PRT
   <213> Homo sapiens
<400> 43
<210> 44
   <211> 257
   <212> PRT
   <213> Homo sapiens
<400> 44
<210> 45
   <211> 221
   <212> PRT
   <213> Homo sapiens
<400> 45
   Met Ser Tyr Gly Arg Pro Pro Pro Asp Val Glu Gly Met Thr Ser Leu
   1               5                   10                  15
   Lys Val Asp Asn Leu Thr Tyr Arg Thr Ser Pro Asp Thr Leu Arg Arg
               20                  25                  30
   Val Phe Glu Lys Tyr Gly Arg Val Gly Asp Val Tyr Ile Pro Arg Asp
           35                  40                  45
   Arg Tyr Thr Lys Glu Ser Arg Gly Phe Ala Phe Val Arg Phe His Asp
       50                  55                  60
   Lys Arg Asp Ala Glu Asp Ala Met Asp Ala Met Asp Gly Ala Val Leu
   65                  70                  75                  80
   Asp Gly Arg Glu Leu Arg Val Gln Met Ala Arg Tyr Gly Arg Pro Pro
                   85                  90                  95
   Asp Ser His His Ser Arg Arg Gly Pro Pro Pro Arg Arg Tyr Gly Gly
               100                 105                 110
   Gly Gly Tyr Gly Arg Arg Ser Arg Ser Pro Arg Arg Arg Arg Arg Ser
           115                 120                 125
   Arg Ser Arg Ser Arg Ser Arg Ser Arg Ser Arg Ser Arg Ser Arg Tyr
       130                 135                 140
   Ser Arg Ser Lys Ser Arg Ser Arg Thr Arg Ser Arg Ser Arg Ser Thr
   145                 150                 155                 160
   Ser Lys Ser Arg Ser Ala Arg Arg Ser Lys Ser Lys Ser Ser Ser Val
                   165                 170                 175
   Ser Arg Ser Arg Ser Arg Ser Arg Ser Arg Ser Arg Ser Arg Ser Pro
               180                 185                 190
   Pro Pro Val Ser Lys Arg Glu Ser Lys Ser Arg Ser Arg Ser Lys Ser
           195                 200                 205
   Pro Pro Lys Ser Pro Glu Glu Glu Gly Ala Val Ser Ser
       210                 215                 220
<210> 46
   <211> 424
   <212> PRT
   <213> Homo sapiens
<400> 46
   Met Ala Ala Gly Pro Ile Ser Glu Arg Asn Gln Asp Ala Thr Val Tyr
   1               5                   10                  15
   Val Gly Gly Leu Asp Glu Lys Val Ser Glu Pro Leu Leu Trp Glu Leu
               20                  25                  30
   Phe Leu Gln Ala Gly Pro Val Val Asn Thr His Met Pro Lys Asp Arg
           35                  40                  45
   Val Thr Gly Gln His Gln Gly Tyr Gly Phe Val Glu Phe Leu Ser Glu
       50                  55                  60
   Glu Asp Ala Asp Tyr Ala Ile Lys Ile Met Asn Met Ile Lys Leu Tyr
   65                  70                  75                  80
   Gly Lys Pro Ile Arg Val Asn Lys Ala Ser Ala His Asn Lys Asn Leu
                   85                  90                  95
   Asp Val Gly Ala Asn Ile Phe Ile Gly Asn Leu Asp Pro Glu Ile Asp
               100                 105                 110
   Glu Lys Leu Leu Tyr Asp Thr Phe Ser Ala Phe Gly Val Ile Leu Gln
           115                 120                 125
   Thr Pro Lys Ile Met Arg Asp Pro Asp Thr Gly Asn Ser Lys Gly Tyr
       130                 135                 140
   Ala Phe Ile Asn Phe Ala Ser Phe Asp Ala Ser Asp Ala Ala Ile Glu
   145                 150                 155                 160
   Ala Met Asn Gly Gln Tyr Leu Cys Asn Arg Pro Ile Thr Val Ser Tyr
   165 170 175
   Ala Phe Lys Lys Asp Ser Lys Gly Glu Arg His Gly Ser Ala Ala Glu
   180 185 190
   Arg Leu Leu Ala Ala Gln Asn Pro Leu Ser Gln Ala Asp Arg Pro His
   195 200 205
   Gln Leu Phe Ala Asp Ala Pro Pro Pro Pro Ser Ala Pro Asn Pro Val
   210 215 220
   Val Ser Ser Leu Gly Ser Gly Leu Pro Pro Pro Gly Met Pro Pro Pro
   225 230 235 240
   Gly Ser Phe Pro Pro Pro Val Pro Pro Pro Gly Ala Leu Pro Pro Gly
   245 250 255
   Ile Pro Pro Ala Met Pro Pro Pro Pro Met Pro Pro Gly Ala Ala Gly
   260 265 270
   His Gly Pro Pro Ser Ala Gly Thr Pro Gly Ala Gly His Pro Gly His
   275 280 285
   Gly His Ser His Pro His Pro Phe Pro Pro Gly Gly Met Pro His Pro
   290 295 300
   Gly Met Ser Gln Met Gln Leu Ala His His Gly Pro His Gly Leu Gly
   305 310 315 320
   His Pro His Ala Gly Pro Pro Gly Ser Gly Gly Gln Pro Pro Pro Arg
   325 330 335
   Pro Pro Pro Gly Met Pro His Pro Gly Pro Pro Pro Met Gly Met Pro
   340 345 350
   Pro Arg Gly Pro Pro Phe Gly Ser Pro Met Gly His Pro Gly Pro Met
   355 360 365
   Pro Pro His Gly Met Arg Gly Pro Pro Pro Leu Met Pro Pro His Gly
   370 375 380
   Tyr Thr Gly Pro Pro Arg Pro Pro Pro Tyr Gly Tyr Gln Arg Gly Pro
   385 390 395 400
   Leu Pro Pro Pro Arg Pro Thr Pro Arg Pro Pro Val Pro Pro Arg Gly
   405 410 415
   Pro Leu Arg Gly Pro Leu Pro Gln
   420
<210> 47
   <211> 590
   <212> PRT
   <213> Homo sapiens
<400> 47
   Met His Leu Ala Arg Leu Val Gly Ser Cys Ser Leu Leu Leu Leu Leu
   1 5 10 15
   Gly Ala Leu Ser Gly Trp Ala Ala Ser Asp Asp Pro Ile Glu Lys Val
   20 25 30
   Ile Glu Gly Ile Asn Arg Gly Leu Ser Asn Ala Glu Arg Glu Val Gly
   35 40 45
   Lys Ala Leu Asp Gly Ile Asn Ser Gly Ile Thr His Ala Gly Arg Glu
   50 55 60
   Val Glu Lys Val Phe Asn Gly Leu Ser Asn Met Gly Ser His Thr Gly
   65 70 75 80
   Lys Glu Leu Asp Lys Gly Val Gln Gly Leu Asn His Gly Met Asp Lys
   85 90 95
   Val Ala His Glu Ile Asn His Gly Ile Gly Gln Ala Gly Lys Glu Ala
   100 105 110
   Glu Lys Leu Gly His Gly Val Asn Asn Ala Ala Gly Gln Val Gly Lys
   115 120 125
   Glu Ala Asp Lys Leu Ile His His Gly Val His His Gly Ala Asn Gln
   130 135 140
   Ala Gly Ser Glu Ala Gly Lys Phe Gly Gln Gly Val Asp Asn Ala Ala
   145 150 155 160
   Gly Gln Ala Gly Asn Glu Ala Gly Arg Phe Gly Gln Gly Val His His
   165 170 175
   Ala Ala Gly Gln Ala Gly Asn Glu Ala Gly Arg Phe Gly Gln Gly Val
   180 185 190
   His His Ala Ala Gly Gln Ala Gly Asn Glu Ala Gly Arg Phe Gly Gln
   195 200 205
   Gly Ala His His Gly Leu Ser Glu Gly Trp Lys Glu Thr Glu Lys Phe
       210                 215                 220
   Gly Gln Gly Ile His His Ala Ala Gly Gln Val Gly Lys Glu Ala Glu
   225                 230                 235                 240
   Lys Phe Gly Gln Gly Ala His His Ala Ala Gly Gln Ala Gly Asn Glu
                   245                 250                 255
   Ala Gly Arg Phe Gly Gln Gly Val His His Gly Leu Ser Glu Gly Trp
               260                 265                 270
   Lys Glu Thr Glu Lys Phe Gly Gln Gly Val His His Thr Ala Gly Gln
           275                 280                 285
   Val Gly Lys Glu Ala Glu Lys Phe Gly Gln Gly Ala His His Ala Ala
       290                 295                 300
   Gly Gln Ala Gly Asn Glu Ala Gly Arg Phe Gly Gln Gly Ala His His
   305                 310                 315                 320
   Ala Ala Gly Gln Ala Gly Asn Glu Ala Gly Arg Phe Gly Gln Gly Val
                   325                 330                 335
   His His Gly Leu Ser Glu Gly Trp Lys Glu Thr Glu Lys Phe Gly Gln
               340                 345                 350
   Gly Val His His Ala Ala Ser Gln Phe Gly Lys Glu Thr Glu Lys Leu
           355                 360                 365
   Gly His Gly Val His His Gly Val Asn Glu Ala Trp Lys Glu Ala Glu
       370                 375                 380
   Lys Phe Gly Gln Gly Val His His Ala Ala Ser Gln Val Gly Lys Glu
   385                 390                 395                 400
   Glu Asp Arg Val Val Gln Gly Leu His His Gly Val Ser Gln Ala Gly
                   405                 410                 415
   Arg Glu Ala Gly Gln Phe Gly His Asp Ile His His Thr Ala Gly Gln
               420                 425                 430
   Ala Gly Lys Glu Gly Asp Ile Ala Val His Gly Val Gln Pro Gly Val
           435                 440                 445
   His Glu Ala Gly Lys Glu Ala Gly Gln Phe Gly Gln Gly Val His His
       450                 455                 460
   Thr Leu Glu Gln Ala Gly Lys Glu Ala Asp Lys Ala Val Gln Gly Phe
   465 470 475 480
   His Thr Gly Val His Gln Ala Gly Lys Glu Ala Glu Lys Leu Gly Gln
   485 490 495
   Gly Val Asn His Ala Ala Asp Gln Ala Gly Lys Glu Val Glu Lys Leu
   500 505 510
   Gly Gln Gly Ala His His Ala Ala Gly Gln Ala Gly Lys Glu Leu Gln
   515 520 525
   Asn Ala His Asn Gly Val Asn Gln Ala Ser Lys Glu Ala Asn Gln Leu
   530 535 540
   Leu Asn Gly Asn His Gln Ser Gly Ser Ser Ser His Gln Gly Gly Ala
   545 550 555 560
   Thr Thr Thr Pro Leu Ala Ser Gly Ala Ser Val Asn Thr Pro Phe Ile
   565 570 575
   Asn Leu Pro Ala Leu Trp Arg Ser Val Ala Asn Ile Met Pro
   580 585 590
<210> 48
   <211> 379
   <212> PRT
   <213> Homo sapiens
<400> 48
   Met Val Phe Phe Thr Cys Asn Ala Cys Gly Glu Ser Val Lys Lys Ile
   1 5 10 15
   Gln Val Glu Lys His Val Ser Val Cys Arg Asn Cys Glu Cys Leu Ser
   20 25 30
   Cys Ile Asp Cys Gly Lys Asp Phe Trp Gly Asp Asp Tyr Lys Asn His
   35 40 45
   Val Lys Cys Ile Ser Glu Asp Gln Lys Tyr Gly Gly Lys Gly Tyr Glu
   50 55 60
   Gly Lys Thr His Lys Gly Asp Ile Lys Gln Gln Ala Trp Ile Gln Lys
   65 70 75 80
   Ile Ser Glu Leu Ile Lys Arg Pro Asn Val Ser Pro Lys Val Arg Glu
   85 90 95
   Leu Leu Glu Gln Ile Ser Ala Phe Asp Asn Val Pro Arg Lys Lys Ala
   100 105 110
   Lys Phe Gln Asn Trp Met Lys Asn Ser Leu Lys Val His Asn Glu Ser
   115 120 125
   Ile Leu Asp Gln Val Trp Asn Ile Phe Ser Glu Ala Ser Asn Ser Glu
   130 135 140
   Pro Val Asn Lys Glu Gln Asp Gln Arg Pro Leu His Pro Val Ala Asn
   145 150 155 160
   Pro His Ala Glu Ile Ser Thr Lys Val Pro Ala Ser Lys Val Lys Asp
   165 170 175
   Ala Val Glu Gln Gln Gly Glu Val Lys Lys Asn Lys Arg Glu Arg Lys
   180 185 190
   Glu Glu Arg Gln Lys Lys Arg Lys Arg Glu Lys Lys Glu Leu Lys Leu
   195 200 205
   Glu Asn His Gln Glu Asn Ser Arg Asn Gln Lys Pro Lys Lys Arg Lys
   210 215 220
   Lys Gly Gln Glu Ala Asp Leu Glu Ala Gly Gly Glu Glu Val Pro Glu
   225 230 235 240
   Ala Asn Gly Ser Ala Gly Lys Arg Ser Lys Lys Lys Lys Gln Arg Lys
   245 250 255
   Asp Ser Ala Ser Glu Glu Glu Ala His Val Gly Ala Gly Lys Arg Lys
   260 265 270
   Arg Arg His Ser Glu Val Glu Thr Asp Ser Lys Lys Lys Lys Met Lys
   275 280 285
   Leu Pro Glu His Pro Glu Gly Gly Glu Pro Glu Asp Asp Glu Ala Pro
   290 295 300
   Ala Lys Gly Lys Phe Asn Trp Lys Gly Thr Ile Lys Ala Ile Leu Lys
   305 310 315 320
   Gln Ala Pro Asp Asn Glu Ile Thr Ile Lys Lys Leu Arg Lys Lys Val
   325 330 335
   Leu Ala Gln Tyr Tyr Thr Val Thr Asp Glu His His Arg Ser Glu Glu
   340 345 350
   Glu Leu Leu Val Ile Phe Asn Lys Lys Ile Ser Lys Asn Pro Thr Phe
   355 360 365
   Lys Leu Leu Lys Asp Lys Val Lys Leu Val Lys
   370 375
<210> 49
   <211> 331
   <212> PRT
   <213> Homo sapiens
<400> 49
   Met Gly Gly Glu Gln Glu Glu Glu Arg Phe Asp Gly Met Leu Leu Ala
   1 5 10 15
   Met Ala Gln Gln His Glu Gly Gly Val Gln Glu Leu Val Asn Thr Phe
   20 25 30
   Phe Ser Phe Leu Arg Arg Lys Thr Asp Phe Phe Ile Gly Gly Glu Glu
   35 40 45
   Gly Met Ala Glu Lys Leu Ile Thr Gln Thr Phe Ser His His Asn Gln
   50 55 60
   Leu Ala Gln Lys Thr Arg Arg Glu Lys Arg Ala Arg Gln Glu Ala Glu
   65 70 75 80
   Arg Arg Glu Lys Ala Glu Arg Ala Ala Arg Leu Ala Lys Glu Ala Lys
   85 90 95
   Ser Glu Thr Ser Gly Pro Gln Ile Lys Glu Leu Thr Asp Glu Glu Ala
   100 105 110
   Glu Arg Leu Gln Leu Glu Ile Asp Gln Lys Lys Asp Ala Glu Asn His
   115 120 125
   Glu Ala Gln Leu Lys Asn Gly Ser Leu Asp Ser Pro Gly Lys Gln Asp
   130 135 140
   Thr Glu Glu Asp Glu Glu Glu Asp Glu Lys Asp Lys Gly Lys Leu Lys
   145 150 155 160
   Pro Asn Leu Gly Asn Gly Ala Asp Leu Pro Asn Tyr Arg Trp Thr Gln
   165 170 175
   Thr Leu Ser Glu Leu Asp Leu Ala Val Pro Phe Cys Val Asn Phe Arg
   180 185 190
   Leu Lys Gly Lys Asp Met Val Val Asp Ile Gln Arg Arg His Leu Arg
           195                 200                 205
   Val Gly Leu Lys Gly Gln Pro Ala Ile Ile Asp Gly Glu Leu Tyr Asn
       210                 215                 220
   Glu Val Lys Val Glu Glu Ser Ser Trp Leu Ile Glu Asp Gly Lys Val
   225                 230                 235                 240
   Val Thr Val His Leu Glu Lys Ile Asn Lys Met Glu Trp Trp Ser Arg
                   245                 250                 255
   Leu Val Ser Ser Asp Pro Glu Ile Asn Thr Lys Lys Ile Asn Pro Glu
               260                 265                 270
   Asn Ser Lys Leu Ser Asp Leu Asp Ser Glu Thr Arg Ser Met Val Glu
           275                 280                 285
   Lys Met Met Tyr Asp Gln Arg Gln Lys Ser Met Gly Leu Pro Thr Ser
       290                 295                 300
   Asp Glu Gln Lys Lys Gln Glu Ile Leu Lys Lys Phe Met Asp Gln His
   305                 310                 315                 320
   Pro Glu Met Asp Phe Ser Lys Ala Lys Phe Asn
                   325                 330
<210> 50
   <211> 422
   <212> PRT
   <213> Homo sapiens
<400> 50
<210> 51
   <211> 390
   <212> PRT
   <213> Homo sapiens
<400> 51
   Met Pro Pro Ser Gly Leu Arg Leu Leu Leu Leu Leu Leu Pro Leu Leu
   1               5                   10                  15
   Trp Leu Leu Val Leu Thr Pro Gly Arg Pro Ala Ala Gly Leu Ser Thr
               20                  25                  30
   Cys Lys Thr Ile Asp Met Glu Leu Val Lys Arg Lys Arg Ile Glu Ala
           35                  40                  45
   Ile Arg Gly Gln Ile Leu Ser Lys Leu Arg Leu Ala Ser Pro Pro Ser
       50                  55                  60
   Gln Gly Glu Val Pro Pro Gly Pro Leu Pro Glu Ala Val Leu Ala Leu
   65                  70                  75                  80
   Tyr Asn Ser Thr Arg Asp Arg Val Ala Gly Glu Ser Ala Glu Pro Glu
                   85                  90                  95
   Pro Glu Pro Glu Ala Asp Tyr Tyr Ala Lys Glu Val Thr Arg Val Leu
               100                 105                 110
   Met Val Glu Thr His Asn Glu Ile Tyr Asp Lys Phe Lys Gln Ser Thr
   115 120 125
   His Ser Ile Tyr Met Phe Phe Asn Thr Ser Glu Leu Arg Glu Ala Val
   130 135 140
   Pro Glu Pro Val Leu Leu Ser Arg Ala Glu Leu Arg Leu Leu Arg Leu
   145 150 155 160
   Lys Leu Lys Val Glu Gln His Val Glu Leu Tyr Gln Lys Tyr Ser Asn
   165 170 175
   Asn Ser Trp Arg Tyr Leu Ser Asn Arg Leu Leu Ala Pro Ser Asp Ser
   180 185 190
   Pro Glu Trp Leu Ser Phe Asp Val Thr Gly Val Val Arg Gln Trp Leu
   195 200 205
   Ser Arg Gly Gly Glu Ile Glu Gly Phe Arg Leu Ser Ala His Cys Ser
   210 215 220
   Cys Asp Ser Arg Asp Asn Thr Leu Gln Val Asp Ile Asn Gly Phe Thr
   225 230 235 240
   Thr Gly Arg Arg Gly Asp Leu Ala Thr Ile His Gly Met Asn Arg Pro
   245 250 255
   Phe Leu Leu Leu Met Ala Thr Pro Leu Glu Arg Ala Gln His Leu Gln
   260 265 270
   Ser Ser Arg His Arg Arg Ala Leu Asp Thr Asn Tyr Cys Phe Ser Ser
   275 280 285
   Thr Glu Lys Asn Cys Cys Val Arg Gln Leu Tyr Ile Asp Phe Arg Lys
   290 295 300
   Asp Leu Gly Trp Lys Trp Ile His Glu Pro Lys Gly Tyr His Ala Asn
   305 310 315 320
   Phe Cys Leu Gly Pro Cys Pro Tyr Ile Trp Ser Leu Asp Thr Gln Tyr
   325 330 335
   Ser Lys Val Leu Ala Leu Tyr Asn Gln His Asn Pro Gly Ala Ser Ala
   340 345 350
   Ala Pro Cys Cys Val Pro Gln Ala Leu Glu Pro Leu Pro Ile Val Tyr
   355 360 365
   Tyr Val Gly Arg Lys Pro Lys Val Glu Gln Leu Ser Asn Met Ile Val
   370 375 380
   Arg Ser Cys Lys Cys Ser
   385 390
<210> 52
   <211> 527
   <212> PRT
   <213> Homo sapiens
<400> 52
   Met Ala Asp Ser Arg Asp Pro Ala Ser Asp Gln Met Gln His Trp Lys
   1 5 10 15
   Glu Gln Arg Ala Ala Gln Lys Ala Asp Val Leu Thr Thr Gly Ala Gly
   20 25 30
   Asn Pro Val Gly Asp Lys Leu Asn Val Ile Thr Val Gly Pro Arg Gly
   35 40 45
   Pro Leu Leu Val Gln Asp Val Val Phe Thr Asp Glu Met Ala His Phe
   50 55 60
   Asp Arg Glu Arg Ile Pro Glu Arg Val Val His Ala Lys Gly Ala Gly
   65 70 75 80
   Ala Phe Gly Tyr Phe Glu Val Thr His Asp Ile Thr Lys Tyr Ser Lys
   85 90 95
   Ala Lys Val Phe Glu His Ile Gly Lys Lys Thr Pro Ile Ala Val Arg
   100 105 110
   Phe Ser Thr Val Ala Gly Glu Ser Gly Ser Ala Asp Thr Val Arg Asp
   115 120 125
   Pro Arg Gly Phe Ala Val Lys Phe Tyr Thr Glu Asp Gly Asn Trp Asp
   130 135 140
   Leu Val Gly Asn Asn Thr Pro Ile Phe Phe Ile Arg Asp Pro Ile Leu
   145 150 155 160
   Phe Pro Ser Phe Ile His Ser Gln Lys Arg Asn Pro Gln Thr His Leu
   165 170 175
   Lys Asp Pro Asp Met Val Trp Asp Phe Trp Ser Leu Arg Pro Glu Ser
   180 185 190
   Leu His Gln Val Ser Phe Leu Phe Ser Asp Arg Gly Ile Pro Asp Gly
   195 200 205
   His Arg His Met Asn Gly Tyr Gly Ser His Thr Phe Lys Leu Val Asn
   210 215 220
   Ala Asn Gly Glu Ala Val Tyr Cys Lys Phe His Tyr Lys Thr Asp Gln
   225 230 235 240
   Gly Ile Lys Asn Leu Ser Val Glu Asp Ala Ala Arg Leu Ser Gln Glu
   245 250 255
   Asp Pro Asp Tyr Gly Ile Arg Asp Leu Phe Asn Ala Ile Ala Thr Gly
   260 265 270
   Lys Tyr Pro Ser Trp Thr Phe Tyr Ile Gln Val Met Thr Phe Asn Gln
   275 280 285
   Ala Glu Thr Phe Pro Phe Asn Pro Phe Asp Leu Thr Lys Val Trp Pro
   290 295 300
   His Lys Asp Tyr Pro Leu Ile Pro Val Gly Lys Leu Val Leu Asn Arg
   305 310 315 320
   Asn Pro Val Asn Tyr Phe Ala Glu Val Glu Gln Ile Ala Phe Asp Pro
   325 330 335
   Ser Asn Met Pro Pro Gly Ile Glu Ala Ser Pro Asp Lys Met Leu Gln
   340 345 350
   Gly Arg Leu Phe Ala Tyr Pro Asp Thr His Arg His Arg Leu Gly Pro
   355 360 365
   Asn Tyr Leu His Ile Pro Val Asn Cys Pro Tyr Arg Ala Arg Val Ala
   370 375 380
   Asn Tyr Gln Arg Asp Gly Pro Met Cys Met Gln Asp Asn Gln Gly Gly
   385 390 395 400
   Ala Pro Asn Tyr Tyr Pro Asn Ser Phe Gly Ala Pro Glu Gln Gln Pro
   405 410 415
   Ser Ala Leu Glu His Ser Ile Gln Tyr Ser Gly Glu Val Arg Arg Phe
   420 425 430
   Asn Thr Ala Asn Asp Asp Asn Val Thr Gln Val Arg Ala Phe Tyr Val
   435 440 445
   Asn Val Leu Asn Glu Glu Gln Arg Lys Arg Leu Cys Glu Asn Ile Ala
       450                 455                 460
   Gly His Leu Lys Asp Ala Gln Ile Phe Ile Gln Lys Lys Ala Val Lys
   465                 470                 475                 480
   Asn Phe Thr Glu Val His Pro Asp Tyr Gly Ser His Ile Gln Ala Leu
                   485                 490                 495
   Leu Asp Lys Tyr Asn Ala Glu Lys Pro Lys Asn Ala Ile His Thr Phe
               500                 505                 510
   Val Gln Ser Gly Ser His Leu Ala Ala Arg Glu Lys Ala Asn Leu
           515                 520                 525
<210> 53
   <211> 412
   <212> PRT
   <213> Homo sapiens
<400> 53
   Met Gln Pro Ser Ser Leu Leu Pro Leu Ala Leu Cys Leu Leu Ala Ala
   1               5                   10                  15
   Pro Ala Ser Ala Leu Val Arg Ile Pro Leu His Lys Phe Thr Ser Ile
               20                  25                  30
   Arg Arg Thr Met Ser Glu Val Gly Gly Ser Val Glu Asp Leu Ile Ala
           35                  40                  45
   Lys Gly Pro Val Ser Lys Tyr Ser Gln Ala Val Pro Ala Val Thr Glu
       50                  55                  60
   Gly Pro Ile Pro Glu Val Leu Lys Asn Tyr Met Asp Ala Gln Tyr Tyr
   65                  70                  75                  80
   Gly Glu Ile Gly Ile Gly Thr Pro Pro Gln Cys Phe Thr Val Val Phe
                   85                  90                  95
   Asp Thr Gly Ser Ser Asn Leu Trp Val Pro Ser Ile His Cys Lys Leu
               100                 105                 110
   Leu Asp Ile Ala Cys Trp Ile His His Lys Tyr Asn Ser Asp Lys Ser
           115                 120                 125
   Ser Thr Tyr Val Lys Asn Gly Thr Ser Phe Asp Ile His Tyr Gly Ser
       130                 135                 140
   Gly Ser Leu Ser Gly Tyr Leu Ser Gln Asp Thr Val Ser Val Pro Cys
   145 150 155 160
   Gln Ser Ala Ser Ser Ala Ser Ala Leu Gly Gly Val Lys Val Glu Arg
   165 170 175
   Gln Val Phe Gly Glu Ala Thr Lys Gln Pro Gly Ile Thr Phe Ile Ala
   180 185 190
   Ala Lys Phe Asp Gly Ile Leu Gly Met Ala Tyr Pro Arg Ile Ser Val
   195 200 205
   Asn Asn Val Leu Pro Val Phe Asp Asn Leu Met Gln Gln Lys Leu Val
   210 215 220
   Asp Gln Asn Ile Phe Ser Phe Tyr Leu Ser Arg Asp Pro Asp Ala Gln
   225 230 235 240
   Pro Gly Gly Glu Leu Met Leu Gly Gly Thr Asp Ser Lys Tyr Tyr Lys
   245 250 255
   Gly Ser Leu Ser Tyr Leu Asn Val Thr Arg Lys Ala Tyr Trp Gln Val
   260 265 270
   His Leu Asp Gln Val Glu Val Ala Ser Gly Leu Thr Leu Cys Lys Glu
   275 280 285
   Gly Cys Glu Ala Ile Val Asp Thr Gly Thr Ser Leu Met Val Gly Pro
   290 295 300
   Val Asp Glu Val Arg Glu Leu Gln Lys Ala Ile Gly Ala Val Pro Leu
   305 310 315 320
   Ile Gln Gly Glu Tyr Met Ile Pro Cys Glu Lys Val Ser Thr Leu Pro
   325 330 335
   Ala Ile Thr Leu Lys Leu Gly Gly Lys Gly Tyr Lys Leu Ser Pro Glu
   340 345 350
   Asp Tyr Thr Leu Lys Val Ser Gln Ala Gly Lys Thr Leu Cys Leu Ser
   355 360 365
   Gly Phe Met Gly Met Asp Ile Pro Pro Pro Ser Gly Pro Leu Trp Ile
   370 375 380
   Leu Gly Asp Val Phe Ile Gly Arg Tyr Tyr Thr Val Phe Asp Arg Asp
   385 390 395 400
   Asn Asn Arg Val Gly Phe Ala Glu Ala Ala Arg Leu
   405 410
<210> 54
   <211> 509
   <212> PRT
   <213> Homo sapiens
<400> 54
<210> 55
   <211> 1005
   <212> PRT
   <213> Homo sapiens
<400> 55
   Met Thr Thr Glu Val Gly Ser Val Ser Glu Val Lys Lys Asp Ser Ser
   1               5                   10                  15
   Gln Leu Gly Thr Asp Ala Thr Lys Glu Lys Pro Lys Glu Val Ala Glu
               20                  25                  30
   Asn Gln Gln Asn Gln Ser Ser Asp Pro Glu Glu Glu Lys Gly Ser Gln
           35                  40                  45
   Pro Pro Pro Ala Ala Glu Ser Gln Ser Ser Leu Arg Arg Gln Lys Arg
       50                  55                  60
   Glu Lys Glu Thr Ser Glu Ser Arg Gly Ile Ser Arg Phe Ile Pro Pro
   65                  70                  75                  80
   Trp Leu Lys Lys Gln Lys Ser Tyr Thr Leu Val Val Ala Lys Asp Gly
                   85                  90                  95
   Gly Asp Lys Lys Glu Pro Thr Gln Ala Val Val Glu Glu Gln Val Leu
               100                 105                 110
   Asp Lys Glu Glu Pro Leu Pro Glu Glu Gln Arg Gln Ala Lys Gly Asp
           115                 120                 125
   Ala Glu Glu Met Ala Gln Lys Lys Gln Glu Ile Lys Val Glu Val Lys
       130                 135                 140
   Glu Glu Lys Pro Ser Val Ser Lys Glu Glu Lys Pro Ser Val Ser Lys
   145                 150                 155                 160
   Val Glu Met Gln Pro Thr Glu Leu Val Ser Lys Glu Arg Glu Glu Lys
                   165                 170                 175
   Val Lys Glu Thr Gln Glu Asp Lys Leu Glu Gly Gly Ala Ala Lys Arg
               180                 185                 190
   Glu Thr Lys Glu Val Gln Thr Asn Glu Leu Lys Ala Glu Lys Ala Ser
           195                 200                 205
   Gln Lys Val Thr Lys Lys Thr Lys Thr Val Gln Cys Lys Val Thr Leu
       210                 215                 220
   Leu Asp Gly Thr Glu Tyr Ser Cys Asp Leu Glu Lys His Ala Lys Gly
   225                 230                 235                 240
   Gln Val Leu Phe Asp Lys Val Cys Glu His Leu Asn Leu Leu Glu Lys
                   245                 250                 255
   Asp Tyr Phe Gly Leu Leu Phe Gln Glu Ser Pro Glu Gln Lys Asn Trp
               260                 265                 270
   Leu Asp Pro Ala Lys Glu Ile Lys Arg Gln Leu Arg Asn Leu Pro Trp
           275                 280                 285
   Leu Phe Thr Phe Asn Val Lys Phe Tyr Pro Pro Asp Pro Ser Gln Leu
       290                 295                 300
   Thr Glu Asp Ile Thr Arg Tyr Phe Leu Cys Leu Gln Leu Arg Gln Asp
   305                 310                 315                 320
   Ile Ala Ser Gly Arg Leu Pro Cys Ser Phe Val Thr His Ala Leu Leu
                   325                 330                 335
   Gly Ser Tyr Thr Leu Gln Ala Glu Leu Gly Asp Tyr Asp Pro Glu Glu
               340                 345                 350
   His Gly Ser Ile Asp Leu Ser Glu Phe Gln Phe Ala Pro Thr Gln Thr
           355                 360                 365
   Lys Glu Leu Glu Glu Lys Val Ala Glu Leu His Lys Thr His Arg Gly
       370                 375                 380
   Leu Ser Pro Ala Gln Ala Asp Ser Gln Phe Leu Glu Asn Ala Lys Arg
   385                 390                 395                 400
   Leu Ser Met Tyr Gly Val Asp Leu His His Ala Lys Asp Ser Glu Gly
                   405                 410                 415
   Val Asp Ile Lys Leu Gly Val Cys Ala Asn Gly Leu Leu Ile Tyr Lys
   420 425 430
   Asp Arg Leu Arg Ile Asn Arg Phe Ala Trp Pro Lys Ile Leu Lys Ile
   435 440 445
   Ser Tyr Lys Arg Ser Asn Phe Tyr Ile Lys Val Arg Pro Ala Glu Leu
   450 455 460
   Glu Gln Phe Glu Ser Thr Ile Gly Phe Lys Leu Pro Asn His Arg Ala
   465 470 475 480
   Ala Lys Arg Leu Trp Lys Val Cys Val Glu His His Thr Phe Tyr Arg
   485 490 495
   Leu Val Ser Pro Glu Gln Pro Pro Lys Ala Lys Phe Leu Thr Leu Gly
   500 505 510
   Ser Lys Phe Arg Tyr Ser Gly Arg Thr Gln Ala Gln Thr Arg Gln Ala
   515 520 525
   Ser Thr Leu Ile Asp Arg Pro Ala Pro His Phe Glu Arg Thr Ser Ser
   530 535 540
   Lys Arg Val Ser Arg Ser Leu Asp Gly Ala Pro Ile Gly Val Met Asp
   545 550 555 560
   Gln Ser Leu Met Lys Asp Phe Pro Gly Ala Ala Gly Glu Ile Ser Ala
   565 570 575
   Tyr Gly Pro Gly Leu Val Ser Ile Ala Val Val Gln Asp Gly Asp Gly
   580 585 590
   Arg Arg Glu Val Arg Ser Pro Thr Lys Ala Pro His Leu Gln Leu Ile
   595 600 605
   Glu Gly Lys Lys Asn Ser Leu Arg Val Glu Gly Asp Asn Ile Tyr Val
   610 615 620
   Arg His Ser Asn Leu Met Leu Glu Glu Leu Asp Lys Ala Gln Glu Asp
   625 630 635 640
   Ile Leu Lys His Gln Ala Ser Ile Ser Glu Leu Lys Arg Asn Phe Met
   645 650 655
   Glu Ser Thr Pro Glu Pro Arg Pro Asn Glu Trp Glu Lys Arg Arg Ile
   660 665 670
   Thr Pro Leu Ser Leu Gln Thr Gln Gly Ser Ser His Glu Thr Leu Asn
   675 680 685
   Ile Val Glu Glu Lys Lys Arg Ala Glu Val Gly Lys Asp Glu Arg Val
   690 695 700
   Ile Thr Glu Glu Met Asn Gly Lys Glu Ile Ser Pro Gly Ser Gly Pro
   705 710 715 720
   Gly Glu Ile Arg Lys Val Glu Pro Val Thr Gln Lys Asp Ser Thr Ser
   725 730 735
   Leu Ser Ser Glu Ser Ser Ser Ser Ser Ser Glu Ser Glu Glu Glu Asp
   740 745 750
   Val Gly Glu Tyr Arg Pro His His Arg Val Thr Glu Gly Thr Ile Arg
   755 760 765
   Glu Glu Gln Glu Tyr Glu Glu Glu Val Glu Glu Glu Pro Arg Pro Ala
   770 775 780
   Ala Lys Val Val Glu Arg Glu Glu Ala Val Pro Glu Ala Ser Pro Val
   785 790 795 800
   Thr Gln Ala Gly Ala Ser Val Ile Thr Val Glu Thr Val Ile Gln Glu
   805 810 815
   Asn Val Gly Ala Gln Lys Ile Pro Gly Glu Lys Ser Val His Glu Gly
   820 825 830
   Ala Leu Lys Gln Asp Met Gly Glu Glu Ala Glu Glu Glu Pro Gln Lys
   835 840 845
   Val Asn Gly Glu Val Ser His Val Asp Ile Asp Val Leu Pro Gln Ile
   850 855 860
   Ile Cys Cys Ser Glu Pro Pro Val Val Lys Thr Glu Met Val Thr Ile
   865 870 875 880
   Ser Asp Ala Ser Gln Arg Thr Glu Ile Ser Thr Lys Glu Val Pro Ile
   885 890 895
   Val Gln Thr Glu Thr Lys Thr Ile Thr Tyr Glu Ser Pro Gln Ile Asp
   900 905 910
   Gly Gly Ala Gly Gly Asp Ser Gly Thr Leu Leu Thr Ala Gln Thr Ile
   915 920 925
   Thr Ser Glu Ser Val Ser Thr Thr Thr Thr Thr His Ile Thr Lys Thr
   930 935 940
   Val Lys Gly Gly Ile Ser Glu Thr Arg Ile Glu Lys Arg Ile Val Ile
   945 950 955 960
   Thr Gly Asp Gly Asp Ile Asp His Asp Gln Ala Leu Ala Gln Ala Ile
   965 970 975
   Arg Glu Ala Arg Glu Gln His Pro Asp Met Ser Val Thr Arg Val Val
   980 985 990
   Val His Lys Glu Thr Glu Leu Ala Glu Glu Gly Glu Asp
   995 1000 1005
<210> 56
   <211> 219
   <212> PRT
   <213> Homo sapiens
<400> 56
<210> 57
   <211> 151
   <212> PRT
   <213> Homo sapiens
<400> 57
<210> 58
   <211> 266
   <212> PRT
   <213> Homo sapiens
<400> 58
<210> 59
   <211> 742
   <212> PRT
   <213> Homo sapiens
<400> 59
   Met Lys Asp Asp Phe Ala Glu Glu Glu Glu Val Gln Ser Phe Gly Tyr
   1               5                   10                  15
   Lys Arg Phe Gly Ile Gln Glu Gly Thr Gln Cys Thr Lys Cys Lys Asn
               20                  25                  30
   Asn Trp Ala Leu Lys Phe Ser Ile Ile Leu Leu Tyr Ile Leu Cys Ala
           35                  40                  45
   Leu Leu Thr Ile Thr Val Ala Ile Leu Gly Tyr Lys Val Val Glu Lys
       50                  55                  60
   Met Asp Asn Val Thr Gly Gly Met Glu Thr Ser Arg Gln Thr Tyr Asp
   65                  70                  75                  80
   Asp Lys Leu Thr Ala Val Glu Ser Asp Leu Lys Lys Leu Gly Asp Gln
                   85                  90                  95
   Thr Gly Lys Lys Ala Ile Ser Thr Asn Ser Glu Leu Ser Thr Phe Arg
               100                 105                 110
   Ser Asp Ile Leu Asp Leu Arg Gln Gln Leu Arg Glu Ile Thr Glu Lys
           115                 120                 125
   Thr Ser Lys Asn Lys Asp Thr Leu Glu Lys Leu Gln Ala Ser Gly Asp
       130                 135                 140
   Ala Leu Val Asp Arg Gln Ser Gln Leu Lys Glu Thr Leu Glu Asn Asn
   145                 150                 155                 160
   Ser Phe Leu Ile Thr Thr Val Asn Lys Thr Leu Gln Ala Tyr Asn Gly
   165 170 175
   Tyr Val Thr Asn Leu Gln Gln Asp Thr Ser Val Leu Gln Gly Asn Leu
   180 185 190
   Gln Asn Gln Met Tyr Ser His Asn Val Val Ile Met Asn Leu Asn Asn
   195 200 205
   Leu Asn Leu Thr Gln Val Gln Gln Arg Asn Leu Ile Thr Asn Leu Gln
   210 215 220
   Arg Ser Val Asp Asp Thr Ser Gln Ala Ile Gln Arg Ile Lys Asn Asp
   225 230 235 240
   Phe Gln Asn Leu Gln Gln Val Phe Leu Gln Ala Lys Lys Asp Thr Asp
   245 250 255
   Trp Leu Lys Glu Lys Val Gln Ser Leu Gln Thr Leu Ala Ala Asn Asn
   260 265 270
   Ser Ala Leu Ala Lys Ala Asn Asn Asp Thr Leu Glu Asp Met Asn Ser
   275 280 285
   Gln Leu Asn Ser Phe Thr Gly Gln Met Glu Asn Ile Thr Thr Ile Ser
   290 295 300
   Gln Ala Asn Glu Gln Asn Leu Lys Asp Leu Gln Asp Leu His Lys Asp
   305 310 315 320
   Ala Glu Asn Arg Thr Ala Ile Lys Phe Asn Gln Leu Glu Glu Arg Phe
   325 330 335
   Gln Leu Phe Glu Thr Asp Ile Val Asn Ile Ile Ser Asn Ile Ser Tyr
   340 345 350
   Thr Ala His His Leu Arg Thr Leu Thr Ser Asn Leu Asn Glu Val Arg
   355 360 365
   Thr Thr Cys Thr Asp Thr Leu Thr Lys His Thr Asp Asp Leu Thr Ser
   370 375 380
   Leu Asn Asn Thr Leu Ala Asn Ile Arg Leu Asp Ser Val Ser Leu Arg
   385 390 395 400
   Met Gln Gln Asp Leu Met Arg Ser Arg Leu Asp Thr Glu Val Ala Asn
   405 410 415
   Leu Ser Val Ile Met Glu Glu Met Lys Leu Val Asp Ser Lys His Gly
   420 425 430
   Gln Leu Ile Lys Asn Phe Thr Ile Leu Gln Gly Pro Pro Gly Pro Arg
   435 440 445
   Gly Pro Arg Gly Asp Arg Gly Ser Gln Gly Pro Pro Gly Pro Thr Gly
   450 455 460
   Asn Lys Gly Gln Lys Gly Glu Lys Gly Glu Pro Gly Pro Pro Gly Pro
   465 470 475 480
   Ala Gly Glu Arg Gly Pro Ile Gly Pro Ala Gly Pro Pro Gly Glu Arg
   485 490 495
   Gly Gly Lys Gly Ser Lys Gly Ser Gln Gly Pro Lys Gly Ser Arg Gly
   500 505 510
   Ser Pro Gly Lys Pro Gly Pro Gln Gly Ser Ser Gly Asp Pro Gly Pro
   515 520 525
   Pro Gly Pro Pro Gly Lys Glu Gly Leu Pro Gly Pro Gln Gly Pro Pro
   530 535 540
   Gly Phe Gln Gly Leu Gln Gly Thr Val Gly Glu Pro Gly Val Pro Gly
   545 550 555 560
   Pro Arg Gly Leu Pro Gly Leu Pro Gly Val Pro Gly Met Pro Gly Pro
   565 570 575
   Lys Gly Pro Pro Gly Pro Pro Gly Pro Ser Gly Ala Val Val Pro Leu
   580 585 590
   Ala Leu Gln Asn Glu Pro Thr Pro Ala Pro Glu Asp Asn Gly Cys Pro
   595 600 605
   Pro His Trp Lys Asn Phe Thr Asp Lys Cys Tyr Tyr Phe Ser Val Glu
   610 615 620
   Lys Glu Ile Phe Glu Asp Ala Lys Leu Phe Cys Glu Asp Lys Ser Ser
   625 630 635 640
   His Leu Val Phe Ile Asn Thr Arg Glu Glu Gln Gln Trp Ile Lys Lys
   645 650 655
   Gln Met Val Gly Arg Glu Ser His Trp Ile Gly Leu Thr Asp Ser Glu
   660 665 670
   Arg Glu Asn Glu Trp Lys Trp Leu Asp Gly Thr Ser Pro Asp Tyr Lys
   675 680 685
   Asn Trp Lys Ala Gly Gln Pro Asp Asn Trp Gly His Gly His Gly Pro
   690 695 700
   Gly Glu Asp Cys Ala Gly Leu Ile Tyr Ala Gly Gln Trp Asn Asp Phe
   705 710 715 720
   Gln Cys Glu Asp Val Asn Asn Phe Ile Cys Glu Lys Asp Arg Glu Thr
   725 730 735
   Val Leu Ser Ser Ala Leu
   740
<210> 60
   <211> 1214
   <212> PRT
   <213> Homo sapiens
<400> 60
<210> 61
   <211> 199
   <212> PRT
   <213> Homo sapiens
<400> 61
   Met Ala Asn Arg Gly Pro Ala Tyr Gly Leu Ser Arg Glu Val Gln Gln
   1 5 10 15
   Lys Ile Glu Lys Gln Tyr Asp Ala Asp Leu Glu Gln Ile Leu Ile Gln
   20 25 30
   Trp Ile Thr Thr Gln Cys Arg Lys Asp Val Gly Arg Pro Gln Pro Gly
   35 40 45
   Arg Glu Asn Phe Gln Asn Trp Leu Lys Asp Gly Thr Val Leu Cys Glu
   50 55 60
   Leu Ile Asn Ala Leu Tyr Pro Glu Gly Gln Ala Pro Val Lys Lys Ile
   65 70 75 80
   Gln Ala Ser Thr Met Ala Phe Lys Gln Met Glu Gln Ile Ser Gln Phe
   85 90 95
   Leu Gln Ala Ala Glu Arg Tyr Gly Ile Asn Thr Thr Asp Ile Phe Gln
   100 105 110
   Thr Val Asp Leu Trp Glu Gly Lys Asn Met Ala Cys Val Gln Arg Thr
   115 120 125
   Leu Met Asn Leu Gly Gly Leu Ala Val Ala Arg Asp Asp Gly Leu Phe
       130                 135                 140
   Ser Gly Asp Pro Asn Trp Phe Pro Lys Lys Ser Lys Glu Asn Pro Arg
   145                 150                 155                 160
   Asn Phe Ser Asp Asn Gln Leu Gln Glu Gly Lys Asn Val Ile Gly Leu
                   165                 170                 175
   Gln Met Gly Thr Asn Arg Gly Ala Ser Gln Ala Gly Met Thr Gly Tyr
               180                 185                 190
   Gly Met Pro Arg Gln Ile Leu
           195
<210> 62
   <211> 539
   <212> PRT
   <213> Homo sapiens
<400> 62
   Met Pro Glu Asn Val Ala Pro Arg Ser Gly Ala Thr Ala Gly Ala Ala
   1               5                   10                  15
   Gly Gly Arg Gly Lys Gly Ala Tyr Gln Asp Arg Asp Lys Pro Ala Gln
               20                  25                  30
   Ile Arg Phe Ser Asn Ile Ser Ala Ala Lys Ala Val Ala Asp Ala Ile
           35                  40                  45
   Arg Thr Ser Leu Gly Pro Lys Gly Met Asp Lys Met Ile Gln Asp Gly
       50                  55                  60
   Lys Gly Asp Val Thr Ile Thr Asn Asp Gly Ala Thr Ile Leu Lys Gln
   65                  70                  75                  80
   Met Gln Val Leu His Pro Ala Ala Arg Met Leu Val Glu Leu Ser Lys
                   85                  90                  95
   Ala Gln Asp Ile Glu Ala Gly Asp Gly Thr Thr Ser Val Val Ile Ile
               100                 105                 110
   Ala Gly Ser Leu Leu Asp Ser Cys Thr Lys Leu Leu Gln Lys Gly Ile
           115                 120                 125
   His Pro Thr Ile Ile Ser Glu Ser Phe Gln Lys Ala Leu Glu Lys Gly
       130                 135                 140
   Ile Glu Ile Leu Thr Asp Met Ser Arg Pro Val Glu Leu Ser Asp Arg
   145 150 155 160
   Glu Thr Leu Leu Asn Ser Ala Thr Thr Ser Leu Asn Ser Lys Val Val
   165 170 175
   Ser Gln Tyr Ser Ser Leu Leu Ser Pro Met Ser Val Asn Ala Val Met
   180 185 190
   Lys Val Ile Asp Pro Ala Thr Ala Thr Ser Val Asp Leu Arg Asp Ile
   195 200 205
   Lys Ile Val Lys Lys Leu Gly Gly Thr Ile Asp Asp Cys Glu Leu Val
   210 215 220
   Glu Gly Leu Val Leu Thr Gln Lys Val Ser Asn Ser Gly Ile Thr Arg
   225 230 235 240
   Val Glu Lys Ala Lys Ile Gly Leu Ile Gln Phe Cys Leu Ser Ala Pro
   245 250 255
   Lys Thr Asp Met Asp Asn Gln Ile Val Val Ser Asp Tyr Ala Gln Met
   260 265 270
   Asp Arg Val Leu Arg Glu Glu Arg Ala Tyr Ile Leu Asn Leu Val Lys
   275 280 285
   Gln Ile Lys Lys Thr Gly Cys Asn Val Leu Leu Ile Gln Lys Ser Ile
   290 295 300
   Leu Arg Asp Ala Leu Ser Asp Leu Ala Leu His Phe Leu Asn Lys Met
   305 310 315 320
   Lys Ile Met Val Ile Lys Asp Ile Glu Arg Glu Asp Ile Glu Phe Ile
   325 330 335
   Cys Lys Thr Ile Gly Thr Lys Pro Val Ala His Ile Asp Gln Phe Thr
   340 345 350
   Ala Asp Met Leu Gly Ser Ala Glu Leu Ala Glu Glu Val Asn Leu Asn
   355 360 365
   Gly Ser Gly Lys Leu Leu Lys Ile Thr Gly Cys Ala Ser Pro Gly Lys
   370 375 380
   Thr Val Thr Ile Val Val Arg Gly Ser Asn Lys Leu Val Ile Glu Glu
   385 390 395 400
   Ala Glu Arg Ser Ile His Asp Ala Leu Cys Val Ile Arg Cys Leu Val
   405 410 415
   Lys Lys Arg Ala Leu Ile Ala Gly Gly Gly Ala Pro Glu Ile Glu Leu
   420 425 430
   Ala Leu Arg Leu Thr Glu Tyr Ser Arg Thr Leu Ser Gly Met Glu Ser
   435 440 445
   Tyr Cys Val Arg Ala Phe Ala Asp Ala Met Glu Val Ile Pro Ser Thr
   450 455 460
   Leu Ala Glu Asn Ala Gly Leu Asn Pro Ile Ser Thr Val Thr Glu Leu
   465 470 475 480
   Arg Asn Arg His Ala Gln Gly Glu Lys Thr Ala Gly Ile Asn Val Arg
   485 490 495
   Lys Gly Gly Ile Ser Asn Ile Leu Glu Glu Leu Val Val Gln Pro Leu
   500 505 510
   Leu Val Ser Val Ser Ala Leu Thr Leu Ala Thr Glu Thr Val Arg Ser
   515 520 525
   Ile Leu Lys Ile Asp Asp Val Val Asn Thr Arg
   530 535
<210> 63
   <211> 758
   <212> PRT
   <213> Homo sapiens
<400> 63
   Met Ala Gln Arg Lys Asn Ala Lys Ser Ser Gly Asn Ser Ser Ser Ser
   1 5 10 15
   Gly Ser Gly Ser Gly Ser Thr Ser Ala Gly Ser Ser Ser Pro Gly Ala
   20 25 30
   Arg Arg Glu Thr Lys His Gly Gly His Lys Asn Gly Arg Lys Gly Gly
   35 40 45
   Leu Ser Gly Thr Ser Phe Phe Thr Trp Phe Met Val Ile Ala Leu Leu
   50 55 60
   Gly Val Trp Thr Ser Val Ala Val Val Trp Phe Asp Leu Val Asp Tyr
   65 70 75 80
   Glu Glu Val Leu Gly Lys Leu Gly Ile Tyr Asp Ala Asp Gly Asp Gly
   85 90 95
   Asp Phe Asp Val Asp Asp Ala Lys Val Leu Leu Gly Leu Lys Glu Arg
   100 105 110
   Ser Thr Ser Glu Pro Ala Val Pro Pro Glu Glu Ala Glu Pro His Thr
   115 120 125
   Glu Pro Glu Glu Gln Val Pro Val Glu Ala Glu Pro Gln Asn Ile Glu
   130 135 140
   Asp Glu Ala Lys Glu Gln Ile Gln Ser Leu Leu His Glu Met Val His
   145 150 155 160
   Ala Glu His Val Glu Gly Glu Asp Leu Gln Gln Glu Asp Gly Pro Thr
   165 170 175
   Gly Glu Pro Gln Gln Glu Asp Asp Glu Phe Leu Met Ala Thr Asp Val
   180 185 190
   Asp Asp Arg Phe Glu Thr Leu Glu Pro Glu Val Ser His Glu Glu Thr
   195 200 205
   Glu His Ser Tyr His Val Glu Glu Thr Val Ser Gln Asp Cys Asn Gln
   210 215 220
   Asp Met Glu Glu Met Met Ser Glu Gln Glu Asn Pro Asp Ser Ser Glu
   225 230 235 240
   Pro Val Val Glu Asp Glu Arg Leu His His Asp Thr Asp Asp Val Thr
   245 250 255
   Tyr Gln Val Tyr Glu Glu Gln Ala Val Tyr Glu Pro Leu Glu Asn Glu
   260 265 270
   Gly Ile Glu Ile Thr Glu Val Thr Ala Pro Pro Glu Asp Asn Pro Val
   275 280 285
   Glu Asp Ser Gln Val Ile Val Glu Glu Val Ser Ile Phe Pro Val Glu
   290 295 300
   Glu Gln Gln Glu Val Pro Pro Glu Thr Asn Arg Lys Thr Asp Asp Pro
   305 310 315 320
   Glu Gln Lys Ala Lys Val Lys Lys Lys Lys Pro Lys Leu Leu Asn Lys
   325 330 335
   Phe Asp Lys Thr Ile Lys Ala Glu Leu Asp Ala Ala Glu Lys Leu Arg
   340 345 350
   Lys Arg Gly Lys Ile Glu Glu Ala Val Asn Ala Phe Lys Glu Leu Val
   355 360 365
   Arg Lys Tyr Pro Gln Ser Pro Arg Ala Arg Tyr Gly Lys Ala Gln Cys
   370 375 380
   Glu Asp Asp Leu Ala Glu Lys Arg Arg Ser Asn Glu Val Leu Arg Gly
   385 390 395 400
   Ala Ile Glu Thr Tyr Gln Glu Val Ala Ser Leu Pro Asp Val Pro Ala
   405 410 415
   Asp Leu Leu Lys Leu Ser Leu Lys Arg Arg Ser Asp Arg Gln Gln Phe
   420 425 430
   Leu Gly His Met Arg Gly Ser Leu Leu Thr Leu Gln Arg Leu Val Gln
   435 440 445
   Leu Phe Pro Asn Asp Thr Ser Leu Lys Asn Asp Leu Gly Val Gly Tyr
   450 455 460
   Leu Leu Ile Gly Asp Asn Asp Asn Ala Lys Lys Val Tyr Glu Glu Val
   465 470 475 480
   Leu Ser Val Thr Pro Asn Asp Gly Phe Ala Lys Val His Tyr Gly Phe
   485 490 495
   Ile Leu Lys Ala Gln Asn Lys Ile Ala Glu Ser Ile Pro Tyr Leu Lys
   500 505 510
   Glu Gly Ile Glu Ser Gly Asp Pro Gly Thr Asp Asp Gly Arg Phe Tyr
   515 520 525
   Phe His Leu Gly Asp Ala Met Gln Arg Val Gly Asn Lys Glu Ala Tyr
   530 535 540
   Lys Trp Tyr Glu Leu Gly His Lys Arg Gly His Phe Ala Ser Val Trp
   545 550 555 560
   Gln Arg Ser Leu Tyr Asn Val Asn Gly Leu Lys Ala Gln Pro Trp Trp
   565 570 575
   Thr Pro Lys Glu Thr Gly Tyr Thr Glu Leu Val Lys Ser Leu Glu Arg
                   580                 585                 590
   Asn Trp Lys Leu Ile Arg Asp Glu Gly Leu Ala Val Met Asp Lys Ala
           595                 600                 605
   Lys Gly Leu Phe Leu Pro Glu Asp Glu Asn Leu Arg Glu Lys Gly Asp
       610                 615                 620
   Trp Ser Gln Phe Thr Leu Trp Gln Gln Gly Arg Arg Asn Glu Asn Ala
   625                 630                 635                 640
   Cys Lys Gly Ala Pro Lys Thr Cys Thr Leu Leu Glu Lys Phe Pro Glu
                   645                 650                 655
   Thr Thr Gly Cys Arg Arg Gly Gln Ile Lys Tyr Ser Ile Met His Pro
               660                 665                 670
   Gly Thr His Val Trp Pro His Thr Gly Pro Thr Asn Cys Arg Leu Arg
           675                 680                 685
   Met His Leu Gly Leu Val Ile Pro Lys Glu Gly Cys Lys Ile Arg Cys
       690                 695                 700
   Ala Asn Glu Thr Lys Thr Trp Glu Glu Gly Lys Val Leu Ile Phe Asp
   705                 710                 715                 720
   Asp Ser Phe Glu His Glu Val Trp Gln Asp Ala Ser Ser Phe Arg Leu
                   725                 730                 735
   Ile Phe Ile Val Asp Val Trp His Pro Glu Leu Thr Pro Gln Gln Arg
               740                 745                 750
   Arg Ser Leu Pro Ala Ile
           755
<210> 64
   <211> 491
   <212> PRT
   <213> Homo sapiens
<400> 64
   Met Pro Leu Pro Val Gln Val Phe Asn Leu Gln Gly Ala Val Glu Pro
   1               5                   10                  15
   Met Gln Ile Asp Val Asp Pro Gln Glu Asp Pro Gln Asn Ala Pro Asp
               20                  25                  30
   Val Asn Tyr Val Val Glu Asn Pro Ser Leu Asp Leu Glu Gln Tyr Ala
           35                  40                  45
   Ala Ser Tyr Ser Gly Leu Met Arg Ile Glu Arg Leu Gln Phe Ile Ala
       50                  55                  60
   Asp His Cys Pro Thr Leu Arg Val Glu Ala Leu Lys Met Ala Leu Ser
   65                  70                  75                  80
   Phe Val Gln Arg Thr Phe Asn Val Asp Met Tyr Glu Glu Ile His Arg
                   85                  90                  95
   Lys Leu Ser Glu Ala Thr Arg Ser Ser Leu Arg Glu Leu Gln Asn Ala
               100                 105                 110
   Pro Asp Ala Ile Pro Glu Ser Gly Val Glu Pro Pro Ala Leu Asp Thr
           115                 120                 125
   Ala Trp Val Glu Ala Thr Arg Lys Lys Ala Leu Leu Lys Leu Glu Lys
       130                 135                 140
   Leu Asp Thr Asp Leu Lys Asn Tyr Lys Gly Asn Ser Ile Lys Glu Ser
   145                 150                 155                 160
   Ile Arg Arg Gly His Asp Asp Leu Gly Asp His Tyr Leu Asp Cys Gly
                   165                 170                 175
   Asp Leu Ser Asn Ala Leu Lys Cys Tyr Ser Arg Ala Arg Asp Tyr Cys
               180                 185                 190
   Thr Ser Ala Lys His Val Ile Asn Met Cys Leu Asn Val Ile Lys Val
           195                 200                 205
   Ser Val Tyr Leu Gln Asn Trp Ser His Val Leu Ser Tyr Val Ser Lys
       210                 215                 220
   Ala Glu Ser Thr Pro Glu Ile Ala Glu Gln Arg Gly Glu Arg Asp Ser
   225                 230                 235                 240
   Gln Thr Gln Ala Ile Leu Thr Lys Leu Lys Cys Ala Ala Gly Leu Ala
                   245                 250                 255
   Glu Leu Ala Ala Arg Lys Tyr Lys Gln Ala Ala Lys Cys Leu Leu Leu
               260                 265                 270
   Ala Ser Phe Asp His Cys Asp Phe Pro Glu Leu Leu Ser Pro Ser Asn
           275                 280                 285
   Val Ala Ile Tyr Gly Gly Leu Cys Ala Leu Ala Thr Phe Asp Arg Gln
   290 295 300
   Glu Leu Gln Arg Asn Val Ile Ser Ser Ser Ser Phe Lys Leu Phe Leu
   305 310 315 320
   Glu Leu Glu Pro Gln Val Arg Asp Ile Ile Phe Lys Phe Tyr Glu Ser
   325 330 335
   Lys Tyr Ala Ser Cys Leu Lys Met Leu Asp Glu Met Lys Asp Asn Leu
   340 345 350
   Leu Leu Asp Met Tyr Leu Ala Pro His Val Arg Thr Leu Tyr Thr Gln
   355 360 365
   Ile Arg Asn Arg Ala Leu Ile Gln Tyr Phe Ser Pro Tyr Val Ser Ala
   370 375 380
   Asp Met His Arg Met Ala Ala Ala Phe Asn Thr Thr Val Ala Ala Leu
   385 390 395 400
   Glu Asp Glu Leu Thr Gln Leu Ile Leu Glu Gly Leu Ile Ser Ala Arg
   405 410 415
   Val Asp Ser His Ser Lys Ile Leu Tyr Ala Arg Asp Val Asp Gln Arg
   420 425 430
   Ser Thr Thr Phe Glu Lys Ser Leu Leu Met Gly Lys Glu Phe Gln Arg
   435 440 445
   Arg Ala Lys Ala Met Met Leu Arg Ala Ala Val Leu Arg Asn Gln Ile
   450 455 460
   His Val Lys Ser Pro Pro Arg Glu Gly Ser Gln Gly Glu Leu Thr Pro
   465 470 475 480
   Ala Asn Ser Gln Ser Arg Met Ser Thr Asn Met
   485 490
<210> 65
   <211> 1512
   <212> PRT
   <213> Homo sapiens
<400> 65
<210> 66
   <211> 352
   <212> PRT
   <213> Homo sapiens
<400> 66
   Met Ser Val Ala Gly Leu Lys Lys Gln Phe His Lys Ala Thr Gln Lys
   1 5 10 15
   Val Ser Glu Lys Val Gly Gly Ala Glu Gly Thr Lys Leu Asp Asp Asp
   20 25 30
   Phe Lys Glu Met Glu Arg Lys Val Asp Val Thr Ser Arg Ala Val Met
   35 40 45
   Glu Ile Met Thr Lys Thr Ile Glu Tyr Leu Gln Pro Asn Pro Ala Ser
   50 55 60
   Arg Ala Lys Leu Ser Met Ile Asn Thr Met Ser Lys Ile Arg Gly Gln
   65 70 75 80
   Glu Lys Gly Pro Gly Tyr Pro Gln Ala Glu Ala Leu Leu Ala Glu Ala
   85 90 95
   Met Leu Lys Phe Gly Arg Glu Leu Gly Asp Asp Cys Asn Phe Gly Pro
   100 105 110
   Ala Leu Gly Glu Val Gly Glu Ala Met Arg Glu Leu Ser Glu Val Lys
   115 120 125
   Asp Ser Leu Asp Ile Glu Val Lys Gln Asn Phe Ile Asp Pro Leu Gln
   130 135 140
   Asn Leu His Asp Lys Asp Leu Arg Glu Ile Gln His His Leu Lys Lys
   145 150 155 160
   Leu Glu Gly Arg Arg Leu Asp Phe Asp Tyr Lys Lys Lys Arg Gln Gly
   165 170 175
   Lys Ile Pro Asp Glu Glu Leu Arg Gln Ala Leu Glu Lys Phe Asp Glu
   180 185 190
   Ser Lys Glu Ile Ala Glu Ser Ser Met Phe Asn Leu Leu Glu Met Asp
   195 200 205
   Ile Glu Gln Val Ser Gln Leu Ser Ala Leu Val Gln Ala Gln Leu Glu
   210 215 220
   Tyr His Lys Gln Ala Val Gln Ile Leu Gln Gln Val Thr Val Arg Leu
   225 230 235 240
   Glu Glu Arg Ile Arg Gln Ala Ser Ser Gln Pro Arg Arg Glu Tyr Gln
   245 250 255
   Pro Lys Pro Arg Met Ser Leu Glu Phe Pro Thr Gly Asp Ser Thr Gln
   260 265 270
   Pro Asn Gly Gly Leu Ser His Thr Gly Thr Pro Lys Pro Ser Gly Val
   275 280 285
   Gln Met Asp Gln Pro Cys Cys Arg Ala Leu Tyr Asp Phe Glu Pro Glu
   290 295 300
   Asn Glu Gly Glu Leu Gly Phe Lys Glu Gly Asp Ile Ile Thr Leu Thr
   305 310 315 320
   Asn Gln Ile Asp Glu Asn Trp Tyr Glu Gly Met Leu His Gly His Ser
   325 330 335
   Gly Phe Phe Pro Ile Asn Tyr Val Glu Ile Leu Val Ala Leu Pro His
   340 345 350
<210> 67
   <211> 142
   <212> PRT
   <213> Homo sapiens
<400> 67
<210> 68
   <211> 318
   <212> PRT
   <213> Homo sapiens
<400> 68
   Met Pro Lys Arg Gly Lys Lys Gly Ala Val Ala Glu Asp Gly Asp Glu
   1 5 10 15
   Leu Arg Thr Glu Pro Glu Ala Lys Lys Ser Lys Thr Ala Ala Lys Lys
   20 25 30
   Asn Asp Lys Glu Ala Ala Gly Glu Gly Pro Ala Leu Tyr Glu Asp Pro
   35 40 45
   Pro Asp Gln Lys Thr Ser Pro Ser Gly Lys Pro Ala Thr Leu Lys Ile
   50 55 60
   Cys Ser Trp Asn Val Asp Gly Leu Arg Ala Trp Ile Lys Lys Lys Gly
   65 70 75 80
   Leu Asp Trp Val Lys Glu Glu Ala Pro Asp Ile Leu Cys Leu Gln Glu
   85 90 95
   Thr Lys Cys Ser Glu Asn Lys Leu Pro Ala Glu Leu Gln Glu Leu Pro
   100 105 110
   Gly Leu Ser His Gln Tyr Trp Ser Ala Pro Ser Asp Lys Glu Gly Tyr
   115 120 125
   Ser Gly Val Gly Leu Leu Ser Arg Gln Cys Pro Leu Lys Val Ser Tyr
   130 135 140
   Gly Ile Gly Asp Glu Glu His Asp Gln Glu Gly Arg Val Ile Val Ala
   145 150 155 160
   Glu Phe Asp Ser Phe Val Leu Val Thr Ala Tyr Val Pro Asn Ala Gly
                   165                 170                 175
   Arg Gly Leu Val Arg Leu Glu Tyr Arg Gln Arg Trp Asp Glu Ala Phe
               180                 185                 190
   Arg Lys Phe Leu Lys Gly Leu Ala Ser Arg Lys Pro Leu Val Leu Cys
           195                 200                 205
   Gly Asp Leu Asn Val Ala His Glu Glu Ile Asp Leu Arg Asn Pro Lys
       210                 215                 220
   Gly Asn Lys Lys Asn Ala Gly Phe Thr Pro Gln Glu Arg Gln Gly Phe
   225                 230                 235                 240
   Gly Glu Leu Leu Gln Ala Val Pro Leu Ala Asp Ser Phe Arg His Leu
                   245                 250                 255
   Tyr Pro Asn Thr Pro Tyr Ala Tyr Thr Phe Trp Thr Tyr Met Met Asn
               260                 265                 270
   Ala Arg Ser Lys Asn Val Gly Trp Arg Leu Asp Tyr Phe Leu Leu Ser
           275                 280                 285
   His Ser Leu Leu Pro Ala Leu Cys Asp Ser Lys Ile Arg Ser Lys Ala
       290                 295                 300
   Leu Gly Ser Asp His Cys Pro Ile Thr Leu Tyr Leu Ala Leu
   305                 310                 315
<210> 69
   <211> 263
   <212> PRT
   <213> Homo sapiens
<400> 69
   Met Ala Leu Ala Ser Val Leu Glu Arg Pro Leu Pro Val Asn Gln Arg
   1               5                   10                  15
   Gly Phe Phe Gly Leu Gly Gly Arg Ala Asp Leu Leu Asp Leu Gly Pro
               20                  25                  30
   Gly Ser Leu Ser Asp Gly Leu Ser Leu Ala Ala Pro Gly Trp Gly Val
           35                  40                  45
   Pro Glu Glu Pro Gly Ile Glu Met Leu His Gly Thr Thr Thr Leu Ala
       50                  55                  60
   Phe Lys Phe Arg His Gly Val Ile Val Ala Ala Asp Ser Arg Ala Thr
   65 70 75 80
   Ala Gly Ala Tyr Ile Ala Ser Gln Thr Val Lys Lys Val Ile Glu Ile
   85 90 95
   Asn Pro Tyr Leu Leu Gly Thr Met Ala Gly Gly Ala Ala Asp Cys Ser
   100 105 110
   Phe Trp Glu Arg Leu Leu Ala Arg Gln Cys Arg Ile Tyr Glu Leu Arg
   115 120 125
   Asn Lys Glu Arg Ile Ser Val Ala Ala Ala Ser Lys Leu Leu Ala Asn
   130 135 140
   Met Val Tyr Gln Tyr Lys Gly Met Gly Leu Ser Met Gly Thr Met Ile
   145 150 155 160
   Cys Gly Trp Asp Lys Arg Gly Pro Gly Leu Tyr Tyr Val Asp Ser Glu
   165 170 175
   Gly Asn Arg Ile Ser Gly Ala Thr Phe Ser Val Gly Ser Gly Ser Val
   180 185 190
   Tyr Ala Tyr Gly Val Met Asp Arg Gly Tyr Ser Tyr Asp Leu Glu Val
   195 200 205
   Glu Gln Ala Tyr Asp Leu Ala Arg Arg Ala Ile Tyr Gln Ala Thr Tyr
   210 215 220
   Arg Asp Ala Tyr Ser Gly Gly Ala Val Asn Leu Tyr His Val Arg Glu
   225 230 235 240
   Asp Gly Trp Ile Arg Val Ser Ser Asp Asn Val Ala Asp Leu His Glu
   245 250 255
   Lys Tyr Ser Gly Ser Thr Pro
   260
<210> 70
   <211> 105
   <212> PRT
   <213> Homo sapiens
<400> 70
   Met Val Lys Gln Ile Glu Ser Lys Thr Ala Phe Gln Glu Ala Leu Asp
   1 5 10 15
   Ala Ala Gly Asp Lys Leu Val Val Val Asp Phe Ser Ala Thr Trp Cys
   20 25 30
   Gly Pro Cys Lys Met Ile Lys Pro Phe Phe His Ser Leu Ser Glu Lys
   35 40 45
   Tyr Ser Asn Val Ile Phe Leu Glu Val Asp Val Asp Asp Cys Gln Asp
   50 55 60
   Val Ala Ser Glu Cys Glu Val Lys Cys Met Pro Thr Phe Gln Phe Phe
   65 70 75 80
   Lys Lys Gly Gln Lys Val Gly Glu Phe Ser Gly Ala Asn Lys Glu Lys
   85 90 95
   Leu Glu Ala Thr Ile Asn Glu Leu Val
   100 105
<210> 71
   <211> 894
   <212> PRT
   <213> Homo sapiens
<400> 71
   Met Ala Trp Arg Cys Pro Arg Met Gly Arg Val Pro Leu Ala Trp Cys
   1 5 10 15
   Leu Ala Leu Cys Gly Trp Ala Cys Met Ala Pro Arg Gly Thr Gln Ala
   20 25 30
   Glu Glu Ser Pro Phe Val Gly Asn Pro Gly Asn Ile Thr Gly Ala Arg
   35 40 45
   Gly Leu Thr Gly Thr Leu Arg Cys Gln Leu Gln Val Gln Gly Glu Pro
   50 55 60
   Pro Glu Val His Trp Leu Arg Asp Gly Gln Ile Leu Glu Leu Ala Asp
   65 70 75 80
   Ser Thr Gln Thr Gln Val Pro Leu Gly Glu Asp Glu Gln Asp Asp Trp
   85 90 95
   Ile Val Val Ser Gln Leu Arg Ile Thr Ser Leu Gln Leu Ser Asp Thr
   100 105 110
   Gly Gln Tyr Gln Cys Leu Val Phe Leu Gly His Gln Thr Phe Val Ser
   115 120 125
   Gln Pro Gly Tyr Val Gly Leu Glu Gly Leu Pro Tyr Phe Leu Glu Glu
   130 135 140
   Pro Glu Asp Arg Thr Val Ala Ala Asn Thr Pro Phe Asn Leu Ser Cys
   145 150 155 160
   Gln Ala Gln Gly Pro Pro Glu Pro Val Asp Leu Leu Trp Leu Gln Asp
   165 170 175
   Ala Val Pro Leu Ala Thr Ala Pro Gly His Gly Pro Gln Arg Ser Leu
   180 185 190
   His Val Pro Gly Leu Asn Lys Thr Ser Ser Phe Ser Cys Glu Ala His
   195 200 205
   Asn Ala Lys Gly Val Thr Thr Ser Arg Thr Ala Thr Ile Thr Val Leu
   210 215 220
   Pro Gln Gln Pro Arg Asn Leu His Leu Val Ser Arg Gln Pro Thr Glu
   225 230 235 240
   Leu Glu Val Ala Trp Thr Pro Gly Leu Ser Gly Ile Tyr Pro Leu Thr
   245 250 255
   His Cys Thr Leu Gln Ala Val Leu Ser Asn Asp Gly Met Gly Ile Gln
   260 265 270
   Ala Gly Glu Pro Asp Pro Pro Glu Glu Pro Leu Thr Ser Gln Ala Ser
   275 280 285
   Val Pro Pro His Gln Leu Arg Leu Gly Ser Leu His Pro His Thr Pro
   290 295 300
   Tyr His Ile Arg Val Ala Cys Thr Ser Ser Gln Gly Pro Ser Ser Trp
   305 310 315 320
   Thr His Trp Leu Pro Val Glu Thr Pro Glu Gly Val Pro Leu Gly Pro
   325 330 335
   Pro Glu Asn Ile Ser Ala Thr Arg Asn Gly Ser Gln Ala Phe Val His
   340 345 350
   Trp Gln Glu Pro Arg Ala Pro Leu Gln Gly Thr Leu Leu Gly Tyr Arg
   355 360 365
   Leu Ala Tyr Gln Gly Gln Asp Thr Pro Glu Val Leu Met Asp Ile Gly
   370 375 380
   Leu Arg Gln Glu Val Thr Leu Glu Leu Gln Gly Asp Gly Ser Val Ser
   385 390 395 400
   Asn Leu Thr Val Cys Val Ala Ala Tyr Thr Ala Ala Gly Asp Gly Pro
   405 410 415
   Trp Ser Leu Pro Val Pro Leu Glu Ala Trp Arg Pro Gly Gln Ala Gln
   420 425 430
   Pro Val His Gln Leu Val Lys Glu Pro Ser Thr Pro Ala Phe Ser Trp
   435 440 445
   Pro Trp Trp Tyr Val Leu Leu Gly Ala Val Val Ala Ala Ala Cys Val
   450 455 460
   Leu Ile Leu Ala Leu Phe Leu Val His Arg Arg Lys Lys Glu Thr Arg
   465 470 475 480
   Tyr Gly Glu Val Phe Glu Pro Thr Val Glu Arg Gly Glu Leu Val Val
   485 490 495
   Arg Tyr Arg Val Arg Lys Ser Tyr Ser Arg Arg Thr Thr Glu Ala Thr
   500 505 510
   Leu Asn Ser Leu Gly Ile Ser Glu Glu Leu Lys Glu Lys Leu Arg Asp
   515 520 525
   Val Met Val Asp Arg His Lys Val Ala Leu Gly Lys Thr Leu Gly Glu
   530 535 540
   Gly Glu Phe Gly Ala Val Met Glu Gly Gln Leu Asn Gln Asp Asp Ser
   545 550 555 560
   Ile Leu Lys Val Ala Val Lys Thr Met Lys Ile Ala Ile Cys Thr Arg
   565 570 575
   Ser Glu Leu Glu Asp Phe Leu Ser Glu Ala Val Cys Met Lys Glu Phe
   580 585 590
   Asp His Pro Asn Val Met Arg Leu Ile Gly Val Cys Phe Gln Gly Ser
   595 600 605
   Glu Arg Glu Ser Phe Pro Ala Pro Val Val Ile Leu Pro Phe Met Lys
   610 615 620
   His Gly Asp Leu His Ser Phe Leu Leu Tyr Ser Arg Leu Gly Asp Gln
   625 630 635 640
   Pro Val Tyr Leu Pro Thr Gln Met Leu Val Lys Phe Met Ala Asp Ile
   645 650 655
   Ala Ser Gly Met Glu Tyr Leu Ser Thr Lys Arg Phe Ile His Arg Asp
   660 665 670
   Leu Ala Ala Arg Asn Cys Met Leu Asn Glu Asn Met Ser Val Cys Val
   675 680 685
   Ala Asp Phe Gly Leu Ser Lys Lys Ile Tyr Asn Gly Asp Tyr Tyr Arg
   690 695 700
   Gln Gly Arg Ile Ala Lys Met Pro Val Lys Trp Ile Ala Ile Glu Ser
   705 710 715 720
   Leu Ala Asp Arg Val Tyr Thr Ser Lys Ser Asp Val Trp Ser Phe Gly
   725 730 735
   Val Thr Met Trp Glu Ile Ala Thr Arg Gly Gln Thr Pro Tyr Pro Gly
   740 745 750
   Val Glu Asn Ser Glu Ile Tyr Asp Tyr Leu Arg Gln Gly Asn Arg Leu
   755 760 765
   Lys Gln Pro Ala Asp Cys Leu Asp Gly Leu Tyr Ala Leu Met Ser Arg
   770 775 780
   Cys Trp Glu Leu Asn Pro Gln Asp Arg Pro Ser Phe Thr Glu Leu Arg
   785 790 795 800
   Glu Asp Leu Glu Asn Thr Leu Lys Ala Leu Pro Pro Ala Gln Glu Pro
   805 810 815
   Asp Glu Ile Leu Tyr Val Asn Met Asp Glu Gly Gly Gly Tyr Pro Glu
   820 825 830
   Pro Pro Gly Ala Ala Gly Gly Ala Asp Pro Pro Thr Gln Pro Asp Pro
   835 840 845
   Lys Asp Ser Cys Ser Cys Leu Thr Ala Ala Glu Val His Pro Ala Gly
   850 855 860
   Arg Tyr Val Leu Cys Pro Ser Thr Thr Pro Ser Pro Ala Gln Pro Ala
   865 870 875 880
   Asp Arg Gly Ser Pro Ala Ala Pro Gly Gln Glu Asp Gly Ala
   885 890
<210> 72
   <211> 208
   <212> PRT
   <213> Homo sapiens
<400> 72
<210> 73
   <211> 290
   <212> PRT
   <213> Homo sapiens
<400> 73
<210> 74
   <211> 152
   <212> PRT
   <213> Homo sapiens
<400> 74
<210> 75
   <211> 166
   <212> PRT
   <213> Homo sapiens
<400> 75
   Met Ala Ser Gly Val Ala Val Ser Asp Gly Val Ile Lys Val Phe Asn
   1 5 10 15
   Asp Met Lys Val Arg Lys Ser Ser Thr Pro Glu Glu Val Lys Lys Arg
   20 25 30
   Lys Lys Ala Val Leu Phe Cys Leu Ser Glu Asp Lys Lys Asn Ile Ile
   35 40 45
   Leu Glu Glu Gly Lys Glu Ile Leu Val Gly Asp Val Gly Gln Thr Val
   50 55 60
   Asp Asp Pro Tyr Ala Thr Phe Val Lys Met Leu Pro Asp Lys Asp Cys
   65 70 75 80
   Arg Tyr Ala Leu Tyr Asp Ala Thr Tyr Glu Thr Lys Glu Ser Lys Lys
   85 90 95
   Glu Asp Leu Val Phe Ile Phe Trp Ala Pro Glu Ser Ala Pro Leu Lys
   100 105 110
   Ser Lys Met Ile Tyr Ala Ser Ser Lys Asp Ala Ile Lys Lys Lys Leu
   115 120 125
   Thr Gly Ile Lys His Glu Leu Gln Ala Asn Cys Tyr Glu Glu Val Lys
   130 135 140
   Asp Arg Cys Thr Leu Ala Glu Lys Leu Gly Gly Ser Ala Val Ile Ser
   145 150 155 160
   Leu Glu Gly Lys Pro Leu
   165
<210> 76
   <211> 476
   <212> PRT
   <213> Homo sapiens
<400> 76
   Met Lys Phe Gln Gly Pro Leu Ala Cys Leu Leu Leu Ala Leu Cys Leu
   1               5                   10                  15
   Gly Ser Gly Glu Ala Gly Pro Leu Gln Ser Gly Glu Glu Ser Thr Gly
               20                  25                  30
   Thr Asn Ile Gly Glu Ala Leu Gly His Gly Leu Gly Asp Ala Leu Ser
           35                  40                  45
   Glu Gly Val Gly Lys Ala Ile Gly Lys Glu Ala Gly Gly Ala Ala Gly
       50                  55                  60
   Ser Lys Val Ser Glu Ala Leu Gly Gln Gly Thr Arg Glu Ala Val Gly
   65                  70                  75                  80
   Thr Gly Val Arg Gln Val Pro Gly Phe Gly Val Ala Asp Ala Leu Gly
                   85                  90                  95
   Asn Arg Val Gly Glu Ala Ala His Ala Leu Gly Asn Thr Gly His Glu
               100                 105                 110
   Ile Gly Arg Gln Ala Glu Asp Val Ile Arg His Gly Ala Asp Ala Val
           115                 120                 125
   Arg Gly Ser Trp Gln Gly Val Pro Gly His Asn Gly Ala Trp Glu Thr
       130                 135                 140
   Ser Gly Gly His Gly Ile Phe Gly Ser Gln Gly Gly Leu Gly Gly Gln
   145                 150                 155                 160
   Gly Gln Gly Asn Pro Gly Gly Leu Gly Thr Pro Trp Val His Gly Tyr
                   165                 170                 175
   Pro Gly Asn Ser Ala Gly Ser Phe Gly Met Asn Pro Gln Gly Ala Pro
               180                 185                 190
   Trp Gly Gln Gly Gly Asn Gly Gly Pro Pro Asn Phe Gly Thr Asn Thr
           195                 200                 205
   Gln Gly Ala Val Ala Gln Pro Gly Tyr Gly Ser Val Arg Ala Ser Asn
       210                 215                 220
   Gln Asn Glu Gly Cys Thr Asn Pro Pro Pro Ser Gly Ser Gly Gly Gly
   225                 230                 235                 240
   Ser Ser Asn Ser Gly Gly Gly Ser Gly Ser Gln Ser Gly Ser Ser Gly
                   245                 250                 255
   Ser Gly Ser Asn Gly Asp Asn Asn Asn Gly Ser Ser Ser Gly Gly Ser
   260 265 270
   Ser Ser Gly Ser Ser Ser Gly Gly Ser Ser Gly Gly Ser Ser Gly Gly
   275 280 285
   Ser Ser Gly Asn Ser Gly Gly Ser Arg Gly Asp Ser Gly Ser Glu Ser
   290 295 300
   Ser Trp Gly Ser Ser Thr Gly Ser Ser Ser Gly Asn His Gly Gly Ser
   305 310 315 320
   Gly Gly Gly Asn Gly His Lys Pro Gly Cys Glu Lys Pro Gly Asn Glu
   325 330 335
   Ala Arg Gly Ser Gly Glu Ser Gly Ile Gln Asn Ser Glu Thr Ser Pro
   340 345 350
   Gly Met Phe Asn Phe Asp Thr Phe Trp Lys Asn Phe Lys Ser Lys Leu
   355 360 365
   Gly Phe Ile Asn Trp Asp Ala Ile Asn Lys Asn Gln Val Pro Pro Pro
   370 375 380
   Ser Thr Arg Ala Leu Leu Tyr Phe Ser Arg Leu Trp Glu Asp Phe Lys
   385 390 395 400
   Gln Asn Thr Pro Phe Leu Asn Trp Lys Ala Ile Ile Glu Gly Ala Asp
   405 410 415
   Ala Ser Ser Leu Gln Lys Arg Ala Gly Arg Asp Asp Gln Asn Tyr Asn
   420 425 430
   Tyr Asn Gln His Ala Tyr Pro Thr Ala Tyr Gly Gly Lys Tyr Ser Val
   435 440 445
   Lys Thr Pro Ala Lys Gly Gly Val Ser Pro Ser Ser Ser Ala Ser Arg
   450 455 460
   Val Gln Pro Gly Leu Leu Gln Trp Val Lys Phe Trp
   465 470 475
<210> 77
   <211> 128
   <212> PRT
   <213> Homo sapiens
<400> 77
<210> 78
   <211> 288
   <212> PRT
   <213> Homo sapiens
<400> 78
<210> 79
   <211> 582
   <212> PRT
   <213> Homo sapiens
<400> 79
<210> 80
   <211> 410
   <212> PRT
   <213> Homo sapiens
<400> 80
<210> 81
   <211> 98
   <212> PRT
   <213> Homo sapiens
<400> 81
   Met Ala Ala Glu Pro Leu Thr Glu Leu Glu Glu Ser Ile Glu Thr Val
   1               5                   10                  15
   Val Thr Thr Phe Phe Thr Phe Ala Arg Gln Glu Gly Arg Lys Asp Ser
               20                  25                  30
   Leu Ser Val Asn Glu Phe Lys Glu Leu Val Thr Gln Gln Leu Pro His
           35                  40                  45
   Leu Leu Lys Asp Val Gly Ser Leu Asp Glu Lys Met Lys Ser Leu Asp
       50                  55                  60
   Val Asn Gln Asp Ser Glu Leu Lys Phe Asn Glu Tyr Trp Arg Leu Ile
   65                  70                  75                  80
   Gly Glu Leu Ala Lys Glu Ile Arg Lys Lys Lys Asp Leu Lys Ile Arg
                   85                  90                  95
   Lys Lys
<210> 82
   <211> 246
   <212> PRT
   <213> Homo sapiens
<400> 82
   Met Ser Arg Gly Ser Ser Ala Gly Phe Asp Arg His Ile Thr Ile Phe
   1               5                   10                  15
   Ser Pro Glu Gly Arg Leu Tyr Gln Val Glu Tyr Ala Phe Lys Ala Ile
               20                  25                  30
   Asn Gln Gly Gly Leu Thr Ser Val Ala Val Arg Gly Lys Asp Cys Ala
           35                  40                  45
   Val Ile Val Thr Gln Lys Lys Val Pro Asp Lys Leu Leu Asp Ser Ser
       50                  55                  60
   Thr Val Thr His Leu Phe Lys Ile Thr Glu Asn Ile Gly Cys Val Met
   65                  70                  75                  80
   Thr Gly Met Thr Ala Asp Ser Arg Ser Gln Val Gln Arg Ala Arg Tyr
                   85                  90                  95
   Glu Ala Ala Asn Trp Lys Tyr Lys Tyr Gly Tyr Glu Ile Pro Val Asp
               100                 105                 110
   Met Leu Cys Lys Arg Ile Ala Asp Ile Ser Gln Val Tyr Thr Gln Asn
           115                 120                 125
   Ala Glu Met Arg Pro Leu Gly Cys Cys Met Ile Leu Ile Gly Ile Asp
       130                 135                 140
   Glu Glu Gln Gly Pro Gln Val Tyr Lys Cys Asp Pro Ala Gly Tyr Tyr
   145                 150                 155                 160
   Cys Gly Phe Lys Ala Thr Ala Ala Gly Val Lys Gln Thr Glu Ser Thr
                   165                 170                 175
   Ser Phe Leu Glu Lys Lys Val Lys Lys Lys Phe Asp Trp Thr Phe Glu
               180                 185                 190
   Gln Thr Val Glu Thr Ala Ile Thr Cys Leu Ser Thr Val Leu Ser Ile
           195                 200                 205
   Asp Phe Lys Pro Ser Glu Ile Glu Val Gly Val Val Thr Val Glu Asn
       210                 215                 220
   Pro Lys Phe Arg Ile Leu Thr Glu Ala Glu Ile Asp Ala His Leu Val
   225                 230                 235                 240
   Ala Leu Ala Glu Arg Asp
                   245
<210> 83
   <211> 402
   <212> PRT
   <213> Homo sapiens
<400> 83
<210> 84
   <211> 709
   <212> PRT
   <213> Homo sapiens
<400> 84
   Met Pro Ser Ala Thr Ser His Ser Gly Ser Gly Ser Lys Ser Ser Gly
   1 5 10 15
   Pro Pro Pro Pro Ser Gly Ser Ser Gly Ser Glu Ala Ala Ala Gly Ala
   20 25 30
   Gly Ala Ala Ala Pro Ala Ser Gln His Pro Ala Thr Gly Thr Gly Ala
   35 40 45
   Val Gln Thr Glu Ala Met Lys Gln Ile Leu Gly Val Ile Asp Lys Lys
   50 55 60
   Leu Arg Asn Leu Glu Lys Lys Lys Gly Lys Leu Asp Asp Tyr Gln Glu
   65 70 75 80
   Arg Met Asn Lys Gly Glu Arg Leu Asn Gln Asp Gln Leu Asp Ala Val
   85 90 95
   Ser Lys Tyr Gln Glu Val Thr Asn Asn Leu Glu Phe Ala Lys Glu Leu
   100 105 110
   Gln Arg Ser Phe Met Ala Leu Ser Gln Asp Ile Gln Lys Thr Ile Lys
   115 120 125
   Lys Thr Ala Arg Arg Glu Gln Leu Met Arg Glu Glu Ala Glu Gln Lys
   130 135 140
   Arg Leu Lys Thr Val Leu Glu Leu Gln Tyr Val Leu Asp Lys Leu Gly
   145 150 155 160
   Asp Asp Glu Val Arg Thr Asp Leu Lys Gln Gly Leu Asn Gly Val Pro
   165 170 175
   Ile Leu Ser Glu Glu Glu Leu Ser Leu Leu Asp Glu Phe Tyr Lys Leu
   180 185 190
   Val Asp Pro Glu Arg Asp Met Ser Leu Arg Leu Asn Glu Gln Tyr Glu
   195 200 205
   His Ala Ser Ile His Leu Trp Asp Leu Leu Glu Gly Lys Glu Lys Pro
   210 215 220
   Val Cys Gly Thr Thr Tyr Lys Val Leu Lys Glu Ile Val Glu Arg Val
   225 230 235 240
   Phe Gln Ser Asn Tyr Phe Asp Ser Thr His Asn His Gln Asn Gly Leu
   245 250 255
   Cys Glu Glu Glu Glu Ala Ala Ser Ala Pro Ala Val Glu Asp Gln Val
   260 265 270
   Pro Glu Ala Glu Pro Glu Pro Ala Glu Glu Tyr Thr Glu Gln Ser Glu
   275 280 285
   Val Glu Ser Thr Glu Tyr Val Asn Arg Gln Phe Met Ala Glu Thr Gln
   290 295 300
   Phe Thr Ser Gly Glu Lys Glu Gln Val Asp Glu Trp Thr Val Glu Thr
   305 310 315 320
   Val Glu Val Val Asn Ser Leu Gln Gln Gln Pro Gln Ala Ala Ser Pro
   325 330 335
   Ser Val Pro Glu Pro His Ser Leu Thr Pro Val Ala Gln Ala Asp Pro
   340 345 350
   Leu Val Arg Arg Gln Arg Val Gln Asp Leu Met Ala Gln Met Gln Gly
   355 360 365
   Pro Tyr Asn Phe Ile Gln Asp Ser Met Leu Asp Phe Glu Asn Gln Thr
       370                 375                 380
   Leu Asp Pro Ala Ile Val Ser Ala Gln Pro Met Asn Pro Thr Gln Asn
   385                 390                 395                 400
   Met Asp Met Pro Gln Leu Val Cys Pro Pro Val His Ser Glu Ser Arg
                   405                 410                 415
   Leu Ala Gln Pro Asn Gln Val Pro Val Gln Pro Glu Ala Thr Gln Val
               420                 425                 430
   Pro Leu Val Ser Ser Thr Ser Glu Gly Tyr Thr Ala Ser Gln Pro Leu
           435                 440                 445
   Tyr Gln Pro Ser His Ala Thr Glu Gln Arg Pro Gln Lys Glu Pro Ile
       450                 455                 460
   Asp Gln Ile Gln Ala Thr Ile Ser Leu Asn Thr Asp Gln Thr Thr Ala
   465                 470                 475                 480
   Ser Ser Ser Leu Pro Ala Ala Ser Gln Pro Gln Val Phe Gln Ala Gly
                   485                 490                 495
   Thr Ser Lys Pro Leu His Ser Ser Gly Ile Asn Val Asn Ala Ala Pro
               500                 505                 510
   Phe Gln Ser Met Gln Thr Val Phe Asn Met Asn Ala Pro Val Pro Pro
           515                 520                 525
   Val Asn Glu Pro Glu Thr Leu Lys Gln Gln Asn Gln Tyr Gln Ala Ser
       530                 535                 540
   Tyr Asn Gln Ser Phe Ser Ser Gln Pro His Gln Val Glu Gln Thr Glu
   545                 550                 555                 560
   Leu Gln Gln Glu Gln Leu Gln Thr Val Val Gly Thr Tyr His Gly Ser
                   565                 570                 575
   Pro Asp Gln Ser His Gln Val Thr Gly Asn His Gln Gln Pro Pro Gln
               580                 585                 590
   Gln Asn Thr Gly Phe Pro Arg Ser Asn Gln Pro Tyr Tyr Asn Ser Arg
           595                 600                 605
   Gly Val Ser Arg Gly Gly Ser Arg Gly Ala Arg Gly Leu Met Asn Gly
       610                 615                 620
   Tyr Arg Gly Pro Ala Asn Gly Phe Arg Gly Gly Tyr Asp Gly Tyr Arg
   625 630 635 640
   Pro Ser Phe Ser Asn Thr Pro Asn Ser Gly Tyr Thr Gln Ser Gln Phe
   645 650 655
   Ser Ala Pro Arg Asp Tyr Ser Gly Tyr Gln Arg Asp Gly Tyr Gln Gln
   660 665 670
   Asn Phe Lys Arg Gly Ser Gly Gln Ser Gly Pro Arg Gly Ala Pro Arg
   675 680 685
   Gly Arg Gly Gly Pro Pro Arg Pro Asn Arg Gly Met Pro Gln Met Asn
   690 695 700
   Thr Gln Gln Val Asn
   705
<210> 85
   <211> 437
   <212> PRT
   <213> Homo sapiens
<400> 85
   Met Ser Val Ser Ala Arg Ser Ala Ala Ala Glu Glu Arg Ser Val Asn
   1 5 10 15
   Ser Ser Thr Met Val Ala Gln Gln Lys Asn Leu Glu Gly Tyr Val Gly
   20 25 30
   Phe Ala Asn Leu Pro Asn Gln Val Tyr Arg Lys Ser Val Lys Arg Gly
   35 40 45
   Phe Glu Phe Thr Leu Met Val Val Gly Glu Ser Gly Leu Gly Lys Ser
   50 55 60
   Thr Leu Ile Asn Ser Leu Phe Leu Thr Asp Leu Tyr Ser Pro Glu Tyr
   65 70 75 80
   Pro Gly Pro Ser His Arg Ile Lys Lys Thr Val Gln Val Glu Gln Ser
   85 90 95
   Lys Val Leu Ile Lys Glu Gly Gly Val Gln Leu Leu Leu Thr Ile Val
   100 105 110
   Asp Thr Pro Gly Phe Gly Asp Ala Val Asp Asn Ser Asn Cys Trp Gln
   115 120 125
   Pro Val Ile Asp Tyr Ile Asp Ser Lys Phe Glu Asp Tyr Leu Asn Ala
       130                 135                 140
   Glu Ser Arg Val Asn Arg Arg Gln Met Pro Asp Asn Arg Val Gln Cys
   145                 150                 155                 160
   Cys Leu Tyr Phe Ile Ala Pro Ser Gly His Gly Leu Lys Pro Leu Asp
                   165                 170                 175
   Ile Glu Phe Met Lys Arg Leu His Glu Lys Val Asn Ile Ile Pro Leu
               180                 185                 190
   Ile Ala Lys Ala Asp Thr Leu Thr Pro Glu Glu Cys Gln Gln Phe Lys
           195                 200                 205
   Lys Gln Ile Met Lys Glu Ile Gln Glu His Lys Ile Lys Ile Tyr Glu
       210                 215                 220
   Phe Pro Glu Thr Asp Asp Glu Glu Glu Asn Lys Leu Val Lys Lys Ile
   225                 230                 235                 240
   Lys Asp Arg Leu Pro Leu Ala Val Val Gly Ser Asn Thr Ile Ile Glu
                   245                 250                 255
   Val Asn Gly Lys Arg Val Arg Gly Arg Gln Tyr Pro Trp Gly Val Ala
               260                 265                 270
   Glu Val Glu Asn Gly Glu His Cys Asp Phe Thr Ile Leu Arg Asn Met
           275                 280                 285
   Leu Ile Arg Thr His Met Gln Asp Leu Lys Asp Val Thr Asn Asn Val
       290                 295                 300
   His Tyr Glu Asn Tyr Arg Ser Arg Lys Leu Ala Ala Val Thr Tyr Asn
   305                 310                 315                 320
   Gly Val Asp Asn Asn Lys Asn Lys Gly Gln Leu Thr Lys Ser Pro Leu
                   325                 330                 335
   Ala Gln Met Glu Glu Glu Arg Arg Glu His Val Ala Lys Met Lys Lys
               340                 345                 350
   Met Glu Met Glu Met Glu Gln Val Phe Glu Met Lys Val Lys Glu Lys
           355                 360                 365
   Val Gln Lys Leu Lys Asp Ser Glu Ala Glu Leu Gln Arg Arg His Glu
   370 375 380
   Gln Met Lys Lys Asn Leu Glu Ala Gln His Lys Glu Leu Glu Glu Lys
   385 390 395 400
   Arg Arg Gln Phe Glu Asp Glu Lys Ala Asn Trp Glu Ala Gln Gln Arg
   405 410 415
   Ile Leu Glu Gln Gln Asn Ser Ser Arg Thr Leu Glu Lys Asn Lys Lys
   420 425 430
   Lys Gly Lys Ile Phe
   435
<210> 86
   <211> 478
   <212> PRT
   <213> Homo sapiens
<400> 86
   Met Ala Pro Leu Arg Pro Leu Leu Ile Leu Ala Leu Leu Ala Trp Val
   1               5                   10                  15
   Ala Leu Ala Asp Gln Glu Ser Cys Lys Gly Arg Cys Thr Glu Gly Phe
               20                  25                  30
   Asn Val Asp Lys Lys Cys Gln Cys Asp Glu Leu Cys Ser Tyr Tyr Gln
           35                  40                  45
   Ser Cys Cys Thr Asp Tyr Thr Ala Glu Cys Lys Pro Gln Val Thr Arg
       50                  55                  60
   Gly Asp Val Phe Thr Met Pro Glu Asp Glu Tyr Thr Val Tyr Asp Asp
   65                  70                  75                  80
   Gly Glu Glu Lys Asn Asn Ala Thr Val His Glu Gln Val Gly Gly Pro
                   85                  90                  95
   Ser Leu Thr Ser Asp Leu Gln Ala Gln Ser Lys Gly Asn Pro Glu Gln
               100                 105                 110
   Thr Pro Val Leu Lys Pro Glu Glu Glu Ala Pro Ala Pro Glu Val Gly
           115                 120                 125
   Ala Ser Lys Pro Glu Gly Ile Asp Ser Arg Pro Glu Thr Leu His Pro
       130                 135                 140
   Gly Arg Pro Gln Pro Pro Ala Glu Glu Glu Leu Cys Ser Gly Lys Pro
   145 150 155 160
   Phe Asp Ala Phe Thr Asp Leu Lys Asn Gly Ser Leu Phe Ala Phe Arg
   165 170 175
   Gly Gln Tyr Cys Tyr Glu Leu Asp Glu Lys Ala Val Arg Pro Gly Tyr
   180 185 190
   Pro Lys Leu Ile Arg Asp Val Trp Gly Ile Glu Gly Pro Ile Asp Ala
   195 200 205
   Ala Phe Thr Arg Ile Asn Cys Gln Gly Lys Thr Tyr Leu Phe Lys Gly
   210 215 220
   Ser Gln Tyr Trp Arg Phe Glu Asp Gly Val Leu Asp Pro Asp Tyr Pro
   225 230 235 240
   Arg Asn Ile Ser Asp Gly Phe Asp Gly Ile Pro Asp Asn Val Asp Ala
   245 250 255
   Ala Leu Ala Leu Pro Ala His Ser Tyr Ser Gly Arg Glu Arg Val Tyr
   260 265 270
   Phe Phe Lys Gly Lys Gln Tyr Trp Glu Tyr Gln Phe Gln His Gln Pro
   275 280 285
   Ser Gln Glu Glu Cys Glu Gly Ser Ser Leu Ser Ala Val Phe Glu His
   290 295 300
   Phe Ala Met Met Gln Arg Asp Ser Trp Glu Asp Ile Phe Glu Leu Leu
   305 310 315 320
   Phe Trp Gly Arg Thr Ser Ala Gly Thr Arg Gln Pro Gln Phe Ile Ser
   325 330 335
   Arg Asp Trp His Gly Val Pro Gly Gln Val Asp Ala Ala Met Ala Gly
   340 345 350
   Arg Ile Tyr Ile Ser Gly Met Ala Pro Arg Pro Ser Leu Ala Lys Lys
   355 360 365
   Gln Arg Phe Arg His Arg Asn Arg Lys Gly Tyr Arg Ser Gln Arg Gly
   370 375 380
   His Ser Arg Gly Arg Asn Gln Asn Ser Arg Arg Pro Ser Arg Ala Thr
   385 390 395 400
   Trp Leu Ser Leu Phe Ser Ser Glu Glu Ser Asn Leu Gly Ala Asn Asn
                   405                 410                 415
   Tyr Asp Asp Tyr Arg Met Asp Trp Leu Val Pro Ala Thr Cys Glu Pro
               420                 425                 430
   Ile Gln Ser Val Phe Phe Phe Ser Gly Asp Lys Tyr Tyr Arg Val Asn
           435                 440                 445
   Leu Arg Thr Arg Arg Val Asp Thr Val Asp Pro Pro Tyr Pro Arg Ser
       450                 455                 460
   Ile Ala Gln Tyr Trp Leu Gly Cys Pro Ala Pro Gly His Leu
   465                 470                 475
<210> 87
   <211> 863
   <212> PRT
   <213> Homo sapiens
<400> 87
   Met Ala Arg Arg Ser Ser Phe Gln Ser Cys Gln Ile Ile Ser Leu Phe
   1               5                   10                  15
   Thr Phe Ala Val Gly Val Asn Ile Cys Leu Gly Phe Thr Ala His Arg
               20                  25                  30
   Ile Lys Arg Ala Glu Gly Trp Glu Glu Gly Pro Pro Thr Val Leu Ser
           35                  40                  45
   Asp Ser Pro Trp Thr Asn Ile Ser Gly Ser Cys Lys Gly Arg Cys Phe
       50                  55                  60
   Glu Leu Gln Glu Ala Gly Pro Pro Asp Cys Arg Cys Asp Asn Leu Cys
   65                  70                  75                  80
   Lys Ser Tyr Thr Ser Cys Cys His Asp Phe Asp Glu Leu Cys Leu Lys
                   85                  90                  95
   Thr Ala Arg Gly Trp Glu Cys Thr Lys Asp Arg Cys Gly Glu Val Arg
               100                 105                 110
   Asn Glu Glu Asn Ala Cys His Cys Ser Glu Asp Cys Leu Ala Arg Gly
           115                 120                 125
   Asp Cys Cys Thr Asn Tyr Gln Val Val Cys Lys Gly Glu Ser His Trp
       130                 135                 140
   Val Asp Asp Asp Cys Glu Glu Ile Lys Ala Ala Glu Cys Pro Ala Gly
   145 150 155 160
   Phe Val Arg Pro Pro Leu Ile Ile Phe Ser Val Asp Gly Phe Arg Ala
   165 170 175
   Ser Tyr Met Lys Lys Gly Ser Lys Val Met Pro Asn Ile Glu Lys Leu
   180 185 190
   Arg Ser Cys Gly Thr His Ser Pro Tyr Met Arg Pro Val Tyr Pro Thr
   195 200 205
   Lys Thr Phe Pro Asn Leu Tyr Thr Leu Ala Thr Gly Leu Tyr Pro Glu
   210 215 220
   Ser His Gly Ile Val Gly Asn Ser Met Tyr Asp Pro Val Phe Asp Ala
   225 230 235 240
   Thr Phe His Leu Arg Gly Arg Glu Lys Phe Asn His Arg Trp Trp Gly
   245 250 255
   Gly Gln Pro Leu Trp Ile Thr Ala Thr Lys Gln Gly Val Lys Ala Gly
   260 265 270
   Thr Phe Phe Trp Ser Val Val Ile Pro His Glu Arg Arg Ile Leu Thr
   275 280 285
   Ile Leu Gln Trp Leu Thr Leu Pro Asp His Glu Arg Pro Ser Val Tyr
   290 295 300
   Ala Phe Tyr Ser Glu Gln Pro Asp Phe Ser Gly His Lys Tyr Gly Pro
   305 310 315 320
   Phe Gly Pro Glu Met Thr Asn Pro Leu Arg Glu Ile Asp Lys Ile Val
   325 330 335
   Gly Gln Leu Met Asp Gly Leu Lys Gln Leu Lys Leu His Arg Cys Val
   340 345 350
   Asn Val Ile Phe Val Gly Asp His Gly Met Glu Asp Val Thr Cys Asp
   355 360 365
   Arg Thr Glu Phe Leu Ser Asn Tyr Leu Thr Asn Val Asp Asp Ile Thr
   370 375 380
   Leu Val Pro Gly Thr Leu Gly Arg Ile Arg Ser Lys Phe Ser Asn Asn
   385 390 395 400
   Ala Lys Tyr Asp Pro Lys Ala Ile Ile Ala Asn Leu Thr Cys Lys Lys
   405 410 415
   Pro Asp Gln His Phe Lys Pro Tyr Leu Lys Gln His Leu Pro Lys Arg
   420 425 430
   Leu His Tyr Ala Asn Asn Arg Arg Ile Glu Asp Ile His Leu Leu Val
   435 440 445
   Glu Arg Arg Trp His Val Ala Arg Lys Pro Leu Asp Val Tyr Lys Lys
   450 455 460
   Pro Ser Gly Lys Cys Phe Phe Gln Gly Asp His Gly Phe Asp Asn Lys
   465 470 475 480
   Val Asn Ser Met Gln Thr Val Phe Val Gly Tyr Gly Ser Thr Phe Lys
   485 490 495
   Tyr Lys Thr Lys Val Pro Pro Phe Glu Asn Ile Glu Leu Tyr Asn Val
   500 505 510
   Met Cys Asp Leu Leu Gly Leu Lys Pro Ala Pro Asn Asn Gly Thr His
   515 520 525
   Gly Ser Leu Asn His Leu Leu Arg Thr Asn Thr Phe Arg Pro Thr Met
   530 535 540
   Pro Glu Glu Val Thr Arg Pro Asn Tyr Pro Gly Ile Met Tyr Leu Gln
   545 550 555 560
   Ser Asp Phe Asp Leu Gly Cys Thr Cys Asp Asp Lys Val Glu Pro Lys
   565 570 575
   Asn Lys Leu Asp Glu Leu Asn Lys Arg Leu His Thr Lys Gly Ser Thr
   580 585 590
   Glu Glu Arg His Leu Leu Tyr Gly Arg Pro Ala Val Leu Tyr Arg Thr
   595 600 605
   Arg Tyr Asp Ile Leu Tyr His Thr Asp Phe Glu Ser Gly Tyr Ser Glu
   610 615 620
   Ile Phe Leu Met Pro Leu Trp Thr Ser Tyr Thr Val Ser Lys Gln Ala
   625 630 635 640
   Glu Val Ser Ser Val Pro Asp His Leu Thr Ser Cys Val Arg Pro Asp
   645 650 655
   Val Arg Val Ser Pro Ser Phe Ser Gln Asn Cys Leu Ala Tyr Lys Asn
   660 665 670
   Asp Lys Gln Met Ser Tyr Gly Phe Leu Phe Pro Pro Tyr Leu Ser Ser
   675 680 685
   Ser Pro Glu Ala Lys Tyr Asp Ala Phe Leu Val Thr Asn Met Val Pro
   690 695 700
   Met Tyr Pro Ala Phe Lys Arg Val Trp Asn Tyr Phe Gln Arg Val Leu
   705 710 715 720
   Val Lys Lys Tyr Ala Ser Glu Arg Asn Gly Val Asn Val Ile Ser Gly
   725 730 735
   Pro Ile Phe Asp Tyr Asp Tyr Asp Gly Leu His Asp Thr Glu Asp Lys
   740 745 750
   Ile Lys Gln Tyr Val Glu Gly Ser Ser Ile Pro Val Pro Thr His Tyr
   755 760 765
   Tyr Ser Ile Ile Thr Ser Cys Leu Asp Phe Thr Gln Pro Ala Asp Lys
   770 775 780
   Cys Asp Gly Pro Leu Ser Val Ser Ser Phe Ile Leu Pro His Arg Pro
   785 790 795 800
   Asp Asn Glu Glu Ser Cys Asn Ser Ser Glu Asp Glu Ser Lys Trp Val
   805 810 815
   Glu Glu Leu Met Lys Met His Thr Ala Arg Val Arg Asp Ile Glu His
   820 825 830
   Leu Thr Ser Leu Asp Phe Phe Arg Lys Thr Ser Arg Ser Tyr Pro Glu
   835 840 845
   Ile Leu Thr Leu Lys Thr Tyr Leu His Thr Tyr Glu Ser Glu Ile
   850 855 860
<210> 88
   <211> 1461
   <212> PRT
   <213> Homo sapiens
<400> 88
   Met Ala Ala Glu Arg Gly Ala Arg Arg Leu Leu Ser Thr Pro Ser Phe
   1 5 10 15
   Trp Leu Tyr Cys Leu Leu Leu Leu Gly Arg Arg Ala Pro Gly Ala Ala
               20                  25                  30
   Ala Ala Arg Ser Gly Ser Ala Pro Gln Ser Pro Gly Ala Ser Ile Arg
           35                  40                  45
   Thr Phe Thr Pro Phe Tyr Phe Leu Val Glu Pro Val Asp Thr Leu Ser
       50                  55                  60
   Val Arg Gly Ser Ser Val Ile Leu Asn Cys Ser Ala Tyr Ser Glu Pro
   65                  70                  75                  80
   Ser Pro Lys Ile Glu Trp Lys Lys Asp Gly Thr Phe Leu Asn Leu Val
                   85                  90                  95
   Ser Asp Asp Arg Arg Gln Leu Leu Pro Asp Gly Ser Leu Phe Ile Ser
               100                 105                 110
   Asn Val Val His Ser Lys His Asn Lys Pro Asp Glu Gly Tyr Tyr Gln
           115                 120                 125
   Cys Val Ala Thr Val Glu Ser Leu Gly Thr Ile Ile Ser Arg Thr Ala
       130                 135                 140
   Lys Leu Ile Val Ala Gly Leu Pro Arg Phe Thr Ser Gln Pro Glu Pro
   145                 150                 155                 160
   Ser Ser Val Tyr Ala Gly Asn Asn Ala Ile Leu Asn Cys Glu Val Asn
                   165                 170                 175
   Ala Asp Leu Val Pro Phe Val Arg Trp Glu Gln Asn Arg Gln Pro Leu
               180                 185                 190
   Leu Leu Asp Asp Arg Val Ile Lys Leu Pro Ser Gly Met Leu Val Ile
           195                 200                 205
   Ser Asn Ala Thr Glu Gly Asp Gly Gly Leu Tyr Arg Cys Val Val Glu
       210                 215                 220
   Ser Gly Gly Pro Pro Lys Tyr Ser Asp Glu Val Glu Leu Lys Val Leu
   225                 230                 235                 240
   Pro Asp Pro Glu Val Ile Ser Asp Leu Val Phe Leu Lys Gln Pro Ser
                   245                 250                 255
   Pro Leu Val Arg Val Ile Gly Gln Asp Val Val Leu Pro Cys Val Ala
               260                 265                 270
   Ser Gly Leu Pro Thr Pro Thr Ile Lys Trp Met Lys Asn Glu Glu Ala
           275                 280                 285
   Leu Asp Thr Glu Ser Ser Glu Arg Leu Val Leu Leu Ala Gly Gly Ser
       290                 295                 300
   Leu Glu Ile Ser Asp Val Thr Glu Asp Asp Ala Gly Thr Tyr Phe Cys
   305                 310                 315                 320
   Ile Ala Asp Asn Gly Asn Glu Thr Ile Glu Ala Gln Ala Glu Leu Thr
                   325                 330                 335
   Val Gln Ala Gln Pro Glu Phe Leu Lys Gln Pro Thr Asn Ile Tyr Ala
               340                 345                 350
   His Glu Ser Met Asp Ile Val Phe Glu Cys Glu Val Thr Gly Lys Pro
           355                 360                 365
   Thr Pro Thr Val Lys Trp Val Lys Asn Gly Asp Met Val Ile Pro Ser
       370                 375                 380
   Asp Tyr Phe Lys Ile Val Lys Glu His Asn Leu Gln Val Leu Gly Leu
   385                 390                 395                 400
   Val Lys Ser Asp Glu Gly Phe Tyr Gln Cys Ile Ala Glu Asn Asp Val
                   405                 410                 415
   Gly Asn Ala Gln Ala Gly Ala Gln Leu Ile Ile Leu Glu His Ala Pro
               420                 425                 430
   Ala Thr Thr Gly Pro Leu Pro Ser Ala Pro Arg Asp Val Val Ala Ser
           435                 440                 445
   Leu Val Ser Thr Arg Phe Ile Lys Leu Thr Trp Arg Thr Pro Ala Ser
       450                 455                 460
   Asp Pro His Gly Asp Asn Leu Thr Tyr Ser Val Phe Tyr Thr Lys Glu
   465                 470                 475                 480
   Gly Ile Ala Arg Glu Arg Val Glu Asn Thr Ser His Pro Gly Glu Met
                   485                 490                 495
   Gln Val Thr Ile Gln Asn Leu Met Pro Ala Thr Val Tyr Ile Phe Arg
               500                 505                 510
   Val Met Ala Gln Asn Lys His Gly Ser Gly Glu Ser Ser Ala Pro Leu
   515 520 525
   Arg Val Glu Thr Gln Pro Glu Val Gln Leu Pro Gly Pro Ala Pro Asn
   530 535 540
   Leu Arg Ala Tyr Ala Ala Ser Pro Thr Ser Ile Thr Val Thr Trp Glu
   545 550 555 560
   Thr Pro Val Ser Gly Asn Gly Glu Ile Gln Asn Tyr Lys Leu Tyr Tyr
   565 570 575
   Met Glu Lys Gly Thr Asp Lys Glu Gln Asp Val Asp Val Ser Ser His
   580 585 590
   Ser Tyr Thr Ile Asn Gly Leu Lys Lys Tyr Thr Glu Tyr Ser Phe Arg
   595 600 605
   Val Val Ala Tyr Asn Lys His Gly Pro Gly Val Ser Thr Pro Asp Val
   610 615 620
   Ala Val Arg Thr Leu Ser Asp Val Pro Ser Ala Ala Pro Gln Asn Leu
   625 630 635 640
   Ser Leu Glu Val Arg Asn Ser Lys Ser Ile Met Ile His Trp Gln Pro
   645 650 655
   Pro Ala Pro Ala Thr Gln Asn Gly Gln Ile Thr Gly Tyr Lys Ile Arg
   660 665 670
   Tyr Arg Lys Ala Ser Arg Lys Ser Asp Val Thr Glu Thr Leu Val Ser
   675 680 685
   Gly Thr Gln Leu Ser Gln Leu Ile Glu Gly Leu Asp Arg Gly Thr Glu
   690 695 700
   Tyr Asn Phe Arg Val Ala Ala Leu Thr Ile Asn Gly Thr Gly Pro Ala
   705 710 715 720
   Thr Asp Trp Leu Ser Ala Glu Thr Phe Glu Ser Asp Leu Asp Glu Thr
   725 730 735
   Arg Val Pro Glu Val Pro Ser Ser Leu His Val Arg Pro Leu Val Thr
   740 745 750
   Ser Ile Val Val Ser Trp Thr Pro Pro Glu Asn Gln Asn Ile Val Val
   755 760 765
   Arg Gly Tyr Ala Ile Gly Tyr Gly Ile Gly Ser Pro His Ala Gln Thr
       770                 775                 780
   Ile Lys Val Asp Tyr Lys Gln Arg Tyr Tyr Thr Ile Glu Asn Leu Asp
   785                 790                 795                 800
   Pro Ser Ser His Tyr Val Ile Thr Leu Lys Ala Phe Asn Asn Val Gly
                   805                 810                 815
   Glu Gly Ile Pro Leu Tyr Glu Ser Ala Val Thr Arg Pro His Thr Asp
               820                 825                 830
   Thr Ser Glu Val Asp Leu Phe Val Ile Asn Ala Pro Tyr Thr Pro Val
           835                 840                 845
   Pro Asp Pro Thr Pro Met Met Pro Pro Val Gly Val Gln Ala Ser Ile
       850                 855                 860
   Leu Ser His Asp Thr Ile Arg Ile Thr Trp Ala Asp Asn Ser Leu Pro
   865                 870                 875                 880
   Lys His Gln Lys Ile Thr Asp Ser Arg Tyr Tyr Thr Val Arg Trp Lys
                   885                 890                 895
   Thr Asn Ile Pro Ala Asn Thr Lys Tyr Lys Asn Ala Asn Ala Thr Thr
               900                 905                 910
   Leu Ser Tyr Leu Val Thr Gly Leu Lys Pro Asn Thr Leu Tyr Glu Phe
           915                 920                 925
   Ser Val Met Val Thr Lys Gly Arg Arg Ser Ser Thr Trp Ser Met Thr
       930                 935                 940
   Ala His Gly Thr Thr Phe Glu Leu Val Pro Thr Ser Pro Pro Lys Asp
   945                 950                 955                 960
   Val Thr Val Val Ser Lys Glu Gly Lys Pro Lys Thr Ile Ile Val Asn
                   965                 970                 975
   Trp Gln Pro Pro Ser Glu Ala Asn Gly Lys Ile Thr Gly Tyr Ile Ile
               980                 985                 990
   Tyr Tyr Ser Thr Asp Val Asn Ala  Glu Ile His Asp Trp  Val Ile Glu
           995                 1000                 1005
   Pro Val  Val Gly Asn Arg Leu  Thr His Gln Ile Gln  Glu Leu Thr
       1010                 1015                 1020
   Leu Asp Thr Pro Tyr Tyr Phe Lys Ile Gln Ala Arg Asn Ser Lys
   1025 1030 1035
   Gly Met Gly Pro Met Ser Glu Ala Val Gln Phe Arg Thr Pro Lys
   1040 1045 1050
   Ala Asp Ser Ser Asp Lys Met Pro Asn Asp Gln Ala Ser Gly Ser
   1055 1060 1065
   Gly Gly Lys Gly Ser Arg Leu Pro Asp Leu Gly Ser Asp Tyr Lys
   1070 1075 1080
   Pro Pro Met Ser Gly Ser Asn Ser Pro His Gly Ser Pro Thr Ser
   1085 1090 1095
   Pro Leu Asp Ser Asn Met Leu Leu Val Ile Ile Val Ser Val Gly
   1100 1105 1110
   Val Ile Thr Ile Val Val Val Val Ile Ile Ala Val Phe Cys Thr
   1115 1120 1125
   Arg Arg Thr Thr Ser His Gln Lys Lys Lys Arg Ala Ala Cys Lys
   1130 1135 1140
   Ser Val Asn Gly Ser His Lys Tyr Lys Gly Asn Ser Lys Asp Val
   1145 1150 1155
   Lys Pro Pro Asp Leu Trp Ile His His Glu Arg Leu Glu Leu Lys
   1160 1165 1170
   Pro Ile Asp Lys Ser Pro Asp Pro Asn Pro Ile Met Thr Asp Thr
   1175 1180 1185
   Pro Ile Pro Arg Asn Ser Gln Asp Ile Thr Pro Val Asp Asn Ser
   1190 1195 1200
   Met Asp Ser Asn Ile His Gln Arg Arg Asn Ser Tyr Arg Gly His
   1205 1210 1215
   Glu Ser Glu Asp Ser Met Ser Thr Leu Ala Gly Arg Arg Gly Met
   1220 1225 1230
   Arg Pro Lys Met Met Met Pro Phe Asp Ser Gln Pro Pro Gln Pro
   1235 1240 1245
   Val Ile Ser Ala His Pro Ile His Ser Leu Asp Asn Pro His His
   1250 1255 1260
   His Phe His Ser Ser Ser Leu Ala Ser Pro Ala Arg Ser His Leu
   1265 1270 1275
   Tyr His Pro Gly Ser Pro Trp Pro Ile Gly Thr Ser Met Ser Leu
   1280 1285 1290
   Ser Asp Arg Ala Asn Ser Thr Glu Ser Val Arg Asn Thr Pro Ser
   1295 1300 1305
   Thr Asp Thr Met Pro Ala Ser Ser Ser Gln Thr Cys Cys Thr Asp
   1310 1315 1320
   His Gln Asp Pro Glu Gly Ala Thr Ser Ser Ser Tyr Leu Ala Ser
   1325 1330 1335
   Ser Gln Glu Glu Asp Ser Gly Gln Ser Leu Pro Thr Ala His Val
   1340 1345 1350
   Arg Pro Ser His Pro Leu Lys Ser Phe Ala Val Pro Ala Ile Pro
   1355 1360 1365
   Pro Pro Gly Pro Pro Thr Tyr Asp Pro Ala Leu Pro Ser Thr Pro
   1370 1375 1380
   Leu Leu Ser Gln Gln Ala Leu Asn His His Ile His Ser Val Lys
   1385 1390 1395
   Thr Ala Ser Ile Gly Thr Leu Gly Arg Ser Arg Pro Pro Met Pro
   1400 1405 1410
   Val Val Val Pro Ser Ala Pro Glu Val Gln Glu Thr Thr Arg Met
   1415 1420 1425
   Leu Glu Asp Ser Glu Ser Ser Tyr Glu Pro Asp Glu Leu Thr Lys
   1430 1435 1440
   Glu Met Ala His Leu Glu Gly Leu Met Lys Asp Leu Asn Ala Ile
   1445 1450 1455
   Thr Thr Ala
   1460
<210> 89
   <211> 514
   <212> PRT
   <213> Homo sapiens
<400> 89
   Met Ala Asp Ala Glu Val Ile Ile Leu Pro Lys Lys His Lys Lys Lys
   1               5                   10                  15
   Lys Glu Arg Lys Ser Leu Pro Glu Glu Asp Val Ala Glu Ile Gln His
               20                  25                  30
   Ala Glu Glu Phe Leu Ile Lys Pro Glu Ser Lys Val Ala Lys Leu Asp
           35                  40                  45
   Thr Ser Gln Trp Pro Leu Leu Leu Lys Asn Phe Asp Lys Leu Asn Val
       50                  55                  60
   Arg Thr Thr His Tyr Thr Pro Leu Ala Cys Gly Ser Asn Pro Leu Lys
   65                  70                  75                  80
   Arg Glu Ile Gly Asp Tyr Ile Arg Thr Gly Phe Ile Asn Leu Asp Lys
                   85                  90                  95
   Pro Ser Asn Pro Ser Ser His Glu Val Val Ala Trp Ile Arg Arg Ile
               100                 105                 110
   Leu Arg Val Glu Lys Thr Gly His Ser Gly Thr Leu Asp Pro Lys Val
           115                 120                 125
   Thr Gly Cys Leu Ile Val Cys Ile Glu Arg Ala Thr Arg Leu Val Lys
       130                 135                 140
   Ser Gln Gln Ser Ala Gly Lys Glu Tyr Val Gly Ile Val Arg Leu His
   145                 150                 155                 160
   Asn Ala Ile Glu Gly Gly Thr Gln Leu Ser Arg Ala Leu Glu Thr Leu
                   165                 170                 175
   Thr Gly Ala Leu Phe Gln Arg Pro Pro Leu Ile Ala Ala Val Lys Arg
               180                 185                 190
   Gln Leu Arg Val Arg Thr Ile Tyr Glu Ser Lys Met Ile Glu Tyr Asp
           195                 200                 205
   Pro Glu Arg Arg Leu Gly Ile Phe Trp Val Ser Cys Glu Ala Gly Thr
       210                 215                 220
   Tyr Ile Arg Thr Leu Cys Val His Leu Gly Leu Leu Leu Gly Val Gly
   225                 230                 235                 240
   Gly Gln Met Gln Glu Leu Arg Arg Val Arg Ser Gly Val Met Ser Glu
   245 250 255
   Lys Asp His Met Val Thr Met His Asp Val Leu Asp Ala Gln Trp Leu
   260 265 270
   Tyr Asp Asn His Lys Asp Glu Ser Tyr Leu Arg Arg Val Val Tyr Pro
   275 280 285
   Leu Glu Lys Leu Leu Thr Ser His Lys Arg Leu Val Met Lys Asp Ser
   290 295 300
   Ala Val Asn Ala Ile Cys Tyr Gly Ala Lys Ile Met Leu Pro Gly Val
   305 310 315 320
   Leu Arg Tyr Glu Asp Gly Ile Glu Val Asn Gln Glu Ile Val Val Ile
   325 330 335
   Thr Thr Lys Gly Glu Ala Ile Cys Met Ala Ile Ala Leu Met Thr Thr
   340 345 350
   Ala Val Ile Ser Thr Cys Asp His Gly Ile Val Ala Lys Ile Lys Arg
   355 360 365
   Val Ile Met Glu Arg Asp Thr Tyr Pro Arg Lys Trp Gly Leu Gly Pro
   370 375 380
   Lys Ala Ser Gln Lys Lys Leu Met Ile Lys Gln Gly Leu Leu Asp Lys
   385 390 395 400
   His Gly Lys Pro Thr Asp Ser Thr Pro Ala Thr Trp Lys Gln Glu Tyr
   405 410 415
   Val Asp Tyr Ser Glu Ser Ala Lys Lys Glu Val Val Ala Glu Val Val
   420 425 430
   Lys Ala Pro Gln Val Val Ala Glu Ala Ala Lys Thr Ala Lys Arg Lys
   435 440 445
   Arg Glu Ser Glu Ser Glu Ser Asp Glu Thr Pro Pro Ala Ala Pro Gln
   450 455 460
   Leu Ile Lys Lys Glu Lys Lys Lys Ser Lys Lys Asp Lys Lys Ala Lys
   465 470 475 480
   Ala Gly Leu Glu Ser Gly Ala Glu Pro Gly Asp Gly Asp Ser Asp Thr
   485 490 495
   Thr Lys Lys Lys Lys Lys Lys Lys Lys Ala Lys Glu Val Glu Leu Val
   500 505 510
   Ser Glu
<210> 90
   <211> 383
   <212> PRT
   <213> Homo sapiens
<400> 90
   Met Gly Val Lys Ala Ser Gln Thr Gly Phe Val Val Leu Val Leu Leu
   1 5 10 15
   Gln Cys Cys Ser Ala Tyr Lys Leu Val Cys Tyr Tyr Thr Ser Trp Ser
   20 25 30
   Gln Tyr Arg Glu Gly Asp Gly Ser Cys Phe Pro Asp Ala Leu Asp Arg
   35 40 45
   Phe Leu Cys Thr His Ile Ile Tyr Ser Phe Ala Asn Ile Ser Asn Asp
   50 55 60
   His Ile Asp Thr Trp Glu Trp Asn Asp Val Thr Leu Tyr Gly Met Leu
   65 70 75 80
   Asn Thr Leu Lys Asn Arg Asn Pro Asn Leu Lys Thr Leu Leu Ser Val
   85 90 95
   Gly Gly Trp Asn Phe Gly Ser Gln Arg Phe Ser Lys Ile Ala Ser Asn
   100 105 110
   Thr Gln Ser Arg Arg Thr Phe Ile Lys Ser Val Pro Pro Phe Leu Arg
   115 120 125
   Thr His Gly Phe Asp Gly Leu Asp Leu Ala Trp Leu Tyr Pro Gly Arg
   130 135 140
   Arg Asp Lys Gln His Phe Thr Thr Leu Ile Lys Glu Met Lys Ala Glu
   145 150 155 160
   Phe Ile Lys Glu Ala Gln Pro Gly Lys Lys Gln Leu Leu Leu Ser Ala
   165 170 175
   Ala Leu Ser Ala Gly Lys Val Thr Ile Asp Ser Ser Tyr Asp Ile Ala
   180 185 190
   Lys Ile Ser Gln His Leu Asp Phe Ile Ser Ile Met Thr Tyr Asp Phe
           195                 200                 205
   His Gly Ala Trp Arg Gly Thr Thr Gly His His Ser Pro Leu Phe Arg
       210                 215                 220
   Gly Gln Glu Asp Ala Ser Pro Asp Arg Phe Ser Asn Thr Asp Tyr Ala
   225                 230                 235                 240
   Val Gly Tyr Met Leu Arg Leu Gly Ala Pro Ala Ser Lys Leu Val Met
                   245                 250                 255
   Gly Ile Pro Thr Phe Gly Arg Ser Phe Thr Leu Ala Ser Ser Glu Thr
               260                 265                 270
   Gly Val Gly Ala Pro Ile Ser Gly Pro Gly Ile Pro Gly Arg Phe Thr
           275                 280                 285
   Lys Glu Ala Gly Thr Leu Ala Tyr Tyr Glu Ile Cys Asp Phe Leu Arg
       290                 295                 300
   Gly Ala Thr Val His Arg Ile Leu Gly Gln Gln Val Pro Tyr Ala Thr
   305                 310                 315                 320
   Lys Gly Asn Gln Trp Val Gly Tyr Asp Asp Gln Glu Ser Val Lys Ser
                   325                 330                 335
   Lys Val Gln Tyr Leu Lys Asp Arg Gln Leu Ala Gly Ala Met Val Trp
               340                 345                 350
   Ala Leu Asp Leu Asp Asp Phe Gln Gly Ser Phe Cys Gly Gln Asp Leu
           355                 360                 365
   Arg Phe Pro Leu Thr Asn Ala Ile Lys Asp Ala Leu Ala Ala Thr
       370                 375                 380
<210> 91
   <211> 965
   <212> PRT
   <213> Homo sapiens
<400> 91
   Met Ala Leu Leu Cys Gly Leu Gly Gln Val Thr Leu Arg Ile Trp Val
   1               5                   10                  15
   Ser Leu Pro Ser Gln Ser Glu Asn Gly Ile Gly Phe Leu Ala Ala Arg
               20                  25                  30
   Ala Phe Leu Arg Ser Gly Gly Met Glu Ala Leu Thr Thr Gln Leu Gly
   35 40 45
   Pro Gly Arg Glu Gly Ser Ser Ser Pro Asn Ser Lys Gln Glu Leu Gln
   50 55 60
   Pro Tyr Ser Gly Ser Ser Ala Leu Lys Pro Asn Gln Val Gly Glu Thr
   65 70 75 80
   Ser Leu Tyr Gly Val Pro Ile Val Ser Leu Val Ile Asp Gly Gln Glu
   85 90 95
   Arg Leu Cys Leu Ala Gln Ile Ser Asn Thr Leu Leu Lys Asn Tyr Ser
   100 105 110
   Tyr Asn Glu Ile His Asn Arg Arg Val Ala Leu Gly Ile Thr Cys Val
   115 120 125
   Gln Cys Thr Pro Val Gln Leu Glu Ile Leu Arg Arg Ala Gly Ala Met
   130 135 140
   Pro Ile Ser Ser Arg Arg Cys Gly Met Ile Thr Lys Arg Glu Ala Glu
   145 150 155 160
   Arg Leu Cys Lys Ser Phe Leu Gly Glu His Lys Pro Pro Lys Leu Pro
   165 170 175
   Glu Asn Phe Ala Phe Asp Val Val His Glu Cys Ala Trp Gly Ser Arg
   180 185 190
   Gly Ser Phe Ile Pro Ala Arg Tyr Asn Ser Ser Arg Ala Lys Cys Ile
   195 200 205
   Lys Cys Gly Tyr Cys Ser Met Tyr Phe Ser Pro Asn Lys Phe Ile Phe
   210 215 220
   His Ser His Arg Thr Pro Asp Ala Lys Tyr Thr Gln Pro Asp Ala Ala
   225 230 235 240
   Asn Phe Asn Ser Trp Arg Arg His Leu Lys Leu Ser Asp Lys Ser Ala
   245 250 255
   Thr Asp Glu Leu Ser His Ala Trp Glu Asp Val Lys Ala Met Phe Asn
   260 265 270
   Gly Gly Thr Arg Lys Arg Thr Phe Ser Leu Gln Gly Gly Gly Gly Gly
   275 280 285
   Gly Ala Asn Gly Gly Ser Gly Gly Gln Gly Lys Gly Gly Ala Gly Gly
   290 295 300
   Gly Gly Gly Gly Gly Pro Gly Cys Gly Ala Glu Met Ala Pro Gly Pro
   305 310 315 320
   Pro Pro His Lys Ser Leu Arg Cys Gly Glu Asp Glu Ala Ala Gly Pro
   325 330 335
   Pro Gly Pro Pro Pro Pro His Pro Gln Arg Gly Leu Gly Leu Ala Thr
   340 345 350
   Gly Ala Ser Gly Pro Ala Gly Pro Gly Gly Pro Gly Gly Gly Ala Gly
   355 360 365
   Val Arg Ser Tyr Pro Val Ile Pro Val Pro Ser Lys Gly Phe Gly Leu
   370 375 380
   Leu Gln Lys Leu Pro Pro Pro Leu Phe Pro His Pro Tyr Gly Phe Pro
   385 390 395 400
   Thr Ala Phe Gly Leu Cys Pro Lys Lys Asp Asp Pro Val Leu Gly Ala
   405 410 415
   Gly Glu Pro Lys Gly Gly Pro Gly Thr Gly Ser Gly Gly Gly Gly Ala
   420 425 430
   Gly Thr Gly Gly Gly Ala Gly Gly Pro Gly Ala Ser His Leu Pro Pro
   435 440 445
   Gly Ala Gly Ala Gly Pro Gly Gly Gly Ala Met Phe Trp Gly His Gln
   450 455 460
   Pro Ser Gly Ala Ala Lys Asp Ala Ala Ala Val Ala Ala Ala Ala Ala
   465 470 475 480
   Ala Ala Thr Val Tyr Pro Thr Phe Pro Met Phe Trp Pro Ala Ala Gly
   485 490 495
   Ser Leu Pro Val Pro Ser Tyr Pro Ala Ala Gln Ser Gln Ala Lys Ala
   500 505 510
   Val Ala Ala Ala Val Ala Ala Ala Ala Ala Ala Ala Ala Ala Ala Ala
   515 520 525
   Gly Ser Gly Ala Pro Glu Pro Leu Asp Gly Ala Glu Pro Ala Lys Glu
   530 535 540
   Ser Gly Leu Gly Ala Glu Glu Arg Cys Pro Ser Ala Leu Ser Arg Gly
   545 550 555 560
   Pro Leu Asp Glu Asp Gly Thr Asp Glu Ala Leu Pro Pro Pro Leu Ala
   565 570 575
   Pro Leu Pro Pro Pro Pro Pro Pro Pro Ala Arg Lys Gly Ser Tyr Val
   580 585 590
   Ser Ala Phe Arg Pro Val Val Lys Asp Thr Glu Ser Ile Ala Lys Leu
   595 600 605
   Tyr Gly Ser Ala Arg Glu Ala Tyr Gly Ala Gly Pro Ala Arg Gly Pro
   610 615 620
   Gly Pro Gly Ala Gly Ser Gly Gly Tyr Val Ser Pro Asp Phe Leu Ser
   625 630 635 640
   Glu Gly Ser Ser Ser Tyr Asn Ser Ala Ser Pro Asp Val Asp Thr Ala
   645 650 655
   Asp Glu Pro Glu Val Asp Val Glu Ser Asn Arg Phe Pro Asp Asp Glu
   660 665 670
   Asp Ala Gln Glu Glu Thr Glu Pro Ser Ala Pro Ser Ala Gly Gly Gly
   675 680 685
   Pro Asp Gly Glu Gln Pro Thr Gly Pro Pro Ser Ala Thr Ser Ser Gly
   690 695 700
   Ala Asp Gly Pro Ala Asn Ser Pro Asp Gly Gly Ser Pro Arg Pro Arg
   705 710 715 720
   Arg Arg Leu Gly Pro Pro Pro Ala Gly Arg Pro Ala Phe Gly Asp Leu
   725 730 735
   Ala Ala Glu Asp Leu Val Arg Arg Pro Glu Arg Ser Pro Pro Ser Gly
   740 745 750
   Gly Gly Gly Tyr Glu Leu Arg Glu Pro Cys Gly Pro Leu Gly Gly Pro
   755 760 765
   Ala Pro Ala Lys Val Phe Ala Pro Glu Arg Asp Glu His Val Lys Ser
   770 775 780
   Ala Ala Val Ala Leu Gly Pro Ala Ala Ser Tyr Val Cys Thr Pro Glu
   785                 790                 795                 800
   Ala His Glu Pro Asp Lys Glu Asp Asn His Ser Pro Ala Asp Asp Leu
                   805                 810                 815
   Glu Thr Arg Lys Ser Tyr Pro Asp Gln Arg Ser Ile Ser Gln Pro Ser
               820                 825                 830
   Pro Ala Asn Thr Asp Arg Gly Glu Asp Gly Leu Thr Leu Asp Val Thr
           835                 840                 845
   Gly Thr His Leu Val Glu Lys Asp Ile Glu Asn Leu Ala Arg Glu Glu
       850                 855                 860
   Leu Gln Lys Leu Leu Leu Glu Gln Met Glu Leu Arg Lys Lys Leu Glu
   865                 870                 875                 880
   Arg Glu Phe Gln Ser Leu Lys Asp Asn Phe Gln Asp Gln Met Lys Arg
                   885                 890                 895
   Glu Leu Ala Tyr Arg Glu Glu Met Val Gln Gln Leu Gln Ile Val Arg
               900                 905                 910
   Asp Thr Leu Cys Asn Glu Leu Asp Gln Glu Arg Lys Ala Arg Tyr Ala
           915                 920                 925
   Ile Gln Gln Lys Leu Lys Glu Ala His Asp Ala Leu His His Phe Ser
       930                 935                 940
   Cys Lys Met Leu Thr Pro Arg His Cys Thr Gly Asn Cys Ser Phe Lys
   945                 950                 955                 960
   Pro Pro Leu Leu Pro
                   965
<210> 92
   <211> 709
   <212> PRT
   <213> Homo sapiens
<400> 92
   Met Ala Glu Thr Leu Glu Phe Asn Asp Val Tyr Gln Glu Val Lys Gly
   1               5                   10                  15
   Ser Met Asn Asp Gly Arg Leu Arg Leu Ser Arg Gln Gly Ile Ile Phe
               20                  25                  30
   Lys Asn Ser Lys Thr Gly Lys Val Asp Asn Ile Gln Ala Gly Glu Leu
   35 40 45
   Thr Glu Gly Ile Trp Arg Arg Val Ala Leu Gly His Gly Leu Lys Leu
   50 55 60
   Leu Thr Lys Asn Gly His Val Tyr Lys Tyr Asp Gly Phe Arg Glu Ser
   65 70 75 80
   Glu Phe Glu Lys Leu Ser Asp Phe Phe Lys Thr His Tyr Arg Leu Glu
   85 90 95
   Leu Met Glu Lys Asp Leu Cys Val Lys Gly Trp Asn Trp Gly Thr Val
   100 105 110
   Lys Phe Gly Gly Gln Leu Leu Ser Phe Asp Ile Gly Asp Gln Pro Val
   115 120 125
   Phe Glu Ile Pro Leu Ser Asn Val Ser Gln Cys Thr Thr Gly Lys Asn
   130 135 140
   Glu Val Thr Leu Glu Phe His Gln Asn Asp Asp Ala Glu Val Ser Leu
   145 150 155 160
   Met Glu Val Arg Phe Tyr Val Pro Pro Thr Gln Glu Asp Gly Val Asp
   165 170 175
   Pro Val Glu Ala Phe Ala Gln Asn Val Leu Ser Lys Ala Asp Val Ile
   180 185 190
   Gln Ala Thr Gly Asp Ala Ile Cys Ile Phe Arg Glu Leu Gln Cys Leu
   195 200 205
   Thr Pro Arg Gly Arg Tyr Asp Ile Arg Ile Tyr Pro Thr Phe Leu His
   210 215 220
   Leu His Gly Lys Thr Phe Asp Tyr Lys Ile Pro Tyr Thr Thr Val Leu
   225 230 235 240
   Arg Leu Phe Leu Leu Pro His Lys Asp Gln Arg Gln Met Phe Phe Val
   245 250 255
   Ile Ser Leu Asp Pro Pro Ile Lys Gln Gly Gln Thr Arg Tyr His Phe
   260 265 270
   Leu Ile Leu Leu Phe Ser Lys Asp Glu Asp Ile Ser Leu Thr Leu Asn
   275 280 285
   Met Asn Glu Glu Glu Val Glu Lys Arg Phe Glu Gly Arg Leu Thr Lys
   290 295 300
   Asn Met Ser Gly Ser Leu Tyr Glu Met Val Ser Arg Val Met Lys Ala
   305 310 315 320
   Leu Val Asn Arg Lys Ile Thr Val Pro Gly Asn Phe Gln Gly His Ser
   325 330 335
   Gly Ala Gln Cys Ile Thr Cys Ser Tyr Lys Ala Ser Ser Gly Leu Leu
   340 345 350
   Tyr Pro Leu Glu Arg Gly Phe Ile Tyr Val His Lys Pro Pro Val His
   355 360 365
   Ile Arg Phe Asp Glu Ile Ser Phe Val Asn Phe Ala Arg Gly Thr Thr
   370 375 380
   Thr Thr Arg Ser Phe Asp Phe Glu Ile Glu Thr Lys Gln Gly Thr Gln
   385 390 395 400
   Tyr Thr Phe Ser Ser Ile Glu Arg Glu Glu Tyr Gly Lys Leu Phe Asp
   405 410 415
   Phe Val Asn Ala Lys Lys Leu Asn Ile Lys Asn Arg Gly Leu Lys Glu
   420 425 430
   Gly Met Asn Pro Ser Tyr Asp Glu Tyr Ala Asp Ser Asp Glu Asp Gln
   435 440 445
   His Asp Ala Tyr Leu Glu Arg Met Lys Glu Glu Gly Lys Ile Arg Glu
   450 455 460
   Glu Asn Ala Asn Asp Ser Ser Asp Asp Ser Gly Glu Glu Thr Asp Glu
   465 470 475 480
   Ser Phe Asn Pro Gly Glu Glu Glu Glu Asp Val Ala Glu Glu Phe Asp
   485 490 495
   Ser Asn Ala Ser Ala Ser Ser Ser Ser Asn Glu Gly Asp Ser Asp Arg
   500 505 510
   Asp Glu Lys Lys Arg Lys Gln Leu Lys Lys Ala Lys Met Ala Lys Asp
   515 520 525
   Arg Lys Ser Arg Lys Lys Pro Val Glu Val Lys Lys Gly Lys Asp Pro
   530 535 540
   Asn Ala Pro Lys Arg Pro Met Ser Ala Tyr Met Leu Trp Leu Asn Ala
   545 550 555 560
   Ser Arg Glu Lys Ile Lys Ser Asp His Pro Gly Ile Ser Ile Thr Asp
   565 570 575
   Leu Ser Lys Lys Ala Gly Glu Ile Trp Lys Gly Met Ser Lys Glu Lys
   580 585 590
   Lys Glu Glu Trp Asp Arg Lys Ala Glu Asp Ala Arg Arg Asp Tyr Glu
   595 600 605
   Lys Ala Met Lys Glu Tyr Glu Gly Gly Arg Gly Glu Ser Ser Lys Arg
   610 615 620
   Asp Lys Ser Lys Lys Lys Lys Lys Val Lys Val Lys Met Glu Lys Lys
   625 630 635 640
   Ser Thr Pro Ser Arg Gly Ser Ser Ser Lys Ser Ser Ser Arg Gln Leu
   645 650 655
   Ser Glu Ser Phe Lys Ser Lys Glu Phe Val Ser Ser Asp Glu Ser Ser
   660 665 670
   Ser Gly Glu Asn Lys Ser Lys Lys Lys Arg Arg Arg Ser Glu Asp Ser
   675 680 685
   Glu Glu Glu Glu Leu Ala Ser Thr Pro Pro Ser Ser Glu Asp Ser Ala
   690 695 700
   Ser Gly Ser Asp Glu
   705
<210> 93
   <211> 1712
   <212> PRT
   <213> Homo sapiens
<400> 93
<210> 94
   <211> 152
   <212> PRT
   <213> Homo sapiens
<400> 94
   Met Ala Asn Cys Glu Arg Thr Phe Ile Ala Ile Lys Pro Asp Gly Val
   1 5 10 15
   Gln Arg Gly Leu Val Gly Glu Ile Ile Lys Arg Phe Glu Gln Lys Gly
   20 25 30
   Phe Arg Leu Val Gly Leu Lys Phe Met Gln Ala Ser Glu Asp Leu Leu
   35 40 45
   Lys Glu His Tyr Val Asp Leu Lys Asp Arg Pro Phe Phe Ala Gly Leu
   50 55 60
   Val Lys Tyr Met His Ser Gly Pro Val Val Ala Met Val Trp Glu Gly
   65 70 75 80
   Leu Asn Val Val Lys Thr Gly Arg Val Met Leu Gly Glu Thr Asn Pro
   85 90 95
   Ala Asp Ser Lys Pro Gly Thr Ile Arg Gly Asp Phe Cys Ile Gln Val
   100 105 110
   Gly Arg Asn Ile Ile His Gly Ser Asp Ser Val Glu Ser Ala Glu Lys
   115 120 125
   Glu Ile Gly Leu Trp Phe His Pro Glu Glu Leu Val Asp Tyr Thr Ser
   130 135 140
   Cys Ala Gln Asn Trp Ile Tyr Glu
   145 150
<210> 95
   <211> 764
   <212> PRT
   <213> Homo sapiens
<400> 95
   Met Gly Ser Asn Leu Ser Pro Gln Leu Cys Leu Met Pro Phe Ile Leu
   1 5 10 15
   Gly Leu Leu Ser Gly Gly Val Thr Thr Thr Pro Trp Ser Leu Ala Arg
   20 25 30
   Pro Gln Gly Ser Cys Ser Leu Glu Gly Val Glu Ile Lys Gly Gly Ser
   35 40 45
   Phe Arg Leu Leu Gln Glu Gly Gln Ala Leu Glu Tyr Val Cys Pro Ser
   50 55 60
   Gly Phe Tyr Pro Tyr Pro Val Gln Thr Arg Thr Cys Arg Ser Thr Gly
   65 70 75 80
   Ser Trp Ser Thr Leu Lys Thr Gln Asp Gln Lys Thr Val Arg Lys Ala
                   85                  90                  95
   Glu Cys Arg Ala Ile His Cys Pro Arg Pro His Asp Phe Glu Asn Gly
               100                 105                 110
   Glu Tyr Trp Pro Arg Ser Pro Tyr Tyr Asn Val Ser Asp Glu Ile Ser
           115                 120                 125
   Phe His Cys Tyr Asp Gly Tyr Thr Leu Arg Gly Ser Ala Asn Arg Thr
       130                 135                 140
   Cys Gln Val Asn Gly Arg Trp Ser Gly Gln Thr Ala Ile Cys Asp Asn
   145                 150                 155                 160
   Gly Ala Gly Tyr Cys Ser Asn Pro Gly Ile Pro Ile Gly Thr Arg Lys
                   165                 170                 175
   Val Gly Ser Gln Tyr Arg Leu Glu Asp Ser Val Thr Tyr His Cys Ser
               180                 185                 190
   Arg Gly Leu Thr Leu Arg Gly Ser Gln Arg Arg Thr Cys Gln Glu Gly
           195                 200                 205
   Gly Ser Trp Ser Gly Thr Glu Pro Ser Cys Gln Asp Ser Phe Met Tyr
       210                 215                 220
   Asp Thr Pro Gln Glu Val Ala Glu Ala Phe Leu Ser Ser Leu Thr Glu
   225                 230                 235                 240
   Thr Ile Glu Gly Val Asp Ala Glu Asp Gly His Gly Pro Gly Glu Gln
                   245                 250                 255
   Gln Lys Arg Lys Ile Val Leu Asp Pro Ser Gly Ser Met Asn Ile Tyr
               260                 265                 270
   Leu Val Leu Asp Gly Ser Asp Ser Ile Gly Ala Ser Asn Phe Thr Gly
           275                 280                 285
   Ala Lys Lys Cys Leu Val Asn Leu Ile Glu Lys Val Ala Ser Tyr Gly
       290                 295                 300
   Val Lys Pro Arg Tyr Gly Leu Val Thr Tyr Ala Thr Tyr Pro Lys Ile
   305                 310                 315                 320
   Trp Val Lys Val Ser Glu Ala Asp Ser Ser Asn Ala Asp Trp Val Thr
                   325                 330                 335
   Lys Gln Leu Asn Glu Ile Asn Tyr Glu Asp His Lys Leu Lys Ser Gly
   340 345 350
   Thr Asn Thr Lys Lys Ala Leu Gln Ala Val Tyr Ser Met Met Ser Trp
   355 360 365
   Pro Asp Asp Val Pro Pro Glu Gly Trp Asn Arg Thr Arg His Val Ile
   370 375 380
   Ile Leu Met Thr Asp Gly Leu His Asn Met Gly Gly Asp Pro Ile Thr
   385 390 395 400
   Val Ile Asp Glu Ile Arg Asp Leu Leu Tyr Ile Gly Lys Asp Arg Lys
   405 410 415
   Asn Pro Arg Glu Asp Tyr Leu Asp Val Tyr Val Phe Gly Val Gly Pro
   420 425 430
   Leu Val Asn Gln Val Asn Ile Asn Ala Leu Ala Ser Lys Lys Asp Asn
   435 440 445
   Glu Gln His Val Phe Lys Val Lys Asp Met Glu Asn Leu Glu Asp Val
   450 455 460
   Phe Tyr Gln Met Ile Asp Glu Ser Gln Ser Leu Ser Leu Cys Gly Met
   465 470 475 480
   Val Trp Glu His Arg Lys Gly Thr Asp Tyr His Lys Gln Pro Trp Gln
   485 490 495
   Ala Lys Ile Ser Val Ile Arg Pro Ser Lys Gly His Glu Ser Cys Met
   500 505 510
   Gly Ala Val Val Ser Glu Tyr Phe Val Leu Thr Ala Ala His Cys Phe
   515 520 525
   Thr Val Asp Asp Lys Glu His Ser Ile Lys Val Ser Val Gly Gly Glu
   530 535 540
   Lys Arg Asp Leu Glu Ile Glu Val Val Leu Phe His Pro Asn Tyr Asn
   545 550 555 560
   Ile Asn Gly Lys Lys Glu Ala Gly Ile Pro Glu Phe Tyr Asp Tyr Asp
   565 570 575
   Val Ala Leu Ile Lys Leu Lys Asn Lys Leu Lys Tyr Gly Gln Thr Ile
   580 585 590
   Arg Pro Ile Cys Leu Pro Cys Thr Glu Gly Thr Thr Arg Ala Leu Arg
           595                 600                 605
   Leu Pro Pro Thr Thr Thr Cys Gln Gln Gln Lys Glu Glu Leu Leu Pro
       610                 615                 620
   Ala Gln Asp Ile Lys Ala Leu Phe Val Ser Glu Glu Glu Lys Lys Leu
   625                 630                 635                 640
   Thr Arg Lys Glu Val Tyr Ile Lys Asn Gly Asp Lys Lys Gly Ser Cys
                   645                 650                 655
   Glu Arg Asp Ala Gln Tyr Ala Pro Gly Tyr Asp Lys Val Lys Asp Ile
               660                 665                 670
   Ser Glu Val Val Thr Pro Arg Phe Leu Cys Thr Gly Gly Val Ser Pro
           675                 680                 685
   Tyr Ala Asp Pro Asn Thr Cys Arg Gly Asp Ser Gly Gly Pro Leu Ile
       690                 695                 700
   Val His Lys Arg Ser Arg Phe Ile Gln Val Gly Val Ile Ser Trp Gly
   705                 710                 715                 720
   Val Val Asp Val Cys Lys Asn Gln Lys Arg Gln Lys Gln Val Pro Ala
                   725                 730                 735
   His Ala Arg Asp Phe His Ile Asn Leu Phe Gln Val Leu Pro Trp Leu
               740                 745                 750
   Lys Glu Lys Leu Gln Asp Glu Asp Leu Gly Phe Leu
           755                 760
<210> 96
   <211> 472
   <212> PRT
   <213> Homo sapiens
<400> 96
   Met Ala Gly Gly Glu Ala Gly Val Thr Leu Gly Gln Pro His Leu Ser
   1               5                   10                  15
   Arg Gln Asp Leu Thr Thr Leu Asp Val Thr Lys Leu Thr Pro Leu Ser
               20                  25                  30
   His Glu Val Ile Ser Arg Gln Ala Thr Ile Asn Ile Gly Thr Ile Gly
           35                  40                  45
   His Val Ala His Gly Lys Ser Thr Val Val Lys Ala Ile Ser Gly Val
   50 55 60
   His Thr Val Arg Phe Lys Asn Glu Leu Glu Arg Asn Ile Thr Ile Lys
   65 70 75 80
   Leu Gly Tyr Ala Asn Ala Lys Ile Tyr Lys Leu Asp Asp Pro Ser Cys
   85 90 95
   Pro Arg Pro Glu Cys Tyr Arg Ser Cys Gly Ser Ser Thr Pro Asp Glu
   100 105 110
   Phe Pro Thr Asp Ile Pro Gly Thr Lys Gly Asn Phe Lys Leu Val Arg
   115 120 125
   His Val Ser Phe Val Asp Cys Pro Gly His Asp Ile Leu Met Ala Thr
   130 135 140
   Met Leu Asn Gly Ala Ala Val Met Asp Ala Ala Leu Leu Leu Ile Ala
   145 150 155 160
   Gly Asn Glu Ser Cys Pro Gln Pro Gln Thr Ser Glu His Leu Ala Ala
   165 170 175
   Ile Glu Ile Met Lys Leu Lys His Ile Leu Ile Leu Gln Asn Lys Ile
   180 185 190
   Asp Leu Val Lys Glu Ser Gln Ala Lys Glu Gln Tyr Glu Gln Ile Leu
   195 200 205
   Ala Phe Val Gln Gly Thr Val Ala Glu Gly Ala Pro Ile Ile Pro Ile
   210 215 220
   Ser Ala Gln Leu Lys Tyr Asn Ile Glu Val Val Cys Glu Tyr Ile Val
   225 230 235 240
   Lys Lys Ile Pro Val Pro Pro Arg Asp Phe Thr Ser Glu Pro Arg Leu
   245 250 255
   Ile Val Ile Arg Ser Phe Asp Val Asn Lys Pro Gly Cys Glu Val Asp
   260 265 270
   Asp Leu Lys Gly Gly Val Ala Gly Gly Ser Ile Leu Lys Gly Val Leu
   275 280 285
   Lys Val Gly Gln Glu Ile Glu Val Arg Pro Gly Ile Val Ser Lys Asp
   290 295 300
   Ser Glu Gly Lys Leu Met Cys Lys Pro Ile Phe Ser Lys Ile Val Ser
   305 310 315 320
   Leu Phe Ala Glu His Asn Asp Leu Gln Tyr Ala Ala Pro Gly Gly Leu
   325 330 335
   Ile Gly Val Gly Thr Lys Ile Asp Pro Thr Leu Cys Arg Ala Asp Arg
   340 345 350
   Met Val Gly Gln Val Leu Gly Ala Val Gly Ala Leu Pro Glu Ile Phe
   355 360 365
   Thr Glu Leu Glu Ile Ser Tyr Phe Leu Leu Arg Arg Leu Leu Gly Val
   370 375 380
   Arg Thr Glu Gly Asp Lys Lys Ala Ala Lys Val Gln Lys Leu Ser Lys
   385 390 395 400
   Asn Glu Val Leu Met Val Asn Ile Gly Ser Leu Ser Thr Gly Gly Arg
   405 410 415
   Val Ser Ala Val Lys Ala Asp Leu Gly Lys Ile Val Leu Thr Asn Pro
   420 425 430
   Val Cys Thr Glu Val Gly Glu Lys Ile Ala Leu Ser Arg Arg Val Glu
   435 440 445
   Lys His Trp Arg Leu Ile Gly Trp Gly Gln Ile Arg Arg Gly Val Thr
   450 455 460
   Ile Lys Pro Thr Val Asp Asp Asp
   465 470
<210> 97
   <211> 572
   <212> PRT
   <213> Homo sapiens
<400> 97
<210> 98
   <211> 2115
   <212> PRT
   <213> Homo sapiens
<400> 98
   Met Thr Leu His Ala Thr Arg Gly Ala Ala Leu Leu Ser Trp Val Asn
   1               5                   10                  15
   Ser Leu His Val Ala Asp Pro Val Glu Ala Val Leu Gln Leu Gln Asp
               20                  25                  30
   Cys Ser Ile Phe Ile Lys Ile Ile Asp Arg Ile His Gly Thr Glu Glu
           35                  40                  45
   Gly Gln Gln Ile Leu Lys Gln Pro Val Ser Glu Arg Leu Asp Phe Val
       50                  55                  60
   Cys Ser Phe Leu Gln Lys Asn Arg Lys His Pro Ser Ser Pro Glu Cys
   65                  70                  75                  80
   Leu Val Ser Ala Gln Lys Val Leu Glu Gly Ser Glu Leu Glu Leu Ala
                   85                  90                  95
   Lys Met Thr Met Leu Leu Leu Tyr His Ser Thr Met Ser Ser Lys Ser
               100                 105                 110
   Pro Arg Asp Trp Glu Gln Phe Glu Tyr Lys Ile Gln Ala Glu Leu Ala
           115                 120                 125
   Val Ile Leu Lys Phe Val Leu Asp His Glu Asp Gly Leu Asn Leu Asn
       130                 135                 140
   Glu Asp Leu Glu Asn Phe Leu Gln Lys Ala Pro Val Pro Ser Thr Cys
   145                 150                 155                 160
   Ser Ser Thr Phe Pro Glu Glu Leu Ser Pro Pro Ser His Gln Ala Lys
                   165                 170                 175
   Arg Glu Ile Arg Phe Leu Glu Leu Gln Lys Val Ala Ser Ser Ser Ser
               180                 185                 190
   Gly Asn Asn Phe Leu Ser Gly Ser Pro Ala Ser Pro Met Gly Asp Ile
           195                 200                 205
   Leu Gln Thr Pro Gln Phe Gln Met Arg Arg Leu Lys Lys Gln Leu Ala
       210                 215                 220
   Asp Glu Arg Ser Asn Arg Asp Glu Leu Glu Leu Glu Leu Ala Glu Asn
   225                 230                 235                 240
   Arg Lys Leu Leu Thr Glu Lys Asp Ala Gln Ile Ala Met Met Gln Gln
                   245                 250                 255
   Arg Ile Asp Arg Leu Ala Leu Leu Asn Glu Lys Gln Ala Ala Ser Pro
               260                 265                 270
   Leu Glu Pro Lys Glu Leu Glu Glu Leu Arg Asp Lys Asn Glu Ser Leu
           275                 280                 285
   Thr Met Arg Leu His Glu Thr Leu Lys Gln Cys Gln Asp Leu Lys Thr
       290                 295                 300
   Glu Lys Ser Gln Met Asp Arg Lys Ile Asn Gln Leu Ser Glu Glu Asn
   305                 310                 315                 320
   Gly Asp Leu Ser Phe Lys Leu Arg Glu Phe Ala Ser His Leu Gln Gln
                   325                 330                 335
   Leu Gln Asp Ala Leu Asn Glu Leu Thr Glu Glu His Ser Lys Ala Thr
               340                 345                 350
   Gln Glu Trp Leu Glu Lys Gln Ala Gln Leu Glu Lys Glu Leu Ser Ala
           355                 360                 365
   Ala Leu Gln Asp Lys Lys Cys Leu Glu Glu Lys Asn Glu Ile Leu Gln
       370                 375                 380
   Gly Lys Leu Ser Gln Leu Glu Glu His Leu Ser Gln Leu Gln Asp Asn
   385                 390                 395                 400
   Pro Pro Gln Glu Lys Gly Glu Val Leu Gly Asp Val Leu Gln Leu Glu
                   405                 410                 415
   Thr Leu Lys Gln Glu Ala Ala Thr Leu Ala Ala Asn Asn Thr Gln Leu
               420                 425                 430
   Gln Ala Arg Val Glu Met Leu Glu Thr Glu Arg Gly Gln Gln Glu Ala
           435                 440                 445
   Lys Leu Leu Ala Glu Arg Gly His Phe Glu Glu Glu Lys Gln Gln Leu
   450 455 460
   Ser Ser Leu Ile Thr Asp Leu Gln Ser Ser Ile Ser Asn Leu Ser Gln
   465 470 475 480
   Ala Lys Glu Glu Leu Glu Gln Ala Ser Gln Ala His Gly Ala Arg Leu
   485 490 495
   Thr Ala Gln Val Ala Ser Leu Thr Ser Glu Leu Thr Thr Leu Asn Ala
   500 505 510
   Thr Ile Gln Gln Gln Asp Gln Glu Leu Ala Gly Leu Lys Gln Gln Ala
   515 520 525
   Lys Glu Lys Gln Ala Gln Leu Ala Gln Thr Leu Gln Gln Gln Glu Gln
   530 535 540
   Ala Ser Gln Gly Leu Arg His Gln Val Glu Gln Leu Ser Ser Ser Leu
   545 550 555 560
   Lys Gln Lys Glu Gln Gln Leu Lys Glu Val Ala Glu Lys Gln Glu Ala
   565 570 575
   Thr Arg Gln Asp His Ala Gln Gln Leu Ala Thr Ala Ala Glu Glu Arg
   580 585 590
   Glu Ala Ser Leu Arg Glu Arg Asp Ala Ala Leu Lys Gln Leu Glu Ala
   595 600 605
   Leu Glu Lys Glu Lys Ala Ala Lys Leu Glu Ile Leu Gln Gln Gln Leu
   610 615 620
   Gln Val Ala Asn Glu Ala Arg Asp Ser Ala Gln Thr Ser Val Thr Gln
   625 630 635 640
   Ala Gln Arg Glu Lys Ala Glu Leu Ser Arg Lys Val Glu Glu Leu Gln
   645 650 655
   Ala Cys Val Glu Thr Ala Arg Gln Glu Gln His Glu Ala Gln Ala Gln
   660 665 670
   Val Ala Glu Leu Glu Leu Gln Leu Arg Ser Glu Gln Gln Lys Ala Thr
   675 680 685
   Glu Lys Glu Arg Val Ala Gln Glu Lys Asp Gln Leu Gln Glu Gln Leu
   690 695 700
   Gln Ala Leu Lys Glu Ser Leu Lys Val Thr Lys Gly Ser Leu Glu Glu
   705 710 715 720
   Glu Lys Arg Arg Ala Ala Asp Ala Leu Glu Glu Gln Gln Arg Cys Ile
   725 730 735
   Ser Glu Leu Lys Ala Glu Thr Arg Ser Leu Val Glu Gln His Lys Arg
   740 745 750
   Glu Arg Lys Glu Leu Glu Glu Glu Arg Ala Gly Arg Lys Gly Leu Glu
   755 760 765
   Ala Arg Leu Gln Gln Leu Gly Glu Ala His Gln Ala Glu Thr Glu Val
   770 775 780
   Leu Arg Arg Glu Leu Ala Glu Ala Met Ala Ala Gln His Thr Ala Glu
   785 790 795 800
   Ser Glu Cys Glu Gln Leu Val Lys Glu Val Ala Ala Trp Arg Glu Arg
   805 810 815
   Tyr Glu Asp Ser Gln Gln Glu Glu Ala Gln Tyr Gly Ala Met Phe Gln
   820 825 830
   Glu Gln Leu Met Thr Leu Lys Glu Glu Cys Glu Lys Ala Arg Gln Glu
   835 840 845
   Leu Gln Glu Ala Lys Glu Lys Val Ala Gly Ile Glu Ser His Ser Glu
   850 855 860
   Leu Gln Ile Ser Arg Gln Gln Asn Glu Leu Ala Glu Leu His Ala Asn
   865 870 875 880
   Leu Ala Arg Ala Leu Gln Gln Val Gln Glu Lys Glu Val Arg Ala Gln
   885 890 895
   Lys Leu Ala Asp Asp Leu Ser Thr Leu Gln Glu Lys Met Ala Ala Thr
   900 905 910
   Ser Lys Glu Val Ala Arg Leu Glu Thr Leu Val Arg Lys Ala Gly Glu
   915 920 925
   Gln Gln Glu Thr Ala Ser Arg Glu Leu Val Lys Glu Pro Ala Arg Ala
   930 935 940
   Gly Asp Arg Gln Pro Glu Trp Leu Glu Glu Gln Gln Gly Arg Gln Phe
   945 950 955 960
   Cys Ser Thr Gln Ala Ala Leu Gln Ala Met Glu Arg Glu Ala Glu Gln
   965 970 975
   Met Gly Asn Glu Leu Glu Arg Leu Arg Ala Ala Leu Met Glu Ser Gln
   980 985 990
   Gly Gln Gln Gln Glu Glu Arg Gly Gln Gln Glu Arg Glu Val Ala Arg
   995 1000 1005
   Leu Thr Gln Glu Arg Gly Arg Ala Gln Ala Asp Leu Ala Leu Glu
   1010 1015 1020
   Lys Ala Ala Arg Ala Glu Leu Glu Met Arg Leu Gln Asn Ala Leu
   1025 1030 1035
   Asn Glu Gln Arg Val Glu Phe Ala Thr Leu Gln Glu Ala Leu Ala
   1040 1045 1050
   His Ala Leu Thr Glu Lys Glu Gly Lys Asp Gln Glu Leu Ala Lys
   1055 1060 1065
   Leu Arg Gly Leu Glu Ala Ala Gln Ile Lys Glu Leu Glu Glu Leu
   1070 1075 1080
   Arg Gln Thr Val Lys Gln Leu Lys Glu Gln Leu Ala Lys Lys Glu
   1085 1090 1095
   Lys Glu His Ala Ser Gly Ser Gly Ala Gln Ser Glu Ala Ala Gly
   1100 1105 1110
   Arg Thr Glu Pro Thr Gly Pro Lys Leu Glu Ala Leu Arg Ala Glu
   1115 1120 1125
   Val Ser Lys Leu Glu Gln Gln Cys Gln Lys Gln Gln Glu Gln Ala
   1130 1135 1140
   Asp Ser Leu Glu Arg Ser Leu Glu Ala Glu Arg Ala Ser Arg Ala
   1145 1150 1155
   Glu Arg Asp Ser Ala Leu Glu Thr Leu Gln Gly Gln Leu Glu Glu
   1160 1165 1170
   Lys Ala Gln Glu Leu Gly His Ser Gln Ser Ala Leu Ala Ser Ala
   1175 1180 1185
   Gln Arg  Glu Leu Ala Ala Phe  Arg Thr Lys Val Gln  Asp His Ser
       1190                 1195                 1200
   Lys Ala  Glu Asp Glu Trp Lys  Ala Gln Val Ala Arg  Gly Arg Gln
       1205                 1210                 1215
   Glu Ala  Glu Arg Lys Asn Ser  Leu Ile Ser Ser Leu  Glu Glu Glu
       1220                 1225                 1230
   Val Ser  Ile Leu Asn Arg Gln  Val Leu Glu Lys Glu  Gly Glu Ser
       1235                 1240                 1245
   Lys Glu  Leu Lys Arg Leu Val  Met Ala Glu Ser Glu  Lys Ser Gln
       1250                 1255                 1260
   Lys Leu  Glu Glu Arg Leu Arg  Leu Leu Gln Ala Glu  Thr Ala Ser
       1265                 1270                 1275
   Asn Ser  Ala Arg Ala Ala Glu  Arg Ser Ser Ala Leu  Arg Glu Glu
       1280                 1285                 1290
   Val Gln  Ser Leu Arg Glu Glu  Ala Glu Lys Gln Arg  Val Ala Ser
       1295                 1300                 1305
   Glu Asn  Leu Arg Gln Glu Leu  Thr Ser Gln Ala Glu  Arg Ala Glu
       1310                 1315                 1320
   Glu Leu  Gly Gln Glu Leu Lys  Ala Trp Gln Glu Lys  Phe Phe Gln
       1325                 1330                 1335
   Lys Glu  Gln Ala Leu Ser Thr  Leu Gln Leu Glu His  Thr Ser Thr
       1340                 1345                 1350
   Gln Ala  Leu Val Ser Glu Leu  Leu Pro Ala Lys His  Leu Cys Gln
       1355                 1360                 1365
   Gln Leu  Gln Ala Glu Gln Ala  Ala Ala Glu Lys Arg  His Arg Glu
       1370                 1375                 1380
   Glu Leu  Glu Gln Ser Lys Gln  Ala Ala Gly Gly Leu  Arg Ala Glu
       1385                 1390                 1395
   Leu Leu  Arg Ala Gln Arg Glu  Leu Gly Glu Leu Ile  Pro Leu Arg
       1400                 1405                 1410
   Gln Lys  Val Ala Glu Gln Glu  Arg Thr Ala Gln Gln  Leu Arg Ala
       1415                 1420                 1425
   Glu Lys Ala Ser Tyr Ala Glu Gln Leu Ser Met Leu Lys Lys Ala
   1430 1435 1440
   His Gly Leu Leu Ala Glu Glu Asn Arg Gly Leu Gly Glu Arg Ala
   1445 1450 1455
   Asn Leu Gly Arg Gln Phe Leu Glu Val Glu Leu Asp Gln Ala Arg
   1460 1465 1470
   Glu Lys Tyr Val Gln Glu Leu Ala Ala Val Arg Ala Asp Ala Glu
   1475 1480 1485
   Thr Arg Leu Ala Glu Val Gln Arg Glu Ala Gln Ser Thr Ala Arg
   1490 1495 1500
   Glu Leu Glu Val Met Thr Ala Lys Tyr Glu Gly Ala Lys Val Lys
   1505 1510 1515
   Val Leu Glu Glu Arg Gln Arg Phe Gln Glu Glu Arg Gln Lys Leu
   1520 1525 1530
   Thr Ala Gln Val Glu Gln Leu Glu Val Phe Gln Arg Glu Gln Thr
   1535 1540 1545
   Lys Gln Val Glu Glu Leu Ser Lys Lys Leu Ala Asp Ser Asp Gln
   1550 1555 1560
   Ala Ser Lys Val Gln Gln Gln Lys Leu Lys Ala Val Gln Ala Gln
   1565 1570 1575
   Gly Gly Glu Ser Gln Gln Glu Ala Gln Arg Leu Gln Ala Gln Leu
   1580 1585 1590
   Asn Glu Leu Gln Ala Gln Leu Ser Gln Lys Glu Gln Ala Ala Glu
   1595 1600 1605
   His Tyr Lys Leu Gln Met Glu Lys Ala Lys Thr His Tyr Asp Ala
   1610 1615 1620
   Lys Lys Gln Gln Asn Gln Glu Leu Gln Glu Gln Leu Arg Ser Leu
   1625 1630 1635
   Glu Gln Leu Gln Lys Glu Asn Lys Glu Leu Arg Ala Glu Ala Glu
   1640 1645 1650
   Arg Leu Gly His Glu Leu Gln Gln Ala Gly Leu Lys Thr Lys Glu
   1655 1660 1665
   Ala Glu Gln Thr Cys Arg His Leu Thr Ala Gln Val Arg Ser Leu
   1670 1675 1680
   Glu Ala Gln Val Ala His Ala Asp Gln Gln Leu Arg Asp Leu Gly
   1685 1690 1695
   Lys Phe Gln Val Ala Thr Asp Ala Leu Lys Ser Arg Glu Pro Gln
   1700 1705 1710
   Ala Lys Pro Gln Leu Asp Leu Ser Ile Asp Ser Leu Asp Leu Ser
   1715 1720 1725
   Cys Glu Glu Gly Thr Pro Leu Ser Ile Thr Ser Lys Leu Pro Arg
   1730 1735 1740
   Thr Gln Pro Asp Gly Thr Ser Val Pro Gly Glu Pro Ala Ser Pro
   1745 1750 1755
   Ile Ser Gln Arg Leu Pro Pro Lys Val Glu Ser Leu Glu Ser Leu
   1760 1765 1770
   Tyr Phe Thr Pro Ile Pro Ala Arg Ser Gln Ala Pro Leu Glu Ser
   1775 1780 1785
   Ser Leu Asp Ser Leu Gly Asp Val Phe Leu Asp Ser Gly Arg Lys
   1790 1795 1800
   Thr Arg Ser Ala Arg Arg Arg Thr Thr Gln Ile Ile Asn Ile Thr
   1805 1810 1815
   Met Thr Lys Lys Leu Asp Val Glu Glu Pro Asp Ser Ala Asn Ser
   1820 1825 1830
   Ser Phe Tyr Ser Thr Arg Ser Ala Pro Ala Ser Gln Ala Ser Leu
   1835 1840 1845
   Arg Ala Thr Ser Ser Thr Gln Ser Leu Ala Arg Leu Gly Ser Pro
   1850 1855 1860
   Asp Tyr Gly Asn Ser Ala Leu Leu Ser Leu Pro Gly Tyr Arg Pro
   1865 1870 1875
   Thr Thr Arg Ser Ser Ala Arg Arg Ser Gln Ala Gly Val Ser Ser
   1880 1885 1890
   Gly Ala  Pro Pro Gly Arg Asn  Ser Phe Tyr Met Gly  Thr Cys Gln
       1895                 1900                 1905
   Asp Glu  Pro Glu Gln Leu Asp  Asp Trp Asn Arg Ile  Ala Glu Leu
       1910                 1915                 1920
   Gln Gln  Arg Asn Arg Val Cys  Pro Pro His Leu Lys  Thr Cys Tyr
       1925                 1930                 1935
   Pro Leu  Glu Ser Arg Pro Ser  Leu Ser Leu Gly Thr  Ile Thr Asp
       1940                 1945                 1950
   Glu Glu  Met Lys Thr Gly Asp  Pro Gln Glu Thr Leu  Arg Arg Ala
       1955                 1960                 1965
   Ser Met  Gln Pro Ile Gln Ile  Ala Glu Gly Thr Gly  Ile Thr Thr
       1970                 1975                 1980
   Arg Gln  Gln Arg Lys Arg Val  Ser Leu Glu Pro His  Gln Gly Pro
       1985                 1990                 1995
   Gly Thr  Pro Glu Ser Lys Lys  Ala Thr Ser Cys Phe  Pro Arg Pro
       2000                 2005                 2010
   Met Thr  Pro Arg Asp Arg His  Glu Gly Arg Lys Gln  Ser Thr Thr
       2015                 2020                 2025
   Glu Ala  Gln Lys Lys Ala Ala  Pro Ala Ser Thr Lys  Gln Ala Asp
       2030                 2035                 2040
   Arg Arg  Gln Ser Met Ala Phe  Ser Ile Leu Asn Thr  Pro Lys Lys
       2045                 2050                 2055
   Leu Gly  Asn Ser Leu Leu Arg  Arg Gly Ala Ser Lys  Lys Ala Leu
       2060                 2065                 2070
   Ser Lys  Ala Ser Pro Asn Thr  Arg Ser Gly Thr Arg  Arg Ser Pro
       2075                 2080                 2085
   Arg Ile  Ala Thr Thr Thr Ala  Ser Ala Ala Thr Ala  Ala Ala Ile
       2090                 2095                 2100
   Gly Ala  Thr Pro Arg Ala Lys  Gly Lys Ala Lys His
       2105                 2110                 2115
<210> 99
   <211> 1357
   <212> PRT
   <213> Homo sapiens
<400> 99
   Met Glu Phe Tyr Glu Ser Ala Tyr Phe Ile Val Leu Ile Pro Ser Ile
   1               5                   10                  15
   Val Ile Thr Val Ile Phe Leu Phe Phe Trp Leu Phe Met Lys Glu Thr
               20                  25                  30
   Leu Tyr Asp Glu Val Leu Ala Lys Gln Lys Arg Glu Gln Lys Leu Ile
           35                  40                  45
   Pro Thr Lys Thr Asp Lys Lys Lys Ala Glu Lys Lys Lys Asn Lys Lys
       50                  55                  60
   Lys Glu Ile Gln Asn Gly Asn Leu His Glu Ser Asp Ser Glu Ser Val
   65                  70                  75                  80
   Pro Arg Asp Phe Lys Leu Ser Asp Ala Leu Ala Val Glu Asp Asp Gln
                   85                  90                  95
   Val Ala Pro Val Pro Leu Asn Val Val Glu Thr Ser Ser Ser Val Arg
               100                 105                 110
   Glu Arg Lys Lys Lys Glu Lys Lys Gln Lys Pro Val Leu Glu Glu Gln
           115                 120                 125
   Val Ile Lys Glu Ser Asp Ala Ser Lys Ile Pro Gly Lys Lys Val Glu
       130                 135                 140
   Pro Val Pro Val Thr Lys Gln Pro Thr Pro Pro Ser Glu Ala Ala Ala
   145                 150                 155                 160
   Ser Lys Lys Lys Pro Gly Gln Lys Lys Ser Lys Asn Gly Ser Asp Asp
                   165                 170                 175
   Gln Asp Lys Lys Val Glu Thr Leu Met Val Pro Ser Lys Arg Gln Glu
               180                 185                 190
   Ala Leu Pro Leu His Gln Glu Thr Lys Gln Glu Ser Gly Ser Gly Lys
           195                 200                 205
   Lys Lys Ala Ser Ser Lys Lys Gln Lys Thr Glu Asn Val Phe Val Asp
       210                 215                 220
   Glu Pro Leu Ile His Ala Thr Thr Tyr Ile Pro Leu Met Asp Asn Ala
   225                 230                 235                 240
   Asp Ser Ser Pro Val Val Asp Lys Arg Glu Val Ile Asp Leu Leu Lys
   245 250 255
   Pro Asp Gln Val Glu Gly Ile Gln Lys Ser Gly Thr Lys Lys Leu Lys
   260 265 270
   Thr Glu Thr Asp Lys Glu Asn Ala Glu Val Lys Phe Lys Asp Phe Leu
   275 280 285
   Leu Ser Leu Lys Thr Met Met Phe Ser Glu Asp Glu Ala Leu Cys Val
   290 295 300
   Val Asp Leu Leu Lys Glu Lys Ser Gly Val Ile Gln Asp Ala Leu Lys
   305 310 315 320
   Lys Ser Ser Lys Gly Glu Leu Thr Thr Leu Ile His Gln Leu Gln Glu
   325 330 335
   Lys Asp Lys Leu Leu Ala Ala Val Lys Glu Asp Ala Ala Ala Thr Lys
   340 345 350
   Asp Arg Cys Lys Gln Leu Thr Gln Glu Met Met Thr Glu Lys Glu Arg
   355 360 365
   Ser Asn Val Val Ile Thr Arg Met Lys Asp Arg Ile Gly Thr Leu Glu
   370 375 380
   Lys Glu His Asn Val Phe Gln Asn Lys Ile His Val Ser Tyr Gln Glu
   385 390 395 400
   Thr Gln Gln Met Gln Met Lys Phe Gln Gln Val Arg Glu Gln Met Glu
   405 410 415
   Ala Glu Ile Ala His Leu Lys Gln Glu Asn Gly Ile Leu Arg Asp Ala
   420 425 430
   Val Ser Asn Thr Thr Asn Gln Leu Glu Ser Lys Gln Ser Ala Glu Leu
   435 440 445
   Asn Lys Leu Arg Gln Asp Tyr Ala Arg Leu Val Asn Glu Leu Thr Glu
   450 455 460
   Lys Thr Gly Lys Leu Gln Gln Glu Glu Val Gln Lys Lys Asn Ala Glu
   465 470 475 480
   Gln Ala Ala Thr Gln Leu Lys Val Gln Leu Gln Glu Ala Glu Arg Arg
   485 490 495
   Trp Glu Glu Val Gln Ser Tyr Ile Arg Lys Arg Thr Ala Glu His Glu
   500 505 510
   Ala Ala Gln Gln Asp Leu Gln Ser Lys Phe Val Ala Lys Glu Asn Glu
   515 520 525
   Val Gln Ser Leu His Ser Lys Leu Thr Asp Thr Leu Val Ser Lys Gln
   530 535 540
   Gln Leu Glu Gln Arg Leu Met Gln Leu Met Glu Ser Glu Gln Lys Arg
   545 550 555 560
   Val Asn Lys Glu Glu Ser Leu Gln Met Gln Val Gln Asp Ile Leu Glu
   565 570 575
   Gln Asn Glu Ala Leu Lys Ala Gln Ile Gln Gln Phe His Ser Gln Ile
   580 585 590
   Ala Ala Gln Thr Ser Ala Ser Val Leu Ala Glu Glu Leu His Lys Val
   595 600 605
   Ile Ala Glu Lys Asp Lys Gln Ile Lys Gln Thr Glu Asp Ser Leu Ala
   610 615 620
   Ser Glu Arg Asp Arg Leu Thr Ser Lys Glu Glu Glu Leu Lys Asp Ile
   625 630 635 640
   Gln Asn Met Asn Phe Leu Leu Lys Ala Glu Val Gln Lys Leu Gln Ala
   645 650 655
   Leu Ala Asn Glu Gln Ala Ala Ala Ala His Glu Leu Glu Lys Met Gln
   660 665 670
   Gln Ser Val Tyr Val Lys Asp Asp Lys Ile Arg Leu Leu Glu Glu Gln
   675 680 685
   Leu Gln His Glu Ile Ser Asn Lys Met Glu Glu Phe Lys Ile Leu Asn
   690 695 700
   Asp Gln Asn Lys Ala Leu Lys Ser Glu Val Gln Lys Leu Gln Thr Leu
   705 710 715 720
   Val Ser Glu Gln Pro Asn Lys Asp Val Val Glu Gln Met Glu Lys Cys
   725 730 735
   Ile Gln Glu Lys Asp Glu Lys Leu Lys Thr Val Glu Glu Leu Leu Glu
   740 745 750
   Thr Gly Leu Ile Gln Val Ala Thr Lys Glu Glu Glu Leu Asn Ala Ile
   755 760 765
   Arg Thr Glu Asn Ser Ser Leu Thr Lys Glu Val Gln Asp Leu Lys Ala
   770 775 780
   Lys Gln Asn Asp Gln Val Ser Phe Ala Ser Leu Val Glu Glu Leu Lys
   785 790 795 800
   Lys Val Ile His Glu Lys Asp Gly Lys Ile Lys Ser Val Glu Glu Leu
   805 810 815
   Leu Glu Ala Glu Leu Leu Lys Val Ala Asn Lys Glu Lys Thr Val Gln
   820 825 830
   Asp Leu Lys Gln Glu Ile Lys Ala Leu Lys Glu Glu Ile Gly Asn Val
   835 840 845
   Gln Leu Glu Lys Ala Gln Gln Leu Ser Ile Thr Ser Lys Val Gln Glu
   850 855 860
   Leu Gln Asn Leu Leu Lys Gly Lys Glu Glu Gln Met Asn Thr Met Lys
   865 870 875 880
   Ala Val Leu Glu Glu Lys Glu Lys Asp Leu Ala Asn Thr Gly Lys Trp
   885 890 895
   Leu Gln Asp Leu Gln Glu Glu Asn Glu Ser Leu Lys Ala His Val Gln
   900 905 910
   Glu Val Ala Gln His Asn Leu Lys Glu Ala Ser Ser Ala Ser Gln Phe
   915 920 925
   Glu Glu Leu Glu Ile Val Leu Lys Glu Lys Glu Asn Glu Leu Lys Arg
   930 935 940
   Leu Glu Ala Met Leu Lys Glu Arg Glu Ser Asp Leu Ser Ser Lys Thr
   945 950 955 960
   Gln Leu Leu Gln Asp Val Gln Asp Glu Asn Lys Leu Phe Lys Ser Gln
   965 970 975
   Ile Glu Gln Leu Lys Gln Gln Asn Tyr Gln Gln Ala Ser Ser Phe Pro
   980 985 990
   Pro His Glu Glu Leu Leu Lys Val Ile Ser Glu Arg Glu Lys Glu Ile
   995 1000 1005
   Ser Gly Leu Trp Asn Glu Leu Asp Ser Leu Lys Asp Ala Val Glu
   1010 1015 1020
   His Gln Arg Lys Lys Asn Asn Asp Leu Arg Glu Lys Asn Trp Glu
   1025 1030 1035
   Ala Met Glu Ala Leu Ala Ser Thr Glu Lys Met Leu Gln Asp Lys
   1040 1045 1050
   Val Asn Lys Thr Ser Lys Glu Arg Gln Gln Gln Val Glu Ala Val
   1055 1060 1065
   Glu Leu Glu Ala Lys Glu Val Leu Lys Lys Leu Phe Pro Lys Val
   1070 1075 1080
   Ser Val Pro Ser Asn Leu Ser Tyr Gly Glu Trp Leu His Gly Phe
   1085 1090 1095
   Glu Lys Lys Ala Lys Glu Cys Met Ala Gly Thr Ser Gly Ser Glu
   1100 1105 1110
   Glu Val Lys Val Leu Glu His Lys Leu Lys Glu Ala Asp Glu Met
   1115 1120 1125
   His Thr Leu Leu Gln Leu Glu Cys Glu Lys Tyr Lys Ser Val Leu
   1130 1135 1140
   Ala Glu Thr Glu Gly Ile Leu Gln Lys Leu Gln Arg Ser Val Glu
   1145 1150 1155
   Gln Glu Glu Asn Lys Trp Lys Val Lys Val Asp Glu Ser His Lys
   1160 1165 1170
   Thr Ile Lys Gln Met Gln Ser Ser Phe Thr Ser Ser Glu Gln Glu
   1175 1180 1185
   Leu Glu Arg Leu Arg Ser Glu Asn Lys Asp Ile Glu Asn Leu Arg
   1190 1195 1200
   Arg Glu Arg Glu His Leu Glu Met Glu Leu Glu Lys Ala Glu Met
   1205 1210 1215
   Glu Arg Ser Thr Tyr Val Thr Glu Val Arg Glu Leu Lys Asp Leu
   1220 1225 1230
   Leu Thr Glu Leu Gln Lys Lys Leu Asp Asp Ser Tyr Ser Glu Ala
   1235 1240 1245
   Val Arg Gln Asn Glu Glu Leu Asn Leu Leu Lys Ala Gln Leu Asn
   1250 1255 1260
   Glu Thr Leu Thr Lys Leu Arg Thr Glu Gln Asn Glu Arg Gln Lys
   1265 1270 1275
   Val Ala Gly Asp Leu His Lys Ala Gln Gln Ser Leu Glu Leu Ile
   1280 1285 1290
   Gln Ser Lys Ile Val Lys Ala Ala Gly Asp Thr Thr Val Ile Glu
   1295 1300 1305
   Asn Ser Asp Val Ser Pro Glu Thr Glu Ser Ser Glu Lys Glu Thr
   1310 1315 1320
   Met Ser Val Ser Leu Asn Gln Thr Val Thr Gln Leu Gln Gln Leu
   1325 1330 1335
   Leu Gln Ala Val Asn Gln Gln Leu Thr Lys Glu Lys Glu His Tyr
   1340 1345 1350
   Gln Val Leu Glu
   1355
<210> 100
   <211> 393
   <212> PRT
   <213> Homo sapiens
<400> 100
   Met Arg Thr Leu Phe Asn Leu Leu Trp Leu Ala Leu Ala Cys Ser Pro
   1 5 10 15
   Val His Thr Thr Leu Ser Lys Ser Asp Ala Lys Lys Ala Ala Ser Lys
   20 25 30
   Thr Leu Leu Glu Lys Ser Gln Phe Ser Asp Lys Pro Val Gln Asp Arg
   35 40 45
   Gly Leu Val Val Thr Asp Leu Lys Ala Glu Ser Val Val Leu Glu His
   50 55 60
   Arg Ser Tyr Cys Ser Ala Lys Ala Arg Asp Arg His Phe Ala Gly Asp
   65 70 75 80
   Val Leu Gly Tyr Val Thr Pro Trp Asn Ser His Gly Tyr Asp Val Thr
   85 90 95
   Lys Val Phe Gly Ser Lys Phe Thr Gln Ile Ser Pro Val Trp Leu Gln
   100 105 110
   Leu Lys Arg Arg Gly Arg Glu Met Phe Glu Val Thr Gly Leu His Asp
   115 120 125
   Val Asp Gln Gly Trp Met Arg Ala Val Arg Lys His Ala Lys Gly Leu
   130 135 140
   His Ile Val Pro Arg Leu Leu Phe Glu Asp Trp Thr Tyr Asp Asp Phe
   145 150 155 160
   Arg Asn Val Leu Asp Ser Glu Asp Glu Ile Glu Glu Leu Ser Lys Thr
   165 170 175
   Val Val Gln Val Ala Lys Asn Gln His Phe Asp Gly Phe Val Val Glu
   180 185 190
   Val Trp Asn Gln Leu Leu Ser Gln Lys Arg Val Gly Leu Ile His Met
   195 200 205
   Leu Thr His Leu Ala Glu Ala Leu His Gln Ala Arg Leu Leu Ala Leu
   210 215 220
   Leu Val Ile Pro Pro Ala Ile Thr Pro Gly Thr Asp Gln Leu Gly Met
   225 230 235 240
   Phe Thr His Lys Glu Phe Glu Gln Leu Ala Pro Val Leu Asp Gly Phe
   245 250 255
   Ser Leu Met Thr Tyr Asp Tyr Ser Thr Ala His Gln Pro Gly Pro Asn
   260 265 270
   Ala Pro Leu Ser Trp Val Arg Ala Cys Val Gln Val Leu Asp Pro Lys
   275 280 285
   Ser Lys Trp Arg Ser Lys Ile Leu Leu Gly Leu Asn Phe Tyr Gly Met
   290 295 300
   Asp Tyr Ala Thr Ser Lys Asp Ala Arg Glu Pro Val Val Gly Ala Arg
   305 310 315 320
   Tyr Ile Gln Thr Leu Lys Asp His Arg Pro Arg Met Val Trp Asp Ser
   325 330 335
   Gln Ala Ser Glu His Phe Phe Glu Tyr Lys Lys Ser Arg Ser Gly Arg
   340 345 350
   His Val Val Phe Tyr Pro Thr Leu Lys Ser Leu Gln Val Arg Leu Glu
   355 360 365
   Leu Ala Arg Glu Leu Gly Val Gly Val Ser Ile Trp Glu Leu Gly Gln
   370 375 380
   Gly Leu Asp Tyr Phe Tyr Asp Leu Leu
   385 390
<210> 101
   <211> 493
   <212> PRT
   <213> Homo sapiens
<400> 101
<210> 102
   <211> 245
   <212> PRT
   <213> Homo sapiens
<400> 102
   Met Asp Lys Asn Glu Leu Val Gln Lys Ala Lys Leu Ala Glu Gln Ala
   1 5 10 15
   Glu Arg Tyr Asp Asp Met Ala Ala Cys Met Lys Ser Val Thr Glu Gln
   20 25 30
   Gly Ala Glu Leu Ser Asn Glu Glu Arg Asn Leu Leu Ser Val Ala Tyr
   35 40 45
   Lys Asn Val Val Gly Ala Arg Arg Ser Ser Trp Arg Val Val Ser Ser
   50 55 60
   Ile Glu Gln Lys Thr Glu Gly Ala Glu Lys Lys Gln Gln Met Ala Arg
   65 70 75 80
   Glu Tyr Arg Glu Lys Ile Glu Thr Glu Leu Arg Asp Ile Cys Asn Asp
   85 90 95
   Val Leu Ser Leu Leu Glu Lys Phe Leu Ile Pro Asn Ala Ser Gln Ala
   100 105 110
   Glu Ser Lys Val Phe Tyr Leu Lys Met Lys Gly Asp Tyr Tyr Arg Tyr
   115 120 125
   Leu Ala Glu Val Ala Ala Gly Asp Asp Lys Lys Gly Ile Val Asp Gln
       130                 135                 140
   Ser Gln Gln Ala Tyr Gln Glu Ala Phe Glu Ile Ser Lys Lys Glu Met
   145                 150                 155                 160
   Gln Pro Thr His Pro Ile Arg Leu Gly Leu Ala Leu Asn Phe Ser Val
                   165                 170                 175
   Phe Tyr Tyr Glu Ile Leu Asn Ser Pro Glu Lys Ala Cys Ser Leu Ala
               180                 185                 190
   Lys Thr Ala Phe Asp Glu Ala Ile Ala Glu Leu Asp Thr Leu Ser Glu
           195                 200                 205
   Glu Ser Tyr Lys Asp Ser Thr Leu Ile Met Gln Leu Leu Arg Asp Asn
       210                 215                 220
   Leu Thr Leu Trp Thr Ser Asp Thr Gln Gly Asp Glu Ala Glu Ala Gly
   225                 230                 235                 240
   Glu Gly Gly Glu Asn
                   245
<210> 103
   <211> 334
   <212> PRT
   <213> Homo sapiens
<400> 103
   Met Ala Thr Leu Lys Glu Lys Leu Ile Ala Pro Val Ala Glu Glu Glu
   1               5                   10                  15
   Ala Thr Val Pro Asn Asn Lys Ile Thr Val Val Gly Val Gly Gln Val
               20                  25                  30
   Gly Met Ala Cys Ala Ile Ser Ile Leu Gly Lys Ser Leu Ala Asp Glu
           35                  40                  45
   Leu Ala Leu Val Asp Val Leu Glu Asp Lys Leu Lys Gly Glu Met Met
       50                  55                  60
   Asp Leu Gln His Gly Ser Leu Phe Leu Gln Thr Pro Lys Ile Val Ala
   65                  70                  75                  80
   Asp Lys Asp Tyr Ser Val Thr Ala Asn Ser Lys Ile Val Val Val Thr
                   85                  90                  95
   Ala Gly Val Arg Gln Gln Glu Gly Glu Ser Arg Leu Asn Leu Val Gln
   100 105 110
   Arg Asn Val Asn Val Phe Lys Phe Ile Ile Pro Gln Ile Val Lys Tyr
   115 120 125
   Ser Pro Asp Cys Ile Ile Ile Val Val Ser Asn Pro Val Asp Ile Leu
   130 135 140
   Thr Tyr Val Thr Trp Lys Leu Ser Gly Leu Pro Lys His Arg Val Ile
   145 150 155 160
   Gly Ser Gly Cys Asn Leu Asp Ser Ala Arg Phe Arg Tyr Leu Met Ala
   165 170 175
   Glu Lys Leu Gly Ile His Pro Ser Ser Cys His Gly Trp Ile Leu Gly
   180 185 190
   Glu His Gly Asp Ser Ser Val Ala Val Trp Ser Gly Val Asn Val Ala
   195 200 205
   Gly Val Ser Leu Gln Glu Leu Asn Pro Glu Met Gly Thr Asp Asn Asp
   210 215 220
   Ser Glu Asn Trp Lys Glu Val His Lys Met Val Val Glu Ser Ala Tyr
   225 230 235 240
   Glu Val Ile Lys Leu Lys Gly Tyr Thr Asn Trp Ala Ile Gly Leu Ser
   245 250 255
   Val Ala Asp Leu Ile Glu Ser Met Leu Lys Asn Leu Ser Arg Ile His
   260 265 270
   Pro Val Ser Thr Met Val Lys Gly Met Tyr Gly Ile Glu Asn Glu Val
   275 280 285
   Phe Leu Ser Leu Pro Cys Ile Leu Asn Ala Arg Gly Leu Thr Ser Val
   290 295 300
   Ile Asn Gln Lys Leu Lys Asp Asp Glu Val Ala Gln Leu Lys Lys Ser
   305 310 315 320
   Ala Asp Thr Leu Trp Asp Ile Gln Lys Asp Leu Lys Asp Leu
   325 330
<210> 104
   <211> 298
   <212> PRT
   <213> Homo sapiens
<400> 104
   Met Ser Leu Tyr Pro Ser Leu Glu Asp Leu Lys Val Asp Lys Val Ile
   1               5                   10                  15
   Gln Ala Gln Thr Ala Phe Ser Ala Asn Pro Ala Asn Pro Ala Ile Leu
               20                  25                  30
   Ser Glu Ala Ser Ala Pro Ile Pro His Asp Gly Asn Leu Tyr Pro Arg
           35                  40                  45
   Leu Tyr Pro Glu Leu Ser Gln Tyr Met Gly Leu Ser Leu Asn Glu Glu
       50                  55                  60
   Glu Ile Arg Ala Asn Val Ala Val Val Ser Gly Ala Pro Leu Gln Gly
   65                  70                  75                  80
   Gln Leu Val Ala Arg Pro Ser Ser Ile Asn Tyr Met Val Ala Pro Val
                   85                  90                  95
   Thr Gly Asn Asp Val Gly Ile Arg Arg Ala Glu Ile Lys Gln Gly Ile
               100                 105                 110
   Arg Glu Val Ile Leu Cys Lys Asp Gln Asp Gly Lys Ile Gly Leu Arg
           115                 120                 125
   Leu Lys Ser Ile Asp Asn Gly Ile Phe Val Gln Leu Val Gln Ala Asn
       130                 135                 140
   Ser Pro Ala Ser Leu Val Gly Leu Arg Phe Gly Asp Gln Val Leu Gln
   145                 150                 155                 160
   Ile Asn Gly Glu Asn Cys Ala Gly Trp Ser Ser Asp Lys Ala His Lys
                   165                 170                 175
   Val Leu Lys Gln Ala Phe Gly Glu Lys Ile Thr Met Thr Ile Arg Asp
               180                 185                 190
   Arg Pro Phe Glu Arg Thr Ile Thr Met His Lys Asp Ser Thr Gly His
           195                 200                 205
   Val Gly Phe Ile Phe Lys Asn Gly Lys Ile Thr Ser Ile Val Lys Asp
       210                 215                 220
   Ser Ser Ala Ala Arg Asn Gly Leu Leu Thr Glu His Asn Ile Cys Glu
   225                 230                 235                 240
   Ile Asn Gly Gln Asn Val Ile Gly Leu Lys Asp Ser Gln Ile Ala Asp
   245 250 255
   Ile Leu Ser Thr Ser Gly Thr Val Val Thr Ile Thr Ile Met Pro Ala
   260 265 270
   Phe Ile Phe Glu His Ile Ile Lys Arg Met Ala Pro Ser Ile Met Lys
   275 280 285
   Ser Leu Met Asp His Thr Ile Pro Glu Val
   290 295
<210> 105
   <211> 2541
   <212> PRT
   <213> Homo sapiens
<400> 105
   Met Val Ala Leu Ser Leu Lys Ile Ser Ile Gly Asn Val Val Lys Thr
   1 5 10 15
   Met Gln Phe Glu Pro Ser Thr Met Val Tyr Asp Ala Cys Arg Ile Ile
   20 25 30
   Arg Glu Arg Ile Pro Glu Ala Pro Ala Gly Pro Pro Ser Asp Phe Gly
   35 40 45
   Leu Phe Leu Ser Asp Asp Asp Pro Lys Lys Gly Ile Trp Leu Glu Ala
   50 55 60
   Gly Lys Ala Leu Asp Tyr Tyr Met Leu Arg Asn Gly Asp Thr Met Glu
   65 70 75 80
   Tyr Arg Lys Lys Gln Arg Pro Leu Lys Ile Arg Met Leu Asp Gly Thr
   85 90 95
   Val Lys Thr Ile Met Val Asp Asp Ser Lys Thr Val Thr Asp Met Leu
   100 105 110
   Met Thr Ile Cys Ala Arg Ile Gly Ile Thr Asn His Asp Glu Tyr Ser
   115 120 125
   Leu Val Arg Glu Leu Met Glu Glu Lys Lys Glu Glu Ile Thr Gly Thr
   130 135 140
   Leu Arg Lys Asp Lys Thr Leu Leu Arg Asp Glu Lys Lys Met Glu Lys
   145 150 155 160
   Leu Lys Gln Lys Leu His Thr Asp Asp Glu Leu Asn Trp Leu Asp His
   165 170 175
   Gly Arg Thr Leu Arg Glu Gln Gly Val Glu Glu His Glu Thr Leu Leu
   180 185 190
   Leu Arg Arg Lys Phe Phe Tyr Ser Asp Gln Asn Val Asp Ser Arg Asp
   195 200 205
   Pro Val Gln Leu Asn Leu Leu Tyr Val Gln Ala Arg Asp Asp Ile Leu
   210 215 220
   Asn Gly Ser His Pro Val Ser Phe Asp Lys Ala Cys Glu Phe Ala Gly
   225 230 235 240
   Phe Gln Cys Gln Ile Gln Phe Gly Pro His Asn Glu Gln Lys His Lys
   245 250 255
   Ala Gly Phe Leu Asp Leu Lys Asp Phe Leu Pro Lys Glu Tyr Val Lys
   260 265 270
   Gln Lys Gly Glu Arg Lys Ile Phe Gln Ala His Lys Asn Cys Gly Gln
   275 280 285
   Met Ser Glu Ile Glu Ala Lys Val Arg Tyr Val Lys Leu Ala Arg Ser
   290 295 300
   Leu Lys Thr Tyr Gly Val Ser Phe Phe Leu Val Lys Glu Lys Met Lys
   305 310 315 320
   Gly Lys Asn Lys Leu Val Pro Arg Leu Leu Gly Ile Thr Lys Glu Cys
   325 330 335
   Val Met Arg Val Asp Glu Lys Thr Lys Glu Val Ile Gln Glu Trp Asn
   340 345 350
   Leu Thr Asn Ile Lys Arg Trp Ala Ala Ser Pro Lys Ser Phe Thr Leu
   355 360 365
   Asp Phe Gly Asp Tyr Gln Asp Gly Tyr Tyr Ser Val Gln Thr Thr Glu
   370 375 380
   Gly Glu Gln Ile Ala Gln Leu Ile Ala Gly Tyr Ile Asp Ile Ile Leu
   385 390 395 400
   Lys Lys Lys Lys Ser Lys Asp His Phe Gly Leu Glu Gly Asp Glu Glu
   405 410 415
   Ser Thr Met Leu Glu Asp Ser Val Ser Pro Lys Lys Ser Thr Val Leu
   420 425 430
   Gln Gln Gln Tyr Asn Arg Val Gly Lys Val Glu His Gly Ser Val Ala
   435 440 445
   Leu Pro Ala Ile Met Arg Ser Gly Ala Ser Gly Pro Glu Asn Phe Gln
   450 455 460
   Val Gly Ser Met Pro Pro Ala Gln Gln Gln Ile Thr Ser Gly Gln Met
   465 470 475 480
   His Arg Gly His Met Pro Pro Leu Thr Ser Ala Gln Gln Ala Leu Thr
   485 490 495
   Gly Thr Ile Asn Ser Ser Met Gln Ala Val Gln Ala Ala Gln Ala Thr
   500 505 510
   Leu Asp Asp Phe Asp Thr Leu Pro Pro Leu Gly Gln Asp Ala Ala Ser
   515 520 525
   Lys Ala Trp Arg Lys Asn Lys Met Asp Glu Ser Lys His Glu Ile His
   530 535 540
   Ser Gln Val Asp Ala Ile Thr Ala Gly Thr Ala Ser Val Val Asn Leu
   545 550 555 560
   Thr Ala Gly Asp Pro Ala Glu Thr Asp Tyr Thr Ala Val Gly Cys Ala
   565 570 575
   Val Thr Thr Ile Ser Ser Asn Leu Thr Glu Met Ser Arg Gly Val Lys
   580 585 590
   Leu Leu Ala Ala Leu Leu Glu Asp Glu Gly Gly Ser Gly Arg Pro Leu
   595 600 605
   Leu Gln Ala Ala Lys Gly Leu Ala Gly Ala Val Ser Glu Leu Leu Arg
   610 615 620
   Ser Ala Gln Pro Ala Ser Ala Glu Pro Arg Gln Asn Leu Leu Gln Ala
   625 630 635 640
   Ala Gly Asn Val Gly Gln Ala Ser Gly Glu Leu Leu Gln Gln Ile Gly
   645 650 655
   Glu Ser Asp Thr Asp Pro His Phe Gln Asp Ala Leu Met Gln Leu Ala
   660 665 670
   Lys Ala Val Ala Ser Ala Ala Ala Ala Leu Val Leu Lys Ala Lys Ser
   675 680 685
   Val Ala Gln Arg Thr Glu Asp Ser Gly Leu Gln Thr Gln Val Ile Ala
   690 695 700
   Ala Ala Thr Gln Cys Ala Leu Ser Thr Ser Gln Leu Val Ala Cys Thr
   705 710 715 720
   Lys Val Val Ala Pro Thr Ile Ser Ser Pro Val Cys Gln Glu Gln Leu
   725 730 735
   Val Glu Ala Gly Arg Leu Val Ala Lys Ala Val Glu Gly Cys Val Ser
   740 745 750
   Ala Ser Gln Ala Ala Thr Glu Asp Gly Gln Leu Leu Arg Gly Val Gly
   755 760 765
   Ala Ala Ala Thr Ala Val Thr Gln Ala Leu Asn Glu Leu Leu Gln His
   770 775 780
   Val Lys Ala His Ala Thr Gly Ala Gly Pro Ala Gly Arg Tyr Asp Gln
   785 790 795 800
   Ala Thr Asp Thr Ile Leu Thr Val Thr Glu Asn Ile Phe Ser Ser Met
   805 810 815
   Gly Asp Ala Gly Glu Met Val Arg Gln Ala Arg Ile Leu Ala Gln Ala
   820 825 830
   Thr Ser Asp Leu Val Asn Ala Ile Lys Ala Asp Ala Glu Gly Glu Ser
   835 840 845
   Asp Leu Glu Asn Ser Arg Lys Leu Leu Ser Ala Ala Lys Ile Leu Ala
   850 855 860
   Asp Ala Thr Ala Lys Met Val Glu Ala Ala Lys Gly Ala Ala Ala His
   865 870 875 880
   Pro Asp Ser Glu Glu Gln Gln Gln Arg Leu Arg Glu Ala Ala Glu Gly
   885 890 895
   Leu Arg Met Ala Thr Asn Ala Ala Ala Gln Asn Ala Ile Lys Lys Lys
   900 905 910
   Leu Val Gln Arg Leu Glu His Ala Ala Lys Gln Ala Ala Ala Ser Ala
           915                 920                 925
   Thr Gln Thr Ile Ala Ala Ala Gln His Ala Ala Ser Thr Pro Lys Ala
       930                 935                 940
   Ser Ala Gly Pro Gln Pro Leu Leu Val Gln Ser Cys Lys Ala Val Ala
   945                 950                 955                 960
   Glu Gln Ile Pro Leu Leu Val Gln Gly Val Arg Gly Ser Gln Ala Gln
                   965                 970                 975
   Pro Asp Ser Pro Ser Ala Gln Leu Ala Leu Ile Ala Ala Ser Gln Ser
               980                 985                 990
   Phe Leu Gln Pro Gly Gly Lys Met  Val Ala Ala Ala Lys  Ala Ser Val
           995                 1000                1005
   Pro Thr  Ile Gln Asp Gln Ala  Ser Ala Met Gln Leu  Ser Gln Cys
       1010                 1015                 1020
   Ala Lys  Asn Leu Gly Thr Ala  Leu Ala Glu Leu Arg  Thr Ala Ala
       1025                 1030                 1035
   Gln Lys  Ala Gln Glu Ala Cys  Gly Pro Leu Glu Met  Asp Ser Ala
       1040                 1045                 1050
   Leu Ser  Val Val Gln Asn Leu  Glu Lys Asp Leu Gln  Glu Val Lys
       1055                 1060                 1065
   Ala Ala  Ala Arg Asp Gly Lys  Leu Lys Pro Leu Pro  Gly Glu Thr
       1070                 1075                 1080
   Met Glu  Lys Cys Thr Gln Asp  Leu Gly Asn Ser Thr  Lys Ala Val
       1085                 1090                 1095
   Ser Ser  Ala Ile Ala Gln Leu  Leu Gly Glu Val Ala  Gln Gly Asn
       1100                 1105                 1110
   Glu Asn  Tyr Ala Gly Ile Ala  Ala Arg Asp Val Ala  Gly Gly Leu
       1115                 1120                 1125
   Arg Ser  Leu Ala Gln Ala Ala  Arg Gly Val Ala Ala  Leu Thr Ser
       1130                 1135                 1140
   Asp Pro  Ala Val Gln Ala Ile  Val Leu Asp Thr Ala  Ser Asp Val
       1145                 1150                 1155
   Leu Asp Lys Ala Ser Ser Leu Ile Glu Glu Ala Lys Lys Ala Ala
   1160 1165 1170
   Gly His Pro Gly Asp Pro Glu Ser Gln Gln Arg Leu Ala Gln Val
   1175 1180 1185
   Ala Lys Ala Val Thr Gln Ala Leu Asn Arg Cys Val Ser Cys Leu
   1190 1195 1200
   Pro Gly Gln Arg Asp Val Asp Asn Ala Leu Arg Ala Val Gly Asp
   1205 1210 1215
   Ala Ser Lys Arg Leu Leu Ser Asp Ser Leu Pro Pro Ser Thr Gly
   1220 1225 1230
   Thr Phe Gln Glu Ala Gln Ser Arg Leu Asn Glu Ala Ala Ala Gly
   1235 1240 1245
   Leu Asn Gln Ala Ala Thr Glu Leu Val Gln Ala Ser Arg Gly Thr
   1250 1255 1260
   Pro Gln Asp Leu Ala Arg Ala Ser Gly Arg Phe Gly Gln Asp Phe
   1265 1270 1275
   Ser Thr Phe Leu Glu Ala Gly Val Glu Met Ala Gly Gln Ala Pro
   1280 1285 1290
   Ser Gln Glu Asp Arg Ala Gln Val Val Ser Asn Leu Lys Gly Ile
   1295 1300 1305
   Ser Met Ser Ser Ser Lys Leu Leu Leu Ala Ala Lys Ala Leu Ser
   1310 1315 1320
   Thr Asp Pro Ala Ala Pro Asn Leu Lys Ser Gln Leu Ala Ala Ala
   1325 1330 1335
   Ala Arg Ala Val Thr Asp Ser Ile Asn Gln Leu Ile Thr Met Cys
   1340 1345 1350
   Thr Gln Gln Ala Pro Gly Gln Lys Glu Cys Asp Asn Ala Leu Arg
   1355 1360 1365
   Glu Leu Glu Thr Val Arg Glu Leu Leu Glu Asn Pro Val Gln Pro
   1370 1375 1380
   Ile Asn Asp Met Ser Tyr Phe Gly Cys Leu Asp Ser Val Met Glu
   1385 1390 1395
   Asn Ser Lys Val Leu Gly Glu Ala Met Thr Gly Ile Ser Gln Asn
   1400 1405 1410
   Ala Lys Asn Gly Asn Leu Pro Glu Phe Gly Asp Ala Ile Ser Thr
   1415 1420 1425
   Ala Ser Lys Ala Leu Cys Gly Phe Thr Glu Ala Ala Ala Gln Ala
   1430 1435 1440
   Ala Tyr Leu Val Gly Val Ser Asp Pro Asn Ser Gln Ala Gly Gln
   1445 1450 1455
   Gln Gly Leu Val Glu Pro Thr Gln Phe Ala Arg Ala Asn Gln Ala
   1460 1465 1470
   Ile Gln Met Ala Cys Gln Ser Leu Gly Glu Pro Gly Cys Thr Gln
   1475 1480 1485
   Ala Gln Val Leu Ser Ala Ala Thr Ile Val Ala Lys His Thr Ser
   1490 1495 1500
   Ala Leu Cys Asn Ser Cys Arg Leu Ala Ser Ala Arg Thr Thr Asn
   1505 1510 1515
   Pro Thr Ala Lys Arg Gln Phe Val Gln Ser Ala Lys Glu Val Ala
   1520 1525 1530
   Asn Ser Thr Ala Asn Leu Val Lys Thr Ile Lys Ala Leu Asp Gly
   1535 1540 1545
   Ala Phe Thr Glu Glu Asn Arg Ala Gln Cys Arg Ala Ala Thr Ala
   1550 1555 1560
   Pro Leu Leu Glu Ala Val Asp Asn Leu Ser Ala Phe Ala Ser Asn
   1565 1570 1575
   Pro Glu Phe Ser Ser Ile Pro Ala Gln Ile Ser Pro Glu Gly Arg
   1580 1585 1590
   Ala Ala Met Glu Pro Ile Val Ile Ser Ala Lys Thr Met Leu Glu
   1595 1600 1605
   Ser Ala Gly Gly Leu Ile Gln Thr Ala Arg Ala Leu Ala Val Asn
   1610 1615 1620
   Pro Arg Asp Pro Pro Ser Trp Ser Val Leu Ala Gly His Ser Arg
   1625 1630 1635
   Thr Val Ser Asp Ser Ile Lys Lys Leu Ile Thr Ser Met Arg Asp
       1640                1645                1650
   Lys Ala Pro Gly Gln Leu Glu Cys Glu Thr Ala Ile Ala Ala Leu
       1655                1660                1665
   Asn Ser Cys Leu Arg Asp Leu Asp Gln Ala Ser Leu Ala Ala Val
       1670                1675                1680
   Ser Gln Gln Leu Ala Pro Arg Glu Gly Ile Ser Gln Glu Ala Leu
       1685                1690                1695
   His Thr Gln Met Leu Thr Ala Val Gln Glu Ile Ser His Leu Ile
       1700                1705                1710
   Glu Pro Leu Ala Asn Ala Ala Arg Ala Glu Ala Ser Gln Leu Gly
       1715                1720                1725
   His Lys Val Ser Gln Met Ala Gln Tyr Phe Glu Pro Leu Thr Leu
       1730                1735                1740
   Ala Ala Val Gly Ala Ala Ser Lys Thr Leu Ser His Pro Gln Gln
       1745                1750                1755
   Met Ala Leu Leu Asp Gln Thr Lys Thr Leu Ala Glu Ser Ala Leu
       1760                1765                1770
   Gln Leu Leu Tyr Thr Ala Lys Glu Ala Gly Gly Asn Pro Lys Gln
       1775                1780                1785
   Ala Ala His Thr Gln Glu Ala Leu Glu Glu Ala Val Gln Met Met
       1790                1795                1800
   Thr Glu Ala Val Glu Asp Leu Thr Thr Thr Leu Asn Glu Ala Ala
       1805                1810                1815
   Ser Ala Ala Gly Val Val Gly Gly Met Val Asp Ser Ile Thr Gln
       1820                1825                1830
   Ala Ile Asn Gln Leu Asp Glu Gly Pro Met Gly Glu Pro Glu Gly
       1835                1840                1845
   Ser Phe Val Asp Tyr Gln Thr Thr Met Val Arg Thr Ala Lys Ala
       1850                1855                1860
   Ile Ala Val Thr Val Gln Glu Met Val Thr Lys Ser Asn Thr Ser
   1865 1870 1875
   Pro Glu Glu Leu Gly Pro Leu Ala Asn Gln Leu Thr Ser Asp Tyr
   1880 1885 1890
   Gly Arg Leu Ala Ser Glu Ala Lys Pro Ala Ala Val Ala Ala Glu
   1895 1900 1905
   Asn Glu Glu Ile Gly Ser His Ile Lys His Arg Val Gln Glu Leu
   1910 1915 1920
   Gly His Gly Cys Ala Ala Leu Val Thr Lys Ala Gly Ala Leu Gln
   1925 1930 1935
   Cys Ser Pro Ser Asp Ala Tyr Thr Lys Lys Glu Leu Ile Glu Cys
   1940 1945 1950
   Ala Arg Arg Val Ser Glu Lys Val Ser His Val Leu Ala Ala Leu
   1955 1960 1965
   Gln Ala Gly Asn Arg Gly Thr Gln Ala Cys Ile Thr Ala Ala Ser
   1970 1975 1980
   Ala Val Ser Gly Ile Ile Ala Asp Leu Asp Thr Thr Ile Met Phe
   1985 1990 1995
   Ala Thr Ala Gly Thr Leu Asn Arg Glu Gly Thr Glu Thr Phe Ala
   2000 2005 2010
   Asp His Arg Glu Gly Ile Leu Lys Thr Ala Lys Val Leu Val Glu
   2015 2020 2025
   Asp Thr Lys Val Leu Val Gln Asn Ala Ala Gly Ser Gln Glu Lys
   2030 2035 2040
   Leu Ala Gln Ala Ala Gln Ser Ser Val Ala Thr Ile Thr Arg Leu
   2045 2050 2055
   Ala Asp Val Val Lys Leu Gly Ala Ala Ser Leu Gly Ala Glu Asp
   2060 2065 2070
   Pro Glu Thr Gln Val Val Leu Ile Asn Ala Val Lys Asp Val Ala
   2075 2080 2085
   Lys Ala Leu Gly Asp Leu Ile Ser Ala Thr Lys Ala Ala Ala Gly
   2090 2095 2100
   Lys Val Gly Asp Asp Pro Ala Val Trp Gln Leu Lys Asn Ser Ala
   2105 2110 2115
   Lys Val Met Val Thr Asn Val Thr Ser Leu Leu Lys Thr Val Lys
   2120 2125 2130
   Ala Val Glu Asp Glu Ala Thr Lys Gly Thr Arg Ala Leu Glu Ala
   2135 2140 2145
   Thr Thr Glu His Ile Arg Gln Glu Leu Ala Val Phe Cys Ser Pro
   2150 2155 2160
   Glu Pro Pro Ala Lys Thr Ser Thr Pro Glu Asp Phe Ile Arg Met
   2165 2170 2175
   Thr Lys Gly Ile Thr Met Ala Thr Ala Lys Ala Val Ala Ala Gly
   2180 2185 2190
   Asn Ser Cys Arg Gln Glu Asp Val Ile Ala Thr Ala Asn Leu Ser
   2195 2200 2205
   Arg Arg Ala Ile Ala Asp Met Leu Arg Ala Cys Lys Glu Ala Ala
   2210 2215 2220
   Tyr His Pro Glu Val Ala Pro Asp Val Arg Leu Arg Ala Leu His
   2225 2230 2235
   Tyr Gly Arg Glu Cys Ala Asn Gly Tyr Leu Glu Leu Leu Asp His
   2240 2245 2250
   Val Leu Leu Thr Leu Gln Lys Pro Ser Pro Glu Leu Lys Gln Gln
   2255 2260 2265
   Leu Thr Gly His Ser Lys Arg Val Ala Gly Ser Val Thr Glu Leu
   2270 2275 2280
   Ile Gln Ala Ala Glu Ala Met Lys Gly Thr Glu Trp Val Asp Pro
   2285 2290 2295
   Glu Asp Pro Thr Val Ile Ala Glu Asn Glu Leu Leu Gly Ala Ala
   2300 2305 2310
   Ala Ala Ile Glu Ala Ala Ala Lys Lys Leu Glu Gln Leu Lys Pro
   2315 2320 2325
   Arg Ala Lys Pro Lys Glu Ala Asp Glu Ser Leu Asn Phe Glu Glu
   2330 2335 2340
   Gln Ile  Leu Glu Ala Ala Lys  Ser Ile Ala Ala Ala  Thr Ser Ala
       2345                 2350                 2355
   Leu Val  Lys Ala Ala Ser Ala  Ala Gln Arg Glu Leu  Val Ala Gln
       2360                 2365                 2370
   Gly Lys  Val Gly Ala Ile Pro  Ala Asn Ala Leu Asp  Asp Gly Gln
       2375                 2380                 2385
   Trp Ser  Gln Gly Leu Ile Ser  Ala Ala Arg Met Val  Ala Ala Ala
       2390                 2395                 2400
   Thr Asn  Asn Leu Cys Glu Ala  Ala Asn Ala Ala Val  Gln Gly His
       2405                 2410                 2415
   Ala Ser  Gln Glu Lys Leu Ile  Ser Ser Ala Lys Gln  Val Ala Ala
       2420                 2425                 2430
   Ser Thr  Ala Gln Leu Leu Val  Ala Cys Lys Val Lys  Ala Asp Gln
       2435                 2440                 2445
   Asp Ser  Glu Ala Met Lys Arg  Leu Gln Ala Ala Gly  Asn Ala Val
       2450                 2455                 2460
   Lys Arg  Ala Ser Asp Asn Leu  Val Lys Ala Ala Gln  Lys Ala Ala
       2465                 2470                 2475
   Ala Phe  Glu Glu Gln Glu Asn  Glu Thr Val Val Val  Lys Glu Lys
       2480                 2485                 2490
   Met Val  Gly Gly Ile Ala Gln  Ile Ile Ala Ala Gln  Glu Glu Met
       2495                 2500                 2505
   Leu Arg  Lys Glu Arg Glu Leu  Glu Glu Ala Arg Lys  Lys Leu Ala
       2510                 2515                 2520
   Gln Ile  Arg Gln Gln Gln Tyr  Lys Phe Leu Pro Ser  Glu Leu Arg
       2525                 2530                 2535
   Asp Glu  His
       2540
<210> 106
   <211> 470
   <212> PRT
   <213> Homo sapiens
<400> 106
<210> 107
   <211> 770
   <212> PRT
   <213> Homo sapiens
<400> 107
<210> 108
   <211> 895
   <212> PRT
   <213> Homo sapiens
<400> 108
   Met Arg Met Ser Val Gly Leu Ser Leu Leu Leu Pro Leu Ser Gly Arg
   1 5 10 15
   Thr Phe Leu Leu Leu Leu Ser Val Val Met Ala Gln Ser His Trp Pro
   20 25 30
   Ser Glu Pro Ser Glu Ala Val Arg Asp Trp Glu Asn Gln Leu Glu Ala
   35 40 45
   Ser Met His Ser Val Leu Ser Asp Leu His Glu Ala Val Pro Thr Val
   50 55 60
   Val Gly Ile Pro Asp Gly Thr Ala Val Val Gly Arg Ser Phe Arg Val
   65 70 75 80
   Thr Ile Pro Thr Asp Leu Ile Ala Ser Ser Gly Asp Ile Ile Lys Val
   85 90 95
   Ser Ala Ala Gly Lys Glu Ala Leu Pro Ser Trp Leu His Trp Asp Ser
   100 105 110
   Gln Ser His Thr Leu Glu Gly Leu Pro Leu Asp Thr Asp Lys Gly Val
   115 120 125
   His Tyr Ile Ser Val Ser Ala Thr Arg Leu Gly Ala Asn Gly Ser His
   130 135 140
   Ile Pro Gln Thr Ser Ser Val Phe Ser Ile Glu Val Tyr Pro Glu Asp
   145 150 155 160
   His Ser Glu Leu Gln Ser Val Arg Thr Ala Ser Pro Asp Pro Gly Glu
   165 170 175
   Val Val Ser Ser Ala Cys Ala Ala Asp Glu Pro Val Thr Val Leu Thr
   180 185 190
   Val Ile Leu Asp Ala Asp Leu Thr Lys Met Thr Pro Lys Gln Arg Ile
   195 200 205
   Asp Leu Leu His Arg Met Arg Ser Phe Ser Glu Val Glu Leu His Asn
   210 215 220
   Met Lys Leu Val Pro Val Val Asn Asn Arg Leu Phe Asp Met Ser Ala
   225 230 235 240
   Phe Met Ala Gly Pro Gly Asn Ala Lys Lys Val Val Glu Asn Gly Ala
   245 250 255
   Leu Leu Ser Trp Lys Leu Gly Cys Ser Leu Asn Gln Asn Ser Val Pro
   260 265 270
   Asp Ile His Gly Val Glu Ala Pro Ala Arg Glu Gly Ala Met Ser Ala
   275 280 285
   Gln Leu Gly Tyr Pro Val Val Gly Trp His Ile Ala Asn Lys Lys Pro
   290 295 300
   Pro Leu Pro Lys Arg Val Arg Arg Gln Ile His Ala Thr Pro Thr Pro
   305 310 315 320
   Val Thr Ala Ile Gly Pro Pro Thr Thr Ala Ile Gln Glu Pro Pro Ser
   325 330 335
   Arg Ile Val Pro Thr Pro Thr Ser Pro Ala Ile Ala Pro Pro Thr Glu
   340 345 350
   Thr Met Ala Pro Pro Val Arg Asp Pro Val Pro Gly Lys Pro Thr Val
   355 360 365
   Thr Ile Arg Thr Arg Gly Ala Ile Ile Gln Thr Pro Thr Leu Gly Pro
   370 375 380
   Ile Gln Pro Thr Arg Val Ser Glu Ala Gly Thr Thr Val Pro Gly Gln
   385 390 395 400
   Ile Arg Pro Thr Met Thr Ile Pro Gly Tyr Val Glu Pro Thr Ala Val
   405 410 415
   Ala Thr Pro Pro Thr Thr Thr Thr Lys Lys Pro Arg Val Ser Thr Pro
   420 425 430
   Lys Pro Ala Thr Pro Ser Thr Asp Ser Thr Thr Thr Thr Thr Arg Arg
   435 440 445
   Pro Thr Lys Lys Pro Arg Thr Pro Arg Pro Val Pro Arg Val Thr Thr
   450 455 460
   Lys Val Ser Ile Thr Arg Leu Glu Thr Ala Ser Pro Pro Thr Arg Ile
   465 470 475 480
   Arg Thr Thr Thr Ser Gly Val Pro Arg Gly Gly Glu Pro Asn Gln Arg
   485 490 495
   Pro Glu Leu Lys Asn His Ile Asp Arg Val Asp Ala Trp Val Gly Thr
   500 505 510
   Tyr Phe Glu Val Lys Ile Pro Ser Asp Thr Phe Tyr Asp His Glu Asp
   515 520 525
   Thr Thr Thr Asp Lys Leu Lys Leu Thr Leu Lys Leu Arg Glu Gln Gln
   530 535 540
   Leu Val Gly Glu Lys Ser Trp Val Gln Phe Asn Ser Asn Ser Gln Leu
   545 550 555 560
   Met Tyr Gly Leu Pro Asp Ser Ser His Val Gly Lys His Glu Tyr Phe
   565 570 575
   Met His Ala Thr Asp Lys Gly Gly Leu Ser Ala Val Asp Ala Phe Glu
   580 585 590
   Ile His Val His Arg Arg Pro Gln Gly Asp Arg Ala Pro Ala Arg Phe
   595 600 605
   Lys Ala Lys Phe Val Gly Asp Pro Ala Leu Val Leu Asn Asp Ile His
   610 615 620
   Lys Lys Ile Ala Leu Val Lys Lys Leu Ala Phe Ala Phe Gly Asp Arg
   625 630 635 640
   Asn Cys Ser Thr Ile Thr Leu Gln Asn Ile Thr Arg Gly Ser Ile Val
   645 650 655
   Val Glu Trp Thr Asn Asn Thr Leu Pro Leu Glu Pro Cys Pro Lys Glu
   660 665 670
   Gln Ile Ala Gly Leu Ser Arg Arg Ile Ala Glu Asp Asp Gly Lys Pro
   675 680 685
   Arg Pro Ala Phe Ser Asn Ala Leu Glu Pro Asp Phe Lys Ala Thr Ser
   690 695 700
   Ile Thr Val Thr Gly Ser Gly Ser Cys Arg His Leu Gln Phe Ile Pro
   705 710 715 720
   Val Val Pro Pro Arg Arg Val Pro Ser Glu Ala Pro Pro Thr Glu Val
   725 730 735
   Pro Asp Arg Asp Pro Glu Lys Ser Ser Glu Asp Asp Val Tyr Leu His
   740 745 750
   Thr Val Ile Pro Ala Val Val Val Ala Ala Ile Leu Leu Ile Ala Gly
   755 760 765
   Ile Ile Ala Met Ile Cys Tyr Arg Lys Lys Arg Lys Gly Lys Leu Thr
   770 775 780
   Leu Glu Asp Gln Ala Thr Phe Ile Lys Lys Gly Val Pro Ile Ile Phe
   785 790 795 800
   Ala Asp Glu Leu Asp Asp Ser Lys Pro Pro Pro Ser Ser Ser Met Pro
   805 810 815
   Leu Ile Leu Gln Glu Glu Lys Ala Pro Leu Pro Pro Pro Glu Tyr Pro
   820 825 830
   Asn Gln Ser Val Pro Glu Thr Thr Pro Leu Asn Gln Asp Thr Met Gly
   835 840 845
   Glu Tyr Thr Pro Leu Arg Asp Glu Asp Pro Asn Ala Pro Pro Tyr Gln
   850 855 860
   Pro Pro Pro Pro Phe Thr Ala Pro Met Glu Gly Lys Gly Ser Arg Pro
   865 870 875 880
   Lys Asn Met Thr Pro Tyr Arg Ser Pro Pro Pro Tyr Val Pro Pro
   885 890 895
<210> 109
   <211> 23
   <212> RNA
   <213> Homo sapiens
<400> 109
   uguaaacauc cuacacucuc age 23
<210> 110
   <211> 104
   <212> DNA
   <213> Homo sapiens
<400> 110
   attcttctat agttcatgag catgatgatt gggtgttcat gacatgtgtg agatgtgcca 60
   ccctctgaac cttgttccca catcggcaca ttatccatct aacc 104
<210> 111
   <211> 20
   <212> RNA
   <213> Homo sapiens
<400> 111
   agcccccugg ccccaaaccc 20
<210> 112
   <211> 22
   <212> RNA
   <213> Homo sapiens
<400> 112
   uuguacaugg uaggcuuuca uu 22
<210> 113
   <211> 21
   <212> RNA
   <213> Homo sapiens
<400> 113
   ucgaggagcu cacagucuag u 21
<210> 114
   <211> 22
   <212> RNA
   <213> Homo sapiens
<400> 114
   uguaaacauc cccgacugga ag 22
<210> 115
   <211> 22
   <212> RNA
   <213> Homo sapiens
<400> 115
   gaaguuguuc gugguggauu cg 22
<210> 116
   <211> 22
   <212> RNA
   <213> Homo sapiens
<400> 116
   cuuucagucg gauguuugca gc 22
<210> 117
   <211> 22
   <212> RNA
   <213> Homo sapiens
<400> 117
   ugagguagua guuuguacag uu 22
<210> 118
   <211> 22
   <212> RNA
   <213> Homo sapiens
<400> 118
   auauaauaca accugcuaag ug 22
<210> 119
   <211> 22
   <212> RNA
   <213> Homo sapiens
<400> 119
   uagcuuauca gacugauguu ga 22
<210> 120
   <211> 22
   <212> RNA
   <213> Homo sapiens
<400> 120
   ugagguagua gauuguauag uu 22
<210> 121
   <211> 21
   <212> RNA
   <213> Homo sapiens
<400> 121
   gcuggucugc guggugcucg g 21
<210> 122
   <211> 22
   <212> RNA
   <213> Homo sapiens
<400> 122
   uauguaacac gguccacuaa cc 22